# EUROPEAN PATENT APPLICATION

(11) **EP 4 461 724 A1**
(43) Date of publication of application: **13.11.2024**
(21) Application number: 23737303.0
(22) Date of filing: 06.01.2023
(51) Int. Cl.: C07C 231/02, C07C 233/11, C07C 233/15, C07C 233/65, C07C 233/66, C07C 303/36, C07C 311/16, C07D 211/46, C07D 211/64, C07D 233/58, C07D 295/155, C07D 295/192, C07D 307/68

(54) **METHOD FOR PRODUCING AMIDE COMPOUND**

(30) Priority: 06.01.2022 JP 2022000840; 06.07.2022 JP 2022109248
(71) Applicant: CHUGAI SEIYAKU KABUSHIKI KAISHA, Tokyo 115-8543 (JP)
(72) Inventor: ITO, Taisuke, Gotemba-shi, Shizuoka 412-8513 (JP); OSAWA, Taisei, Gotemba-shi, Shizuoka 412-8513 (JP)
(74) Representative: Vossius & Partner Patentanwälte Rechtsanwälte mbB
(86) International application number: PCT/JP2023/000120
(87) International publication number: WO 2023/132352

(57) **Abstract**

The present invention provides a method for producing an amide compound, comprising reacting a carboxylic acid compound and an amine compound in the presence of a condensing agent and a first base to obtain an amide compound, wherein the first base is a compound represented by formula A, and a molar ratio of the first base to the condensing agent (first base/condensing agent) is 1.8 or less.

## Description

### TECHNICAL FIELD

The present invention relates to a method for forming an amide bond from an amine compound and a carboxylic acid compound. The present invention further relates to a method for producing a compound, comprising the step of forming an amide bond from an amine compound and a carboxylic acid compound.

### BACKGROUND ART

Methods for forming an amide bond from an amine compound and a carboxylic acid compound are extremely important reactions in forming the basic backbone of various organic compounds such as pharmaceuticals, pesticides, and polymeric compounds. The methods for forming an amide bond have been studied from a long time ago, and numerous condensing agents have been developed (NPL 1).

In the amide bond formation reactions, a method using a halouronium-based condensing agent has been reported for the cases where the reactivity is low due to the sterically hindered substrates, or the like (NPL 2).

As a condensation method to improve the reactivity, a method using N-methylimidazole (NMI) along with a halouronium-based condensing agent and via an N-acylimidazolium intermediate has been reported (NPL 3).

### CITATION LIST

### NON PATENT LITERATURE

NPL 1: Valeur, E. et al., Chem. Soc. Rev. 2009, 38, 606-631
NPL 2: Due-Hansen, M.E. et al., Org. Biomol. Chem. 2016, 14, 430-433.
NPL 3: Beutner, G.L. et al., Org. Lett. 2018, 20, 4218-4222.

### SUMMARY OF INVENTION

### TECHNICAL PROBLEM

Methods for forming an amide bond from an amine compound and a carboxylic acid compound are widely used in the development and manufacture of pharmaceuticals, and numerous condensing agents have been developed. Methods for improving reactivity for sterically hindered substrates or the like have also been reported (NPLs 2 and 3).

However, in the case of significantly less reactive amide-bond formation, such as when using a carboxylic acid compound with high steric hindrance and/or an amine compound with low nucleophilicity, the existing method using the halouronium-based condensing agent described above fails to obtain the target amide compounds with sufficient yields and/or produces many byproducts. As such, there are problems that have not yet been solved by conventional methods, and there is a need for a more versatile method for forming an amide bond.

In view of such circumstances, the present inventors have intensively studied and completed the present invention. In one aspect, an object of the present invention is to provide a method for producing an amide compound capable of improving yield and/or reducing byproducts in a reaction forming an amide bond from an amine compound and a carboxylic acid compound. In one aspect, another object of the present invention is to provide a method for synthesizing a compound, comprising the method described above. In one aspect, an object of the present invention is to provide a method of improving yield and/or a method of reducing byproducts that is applicable in a method of forming an amide bond from an amine compound and a carboxylic acid compound.

### SOLUTION TO PROBLEM

The present inventors have found that, in a reaction using a condensing agent to prepare an amide compound from an amine compound and a carboxylic acid compound by forming an amide bond by a dehydration condensation reaction of an amino group and a carboxy group, the use of a specific condensing agent and the adjustment of the amount of bases used make the reaction proceed in high yields and with less byproducts, and have completed the present invention. Specifically, as the results of examining the equivalents used for a uronium-based condensing agents or 2-halo-N-alkyl pyridinium-based condensing agent, a first base (such as NMI), and an optional second base (such as dimethylaniline), the present inventors have found that the amide compound of interest can be produced in higher yields and with less byproducts than previously reported reaction conditions (e.g., conditions described in NPL 3). Furthermore, the present inventors have found that this condition is applicable to reactions forming amide bonds from various amine compounds and carboxylic acid compounds, and have completed the present invention.

The present description encompasses the following disclosures of the invention.

[A-1] A method for producing an amide compound, comprising reacting a carboxylic acid compound and an amine compound in the presence of a condensing agent and a first base to obtain an amide compound,
wherein the first base is represented by formula A: 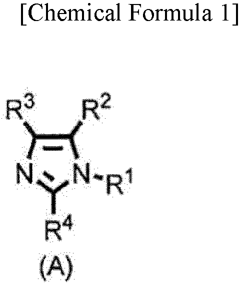
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
two or more of the first base may be used, and
wherein a molar ratio of the first base to the condensing agent (first base/condensing agent) is 1.8 or less.

[A-2] The method according to [A-1], wherein a molar ratio of the first base to the condensing agent is 1.7 or less, 1.6 or less, 1.5 or less, 1.4 or less, 1.3 or less, 1.2 or less, 1.1 or less, or 1.0 or less.

[A-3] The method according to [A-1] or [A-2], wherein the condensing agent is at least one selected from a uronium-based condensing agent or a 2-halo-N-alkyl pyridinium-based condensing agent.

[A-4] The method according to [A-1] or [A-2], wherein the condensing agent is a uronium-based condensing agent.

[A-5] The method according to any of [A-1] to [A-4], wherein the uronium-based condensing agent is a halouronium-based condensing agent, such as a halouronium-based condensing agent selected from the group consisting of
fluoro-N,N,N',N'-tetramethylformamidinium hexafluorophosphate (TFFH),
chloro-N,N,N',N'-tetramethylformamidinium hexafluorophosphate (TCFH),
1 -(chloro-1 -pyrrolidinylmethylene)pyrrolidinium hexafluorophosphate (PyClU),
1 -(chloro-1 -pyrrolidinylmethylene)pyrrolidinium tetrafluoroborate (TPyClU),
chlorodipiperidinocarbenium hexafluorophosphate (PipClU),
2-chloro-1,3-dimethylimidazolinium chloride (DMC),
2-chloro-1,3-dimethylimidazolinium hexafluorophosphate (CIP), and
2-chloro-1,3-dimethylimidazolinium tetrafluoroborate (CIB), and two or more of the halouronium-based condensing agent may be used.

[A-6] The method according to [A-1] or [A-2], wherein the condensing agent is a 2-halo-N-alkyl pyridinium-based condensing agent.

[A-7] The method according to [A-6], wherein the 2-halo-N-alkyl pyridinium-based condensing agent is a 2-halo-N-alkyl pyridinium-based condensing agent, for example, selected from the group consisting of 2-chloro-1-methylpyridinium iodide, 2-bromo-1-ethyl pyridinium tetrafluoroborate, and 2-fluoro-1-methylpyridinium p-toluenesulfonate, and two or more of the 2-halo-N-alkyl pyridinium-based condensing agent may be used.

[A-8] The method according to any of [A-1] to [A-7], wherein the reaction is performed further in the presence of a second base.

[A-9] The method according to any of [A-1] to [A-8], wherein the reaction is performed in the presence of an excess amount of the amine compound as a second base relative to the carboxylic acid compound.

[A-10] The method according to [A-8] or [A-9], wherein the second base is an organic base having a pKa of a conjugated acid in water of 11 or less.

[A-11] The method according to any of [A-8] to [A-10], wherein the pKa of a conjugated acid of the second base in water is 0 to 11, 1 to 9, 2 to 8, 3 to 8, or 3.7 to 7.9.

[A-12] The method according to any of [A-1] to [A-11], wherein the second base is selected from the group consisting of formulas B1 and B2: 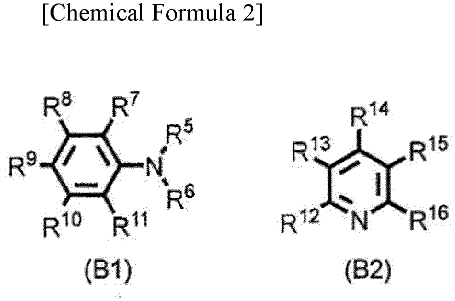
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms, and
two or more of the second base may be used.

[A-13] The method according to any of [A-8] to [A-12], wherein the second base is selected from the group consisting of formula B 1: 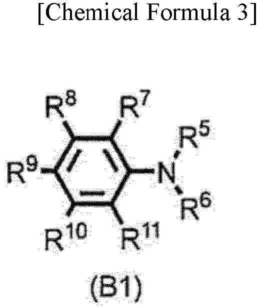
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl; and
R⁷, R⁸, R⁹, R¹⁰, and R¹¹ are each independently a hydrogen atom, C₁₋₆ alkyl optionally substituted with one or more halogen atoms, C₆₋₁₀ aryl optionally substituted with one or more halogen atoms, a halogen atom, or cyano, and
two or more of the second base may be used.

[A-14] The method according to [A-12] or [A-13], wherein R⁵ and R⁶ together with a nitrogen atom to which they are attached form a 5- to 7-membered saturated heterocycle; or
R⁵ and R⁷, and R⁶ and R¹¹ together with a nitrogen atom and a carbon atom to which they are attached form a 5- to 7-membered non-aromatic heterocycle, or
R⁵ and R⁶ are each independently C₁₋₆ alkyl;
R⁷, R⁸, R⁹, R¹⁰, and R¹¹ are each independently a hydrogen atom, C₁₋₆ alkyl optionally substituted with one or more halogen atoms, or a halogen atom; and
R¹², R¹³, R¹⁴, R¹⁵, and R¹⁶ are each independently a hydrogen atom, or C₁₋₆ alkyl optionally substituted with one or more halogen atoms.

[A-15] The method according to any of [A-8] to [A-12], wherein the second base is selected from the group consisting of N,N-dimethylaniline, N,N,2,4,6-pentamethylaniline, julolidine, collidine, and 2,6-lutidine in which a benzene ring of each is optionally substituted with a halogen atom or trifluoromethyl, and
two or more of the second base may be used.

[A-16] The method according to any of [A-8] to [A-15], wherein, in the first base, the R¹ is C₁₋₆ alkyl or C₆₋₁₀ aryl, and the R², R³ and R⁴ are each independently a hydrogen atom, C₁₋₆ alkyl or C₆₋₁₀ aryl.

[A-17] The method according to any of [A-8] to [A-16], wherein the first base is selected from the group consisting of N-methylimidazole, tetramethylimidazole, and N-phenylimidazole, and two or more of the first base may be used.

[A-18] The method according to any of [A-1] to [A-17], wherein 2 or more equivalents of the amine compound are used relative to the carboxylic acid compound.

[A-19] The method according to any of [A-1] to [A-18], wherein the reaction is performed in the presence of a solvent.

[A-20] The method according to [A-19], wherein the solvent is selected from the group consisting of a halogen-based solvent, a nitrile-based solvent, an amide-based solvent, an ether-based solvent, and an aromatic hydrocarbon-based solvent, and two or more of the solvent may be used.

[A-21] The method according to [A-19] or [A-20], wherein the solvent is selected from the group consisting of a halogen-based solvent, a nitrile-based solvent, an ether-based solvent, and an amide-based solvent, and two or more of the solvent may be used.

[A-22] The method according to any of [A-19] to [A-21], wherein the solvent is selected from the group consisting of a halogen-based solvent, a nitrile-based solvent, and an amide-based solvent, and two or more of the solvent may be used.

[A-23] The method according to any of [A-20] to [A-22], wherein the halogen-based solvent is selected from the group consisting of dichloromethane, chloroform, and 1,2-dichloroethane, and two or more of the halogen-based solvent may be used.

[A-24] The method according to any of [A-20] to [A-23], wherein the halogen-based solvent is dichloromethane.

[A-25] The method according to any of [A-20] to [A-22], wherein the nitrile-based solvent is selected from the group consisting of acetonitrile, propionitrile, and benzonitrile, and two or more of the nitrile-based solvent may be used.

[A-26] The method according to any of [A-20] to [A-22], wherein the nitrile-based solvent is acetonitrile.

[A-27] The method according to any of [A-20] to [A-22], wherein the amide-based solvent is selected from the group consisting of N-methylpyrrolidone, N,N-dimethylacetamide, N,N-dimethylformamide, N,N-dimethylpropionamide, N,N-dimethylisobutylamide, N,N-diethylacetamide, N,N-diethylpropionamide, 1-ethyl-2-pyrrolidinone, 1-octyl-2-pyrrolidinone, 1-cyclohexyl-2-pyrrolidinone, and N-methylcaprolactam, and two or more of the amide-based solvent may be used.

[A-28] The method according to [A-20] or [A-21], wherein the ether-based solvent is selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, and 4-methyltetrahydropyran, and two or more of the ether-based solvent may be used.

[A-29] The method according to [A-20] or [A-21], wherein the ether-based solvent is tetrahydrofuran.

[A-30] The method according to [A-20], wherein the aromatic hydrocarbon-based solvent is selected from the group consisting of benzene, toluene, and xylene, and two or more of the aromatic hydrocarbon-based solvent may be used.

[A-31] The method according to any of [A-1] to [A-30], wherein an equivalent ratio of the condensing agent to the carboxylic acid compound is condensing agent/carboxylic acid compound = 20/1 to 1/1.

[A-32] The method according to any of [A-1] to [A-31], wherein a molar ratio of the condensing agent to the carboxylic acid is condensing agent/carboxylic acid = 15/1 to 1/1, 10/1 to 1/1, or 5/1 to 1/1.

[A-33] The method according to any of [A-1] to [A-32], wherein the reaction is performed at a reaction temperature of 0°C to 100°C.

[A-34] The method according to [A-33], wherein the reaction temperature is 10°C to 80°C, 10°C to 60°C, 15°C to 50°C, 15°C to 40°C, or 20°C to 40°C.

[A-35] The method according to [A-33], wherein the reaction temperature is 10°C to 80°C, 10°C to 60°C, 15°C to 40°C, or 20°C to 30°C.

[A-36] The method according to any of [A-1] to [A-35], wherein the carboxylic acid compound is a resin for solid-phase synthesis to which the carboxylic acid compound is attached via a linker, or the amine compound is a resin for solid-phase synthesis to which the amine compound is attached via a linker.

[A-37] The method according to any of [A-1] to [A-36], wherein the carboxylic acid compound is a resin for solid-phase synthesis to which two or more different carboxylic acid compounds are attached via a linker, or the amine compound is a resin for solid-phase synthesis to which two or more different amine compounds are attached via a linker.

[A-38] The method according to any of [A-1] to [A-37], wherein the carboxylic acid compound is a resin for solid-phase synthesis to which the carboxylic acid compound is attached via a linker.

[A-39] The method according to any of [A-1] to [A-37], wherein the amine compound is a resin for solid-phase synthesis to which the amine compound is attached via a linker.

[A-40] The method according to any of [A-1] to [A-39],
wherein the carboxylic acid compound is a compound or a mixture of two or more compounds represented by formula A1 or formula A2: 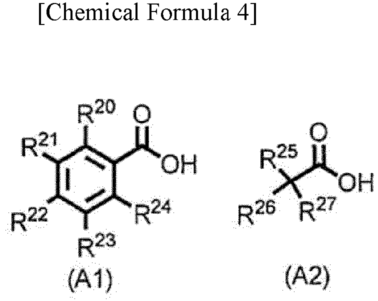
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the carboxylic acid compound does not have a group involved in an amide bond formation reaction other than a carboxy group represented by formula A1 or formula A2.

[A-41] The method according to [A-40], wherein the carboxylic acid compound is a compound or a mixture of two or more compounds represented by the formula A1, or a resin for solid-phase synthesis to which the compound is attached via a linker, and wherein the carboxylic acid compound does not have a group involved in an amide bond formation reaction other than a carboxy group represented by formula A1.

[A-42] The method according to [A-41], wherein the R²⁰ and R²⁴ in the carboxylic acid compound are each independently C₁₋₆ alkyl or C₆₋₁₀ aryl.

[A-43] The method according to any of [A-1] to [A-37],
wherein the amine compound is a compound or a mixture of two or more compounds represented by formula A3 or formula A4: 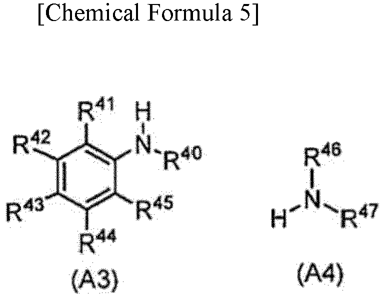
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group involved in an amide bond formation reaction other than an amino group represented by formula A3 or formula A4.

[A-44] The method according to [A-43], wherein the amine compound is a compound or a mixture of two or more compounds represented by formula A3, or a resin for solid-phase synthesis to which the compound is attached via a linker, and wherein the amine compound does not have a group involved in an amide bond formation reaction other than an amino group represented by formula A3.

[A-45] The method according to [A-44], wherein the R⁴¹ and R⁴⁵ in the amine compound are each independently C₁₋₆ alkyl or C₆₋₁₀ aryl.

[A-46] The method according to any of [A-1] to [A-45],
wherein the uronium-based condensing agent is a compound or a mixture of two or more compounds represented by formula C1: 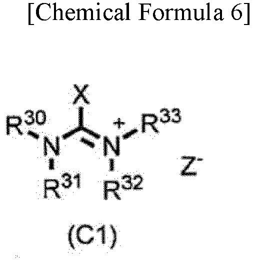
wherein R³⁰, R³¹, R³², and R³³ are each independently C₁₋₆ alkyl; or
R³⁰ and R³¹ and/or R³² and R³³ form a 5- to 7-membered saturated heterocycle, wherein the heterocycle may comprise one ring heteroatom selected from O or S; or
R³¹ and R³² form a 5- to 7-membered saturated heterocycle, wherein the heterocycle may comprise one ring heteroatom selected from O or S;
X is a leaving group; and
Z is a counter anion.

[A-47] The method according to [A-46], wherein X is a halogen atom.

[A-48] The method according to [A-46] or [A-42], wherein X is a fluorine atom or a chlorine atom.

[A-49] The method according to any of [A-1], [A-2], and [A-6] to [A-48],
wherein the 2-halo-N-alkyl pyridinium-based condensing agent is a compound or a mixture of two or more compounds represented by formula C3: 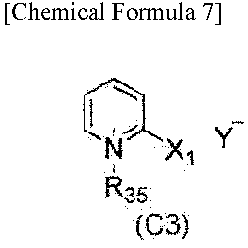
wherein R³⁵ is C₁₋₆ alkyl, X₁ is halogen, and Y is a counter anion.

[A-50] The method according to any of [A-1] to [A-49], wherein the reaction is performed in a mixture comprising two or more different carboxylic acid compounds and/or two or more different amine compounds as substrates.

[A-51] The method according to any of [A-1] to [A-50], wherein the reaction is performed in a mixture comprising two or more different carboxylic acid compounds as substrates.

[A-52] The method according to any of [A-1] to [A-51], wherein the reaction is performed in a mixture comprising two or more different amine compounds as substrates.

[A-53] The method according to any of [A-45] to [A-52], wherein the mixture comprises, as a substrate, a resin for solid-phase synthesis to which two or more different carboxylic acid compounds are attached via a linker, or a resin for solid-phase synthesis to which two or more different amine compounds are attached via a linker, and wherein the carboxylic acid compounds or amine compounds attached to the individual resins are the same.

[A-54] The method according to any of [A-1] to [A-11] and [A-18] to [A-53], wherein the reaction is performed in the presence of an excess amount of the amine compound as a second base relative to the carboxylic acid compound, and wherein the amine compound is a compound or a mixture of two or more compounds represented by formula A3 described in [A-43].

[A-55] A method for producing a compound constituting a compound library, the method comprising producing an amide compound by the method according to any of [A-1] to [A-54].

[B-1] A method for forming an amide bond by dehydration condensation of a carboxy group and an amino group, comprising reacting a carboxylic acid compound and an amine compound in the presence of a condensing agent and a first base to form an amide bond,
wherein the first base is a compound or a mixture of two or more compounds represented by formula A: 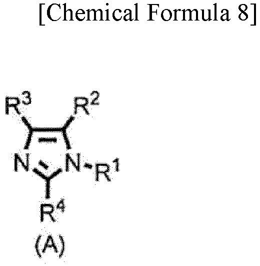
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
wherein a molar ratio of the first base to the condensing agent (first base/condensing agent) is 1.8 or less.

[B-2] The method according to [B-1], wherein a molar ratio of the first base to the condensing agent is 1.7 or less, 1.6 or less, 1.5 or less, 1.4 or less, 1.3 or less, 1.2 or less, 1.1 or less, or 1.0 or less.

[B-3] The method according to [B-1] or [B-2], wherein the condensing agent is at least one selected from a uronium-based condensing agent or a 2-halo-N-alkyl pyridinium-based condensing agent.

[B-4] The method according to [B-1] or [B-2], wherein the condensing agent is a uronium-based condensing agent.

[B-5] The method according to [B-1] or [B-2], wherein the uronium-based condensing agent is a halouronium-based condensing agent, such as a halouronium-based condensing agent selected from the group consisting of
fluoro-N,N,N',N'-tetramethylformamidinium hexafluorophosphate (TFFH),
chloro-N,N,N',N'-tetramethylformamidinium hexafluorophosphate (TCFH),
1-(chloro-1-pyrrolidinylmethylene)pyrrolidinium hexafluorophosphate (PyClU),
1-(chloro-1-pyrrolidinylmethylene)pyrrolidinium tetrafluoroborate (TPyClU),
chlorodipiperidinocarbenium hexafluorophosphate (PipClU),
2-chloro-1,3-dimethylimidazolinium chloride (DMC),
2-chloro-1,3-dimethylimidazolinium hexafluorophosphate (CIP), and
2-chloro-1,3-dimethylimidazolinium tetrafluoroborate (CIB), and two or more of the halouronium-based condensing agent may be used.

[B-6] The method according to [B-1] or [B-2], wherein the condensing agent is a 2-halo-N-alkyl pyridinium-based condensing agent.

[B-7] The method according to [B-1] or [B-2], wherein the 2-halo-N-alkyl pyridinium-based condensing agent is the 2-halo-N-alkyl pyridinium-based condensing agent, for example, selected from the group consisting of 2-chloro-1-methylpyridinium iodide, 2-bromo-1-ethyl pyridinium tetrafluoroborate, and 2-fluoro-1-methylpyridinium p-toluenesulfonate, and two or more of the 2-halo-N-alkyl pyridinium-based condensing agent may be used.

[B-8] The method according to any of [B-1] to [B-7], wherein the reaction is performed further in the presence of a second base.

[B-9] The method according to any of [B-1] to [B-8], wherein the reaction is performed in the presence of an excess amount of the amine compound as a second base.

[B-10] The method according to [B-8] or [B-9], wherein the second base is an organic base having a pKa of a conjugated acid in water of 11 or less.

[B-11] The method according to any of [B-8] to [B-10], wherein the pKa of a conjugated acid of the second base in water is 0 to 11, 1 to 9, 2 to 8, 3 to 8, or 3.7 to 7.9.

[B-12] The method according to any of [B-8] to [B-11], wherein the second base is selected from the group consisting of formulas B1 and B2: 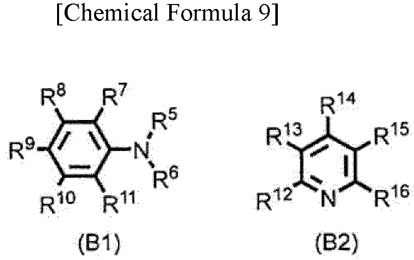
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms, and
two or more of the second base may be used.

[B-13] The method according to any of [B-8] to [B-12], wherein the second base is selected from the group consisting of formula B1: 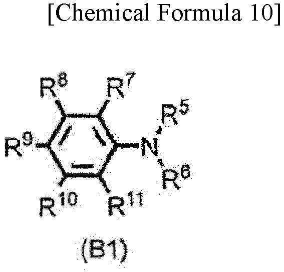
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl; and
R⁷, R⁸, R⁹, R¹⁰, and R¹¹ are each independently a hydrogen atom, C₁₋₆ alkyl optionally substituted with one or more halogen atoms, C₆₋₁₀ aryl optionally substituted with one or more halogen atoms, a halogen atom, or cyano, and
two or more of the second base may be used.

[B-14] The method according to [B-12] or [B-13], wherein R⁵ and R⁶ together with a nitrogen atom to which they are attached form a 5- to 7-membered saturated heterocycle; or
R⁵ and R⁷, and R⁶ and R¹¹ together with a nitrogen atom and a carbon atom to which they are attached form a 5- to 7-membered non-aromatic heterocycle, or
R⁵ and R⁶ are each independently C₁₋₆ alkyl;
R⁷, R⁸, R⁹, R¹⁰, and R¹¹ are each independently a hydrogen atom, C₁₋₆ alkyl optionally substituted with one or more halogen atoms, or a halogen atom; and
R¹², R¹³, R¹⁴, R¹⁵, and R¹⁶ are each independently a hydrogen atom, or C₁₋₆ alkyl optionally substituted with one or more halogen atoms.

[B-15] The method according to any of [B-8] to [B-12], wherein the second base is selected from the group consisting of N,N-dimethylaniline, N,N,2,4,6-pentamethylaniline, julolidine, collidine, and 2,6-lutidine in which a benzene ring of each is optionally substituted with a halogen atom or trifluoromethyl, and
two or more of the second base may be used.

[B-16] The method according to any of [B-8] to [B-15], wherein, in the first base, the R¹ is C₁₋₆ alkyl or C₆₋₁₀ aryl, and the R², R³ and R⁴ are each independently a hydrogen atom, C₁₋₆ alkyl or C₆₋₁₀ aryl.

[B-17] The method according to any of [B-8] to [B-16], wherein the first base is selected from the group consisting of N-methylimidazole, tetramethylimidazole, and N-phenylimidazole, and two or more of the first base may be used.

[B-18] The method according to any of [B-1] to [B-17], wherein 2 or more equivalents of the amine compound are used relative to the carboxylic acid compound.

[B-19] The method according to any of [B-1] to [B-18], wherein the reaction is performed in the presence of a solvent.

[B-20] The method according to [B-19], wherein the solvent is selected from the group consisting of a halogen-based solvent, a nitrile-based solvent, an amide-based solvent, an ether-based solvent, and an aromatic hydrocarbon-based solvent, and two or more of the solvent may be used.

[B-21] The method according to [B-19] or [B-20], wherein the solvent is selected from the group consisting of a halogen-based solvent, a nitrile-based solvent, an ether-based solvent, and an amide-based solvent, and two or more of the solvent may be used.

[B-22] The method according to any of [B-19] to [B-21], wherein the solvent is selected from the group consisting of a halogen-based solvent, a nitrile-based solvent, and an amide-based solvent, and two or more of the solvent may be used.

[B-23] The method according to any of [B-20] to [B-22], wherein the halogen-based solvent is selected from the group consisting of dichloromethane, chloroform, and 1,2-dichloroethane, and two or more of the halogen-based solvent may be used.

[B-24] The method according to any of [B-20] to [B-22], wherein the halogen-based solvent is dichloromethane.

[B-25] The method according to any of [B-20] to [B-22], wherein the nitrile-based solvent is selected from the group consisting of acetonitrile, propionitrile, and benzonitrile, and two or more of the nitrile-based solvent may be used.

[B-26] The method according to any of [B-20] to [B-22], wherein the nitrile-based solvent is acetonitrile.

[B-27] The method according to any of [B-20] to [B-22], wherein the amide-based solvent is selected from the group consisting of N-methylpyrrolidone, N,N-dimethylacetamide, N,N-dimethylformamide, N,N-dimethylpropionamide, N,N-dimethylisobutylamide, N,N-diethylacetamide, N,N-diethylpropionamide, 1-ethyl-2-pyrrolidinone, 1-octyl-2-pyrrolidinone, 1-cyclohexyl-2-pyrrolidinone, and N-methylcaprolactam, and two or more of the amide-based solvent may be used.

[B-28] The method according to [B-20] or [B-21], wherein the ether-based solvent is selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, and 4-methyltetrahydropyran, and two or more of the ether-based solvent may be used.

[B-29] The method according to [B-20] or [B-21], wherein the ether-based solvent is tetrahydrofuran.

[B-30] The method according to any of [B-20] to [B-22], wherein the aromatic hydrocarbon-based solvent is selected from the group consisting of benzene, toluene, and xylene, and two or more of the aromatic hydrocarbon-based solvent may be used.

[B-31] The method according to any of [B-1] to [B-30], wherein an equivalent ratio of the condensing agent to the carboxylic acid compound is condensing agent/carboxylic acid compound = 20/1 to 1/1.

[B-32] The method according to any of [B-1] to [B-31], wherein a molar ratio of the condensing agent to the carboxylic acid is condensing agent/carboxylic acid = 15/1 to 1/1, 10/1 to 1/1, or 5/1 to 1/1.

[B-33] The method according to any of [B-1] to [B-32], wherein the reaction is performed at a reaction temperature of 0°C to 100°C.

[B-34] The method according to [B-33], wherein the reaction temperature is 10°C to 80°C, 10°C to 60°C, 15°C to 50°C, 15°C to 40°C, or 20°C to 40°C.

[B-35] The method according to [B-33], wherein the reaction temperature is 10°C to 80°C, 10°C to 60°C, 15°C to 40°C, or 20°C to 30°C.

[B-36] The method according to any of [B-1] to [B-35], wherein the carboxylic acid compound is a resin for solid-phase synthesis to which the carboxylic acid compound is attached via a linker, or the amine compound is a resin for solid-phase synthesis to which the amine compound is attached via a linker.

[B-37] The method according to any of [B-1] to [B-36], wherein the carboxylic acid compound is a resin for solid-phase synthesis to which two or more different carboxylic acid compounds are attached via a linker, or the amine compound is a resin for solid-phase synthesis to which two or more different amine compounds are attached via a linker.

[B-38] The method according to any of [B-1] to [B-37], wherein the carboxylic acid compound is a resin for solid-phase synthesis to which the carboxylic acid compound is attached via a linker.

[B-39] The method according to any of [B-1] to [B-37], wherein the amine compound is a resin for solid-phase synthesis to which the amine compound is attached via a linker.

[B-40] The method according to any of [B-1] to [B-39],
wherein the carboxylic acid compound is a compound or a mixture of two or more compounds represented by formula A1 or formula A2: 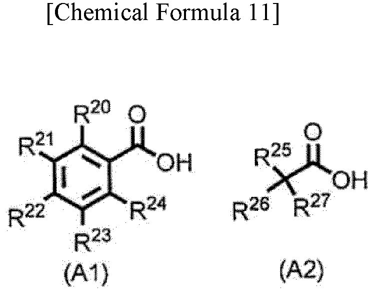
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the carboxylic acid compound does not have a group involved in an amide bond formation reaction other than a carboxy group represented by formula A1 or formula A2.

[B-41] The method according to [B-40], wherein the carboxylic acid compound is a compound or a mixture of two or more compounds represented by the formula A1, or a resin for solid-phase synthesis to which the compound is attached via a linker, and wherein the carboxylic acid compound does not have a group involved in an amide bond formation reaction other than a carboxy group represented by formula A1.

[B-42] The method according to [B-41], wherein the R²⁰ and R²⁴ in the carboxylic acid compound are each independently C₁₋₆ alkyl or C₆₋₁₀ aryl.

[B-43] The method according to any of [B-1] to [B-42],
wherein the amine compound is a compound or a mixture of two or more compounds represented by formula A3 or formula A4: 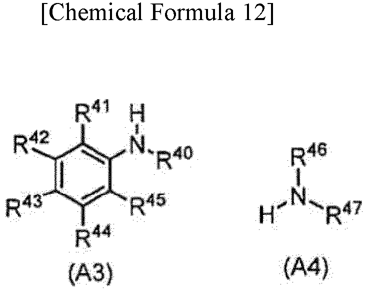
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group involved in an amide bond formation reaction other than an amino group represented by formula A3 or formula A4.

[B-44] The method according to [B-43], wherein the amine compound is a compound or a mixture of two or more compounds represented by the formula A3, or a resin for solid-phase synthesis to which the compound is attached via a linker, and wherein the amine compound does not have a group involved in an amide bond formation reaction other than an amino group represented by formula A3.

[B-45] The method according to [B-44], wherein the R⁴¹ and R⁴⁵ in the amine compound are each independently C₁₋₆ alkyl or C₆₋₁₀ aryl.

[B-46] The method according to any of [B-1] to [B-45],
wherein the uronium-based condensing agent is a compound or a mixture of two or more compounds represented by formula C1: 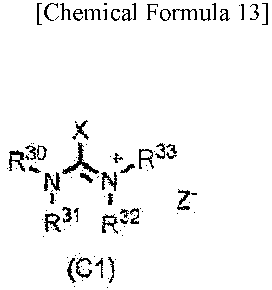
wherein R³⁰, R³¹, R³², and R³³ are each independently C₁₋₆ alkyl; or
R³⁰ and R³¹ and/or R³² and R³³ form a 5- to 7-membered saturated heterocycle, wherein the heterocycle may comprise one ring heteroatom selected from O or S; or
R³¹ and R³² form a 5- to 7-membered non-aromatic heterocycle, wherein the heterocycle may comprise one ring heteroatom selected from O or S;
X is a leaving group; and
Z⁻ is a counter anion.

[B-47] The method according to [B-46], wherein X is a halogen atom.

[B-48] The method according to [B-46] or [B-47], wherein X is a fluorine atom or a chlorine atom.

[B-49] The method according to any of [B-1], [B-2], and [B-6] to [B-48],
wherein the 2-halo-N-alkyl pyridinium-based condensing agent is a compound or a mixture of two or more compounds represented by formula C3: 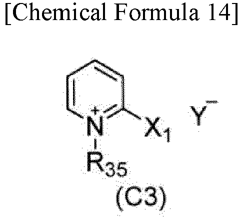
wherein R³⁵ is C₁₋₆ alkyl, X₁ is halogen, and Y is a counter anion.

[B-50] The method according to any of [B-1] to [B-49], wherein the reaction is performed in a mixture comprising two or more different carboxylic acid compounds and/or two or more different amine compounds as substrates.

[B-51] The method according to any of [B-1] to [B-50], wherein the reaction is performed in a mixture comprising two or more different carboxylic acid compounds as substrates.

[B-52] The method according to any of [B-1] to [B-41], wherein the reaction is performed in a mixture comprising two or more different amine compounds as substrates.

[B-53] The method according to any of [B-50] to [B-52], wherein the mixture comprises, as a substrate, a resin for solid-phase synthesis to which two or more different carboxylic acid compounds are attached via a linker, or a resin for solid-phase synthesis to which two or more different amine compounds are attached via a linker, and wherein the carboxylic acid compounds or amine compounds attached to the individual resins are the same.

[B-54] The method according to any of [B-1] to [B-11] and [B-18] to [B-53], wherein the reaction is performed in the presence of an excess amount of the amine compound as a second base relative to the carboxylic acid compound, and wherein the amine compound is a compound or a mixture of two or more compounds represented by formula A3 described in [B-43].

[C-1] A composition comprising a condensing agent for use in a method for forming an amide bond by dehydration condensation of a carboxy group and an amino group, the method comprising reacting a carboxylic acid compound and an amine compound in the presence of a condensing agent and a first base to form an amide bond,
wherein the first base is a compound or a mixture of two or more compounds represented by formula A: 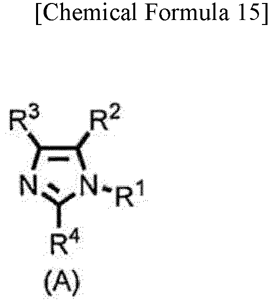
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
wherein a molar ratio of the first base to the condensing agent (first base/condensing agent) is 1.8 or less.

[C-2] The composition according to [C-1], wherein a molar ratio of the first base to the uronium-based condensing agent is 1.7 or less, 1.6 or less, 1.5 or less, 1.4 or less, 1.3 or less, 1.2 or less, 1.1 or less, or 1.0 or less.

[C-3] The composition according to [C-1] or [C-2], wherein the condensing agent is at least one selected from a uronium-based condensing agent or a 2-halo-N-alkyl pyridinium-based condensing agent.

[C-4] The composition according to [C-1] or [C-2], wherein the condensing agent is a uronium-based condensing agent.

[C-5] The composition according to [C-1] or [C-2], wherein the uronium-based condensing agent is a halouronium-based condensing agent, such as a halouronium-based condensing agent selected from the group consisting of
fluoro-N,N,N',N'-tetramethylformamidinium hexafluorophosphate (TFFH),
chloro-N,N,N',N'-tetramethylformamidinium hexafluorophosphate (TCFH),
1-(chloro-1-pyrrolidinylmethylene)pyrrolidinium hexafluorophosphate (PyClU),
1-(chloro-1-pyrrolidinylmethylene)pyrrolidinium tetrafluoroborate (TPyClU),
chlorodipiperidinocarbenium hexafluorophosphate (PipClU),
2-chloro-1,3-dimethylimidazolinium chloride (DMC),
2-chloro-1,3-dimethylimidazolinium hexafluorophosphate (CIP), and
2-chloro-1,3-dimethylimidazolinium tetrafluoroborate (CIB), and two or more of the halouronium-based condensing agent may be used.

[C-6] The method according to [C-1] or [C-2], wherein the condensing agent is a 2-halo-N-alkyl pyridinium-based condensing agent.

[C-7] The method according to [C-1] or [C-2], wherein the 2-halo-N-alkyl pyridinium-based condensing agent is a 2-halo-N-alkyl pyridinium-based condensing agent, for example, selected from the group consisting of 2-chloro-1-methylpyridinium iodide, 2-bromo-1-ethyl pyridinium tetrafluoroborate, and 2-fluoro-1-methylpyridinium p-toluenesulfonate, and two or more of the 2-halo-N-alkyl pyridinium-based condensing agent may be used.

[C-8] The composition according to any of [C-1] to [C-7], wherein the reaction is performed further in the presence of a second base.

[C-9] The composition according to any of [C-1] to [C-8], wherein the reaction is performed in the presence of an excess amount of the amine compound as a second base.

[C-10] The composition according to [C-8] or [C-9], wherein the second base is an organic base having a pKa of a conjugated acid in water of 11 or less.

[C-11] The composition according to any of [C-8] to [C-10], wherein the pKa of a conjugated acid of the second base in water is 0 to 11, 1 to 9, 2 to 8, 3 to 8, or 3.7 to 7.9.

[C-12] The composition according to any of [C-8] to [C-11], wherein the second base is selected from the group consisting of formulas B1 and B2: 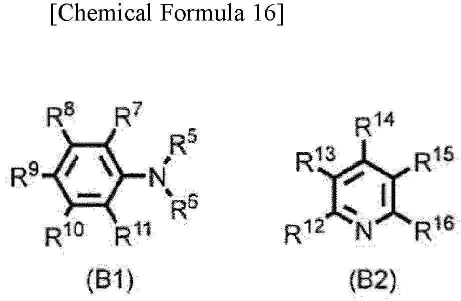
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms, and
two or more of the second base may be used.

[C-13] The composition according to any of [C-8] to [C-12], wherein the second base is selected from the group consisting of formula B1: 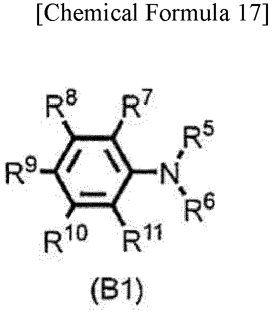
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl; and
R⁷, R⁸, R⁹, R¹⁰, and R¹¹ are each independently a hydrogen atom, C₁₋₆ alkyl optionally substituted with one or more halogen atoms, C₆₋₁₀ aryl optionally substituted with one or more halogen atoms, a halogen atom, or cyano, and
two or more of the second base may be used.

[C-14] The composition according to [C-12] or [C-13], wherein R⁵ and R⁶ together with a nitrogen atom to which they are attached form a 5- to 7-membered saturated heterocycle; or
R⁵ and R⁷, and R⁶ and R¹¹ together with a nitrogen atom and a carbon atom to which they are attached form a 5- to 7-membered non-aromatic heterocycle, or
R⁵ and R⁶ are each independently C₁₋₆ alkyl;
R⁷, R⁸, R⁹, R¹⁰, and R¹¹ are each independently a hydrogen atom, C₁₋₆ alkyl optionally substituted with one or more halogen atoms, or a halogen atom; and
R¹², R¹³, R¹⁴, R¹⁵, and R¹⁶ are each independently a hydrogen atom, or C₁₋₆ alkyl optionally substituted with one or more halogen atoms.

[C-15] The composition according to any of [C-8] to [C-12], wherein the second base is selected from the group consisting of N,N-dimethylaniline, N,N,2,4,6-pentamethylaniline, julolidine, collidine, and 2,6-lutidine in which a benzene ring of each is optionally substituted with a halogen atom or trifluoromethyl, and
two or more of the second base may be used.

[C-16] The composition according to any of [C-8] to [C-15], wherein, in the first base, the R¹ is C₁₋₆ alkyl or C₆₋₁₀ aryl, and the R², R³ and R⁴ are each independently a hydrogen atom, C₁₋₆ alkyl or C₆₋₁₀ aryl.

[C-17] The composition according to any of [C-8] to [C-16], wherein the first base is selected from the group consisting of N-methylimidazole, tetramethylimidazole, and N-phenylimidazole, and two or more of the first base may be used.

[C-18] The composition according to any of [C-1] to [C-17], wherein 2 or more equivalents of the amine compound are used relative to the carboxylic acid compound.

[C-19] The composition according to any of [C-1] to [C-18], wherein the reaction is performed in the presence of a solvent.

[C-20] The composition according to [C-19], wherein the solvent is selected from the group consisting of a halogen-based solvent, a nitrile-based solvent, an amide-based solvent, an ether-based solvent, and an aromatic hydrocarbon-based solvent, and two or more of the solvent may be used.

[C-21] The composition according to [C-19] or [C-20], wherein the solvent is selected from the group consisting of a halogen-based solvent, a nitrile-based solvent, an ether-based solvent, and an amide-based solvent, and two or more of the solvent may be used.

[C-22] The composition according to any of [C-19] to [C-21], wherein the solvent is selected from the group consisting of a halogen-based solvent, a nitrile-based solvent, and an amide-based solvent, and two or more of the solvent may be used.

[C-23] The composition according to any of [C-20] to [C-22], wherein the halogen-based solvent is selected from the group consisting of dichloromethane, chloroform, and 1,2-dichloroethane, and two or more of the halogen-based solvent may be used.

[C-24] The composition according to any of [C-20] to [C-22], wherein the halogen-based solvent is dichloromethane.

[C-25] The composition according to any of [C-20] to [C-22], wherein the nitrile-based solvent is selected from the group consisting of acetonitrile, propionitrile, and benzonitrile, and two or more of the nitrile-based solvent may be used.

[C-27] The composition according to any of [C-20] to [C-22], wherein the amide-based solvent is selected from the group consisting of N-methylpyrrolidone, N,N-dimethylacetamide, N,N-dimethylformamide, N,N-dimethylpropionamide, N,N-dimethylisobutylamide, N,N-diethylacetamide, N,N-diethylpropionamide, 1-ethyl-2-pyrrolidinone, 1-octyl-2-pyrrolidinone, 1-cyclohexyl-2-pyrrolidinone, and N-methylcaprolactam, and two or more of the amide-based solvent may be used.

[C-28] The composition according to [C-20], wherein the ether-based solvent is selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, and 4-methyltetrahydropyran, and two or more of the ether-based solvent may be used.

[C-29] The composition according to [C-20] or [C-21], wherein the ether-based solvent is tetrahydrofuran.

[C-30] The composition according to any of [C-20] to [C-22], wherein the aromatic hydrocarbon-based solvent is selected from the group consisting of benzene, toluene, and xylene, and two or more of the aromatic hydrocarbon-based solvent may be used.

[C-31] The composition according to any of [C-1] to [C-30], wherein an equivalent ratio of the condensing agent to the carboxylic acid compound is condensing agent/carboxylic acid compound = 20/1 to 1/1.

[C-32] The composition according to any of [C-1] to [C-31], wherein a molar ratio of the condensing agent to the carboxylic acid is condensing agent/carboxylic acid = 15/1 to 1/1, 10/1 to 1/1, or 5/1 to 1/1.

[C-33] The composition according to any of [C-1] to [C-32], wherein the reaction is performed at a reaction temperature of 0°C to 100°C.

[C-34] The composition according to [C-33], wherein the reaction temperature is 10°C to 80°C, 10°C to 60°C, 15°C to 40°C, or 20°C to 30°C.

[C-35] The composition according to [C-33], wherein the reaction temperature is 10°C to 80°C, 10°C to 60°C, 15°C to 40°C, or 20°C to 30°C.

[C-36] The composition according to any of [C-1] to [C-35], wherein the carboxylic acid compound is a resin for solid-phase synthesis to which the carboxylic acid compound is attached via a linker, or the amine compound is a resin for solid-phase synthesis to which the amine compound is attached via a linker.

[C-37] The composition according to any of [C-1] to [C-36], wherein the carboxylic acid compound is a resin for solid-phase synthesis to which two or more different carboxylic acid compounds are attached via a linker, or the amine compound is a resin for solid-phase synthesis to which two or more different amine compounds are attached via a linker.

[C-38] The composition according to any of [C-1] to [C-37], wherein the carboxylic acid compound is a resin for solid-phase synthesis to which the carboxylic acid compound is attached via a linker.

[C-39] The composition according to any of [C-1] to [C-37], wherein the amine compound is a resin for solid-phase synthesis to which the amine compound is attached via a linker.

[C-40] The composition according to any of [C-1] to [C-39],
wherein the carboxylic acid compound is a compound or a mixture of two or more compounds represented by formula A1 or formula A2: 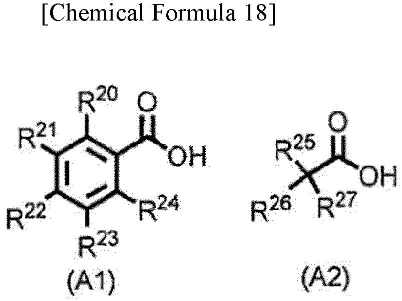
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with any substituent, or
a resin for solid-phase synthesis to which the compound is attached via a linker, and
wherein the carboxylic acid compound does not have a group involved in an amide bond formation reaction other than a carboxy group represented by formula A1 or formula A2.

[C-41] The composition according to [C-40], wherein the carboxylic acid compound is a compound or a mixture of two or more compounds represented by the formula A1, or a resin for solid-phase synthesis to which the compound is attached via a linker, and wherein the carboxylic acid compound does not have a group involved in an amide bond formation reaction other than a carboxy group represented by formula A1.

[C-42] The composition according to [C-41], wherein the R²⁰ and R²⁴ in the carboxylic acid compound are each independently C₁₋₆ alkyl or C₆₋₁₀ aryl.

[C-43] The composition according to any of [C-1] to [C-42],
wherein the amine compound is a compound or a mixture of two or more compounds represented by formula A3 or formula A4: 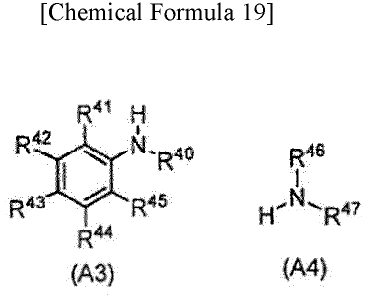
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group other than an amino group represented by formula A3 or formula A4 that is involved in an amide bond formation reaction.

[C-44] The composition according to [C-43], wherein the amine compound is a compound or a mixture of two or more compounds represented by the formula A3, or a resin for solid-phase synthesis to which the compound is attached via a linker, and wherein the amine compound does not have a group involved in an amide bond formation reaction other than an amino group represented by formula A3.

[C-45] The composition according to [C-44], wherein the R⁴¹ and R⁴⁵ in the amine compound are each independently C₁₋₆ alkyl or C₆₋₁₀ aryl.

[C-46] The composition according to any of [C-1] to [C-45],
wherein the uronium-based condensing agent is a compound or a mixture of two or more compounds represented by formula C1: 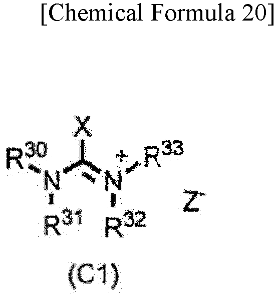
wherein R³⁰, R³¹, R³², and R³³ are each independently C₁₋₆ alkyl; or
R³¹ and R³² and/or R³² and R³³ form a 5- to 7-membered non-aromatic heterocycle, wherein the heterocycle may comprise one ring heteroatom selected from O or S; or
R³¹ and R³² form a 5- to 7-membered non-aromatic heterocycle, wherein the heterocycle may comprise one ring heteroatom selected from O or S;
X is a leaving group; and
Z⁻ is a counter anion.

[C-47] The composition according to [C-46], wherein X is a halogen atom.

[C-48] The composition according to [C-46] or [C-47], wherein X is a fluorine atom or a chlorine atom.

[C-49] The composition according to any of [C-1], [C-2], and [C-6] to [C-48],
wherein the 2-halo-N-alkyl pyridinium-based condensing agent is a compound or a mixture of two or more compounds represented by formula C3: 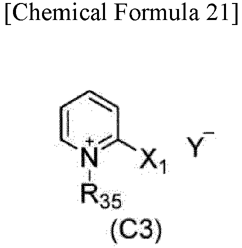
wherein R³⁵ is C₁₋₆ alkyl, X₁ is halogen, and Y is a counter anion.

[C-50] The composition according to any of [C-1] to [C-49], wherein the reaction is performed in a mixture comprising two or more different carboxylic acid compounds and/or two or more different amine compounds as substrates.

[C-51] The composition according to any of [C-1] to [C-50], wherein the reaction is performed in a mixture comprising two or more different carboxylic acid compounds as substrates.

[C-52] The composition according to any of [C-1] to [C-50], wherein the reaction is performed in a mixture comprising two or more different amine compounds as substrates.

[C-53] The composition according to any of [C-50] to [C-52], wherein the mixture comprises, as a substrate, a resin for solid-phase synthesis to which two or more different carboxylic acid compounds are attached via a linker, or a resin for solid-phase synthesis to which two or more different amine compounds are attached via a linker, and wherein the carboxylic acid compounds or amine compounds attached to the individual resins are the same.

[C-54] The composition according to any of [C-1] to [C-11] and [C-18] to [C-53], wherein the reaction is performed in the presence of an excess amount of the amine compound as a second base relative to the carboxylic acid compound, and wherein the amine compound is a compound or a mixture of two or more compounds represented by formula A3 described in [C-48].

[C-55] The composition according to any of [C-1] to [C-54] for use in the method according to any of [A-1] to [A-54] and [B-1] to [B-54].

[D-1] Use of a condensing agent in a method for forming an amide bond by dehydration condensation of a carboxy group and an amino group, the method comprising reacting a carboxylic acid compound and an amine compound in the presence of a condensing agent and a first base to form an amide bond,
wherein the first base is a compound represented by formula A: 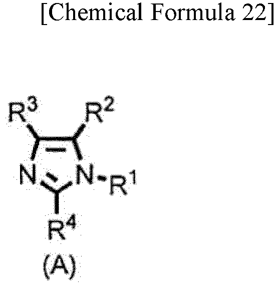
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
wherein a molar ratio of the first base to the condensing agent (first base/condensing agent) is 1.8 or less.

[D-2] The use according to [D-1], wherein a molar ratio of the first base to the condensing agent is 1.7 or less, 1.6 or less, 1.5 or less, 1.4 or less, 1.3 or less, 1.2 or less, 1.1 or less, or 1.0 or less.

[D-3] The use according to [D-1] or [D-2], wherein the condensing agent is at least one selected from a uronium-based condensing agent or a 2-halo-N-alkyl pyridinium-based condensing agent.

[D-4] The use according to [D-1] or [D-2], wherein the condensing agent is a uronium-based condensing agent.

[D-5] The use according to [D-1] or [D-2], wherein the uronium-based condensing agent is a halouronium-based condensing agent, such as a halouronium-based condensing agent selected from the group consisting of
fluoro-N,N,N',N'-tetramethylformamidinium hexafluorophosphate (TFFH),
chloro-N,N,N',N'-tetramethylformamidinium hexafluorophosphate (TCFH),
1-(chloro-1-pyrrolidinylmethylene)pyrrolidinium hexafluorophosphate (PyClU),
1-(chloro-1-pyrrolidinylmethylene)pyrrolidinium tetrafluoroborate (TPyClU),
chlorodipiperidinocarbenium hexafluorophosphate (PipClU),
2-chloro-1,3-dimethylimidazolinium chloride (DMC),
2-chloro-1,3-dimethylimidazolinium hexafluorophosphate (CIP), and
2-chloro-1,3-dimethylimidazolinium tetrafluoroborate (CIB), and two or more of the halouronium-based condensing agent may be used.

[D-6] The use according to [D-1] or [D-2], wherein the condensing agent is a 2-halo-N-alkyl pyridinium-based condensing agent.

[D-7] The use according to [D-1] or [D-2], wherein the 2-halo-N-alkyl pyridinium-based condensing agent is a 2-halo-N-alkyl pyridinium-based condensing agent, for example, selected from the group consisting of 2-chloro-1-methylpyridinium iodide, 2-bromo-1-ethyl pyridinium tetrafluoroborate, and 2-fluoro-1-methylpyridinium p-toluenesulfonate, and two or more of the 2-halo-N-alkyl pyridinium-based condensing agent may be used.

[D-8] The use according to any of [D-1] to [D-7], wherein the reaction is performed further in the presence of a second base.

[D-9] The use according to any of [D-1] to [D-8], wherein the reaction is performed in the presence of an excess amount of the amine compound as a second base relative to the carboxylic acid compound.

[D-10] The use according to [D-8] or [D-9], wherein the second base is an organic base having a pKa of a conjugated acid in water of 11 or less.

[D-11] The use according to any of [D-8] to [D-10], wherein the pKa of a conjugated acid of the second base in water is 0 to 11, 1 to 9, 2 to 8, 3 to 8, or 3.7 to 7.9.

[D-12] The use according to any of [D-8] to [D-11], wherein the second base is selected from the group consisting of formulas B1 and B2: 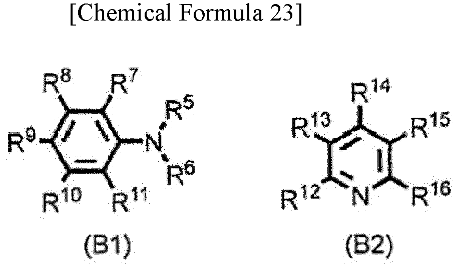
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl may be substituted with one or more halogen atoms, and
two or more of the second base may be used.

[D-13] The use according to any of [D-8] to [D-12], wherein the second base is selected from the group consisting of formula B 1: 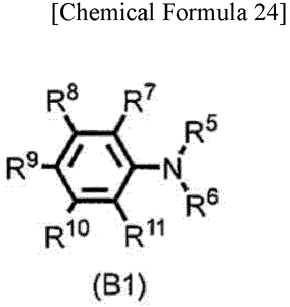
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl; and
R⁷, R⁸, R⁹, R¹⁰, and R¹¹ are each independently a hydrogen atom, C₁₋₆ alkyl optionally substituted with one or more halogen atoms, C₆₋₁₀ aryl optionally substituted with one or more halogen atoms, a halogen atom, or cyano, and
two or more of the second base may be used.

[D-14] The use according to [D-12] or [D-13], wherein R⁵ and R⁶ together with a nitrogen atom to which they are attached form a 5- to 7-membered saturated heterocycle; or
R⁵ and R⁷, and R⁶ and R¹¹ together with a nitrogen atom and a carbon atom to which they are attached form a 5- to 7-membered non-aromatic heterocycle, or
R⁵ and R⁶ are each independently C₁₋₆ alkyl;
R⁷, R⁸, R⁹, R¹⁰, and R¹¹ are each independently a hydrogen atom, C₁₋₆ alkyl optionally substituted with one or more halogen atoms, or a halogen atom; and
R¹², R¹³, R¹⁴, R¹⁵, and R¹⁶ are each independently a hydrogen atom, or C₁₋₆ alkyl optionally substituted with one or more halogen atoms.

[D-15] The use according to any of [D-8] to [D-12], wherein the second base is selected from the group consisting of N,N-dimethylaniline, N,N,2,4,6-pentamethylaniline, julolidine, collidine, and 2,6-lutidine in which a benzene ring of each is optionally substituted with a halogen atom or trifluoromethyl, and two or more of the second base may be used.

[D-16] The use according to any of [D-8] to [D-15], wherein, in the first base, the R¹ is C₁₋₆ alkyl or C₆₋₁₀ aryl, and the R², R³ and R⁴ are each independently a hydrogen atom, C₁₋₆ alkyl or C₆₋₁₀ aryl.

[D-17] The use according to any of [D-8] to [D-16], wherein the first base is selected from the group consisting of N-methylimidazole, tetramethylimidazole, and N-phenylimidazole, and two or more of the first base may be used.

[D-18] The use according to any of [D-1] to [D-17], wherein a molar ratio of the amine compound to the carboxylic acid compound is 2.0 or more.

[D-19] The use according to any of [D-1] to [D-18], wherein the reaction is performed in the presence of a solvent.

[D-20] The use according to [D-19], wherein the solvent is selected from the group consisting of a halogen-based solvent, a nitrile-based solvent, an amide-based solvent, an ether-based solvent, and an aromatic hydrocarbon-based solvent, and two or more of the solvent may be used.

[D-21] The use according to [D-19] or [D-20], wherein the solvent is selected from the group consisting of a halogen-based solvent, a nitrile-based solvent, an ether-based solvent, and an amide-based solvent, and two or more of the solvent may be used.

[D-22] The use according to any of [D-19] to [D-21], wherein the solvent is selected from the group consisting of a halogen-based solvent, a nitrile-based solvent, and an amide-based solvent, and two or more of the solvent may be used.

[D-23] The use according to any of [D-20] to [D-22], wherein the halogen-based solvent is selected from the group consisting of dichloromethane, chloroform, and 1,2-dichloroethane, and two or more of the halogen-based solvent may be used.

[D-24] The use according to any of [D-20] to [D-22], wherein the halogen-based solvent is dichloromethane.

[D-25] The use according to any of [D-20] to [D-23], wherein the nitrile-based solvent is selected from the group consisting of acetonitrile, propionitrile, and benzonitrile, and two or more of the nitrile-based solvent may be used.

[D-26] The use according to any of [D-20] to [D-22], wherein the nitrile-based solvent is acetonitrile.

[D-27] The use according to any of [D-20] to [D-22], wherein the amide-based solvent is selected from the group consisting of N-methylpyrrolidone, N,N-dimethylacetamide, N,N-dimethylformamide, N,N-dimethylpropionamide, N,N-dimethylisobutylamide, N,N-diethylacetamide, N,N-diethylpropionamide, 1-ethyl-2-pyrrolidinone, 1-octyl-2-pyrrolidinone, 1-cyclohexyl-2-pyrrolidinone, and N-methylcaprolactam, and two or more of the amide-based solvent may be used.

[D-28] The use according to [D-20] or [D-21], wherein the ether-based solvent is selected from the group consisting of tetrahydrofuran, diethyl ether, 2-methyltetrahydrofuran, 1,4-dioxane, 1,2-dimethoxyethane, 1,3-dioxolane, diisopropyl ether, cyclopentyl methyl ether, t-butyl methyl ether, and 4-methyltetrahydropyran, and two or more of the ether-based solvent may be used.

[D-29] The use according to [D-20] or [D-21], wherein the ether-based solvent is tetrahydrofuran.

[D-30] The use according to any of [D-20] to [D-22], wherein the aromatic hydrocarbon-based solvent is selected from the group consisting of benzene, toluene, and xylene, and two or more of the aromatic hydrocarbon-based solvent may be used.

[D-31] The use according to any of [D-1] to [D-30], wherein an equivalent ratio of the condensing agent to the carboxylic acid compound is condensing agent/carboxylic acid compound = 20/1 to 1/1.

[D-32] The use according to any of [D-1] to [D-31], wherein a molar ratio of the uronium-based condensing agent to the carboxylic acid is uronium-based condensing agent/carboxylic acid = 15/1 to 1/1, 10/1 to 1/1, or 5/1 to 1/1.

[D-33] The use according to any of [D-1] to [D-32], wherein the reaction is performed at a reaction temperature of 0°C to 100°C.

[D-34] The use according to [D-33], wherein the reaction temperature is 10°C to 80°C, 10°C to 60°C, 15°C to 40°C, or 20°C to 30°C.

[D-35] The use according to [D-33], wherein the reaction temperature is 10°C to 80°C, 10°C to 60°C, 15°C to 40°C, or 20°C to 30°C.

[D-36] The use according to any of [D-1] to [D-35], wherein the carboxylic acid compound is a resin for solid-phase synthesis to which the carboxylic acid compound is attached via a linker, or the amine compound is a resin for solid-phase synthesis to which the amine compound is attached via a linker.

[D-37] The use according to any of [D-1] to [D-36], wherein the carboxylic acid compound is a resin for solid-phase synthesis to which two or more different carboxylic acid compounds are attached via a linker, or the amine compound is a resin for solid-phase synthesis to which two or more different amine compounds are attached via a linker.

[D-38] The use according to any of [D-1] to [D-37], wherein the carboxylic acid compound is a resin for solid-phase synthesis to which the carboxylic acid compound is attached via a linker.

[D-39] The use according to any of [D-1] to [D-37], wherein the amine compound is a resin for solid-phase synthesis to which the amine compound is attached via a linker.

[D-40] The use according to any of [D-1] to [D-39],
wherein the carboxylic acid compound is a compound or a mixture of two or more compounds represented by formula A1 or formula A2: 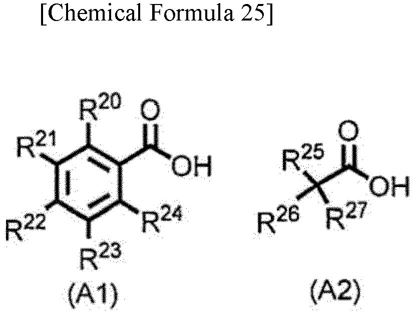
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with any substituent, or
a resin for solid-phase synthesis to which the compound is attached via a linker, and
wherein the carboxylic acid compound does not have a group involved in an amide bond formation reaction other than a carboxy group represented by formula A1 or formula A2.

[D-41] The use according to [D-40], wherein the carboxylic acid compound is a compound or a mixture of two or more compounds represented by the formula A1, or a resin for solid-phase synthesis to which the compound is attached via a linker, and wherein the carboxylic acid compound does not have a group involved in an amide bond formation reaction other than a carboxy group represented by formula A1.

[D-42] The use according to [D-41], wherein the R²⁰ and R²⁴ in the carboxylic acid compound are each independently C₁₋₆ alkyl or C₆₋₁₀ aryl.

[D-43] The use according to any of [D-1] to [D-42],
wherein the amine compound is a compound or a mixture of two or more compounds represented by formula A3 or formula A4: 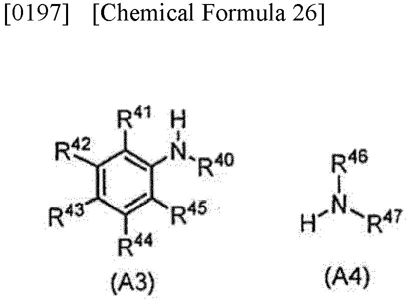
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group involved in an amide bond formation reaction other than an amino group represented by formula A3 or formula A4.

[D-44] The use according to [D-43], wherein the amine compound is a compound or a mixture of two or more compounds represented by the formula A3, or a resin for solid-phase synthesis to which the compound is attached via a linker, and wherein the amine compound does not have a group involved in an amide bond formation reaction other than an amino group represented by formula A3.

[D-45] The use according to [D-44], wherein the R⁴¹ and R⁴⁵ in the amine compound are each independently C₁₋₆ alkyl or C₆₋₁₀ aryl.

[D-46] The use according to any of [D-1] to [D-45],
wherein the uronium-based condensing agent is a compound or a mixture of two or more compounds represented by formula C1: 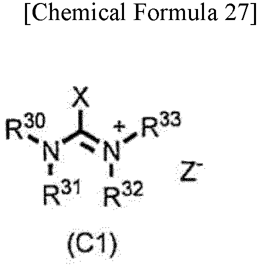
wherein R³⁰, R³¹, R³², and R³³ are each independently C₁₋₆ alkyl; or
R³¹ and R³² and/or R³² and R³³ form a 5- to 7-membered non-aromatic heterocycle, wherein the heterocycle may comprise one ring heteroatom selected from O or S; or
R³¹ and R³² form a 5- to 7-membered non-aromatic heterocycle, wherein the heterocycle may comprise one ring heteroatom selected from O or S;
X is a leaving group; and
Z⁻ is a counter anion.

[D-47] The use according to [D-46], wherein X is a halogen atom.

[D-48] The use according to [D-46] or [D-47], wherein X is a fluorine atom or a chlorine atom.

[D-49] The use according to any of [D-1], [D-2], and [D-6] to [D-48],
wherein the 2-halo-N-alkyl pyridinium-based condensing agent is a compound or a mixture of two or more compounds represented by formula C3: 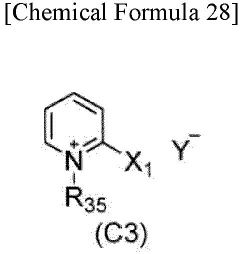
wherein R³⁵ is C₁₋₆ alkyl, X₁ is halogen, and Y is a counter anion.

[D-50] The use according to any of [D-1] to [D-49], wherein the reaction is performed in a mixture comprising two or more different carboxylic acid compounds and/or two or more different amine compounds as substrates.

[D-51] The use according to any of [D-1] to [D-50], wherein the reaction is performed in a mixture comprising two or more different carboxylic acid compounds as substrates.

[D-52] The use according to any of [D-1] to [D-51], wherein the reaction is performed in a mixture comprising two or more different amine compounds as substrates.

[D-53] The use according to any of [D-50] to [D-52], wherein the mixture comprises, as a substrate, a resin for solid-phase synthesis to which two or more different carboxylic acid compounds are attached via a linker, or a resin for solid-phase synthesis to which two or more different amine compounds are attached via a linker, and wherein the carboxylic acid compounds or amine compounds attached to the individual resins are the same.

[D-54] The use according to any of [D-1] to [D-11] and [D-18] to [D-53], wherein the reaction is performed in the presence of an excess amount of the amine compound as a second base relative to the carboxylic acid compound, and wherein the amine compound is a compound or a mixture of two or more compounds represented by formula A3 described in [D-43].

[D-55] The use according to any of [D-1] to [D-54] in the method according to any of [A-1] to [A-54] and [B-1] to [B-54].

[E-1] A method for producing an amide compound, comprising reacting a carboxylic acid compound and an amine compound in the presence of a uronium-based condensing agent and a first base to obtain an amide compound,
wherein the first base is represented by formula A: 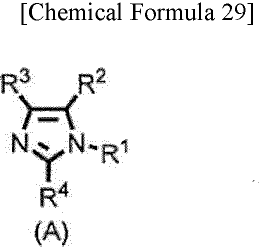
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
two or more of the first base may be used, and
wherein a molar ratio of the first base to the uronium-based condensing agent (first base/uronium-based condensing agent) is 1.8 or less.

[E-2] The method according to [E-1], wherein the uronium-based condensing agent is a halouronium-based condensing agent.

[E-3] The method according to [E-1] or [E-2], wherein the reaction is performed further in the presence of a second base.

[E-4] The method according to any of [E-1] to [E-3], wherein the reaction is performed in the presence of an excess amount of the amine compound as a second base.

[E-5] The method according to [E-3] or [E-4], wherein the second base is an organic base having a pKa of a conjugated acid in water of 11 or less.

[E-6] The method according to any of [E-3] to [E-5], wherein the second base is selected from the group consisting of formulas B 1 and B2: 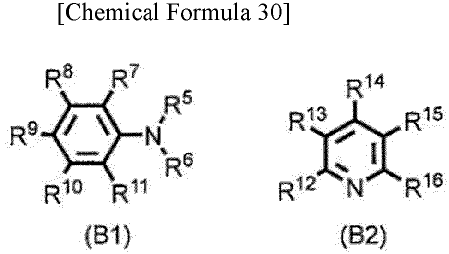
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms, and
two or more of the second base may be used.

[E-7] The method according to any of [E-1] to [E-6], wherein the first base is selected from the group consisting of N-methylimidazole, tetramethylimidazole, and N-phenylimidazole, and two or more of the first base may be used.

[E-8] The method according to any of [E-1] to [E-7], wherein the reaction is performed in the presence of a solvent, and wherein the solvent is selected from the group consisting of a halogen-based solvent, a nitrile-based solvent, an amide-based solvent, an ether-based solvent, and an aromatic hydrocarbon-based solvent, and two or more of the solvent may be used.

[E-9] The method according to any of [E-1] to [E-8], wherein the carboxylic acid compound is a resin for solid-phase synthesis to which the carboxylic acid compound is attached via a linker, or the amine compound is a resin for solid-phase synthesis to which the amine compound is attached via a linker.

[E-10] The method according to any of [E-1] to [E-9],
wherein the carboxylic acid compound is a compound or a mixture of two or more compounds represented by formula A1 or formula A2: 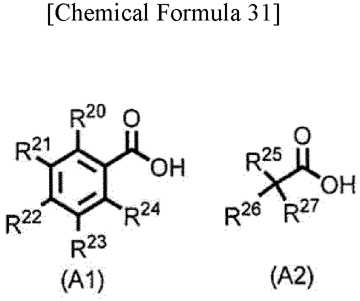
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the carboxylic acid compound does not have a group involved in an amide bond formation reaction other than a carboxy group represented by formula A1 or formula A2.

[E-11] The method according to any of [E-1] to [E-10],
wherein the amine compound is a compound or a mixture of two or more compounds represented by formula A3 or formula A4: 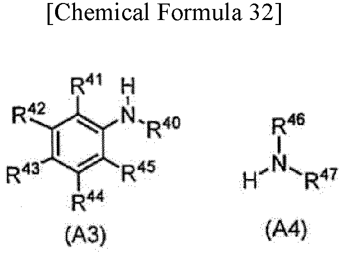
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group involved in an amide bond formation reaction other than an amino group represented by formula A3 or formula A4.

[E-12] The method according to any of [E-1] to [E-11],
wherein the uronium-based condensing agent is a compound or a mixture of two or more compounds represented by formula C 1: 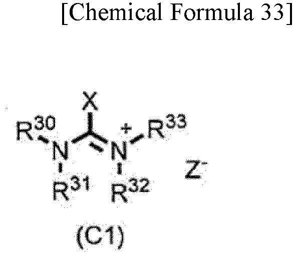
wherein R³⁰, R³¹, R³², and R³³ are each independently C₁₋₆ alkyl; or
R³⁰ and R³¹ and/or R³² and R³³ form a 5- to 7-membered saturated heterocycle, wherein the heterocycle may comprise one ring heteroatom selected from O or S; or
R³¹ and R³² form a 5- to 7-membered saturated heterocycle, wherein the heterocycle may comprise one ring heteroatom selected from O or S;
X is a leaving group; and
Z is a counter anion.

[E-13] The method according to any of [E-1] to [E-12], wherein the reaction is performed in a mixture comprising two or more different carboxylic acid compounds and/or two or more different amine compounds as substrates.

[E-14] A method for producing a compound constituting a compound library, the method comprising producing an amide compound by the method according to any of [E-1] to [E-13].

[E-15] A method for forming an amide bond by dehydration condensation of a carboxy group and an amino group, comprising reacting a carboxylic acid compound and an amine compound in the presence of a uronium-based condensing agent and a first base to form an amide bond,
wherein the first base is a compound or a mixture of two or more compounds represented by formula A: 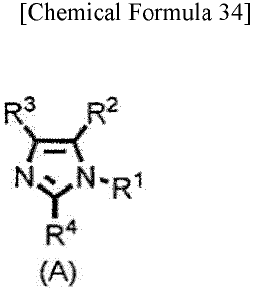
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
wherein a molar ratio of the first base to the uronium-based condensing agent (first base/uronium-based condensing agent) is 1.8 or less.

### ADVANTAGEOUS EFFECTS OF INVENTION

In one aspect, the present invention enables improved conversion rate in an amide-forming reaction from an amine compound and a carboxylic acid compound using a condensing agent, and/or efficient production of the amide compound by the reaction.

### DESCRIPTION OF EMBODIMENTS

In one aspect, the present invention provides a method for producing an amide compound, comprising reacting a carboxylic acid compound and an amine compound in the presence of a uronium-based condensing agent or 2-halo-N-alkyl pyridinium-based condensing agent and a first base to obtain an amide compound. The carboxylic acid compound is not particularly limited as long as it is a compound having one or more carboxy groups that serve as reaction points for amide bond formation reactions. In one embodiment, the carboxylic acid compound has 1 to 5, 1 to 4, 1 to 3, 1 or 2, or one carboxy group within its molecule. The amine compound is not particularly limited as long as it is a compound having one or more amino groups that serve as reaction points for amide bond formation reactions. In one embodiment, the amine compound has 1 to 5, 1 to 4, 1 to 3, 1 or 2, or one amino group within its molecule. The carboxylic acid compound and amine compound can be synthesized by known methods or can be commercially available.

In one aspect, the method for producing an amide compound of the present invention can be applied to an amide-forming reaction in a compound having a carboxy group and an amino group in one molecule. In one embodiment, the amide-forming reaction is a cyclization reaction, the resulting compound is a 4- to 40-membered, 4- to 34-membered, 4- to 12-membered, or 5- to 10-membered cyclic amide compound, and the ring-constituting atoms may comprise one or more heteroatoms selected from O, N, and S.

In one aspect of the present invention, the uronium-based condensing agent is not particularly limited as long as it is a uronium compound that is available for dehydration condensation in an amide bond formation reaction. In one embodiment, the uronium compound can be a known uronium salt. For example, a commercially available uronium compound can be used as the condensing agent. In one embodiment of the present invention, a uronium compound or a mixture of two or more uronium compounds can be used as the uronium-based condensing agent.

As used herein, the uronium compound encompass compounds called amidinium compounds. In one aspect of the present invention, the uronium-based condensing agent can be a compound represented by formula C1: 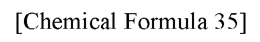 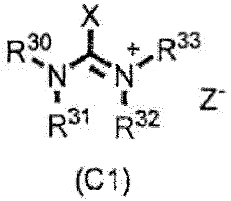
wherein R³⁰, R³¹, R³², and R³³ are each independently C₁₋₆ alkyl; or
R³⁰ and R³¹ and/or R³² and R³³ form a 5- to 7-membered non-aromatic heterocycle, wherein the heterocycle may comprise one ring heteroatom selected from O or S; or
R³¹ and R³² form a 5- to 7-membered non-aromatic heterocycle, wherein the heterocycle may comprise one ring heteroatom selected from O or S;
X is a leaving group; and
Z⁻ is a counter anion.

As used herein, the compound represented by formula C1 may be present as an isomer represented by formula C2: 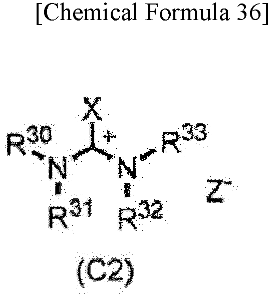 , for example, as a tautomer.

As used herein, the leaving group represented by X is not particularly limited, and examples thereof include a halogen atom and a group represented by the following formula: 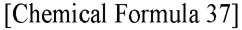 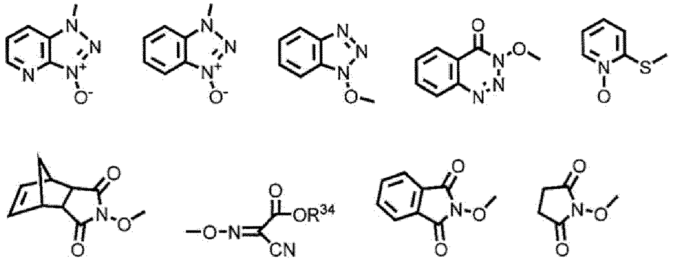 wherein R³⁴ represents C₁₋₆ alkyl, and the benzene and pyridine rings in the above formula are optionally substituted with one or more substituents selected from C₁₋₆ alkyl and a halogen atom.

In one aspect of the present invention, a halouronium compound is used as the uronium-based condensing agent. In one embodiment, the group represented by X of formula C1 is a halogen atom, specifically a fluorine atom, a chlorine atom, a bromine atom, an iodine atom, or the like, more specifically a fluorine atom or a chlorine atom.

As used herein, the counter anion represented by Z⁻ is not particularly limited, and examples thereof include a halide anion such as a fluoride ion, a chloride ion, a bromide ion, or an iodide ions, a hexafluorophosphate ion (PF₆⁻), and a tetrafluoroborate ion (BF₄⁻). More specific examples of the counter anion include a chloride ion, a hexafluorophosphate ion (PF₆⁻), and a tetrafluoroborate ion (BF₄⁻).

In one embodiment of the present invention, as the uronium-based condensing agent, a uronium compound or a mixture of two or more different uronium compounds described below can be used:
1-[Bis(dimethylamino)methylene]-1H-benzotriazolium 3-oxide hexafluorophosphate (HBTU);
1 - [Bis(dimethyl amino)methylene] -1H-benzotriazolium 3-oxide tetrafluoroborate (TBTU);
1-[Bis(dimethylamino)methylene]-1H-1,2,3-triazolo [4,5-b]pyridinium 3-oxide hexafluorophosphate (HATU);
1-[Bis(dimethylamino)methylene]-1H-1,2,3-triazolo [4,5-b]pyridinium 3-oxide tetrafluoroborate (TATU);
O-(6-chlorobenzotriazol-1-yl)-N,N,N',N'-tetramethyluronium tetrafluoroborate (TCTU);
O-(6-chlorobenzotriazol-1-yl)-N,N,N',N'-tetramethyluronium hexafluorophosphate (HCTU);
N,N,N',N'-tetramethyl-O-(3,4-dihydro-4-oxo-1,2,3-benzotriazin-3-yl)uronium tetrafluoroborate (TDBTU);
O-(benzotriazol-1-yl)-N,N,N',N'-bis(tetramethylene)uronium hexafluorophosphate (HBPyU);
O-(benzotriazol-1-yl)-N,N,N',N'-bis(pentamethylene)uronium hexafluorophosphate (HBPipU);
O-[(ethoxycarbonyl)cyanomethyleneamino]-N,N,N',N'-tetramethyluronium tetrafluoroborate (TOTU);
O-[(ethoxycarbonyl)cyanomethyleneamino]-N,N,N',N'-tetramethyluronium hexafluorophosphate (HOTU);
(1-Cyano-2-ethoxy-2-oxoethylideneaminooxy)dimethylamino morpholinocarbenium hexafluorophosphate (COMU);
O-[2-oxo-1(2H)-pyridyl]-N,N,N',N' -tetramethyluronium tetrafluoroborate (TPTU);
2-(5-Norbornene-2,3-dicarboximide)-1,1,3,3-tetramethyluronium tetrafluoroborate (TNTU);
O-(N-succinimidyl)-N,N,N',N'-tetramethyluronium tetrafluoroborate (TSTU);
O-(N-succinimidyl)-N,N,N',N'-tetramethyluronium hexafluorophosphate (HSTU);
Dipyrrolidino(N-succinimidyloxy)carbenium hexafluorophosphate (HSPyU);
N,N,N',N'-tetramethyl-S-(1-oxide-2-pyridyl)thiouronium tetrafluoroborate (TOTT)
N,N,N',N'-tetramethyl-S-(1-oxide-2-pyridyl)thiouronium hexafluorophosphate (HOTT);
Fluoro-N,N,N',N'-tetramethylformamidinium hexafluorophosphate (TFFH),
Chloro-N,N,N',N'-tetramethylformamidinium hexafluorophosphate (TCFH),
1-(Chloro-1-pyrrolidinylmethylene)pyrrolidinium hexafluorophosphate (PyClU),
1-(Chloro-1-pyrrolidinylmethylene)pyrrolidinium tetrafluoroborate (TPyClU),
Chlorodipiperidinocarbenium hexafluorophosphate (PipClU),
2-Chloro-1,3-dimethylimidazolinium chloride (DMC),
2-Chloro-1,3-dimethylimidazolinium hexafluorophosphate (CIP), and
2-Chloro-1,3-dimethylimidazolinium tetrafluoroborate (CIB).

In one embodiment of the present invention, as the uronium-based condensing agent, a halouronium compound or a mixture of two or more different halouronium compounds described below can be used:
Fluoro-N,N,N',N'-tetramethylformamidinium hexafluorophosphate (TFFH),
Chloro-N,N,N',N'-tetramethylformamidinium hexafluorophosphate (TCFH),
1-(Chloro-1-pyrrolidinylmethylene)pyrrolidinium hexafluorophosphate (PyClU),
1-(Chloro-1-pyrrolidinylmethylene)pyrrolidinium tetrafluoroborate (TPyClU),
Chlorodipiperidinocarbenium hexafluorophosphate (PipClU),
2-Chloro-1,3-dimethylimidazolinium chloride (DMC),
2-Chloro-1,3-dimethylimidazolinium hexafluorophosphate (CIP), and
2-Chloro-1,3-dimethylimidazolinium tetrafluoroborate (CIB).

In one aspect of the present invention, the 2-halo-N-alkyl pyridinium-based condensing agent is not particularly limited as long as it is a 2-halo-N-alkyl pyridinium compound that is available for dehydration condensation in an amide bond formation reaction. In one embodiment, the 2-halo-N-alkyl pyridinium compound can be a known 2-halo-N-alkyl pyridinium salt. For example, a commercially available 2-halo-N-alkyl pyridinium compound can be used as the condensing agent. In one embodiment of the present invention, a 2-halo-N-alkyl pyridinium compound or a mixture of two or more 2-halo-N-alkyl pyridinium compounds can be used as the 2-halo-N-alkyl pyridinium-based condensing agent.

In one aspect of the present invention, the 2-halo-N-alkyl pyridinium-based condensing agent can be a compound represented by formula C3: 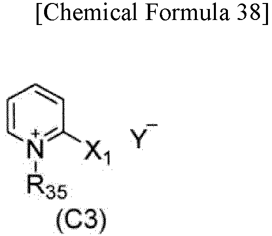 wherein R³⁵ is C₁₋₆ alkyl, X₁ is halogen and Y is a counter anion.

In one embodiment of the present invention, the group represented by X₁ of formula C3 is a halogen atom, specifically a fluorine atom, a chlorine atom, a bromine atom, an iodine atom, or the like, more specifically a fluorine atom, a chlorine atom, or a bromine atom.

As used herein, the counter anion represented by Y⁻ is not particularly limited, and examples thereof include a halide anion such as a fluoride ion, a chloride ion, a bromide ion, or an iodide ions, a hexafluorophosphate ion (PF₆⁻), a tetrafluoroborate ion (BF₄⁻), and a p-toluenesulfonate ion (pTsO⁻). More specific examples of the counter anion include an iodide ion, a tetrafluoroborate ion (BF₄⁻), and a p-toluenesulfonate ion (pTsO⁻).

In one embodiment of the present invention, as the 2-halo-N-alkyl pyridinium-based condensing agent, a 2-halo-N-alkyl pyridinium compounds or a mixture of two or more different 2-halo-N-alkyl pyridinium compounds described below can be used:
2-chloro-1-methylpyridinium iodide;
2-bromo-1-ethylpyridinium tetrafluoroborate; and
2-fluoro-1-methylpyridinium p-toluenesulfonate.

In one embodiment of the present invention, a uronium compound, a halouronium-based compound, or a 2-halo-N-alkyl pyridinium compound can be used as the condensing agent.

In one aspect of the present invention, the amide bond formation reaction is performed by reacting a carboxylic acid compound and an amine compound in the presence of a condensing agent and a first base.

In one embodiment of the present invention, a uronium compound or a halouronium-based compound can be used as the uronium-based condensing agent.

In one aspect of the present invention, the amide bond formation reaction is performed by reacting a carboxylic acid compound and an amine compound in the presence of a condensing agent and a first base.

Here, the first base is a compound or a mixture of two or more different compounds represented by formula A: 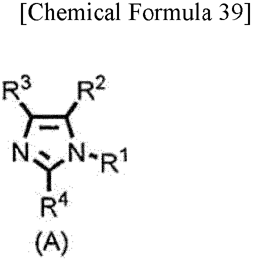
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle. C₁₋₆ alkyl is, for example, methyl and C₆₋₁₀ aryl is, for example, phenyl. In one embodiment of the present invention, R¹ is methyl or phenyl, and R², R³, and R⁴ are each independently a hydrogen atom or methyl.

Examples of the compound utilized as the first base include N-methylimidazole, tetramethylimidazole, N-phenylimidazole, 1-isopropylimidazole, 1-tert-butylimidazole, 1-(2,6-diisopropylphenyl)imidazole, 1,2-dimethylimidazole, 1,4-dimethylimidazole, 5-chloro-1-methylimidazole, 5-bromo-1-methylimidazole, 5-iodo-1-methylimidazole, 2-iodo-1-methylimidazole, 1-methyl-2-(methylthio)imidazole, 1-methylbenzoimidazole, 4-methyl-1,2,4-triazole, and imidazo[1,5-a]pyridine. Specific examples thereof include N-methylimidazole, tetramethylimidazole and N-phenylimidazole.

In one embodiment of the present invention, the molar ratio of the first base to the condensing agent (first base/condensing agent) is 1.8 or less, 1.7 or less, 1.6 or less, 1.5 or less, 1.4 or less, 1.3 or less, 1.2 or less, or 1.1 or less. In one embodiment, the molar ratio (first base/condensing agent) is 0.1 or more, 0.2 or more, 0.3 or more, 0.4 or more, 0.5 or more, 0.6 or more, 0.7 or more, 0.8 or more, or 0.9 or more.

In one embodiment of the present invention, the molar ratio of the first base to the uronium-based condensing agent (first base/condensing agent) is 1.8 or less, 1.7 or less, 1.6 or less, 1.5 or less, 1.4 or less, 1.3 or less, 1.2 or less, or 1.1 or less. In one embodiment, the molar ratio (first base/uronium-based condensing agent) is 0.1 or more, 0.2 or more, 0.3 or more, 0.4 or more, 0.5 or more, 0.6 or more, 0.7 or more, 0.8 or more, or 0.9 or more.

In one aspect of the present invention, the amide bond formation reaction can be performed in the presence of a second base that is different from the first base. In one aspect of the present invention, the second base is an organic base having a pKa of the conjugated acid in water of 11 or less, 10 or less, 9 or less, 8 or less, or 7.9 or less, and is 0 or more, 1 or more, 2 or more, 3 or more, or 3.7 or more. In one aspect of the present invention, the pKa in water of the conjugated acid of the second base is 0 to 11, 1 to 9, 2 to 8, 3 to 8, or 3.7 to 7.9. The pKa of the conjugate acid of the base here can be determined by the conventional methods. For example, the values measured at 25°C by the method described in the 5th series of experimental chemistry "Thermal Measurement and Equilibrium", page 460 (edited by the Chemical Society of Japan, published by Maruzen Publishing Co., Ltd.) can be used. Also, the values described in publicly known document Eur. J. Org. Chem. 2019, 6735-6748, the values calculated by using Advanced Chemistry Development (ACD/Labs) Software V11.02 ((c) 1994-2019 ACD/Labs), ADMET predictor (version 9.5, parameters are all default values), the values described in the catalog of Sigma-Aldrich Co. LLC, the values described in Chemical Book (https://www.chemicalbook.com), or the values described in PubChem https://pubchem.ncbi.nlm.nih.gov) can be appropriately referred to as reference values. The pKa values of conjugated acids in water calculated using ADMET predictor (version 9.5) are, for example, 3.71 for 3-chloro-N,N-dimethylaniline, 3.75 for N,N-dimethyl-4-(trifluoromethyl)aniline, 3.81 for N,N-dimethyl-3-(trifluoromethyl)aniline, 4.45 for 3-bromo-N,N-dimethylaniline, 4.65 for N,N,2,4,6-pentamethylaniline, 4.7 for 4-bromo-N,N-dimethylaniline, 4.8 for 4-fluoro-N,N-dimethylaniline, 5.12 for N,N-dimethyl-p-toluidine, 5.19 for N,N-dimethylaniline, 5.63 for julolidine, 6.64 for N,N-diethylaniline, 6.73 for 2,6-lutidine, and 7.86 for N-methylmorpholine.

In one embodiment of the present invention, the second base is a compound or a mixture of two or more different compounds selected from the group consisting of formulas B1 and B2: 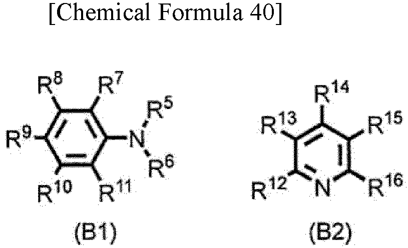
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms.

In one embodiment, R⁵ and R⁷, and R⁶ and R¹¹ together with a nitrogen atom and a carbon atom to which they are attached form a 6-membered non-aromatic heterocycle, or R⁵ and R⁶ are each independently selected from C₁₋₆ alkyl; R⁷, R⁸, R⁹, R¹⁰, and R¹¹ are each independently a hydrogen atom, C₁₋₆ alkyl optionally substituted with one or more fluorine atoms, a halogen atom, or cyano; and R¹², R¹³, R¹⁴, R¹⁵, and R¹⁶ are each independently selected from a hydrogen atom and C₁₋₆ alkyl optionally substituted with one or more fluorine atoms.

In one embodiment, R⁵ and R⁷, and R⁶ and R¹¹ together with a nitrogen atom and a carbon atom to which they are attached form a 6-membered non-aromatic heterocycle, or R⁵ and R⁶ are both methyl; R⁷, R⁸, R⁹, R¹⁰, and R¹¹ are each independently a hydrogen atom, trifluoromethyl, methyl, a fluorine atom, a chlorine atom, and a bromine atom; and R¹², R¹³, R¹⁴, R¹⁵, and R¹⁶ are each independently selected from a hydrogen atom, trifluoromethyl, and methyl.

Examples of the compound utilized as the second base include N,N-dimethylaniline, 4-bromo-N,N-dimethylaniline, 4-fluoro-N,N-dimethylaniline, 3-bromo-N,N-dimethylaniline, 3-chloro-N,N-dimethylaniline, N,N-dimethyl-3-(trifluoromethyl)aniline, N,N,2,4,6-pentamethylaniline, julolidine, collidine, and 2,6-lutidine.

In one embodiment of the present invention, the molar ratio of the second base to the first base (second base/first base) is 100 or less, 70 or less, 50 or less, 20 or less, 10 or less, 5 or less, or 3.5 or less. In one embodiment, the molar ratio (second base/first base) is 0 or more, 0.1 or more, 0.2 or more, 0.5 or more, 0.7 or more, or 1.0 or more.

In one aspect of the present invention, an amine compound that serves as a substrate for an amide formation reaction can be used in an excess amount relative to the carboxylic acid compound to use as the second base. The excess amount here is not particularly limited, for example, it is not particularly limited as long as the amount exceeds the equivalents relative to the carboxylic acid compound. In one embodiment, an amine compound in an amount exceeding an equivalent amount can be used as the second base by using, in addition to the equivalent amount of the amine compound, an amount of the amine compound corresponding to a predetermined molar ratio to the uronium compound or first base that has been already identified. In one embodiment of the present invention, when an amine compound that serves as a substrate is used as the second base, the amine compound is used relative to the carboxylic acid in an amount of 2 to 100 equivalents, 3 to 80 equivalents, 5 to 50 equivalents, 5 to 20 equivalents, or 5 to 12 equivalents. Here, the equivalents relative to the carboxylic acid compound is calculated considering the number of carboxy groups contained in the carboxylic acid compound.

In one embodiment of the present invention, the molar ratio of the second base to the condensing agent (second base/condensing agent) is 20 or less, 10 or less, 7 or less, 5 or less, 4 or less, 3.5 or less, 2 or less, or 1 or less. In one embodiment, the molar ratio (second base/condensing agent) is 0 or more, 0.1 or more, 0.2 or more, 0.5 or more, 0.7 or more, or 1 or more.

In one embodiment of the present invention, the molar ratio of the second base to the uronium-based condensing agent (second base/uronium-based condensing agent) is 20 or less, 10 or less, 7 or less, 5 or less, 4 or less, 3.5 or less, 2 or less, or 1 or less. In one embodiment, the molar ratio (second base/uronium-based condensing agent) is 0 or more, 0.1 or more, 0.2 or more, 0.5 or more, 0.7 or more, or 1 or more.

In one aspect of the present invention, the carboxylic acid compound is a compound or a mixture of two, three, or four or more different compounds represented by formula A1 or formula A2: 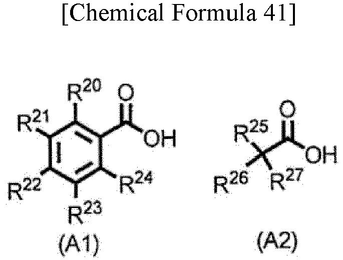 wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent.

In one aspect of the present invention, a resin for solid-phase synthesis in which a carboxylic acid compound or two, three, or four or more different carboxylic acid compounds represented by carboxylic acid compounds are bound via a linker is used as the carboxylic acid compound. In one aspect of the present invention, the carboxylic acid compound is a resin for solid-phase synthesis in which a compound or two or more compounds represented by formula A1 or formula A2 are bound via a linker. In one embodiment, the carboxylic acid compound does not have a group involved in an amide bond formation reaction other than a carboxy group represented by formula A1 or formula A2.

In one aspect of the present invention, the molar ratio of the condensing agent to the carboxylic acid compound is condensing agent/carboxylic acid compound = 20/1 to 1/1, 15/1 to 1/1, 10/1 to 1/1, or 5/1 to 1/1. When the carboxylic acid compound is a resin for solid-phase synthesis, the molar ratio of the condensing agent to the carboxylic acid contained in the compound bound to the resin is condensing agent/carboxylic acid = 20/1 to 1/1, 15/1 to 1/1, 10/1 to 1/1, or 5/1 to 1/1.

In one aspect of the present invention, the molar ratio of the uronium-based condensing agent to the carboxylic acid compound is uronium-based condensing agent/carboxylic acid compound = 20/1 to 1/1, 15/1 to 1/1, 10/1 to 1/1, or 5/1 to 1/1. When the carboxylic acid compound is a resin for solid-phase synthesis, the molar ratio of the uronium-based condensing agent to the carboxylic acid contained in the compound bound to the resin is uronium-based condensing agent/carboxylic acid = 20/1 to 1/1, 15/1 to 1/1, 10/1 to 1/1, or 5/1 to 1/1.

In one aspect of the present invention, the amine compound is a compound or a mixture of two, three, or four or more different compounds represented by formula A3 or formula A4: 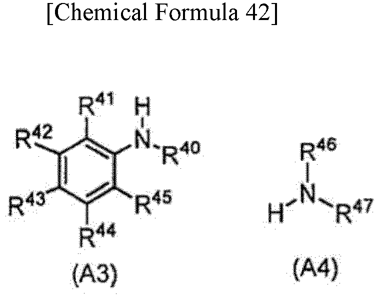
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent. In one embodiment, the amine compound does not have a group involved in an amide bond formation reaction other than an amino group represented by formula A3 or formula A4.

In one aspect of the present invention, a resin for solid-phase synthesis in which an amine compound or two, three, or four or more amine compounds are bound via a linker is used as the amine compound. In one embodiment, the amine compound does not have a group involved in an amide bond formation reaction other than an amino group represented by formula A3 or formula A4.

As used herein, the groups involved in the amide bond formation reaction are not particularly limited. Specifically, the groups involved in the amide bond formation reaction are a carboxy group and an amino group capable of amide bond formation.

In one aspect of the present invention, the molar ratio of the condensing agent to the amine compound is condensing agent/amine compound = 20/1 to 1/1, 15/1 to 1/1, 10/1 to 1/1, or 5/1 to 1/1. When the amine compound is a resin for solid-phase synthesis, the molar ratio of the condensing agent to the amine contained in the compound bound to the resin is condensing agent/amine = 20/1 to 1/1, 15/1 to 1/1, 10/1 to 1/1, or 5/1 to 1/1.

In one aspect of the present invention, the molar ratio of the uronium-based condensing agent to the amine compound is uronium-based condensing agent/amine compound = 20/1 to 1/1, 15/1 to 1/1, 10/1 to 1/1, or 5/1 to 1/1. When the amine compound is a resin for solid-phase synthesis, the molar ratio of the uronium-based condensing agent to the amine contained in the compound bound to the resin is uronium-based condensing agent/amine = 20/1 to 1/1, 15/1 to 1/1, 10/1 to 1/1, or 5/1 to 1/1.

In one embodiment of the present invention, the amide bond formation reaction is performed at a reaction temperature of 0°C to 100°C, 10°C to 80°C, 10°C to 60°C, 15°C to 40°C, or 20°C to 30°C.

In one aspect of the present invention, the amide bond formation reaction is performed in a mixture containing two, three or more, or four or more carboxylic acid compounds and/or two, three or more, or four or more amine compounds as substrates.

In one embodiment, the present invention provides the following:
A method for producing an amide compound, comprising reacting a carboxylic acid compound and an amine compound in the presence of at least one condensing agent selected from the group consisting of fluoro-N,N,N',N'-tetramethylformamidinium hexafluorophosphate (TFFH), chloro-N,N,N',N'-tetramethylformamidinium hexafluorophosphate (TCFH), 1-(chloro-1-pyrrolidinylmethylene)pyrrolidinium hexafluorophosphate (PyClU), 1-(chloro-1-pyrrolidinylmethylene)pyrrolidinium tetrafluoroborate (TPyClU), chlorodipiperidinocarbenium hexafluorophosphate (PipClU), 2-chloro-1,3-dimethylimidazolinium chloride (DMC), 2-chloro-1,3-dimethylimidazolinium hexafluorophosphate (CIP), 2-chloro-1,3-dimethylimidazolinium tetrafluoroborate (CIB), 2-chloro-1-methylpyridinium iodide, 2-bromo-1-ethylpyridinium tetrafluoroborate, and 2-fluoro-1-methylpyridinium p-toluenesulfonate, and at least one first base selected from the group consisting of N-methylimidazole, tetramethylimidazole, and N-phenylimidazole to obtain an amide compound,
wherein a molar ratio of the first base to the condensing agent (first base/condensing agent) is 1.5 or less.

In one embodiment, the present invention provides the following:
A method for producing an amide compound, comprising reacting a carboxylic acid compound and an amine compound in the presence of at least one uronium-based condensing agent selected from the group consisting of fluoro-N,N,N',N'-tetramethylformamidinium hexafluorophosphate (TFFH), chloro-N,N,N',N'-tetramethylformamidinium hexafluorophosphate (TCFH), 1-(chloro-1-pyrrolidinylmethylene)pyrrolidinium hexafluorophosphate (PyClU), 1-(chloro-1-pyrrolidinylmethylene)pyrrolidinium tetrafluoroborate (TPyClU), chlorodipiperidinocarbenium hexafluorophosphate (PipClU), 2-chloro-1,3-dimethylimidazolinium chloride (DMC), 2-chloro-1,3-dimethylimidazolinium hexafluorophosphate (CIP), and 2-chloro-1,3-dimethylimidazolinium tetrafluoroborate (CIB), and at least one first base selected from the group consisting of N-methylimidazole, tetramethylimidazole, and N-phenylimidazole to obtain an amide compound,
wherein a molar ratio of the first base to the uronium-based condensing agent (first base/uronium-based condensing agent) is 1.5 or less.

In one embodiment, the present invention provides the following:
A method for producing an amide compound, comprising reacting a carboxylic acid compound and an amine compound in the presence of at least one uronium-based condensing agent selected from the group consisting of fluoro-N,N,N',N'-tetramethylformamidinium hexafluorophosphate (TFFH), chloro-N,N,N',N'-tetramethylformamidinium hexafluorophosphate (TCFH), 1-(chloro-1-pyrrolidinylmethylene)pyrrolidinium hexafluorophosphate (PyClU), 1-(chloro-1-pyrrolidinylmethylene)pyrrolidinium tetrafluoroborate (TPyClU), chlorodipiperidinocarbenium hexafluorophosphate (PipClU), 2-chloro-1,3-dimethylimidazolinium chloride (DMC), 2-chloro-1,3-dimethylimidazolinium hexafluorophosphate (CIP), and 2-chloro-1,3-dimethylimidazolinium tetrafluoroborate (CIB), and at least one first base selected from the group consisting of N-methylimidazole, tetramethylimidazole, and N-phenylimidazole, and further at least one second base selected from the group consisting of N,N-dimethylaniline, 4-bromo-N,N-dimethylaniline, 4-fluoro-N,N-dimethylaniline, 3-bromo-N,N-dimethylaniline, 3-chloro-N,N-dimethylaniline,N,N-dimethyl-3-(trifluoromethyl)aniline, N,N,2,4,6-pentamethylaniline, julolidine, collidine, and 2,6-lutidine to obtain an amide compound,
wherein a molar ratio of the first base to the uronium-based condensing agent (first base/uronium-based condensing agent) is 1.2 or less.

Examples of C₆₋₁₀ aryl herein include phenyl and naphthyl.

Examples of the 5- to 10-membered heteroaryl containing one or more ring heteroatoms independently selected from O, N and S include pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl.

As used herein, a 5- to 7-membered saturated heterocycle is a saturated heterocycle having 5 to 7 ring atoms and containing one or more heteroatoms selected from O and S as ring atoms. Examples thereof include pyrrolidine, piperidine, morpholine, thiomorpholine, and azepane.

As used herein, a 5- to 7-membered non-aromatic heterocycle is a non-aromatic heterocycle having 5 to 7 ring atoms and encompasses a 5- to 7-membered saturated heterocycle. The 5- to 7-membered non-aromatic heterocycle includes a non-aromatic heterocycle in which one single bond of the 5- to 7-membered saturated heterocycle is replaced by a double bond.

As used herein, C₁₋₆ alkyl is a linear or branched monovalent saturated aliphatic group having 1 to 6 carbon atoms. Specific examples thereof include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, 4-methylpentyl, and 2-ethylbutyl.

As used herein, C₂₋₆ alkenyl is a linear or branched monovalent group having 2 to 6 carbon atoms having one or more double bonds. Examples thereof include ethenyl (vinyl), 1-propenyl, 2-propenyl (allyl), propen-2-yl, and 3-butenyl (homoallyl).

As used herein, C₂₋₆ alkynyl refers to a linear or branched monovalent group having 2 to 6 carbon atoms having one or more triple bonds, and examples thereof include ethynyl, 1-propynyl, 2-propynyl, 1-butynyl, 2-butynyl, and 3-butynyl.

As used herein, C₃₋₈ cycloalkyl refers to a cyclic saturated aliphatic hydrocarbon group having 3 to 8 carbon atoms. Specific examples thereof include cyclopropyl, cyclobutyl, cyclopentyl, and cyclohexyl.

As used herein, C₇₋₁₄ aralkyl refers to aryl-substituted alkyl having a total 7 to 14 carbon atoms. Examples thereof include benzyl, 1-phenethyl, 2-phenethyl, 1-naphthylmethyl, and 2-naphthylmethyl.

As used herein, C₁₋₆ alkoxy refers to a C₁₋₆ alkyl-O- group, wherein the C₁₋₆ alkyl is as already defined. Specific examples thereof include methoxy, ethoxy, 1-propoxy, 2-propoxy, n-butoxy, i-butoxy, sec-butoxy, and t-butoxy.

As used herein, (C₁₋₆ alkoxy)carbonyl refers to a C₁₋₆ alkoxy-C(=O)- group, wherein the C₁₋₆ alkoxy is as already defined.

As used herein, (C₁₋₆ alkyl)carbonyl refers to a C₁₋₆ alkyl-C(=O)- group, wherein the C₁₋₆ alkyl is as already defined.

As used herein, "(C₁₋₆ alkoxy)carbonyl" of (C₁₋₆ alkoxy)carbonylamino is as already defined.

As used herein, "(C₁₋₆ alkyl)" of (C₁₋₆ alkyl)amino is as already defined.

As used herein, "(C₁₋₆ alkyl)" of di(C₁₋₆ alkyl)amino is as already defined and may be the same or different.

As used herein, 4- to 8-membered cyclic amino includes groups such as aziridinyl, azetidinyl, pyrrolidinyl, piperidinyl, piperazinyl, morpholinyl, and the like, which bind with a nitrogen atom.

As used herein, "(C₆₋₁₀ aryl)" of (C₆₋₁₀ aryl)carbonylamino is as already defined.

As defined herein, "5- to 10-membered heteroaryl containing one or more ring heteroatoms independently selected from O, N, and S" of 5- to 10-membered heteroarylcarbonylamino containing one or more ring heteroatoms independently selected from O, N, and S.

As used herein, aminocarbonyl means -CONH₂.

"(C₁₋₆ alkyl)" of (C₁₋₆ alkyl)aminocarbonyl is as already defined.

As used herein, "(C₁₋₆ alkyl)" of di(C₁₋₆ alkyl)aminocarbonyl is as already defined and may be the same or different.

As used herein, 4- to 8-membered cyclic amino of 4- to 8-membered cyclic aminocarbonyl is as already defined, in which the nitrogen atom is bound to the carbonyl.

As used herein, "(C₆₋₁₀ aryl)" of (C₆₋₁₀ aryl)carbonyl is as already defined.

As defined herein, "5- to 10-membered heteroaryl containing one or more ring heteroatoms independently selected from O, N, and S" of 5- to 10-membered heteroarylcarbonyl containing one or more ring heteroatoms independently selected from O, N, and S.

As used herein, "(C₁₋₆ alkyl)" of tri(C₁₋₆ alkyl)silyl is as already defined and may be the same or different. Examples thereof include trimethylsilyl, triethylsilyl, and t-butyldimethylsilyl.

As used herein, "halogen atom" refers to a fluorine atom, a chlorine atom, a bromine atom, an iodine atom, and the like. In the present invention, when a halogen atom is a substituent for an aryl, a heteroaryl, and the like, examples of the preferred halogen atom include a fluorine atom, a chlorine atom, and a bromine atom. In the present invention, when a halogen atom is a substituent for an alkyl or a group containing an alkyl as a part thereof (alkoxy, alkenyl, alkylthio, and the like), examples of the preferred halogen atom include a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio.

Examples of C₁₋₆ alkyl optionally substituted with one or more fluorine atoms include trifluoromethyl.

In one aspect of the present invention, the reaction time of the amide bond formation reaction can be appropriately set by one skilled in the art. For example, the reaction time can be set in the range of 1 minute to 96 hours, 5 minutes to 72 hours, 10 minutes to 48 hours, 15 minutes to 48 hours, or 30 minutes to 24 hours.

In one embodiment of the present invention, the production method comprises removing impurities after amidation. The impurity removal can be performed by methods commonly performed in the art of the present invention. The impurities here include impurities derived from reaction reagents consumed in the reaction, unreacted reaction reagents, degradation products generated by the reaction, coexisting bases, reaction solvents, and the like. Specific examples of the impurities include condensing agents such as 2-chloro-1,3-dimethylimidazolinium hexafluorophosphate (CIP), degradation products derived from condensing agents such as 1,3-dimethyl-2-imidazolidinone, first bases such as NMI, and second bases such as N,N-dimethylaniline. Examples of the methods for removing impurities include liquid-liquid separation, vacuum distillation, methods using solid phase reagents, purification by normal phase or reverse phase silica gel column chromatography, and purification by GPC (molecular sieve).

The liquid-liquid separation for removing impurities can be performed by methods commonly performed in the art of the present invention. The liquid-liquid separation is not particularly limited as long as it is a combination of solvents separated into multiple layers to separate the group of desired products and the group of impurities (unnecessities). For example, the liquid-liquid separation can be performed by combining a solvent selected from an ester-based solvent such as ethyl acetate or isopropyl acetate, an ether-based solvent selected from diethyl ether, diisopropyl ether, t-butyl methyl ether (TBME), cyclopentyl methyl ether (CPME), 2-methyltetrahydrofuran, or 4-methyltetrahydropyran, a halogen-based solvent such as dichloromethane, chloroform, or 1,2-dichloroethane, an aromatic hydrocarbon-based solvent such as benzene or toluene, and a hydrocarbon-based solvent such as hexane, cyclohexane, or heptane, and a solvent selected from water, an acidic aqueous solution such as aqueous hydrochloric acid solution, and a basic aqueous solution such as aqueous sodium bicarbonate solution. A single solvent may be used, or a mixture of multiple solvents may be used. The liquid-liquid separation can also be performed by combining organic solvents that separate into layers, such as combining hexane and acetonitrile, when it is suitable to separate the group of desired products and the group of impurities (unnecessities).

The vacuum distillation for removing impurities can be performed by methods commonly performed in the art of the present invention. The conditions for vacuum distillation can be appropriately set depending on the impurities to be removed. For example, the pressure may be 100 to 400 mbar, 50 to 100 mbar, 5 to 50 mbar, or 0.1 to 5 mbar, and the temperature may be 20 to 100°C, 25 to 50°C, 35 to 45°C, or the like.

The impurity removal using solid phase reagents can be performed by methods commonly performed in the art of the present invention. Examples of the solid phase reagents include macroporous triethylammonium methylpolystyrene carbonate, macroporous polystyrene sulfonic acid, amine-supported silica gel, and carboxylic acid-supported silica gel.

In one aspect of the present invention, an amide compound formation reaction can be performed by reacting a resin for solid-phase synthesis having a side chain containing a carboxy group as a carboxylic acid compound in a liquid containing an amine compound. In one embodiment, the amine compound is used in an excess amount relative to the carboxy group present. For example, 2 or more equivalents, 3 or more equivalents, 5 or more equivalents, 7 or more equivalents, 10 or more equivalents of the amine compound are used relative to the carboxy group present. Here, an excess amount of the amine compound may serve as the second base.

In one aspect of the present invention, an amide compound formation reaction can be performed by reacting a resin for solid-phase synthesis having a side chain containing an amino group as an amine compound in a liquid containing a carboxylic acid compound. In one embodiment, the carboxylic acid compound is used in an excess amount relative to the amino group present. For example, 1.1 or more equivalents, 1.5 or more equivalents, 2 or more equivalents, 3 or more equivalents, 5 or more equivalents, 7 or more equivalents, 10 or more equivalents of the carboxylic acid compound are used relative to the amino group present.

Conversion of a carboxy group or amino group contained in a resin for solid-phase synthesis to an amide group can be performed using a reactor or the like known to those skilled in the art. In one embodiment, the side chain contains a linker moiety from which a compound containing an amide group to be produced by reaction can be cleaved.

In one aspect of the present invention, the amide bond formation reaction can be performed using a resin for solid-phase synthesis in which an amine compound or a carboxylic acid compound is bound via a linker as a substrate. In one embodiment, the amine compound or carboxylic acid compound is supported via a linker on a resin for solid-phase synthesis used as a solid-phase support, and the linker moiety is degraded under predetermined reaction conditions after the amide bond reaction to produce a compound containing the amide moiety.

The resin for solid-phase synthesis used as the solid-phase support is not particularly limited as long as it is commonly used. Examples thereof include a Carboxylic resin, a CTC resin, a Trt resin, a SASRIN resin, a Rink amide resin, a PAL AM resin, a Seiber amide resin, a Merrifield resin, a Wang resin, 2-(4-bromomethylphenoxy)ethyl polystyrene, and a solid phase support having any functional group such as a carboxy group, an amino group, an aminomethyl group, a hydroxy group, or a hydroxymethyl group on polystyrene. Furthermore, the resin for solid-phase synthesis may have a design in which any linker covalently connecting compound 1 or 2 with the support can be used to make cleavage between the linker and the compound. The support is also not particularly limited, and examples thereof include polystyrene and polyethylene glycol (PEG).

Methods and reaction conditions for connecting the compound with a resin for solid-phase synthesis can be appropriately set by one skilled in the art based on the methods described in known literatures and the like. The reaction conditions for cleaving the compound from the resin for solid-phase synthesis can be appropriately set by a person skilled in the art based on the chemical structure of the resin for solid-phase synthesis used. Examples of the reagents used for cleavage can include hydrochloric acid, carboxylic acids such as trifluoroacetic acid (TFA), fluoroalcohols such as 2,2,2-trifluoroethanol (TFE) or 1,1,1,3,3,3-hexafluoroisopropyl alcohol (HFIP), Brensted acid with pKa of 10 or less in water, or any Lewis acid. In one embodiment, a compound cleaved from a resin for solid-phase synthesis can be used as a compound for screening for pharmaceutical exploration.

Hereinafter, the present invention will be described in more detail using Reference Examples and Examples, but the present invention is not limited to these Examples.

### EXAMPLES

All starting materials, reagents, and solvents were obtained from commercial suppliers, or synthesized by known methods. The reagents and solvents were of reagent quality or better and were used as obtained from various commercial sources, unless otherwise noted.

For silica gel of the column chromatography, Biotage(R) SNAP MLtra, Biotage(R) Sfaer D (Duo) (60 µm), Biotage(R) Sfaer HC D (Duo) (20 µm), or the like was appropriately used. For amino silica gel of the column chromatography, Biotage(R) SNAP Isolute NH2 (50 µm), Biotage(R) SNAP Cartridge KP-NH, or the like was appropriately used. For reverse phase silica gel of the column chromatography, Biotage(R) SNAP Mltra C18 (25 µm), Biotage(R) Sfaer C18 (30 µm), or the like was appropriately used.

¹H-NMR and ¹³C-NMR spectra were measured, with or without Me₄Si as the internal standard substance, using ECP-400 (manufactured by JEOL Ltd.), Agilent 400-MR (manufactured by Agilent Technologies), AVANCE3 Cryo-TCI, AVANCE3 400, AVANCE3 HD 400, AVANCE NEO 400, AVANCE3 HD 300, AVANCE3 300, AVANCE2 300, AVANCE NEO 300 (manufactured by Bruker) or the like as appropriate (s = singlet, brs = broad singlet, d = doublet, t = triplet, q = quartet, dd = double doublet, ddd = double double doublet, dt = double triplet, td = triple doublet, and m = multiplet).

The reaction tracking and purity measurement were carried out, unless otherwise noted, by performing retention time measurement and mass spectrometry using 2020 (manufactured by SHIMADZU CORPORATION), under the analysis conditions shown in the following tables.

In Examples, the following abbreviations were used.

**[Table 1]**

| Abbreviation | |
|---|---|
| TFA | Trifluoroacetic acid |
| DCM | Dichloromethane |
| DMF | N,N-Dimethylformamide |
| EtOH | Ethanol |
| MeCN | Acetonitrile |
| MeOH | Methanol |
| NMP | N-Methylpyrrolidone |
| THF | Tetrahydrofuran |
| DIPEA | N,N-diisopropylethylamine |
| DIC | N,N'-diisopropylcarbodiimide |
| HOAt | 1-Hydroxy-7-azabenzotriazole |
| Oxyma | Cyano(hydroxyimino)ethyl acetate |
| TCFH | Chloro-N,N,N',N'-tetramethylformamidinium hexafluorophosphate (CAS: 94790-35-9) |
| PipCIU | Chlorodipiperidinocarbenium hexafluorophosphate (CAS: 161308-40-3) |
| PyCIU | 1-(Chloro-1-pyrrolidinylmethylene)pyrrolidinium hexafluorophosphate (CAS: 135540-11-3) |
| TPyCIU | 1-(Chloro-1-pyrrolidinylmethylene)pyrrolidinium tetrafluoroborate (CAS: 115007-14-2) |
| CIP | 2-Chloro-1,3-dimethylimidazolinium hexafluorophosphate (CAS: 101385-69-7) |
| CIB | 2-Chloro-1,3-dimethylimidazolinium tetrafluoroborate (CAS: 153433-26-2) |
| BEP | 2-Bromo-1-ethylpyridinium tetrafluoroborate (CAS: 878-23-9) |
| BTFFH | Fluoro-N,N,N',N'-bis(tetramethylene)formamidinium hexafluorophosphate (CAS: 164298-25-3) |
| NMI | 1-Methylimidazole |

The analysis conditions of LCMS are shown in the following tables.

**[Table 2]**

| Analysis method | Device | Column | Mobile phase A | Mobile phase B | Gradient | Flow rate |
|---|---|---|---|---|---|---|
| FA05-1 | nexera /2020 | Ascentis Express C18 2.1mmI.D.×50mm, 2.7um | 0.1%FA H2O | 0.1%FA MeCN | A/B=95/5→0/100(1 .5 min)→0/100(0.5 min) | 1 mL/min |
| FA05-long | nexera /2020 | Ascentis Express C18 2.1mmI.D.×50mm, 2.7um | 0.1%FA H2O | 0.1%FA MeCN | A/B=95/5→0/100(4 .5 min)→0/100(0.5 min) | 1 mL/min |
| RPAmide TFA05 | nexera /2020 | Ascentis Express RPAmide 2.1mmI.D.×50mm, 2.7um | 0.05%TF A H20 | 0.05%TF A MeCN | A/B=95/5→0/100(1 .5 min)→0/100(0.5 min) | 1 mL/min |

The descriptions of m/z [M+H]⁺ and (M+H)⁺ noted in the LCMS analysis results in Examples all indicate values detected in positive mode, unless otherwise noted. Also, the UV area% in LCMS indicated values with PDA (190 to 400 nm or 210 to 400 nm), unless otherwise noted. When a specific wavelength (for example, 299 nm) is described, the UV area% at wavelengths up to +/- 4 nm centered on the described wavelength was shown. Note that the blank in the table indicates that it was below the detection limit.

The expression "concentration under reduced pressure" refers to the evaporation and removal of a solvent under reduced pressure with a rotary evaporator, a mechanical oil vacuum pump, or a mechanical oil-free vacuum pump.

The expression "drying overnight under reduced pressure" refers to the evaporation and removal of a solvent under reduced pressure with a rotary evaporator, a mechanical oil vacuum pump, or a mechanical oil-free vacuum pump.

The expressions "overnight" and "all night" refer to about 8 to 14 hours, unless otherwise noted.

The expressions "room temperature" and "rt" refer to about 20 to 25°C, unless otherwise noted.

The solid phase reaction can be performed in an appropriate container, such as a glass vial that can be tightly sealed by a cap equipped with a Teflon (R) packing or a column having a frit filter and an appropriate stopper. The container size is selected as appropriate such that there is sufficient space for the solvent and sufficient room for the resin to be effectively stirred, taking into account that certain resins may be significantly swollen when treated with organic solvents.

Stirring in the solid phase reaction was performed at 50 to 200 rpm using an appropriate shaker (for example, TOKYO RIKAKIKAI CO., LTD., EYELA, MMS-320, MMS-220H, or AS ONE CORPORATION, MyBL-100CS, or TAITEC CORPORATION, M·BR-104) or an agitator (combination of Asahi Glassplant Inc., separable flask, and NAKAMURA SCIENTIFIC INSTRUMENT CO., LTD., sealing mixer UZU, and AQUATECHS Co., Ltd., centrifugal agitator C-Mix) as appropriate in order to ensure adequate mixing, which is a factor generally accepted as important for successful reaction on the resin.

In order to monitor the progress of the reaction on the solid phase, it is necessary to collect the resin from the reaction container. At that time, using a micropipette fitted with a pipette tip cut at the appropriate length from the end, the resin was collected by sucking approximately 10 µL to ensure that the resin was included and was transferred onto the filter of a pipette tip equipped with a filter (for example, Thermo Scientific, tip with ART filter, ART20P, 2149P-05). Thereafter, the compound supported on the resin was cleaved from the resin by the following representative procedures for the resin on the filter. The resin was washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample. The LC sample was then subjected to LCMS measurement to measure the progress of the reaction.

The expression " cleavage" from the solid phase indicates the removal of the compound supported on the resin from the resin, such as a treatment of the resin with a 10% TFA/DCM solution containing 0.02 M pentamethylbenzene to recover the supported compound into the solution.

The compound No. used in Examples was indicated by the combination of arbitrary alphabets, numbers, and symbols. For compounds in the state wherein they are supported on the solid phase, "R" was added at the end, for example, "A02-1R". In contrast, the compound cleaved from the solid phase was indicated as "A02" without "-1R".

The notation 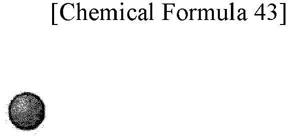 used in the chemical structure representations in Examples refer to polystyrene resin and indicate the state wherein the compound is supported on the solid phase.

In "-1R" used in Examples to indicate that the compound is supported on the solid phase, the number indicates the lot of resin used.

### Notation example of "-1R"

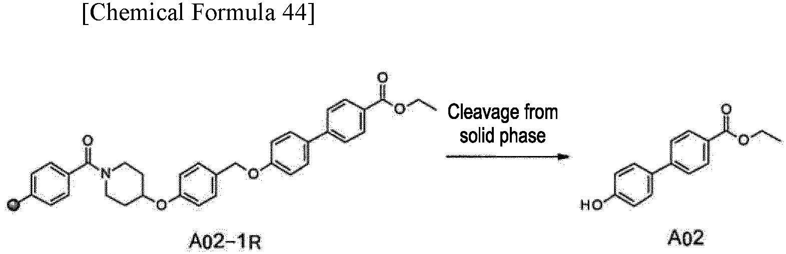

For the solid phase-supported compound used in the solid phase synthesis, the loading rate (mmol/g) is shown, which indicates the amount of support calculated when the cleaved compound is assumed to be 100% supported on the solid phase.

Even when the solid phase-supported compound is the same, the loading rate may vary from lot to lot, but the same compound No. may be used for the compound No.

### Example 1: Substrate synthesis

### Example 1-1: Synthesis of solid phase carboxylic acid compound

### Example 1-1-1: Synthesis of compound D03-1R

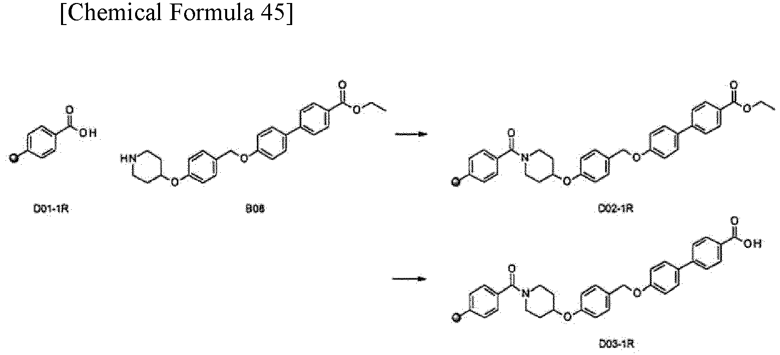

### Amidation reaction

Under a nitrogen atmosphere, to two 150 mL glass vials, Carboxylic Resin (D01-1R) (2.19 mmol/g, 12.0 g) and NMP (120 mL) were added, and the mixture was shaken at room temperature for 1 hour. Ethyl 4-[4-[(4-piperidin-4-yloxyphenyl)methoxy]phenyl]benzoate (B08) (1.28 g, 2.96 mmol), piperidine (2.08 mL, 21.0 mmol), HOAt (3.58 g, 26.3 mmol), and DIC (4.09 mL, 26.3 mmol) were added to each vial, and the mixture was shaken at 40°C for 3 days. Piperidine (5.20 mL, 52.6 mmol), HOAt (7.15 g, 52.6 mmol), and DIC (8.19 mL, 52.6 mmol) were added to each vial, and the mixture was shaken at room temperature all night.

The whole of the suspension of the reaction solution and the solid phase of the two vials was transferred to a 400 mL column equipped with a filter, and washed 3 times with NMP (240 mL), 3 times with MeOH (240 mL), and 3 times with DCM (240 mL), and the resulting solid phase was dried under reduced pressure to obtain compound D02-1R (the loading rate 0.200 mmol/g).

### Hydrolysis

Under nitrogen atmosphere, to a 800 mL column equipped with a filter, the whole of the resulting compound D02-1R and THF (360 mL), MeOH (40 mL), and aqueous KOH solution (5 M, 40 mL, 200 mmol) were added, and the mixture was shaken at 50°C for 21 hours. A portion of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 5 minutes and then filtered, and the filtrate was diluted with NMP (0.25 mL). 50 µL of the resultant was diluted with MeCN (0.25 mL) and then subjected to LCMS measurement to measure the progress of the reaction. As a result, D03 was observed as 100%. The analysis was performed by extracting at the wavelength of 290 nm (± 4 nm).

The whole of the suspension of the reaction solution and the solid phase was filtered through the filter of the column, and washed 3 times with water (500 mL), 3 times with a solution of HOAt in NMP (0.2 M, 500 mL), 3 times with NMP (500 mL), 3 times with MeOH (500 mL), and 3 times with DCM (500 mL), and the resulting solid phase was dried under reduced pressure to obtain compound D03-1R (0.201 mmol/g, 28.7 g).

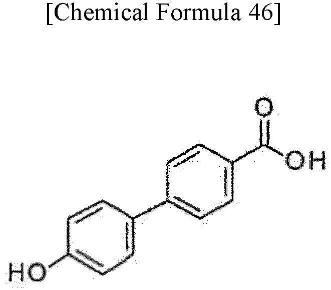

### Compound D03

Maximum wavelength: 294 nm
Retention time: 0.773 min (analysis condition FA05-1)

### Example 1-1-2: Synthesis of compound D05-1R

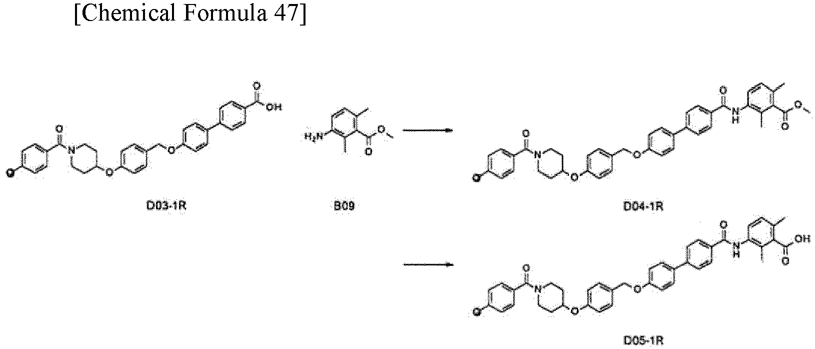

### Amidation reaction

Under a nitrogen atmosphere, to a 20 mL column equipped with a filter, compound D03-1R (0.201 mmol/g, 1.00 g) and NMP (15 mL) were added, and the mixture was shaken at room temperature for 1 hour. To this, methyl 3-amino-2,6-dimethylbenzoate (B09) (72.0 mg, 0.402 mmol), NMI (64.0 µL, 0.804 mmol), and PyClU (0.134 g, 0.402 mmol) were added, and the mixture was shaken at room temperature for 18 hours. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 5 minutes and then filtered, and the filtrate was washed with NMP (0.1 mL). 50 µL of the filtrates combined was diluted with MeCN (0.25 mL) and then subjected to LCMS measurement to measure the progress of the reaction. As a result, D04 was observed as 100%. The analysis was performed by extracting at the wavelength of 290 nm (± 4 nm).

The whole of the suspension of the reaction solution and the solid phase was filtered through the filter of the column, and washed 2 times with NMP (15 mL), 3 times with MeOH (15 mL), and 3 times with DCM (15 mL), and the resulting solid phase was dried under reduced pressure to obtain compound D04-1R (0.195 mmol/g, 1.01 g).

### Hydrolysis

Under nitrogen atmosphere, to a 20 mL column equipped with a filter, the whole of the resulting compound D04-1R and THF (12 mL), MeOH (1.5 mL), and aqueous NaOH solution (5 M, 1.5 mL, 7.5 mmol) were added, and the mixture was shaken at 50°C for 22 hours. The whole of the suspension of the reaction solution and the solid phase was filtered through the filter of the column, and washed 3 times with water (15 mL), 3 times with a solution of HOAt in NMP (0.2 M, 15 mL), 3 times with NMP (15 mL), 3 times with MeOH (15 mL), and 3 times with DCM (15 mL), and the resulting solid phase was dried under reduced pressure. To this, THF (12 mL), MeOH (1.5 mL), and aqueous NaOH solution (5 M, 1.5 mL, 7.5 mmol) were added, and the mixture was shaken at 50°C for 16 hours. Potassium trimethylsilanolate (0.759 g, 5.92 mmol) was added, and the mixture was shaken at room temperature for 20 hours. Potassium trimethylsilanolate (0.759 g, 5.92 mmol) was added, and the mixture was shaken at 60°C for 18 hours and at 80°C for 2 days. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.2 M, 0.05 mL) for 5 minutes and then filtered. The filtrate was washed with NMP (0.05 mL), then the filtrates were combined and diluted with NMP (250 µL). 50 µL of the resultant was diluted with MeCN (0.25 mL) and then subjected to LCMS measurement to measure the progress of the reaction. As a result, D05 was observed as 83%. The analysis was performed by extracting at the wavelength of 290 nm (± 4 nm).

The whole of the suspension of the reaction solution and the solid phase was filtered through the filter of the column, and washed 3 times with water (15 mL), 3 times with a solution of HOAt in NMP (0.2 M, 15 mL), 3 times with NMP (15 mL), 3 times with MeOH (15 mL), and 3 times with DCM (15 mL), and the resulting solid phase was dried under reduced pressure to obtain compound D05-1R (0.195 mmol/g).

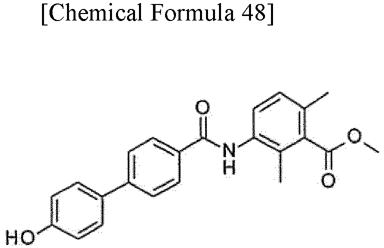

### Compound D04

LRMS: m/z 376 [M+H]⁺
Retention time: 1.021 min (analysis condition FA05-1)

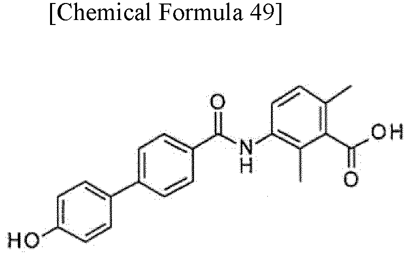

### Compound D05

LRMS: m/z 362 [M+H]⁺
Retention time: 0.849 min (analysis condition FA05-1)

### Example 1-1-3: Synthesis of compound D07-1R

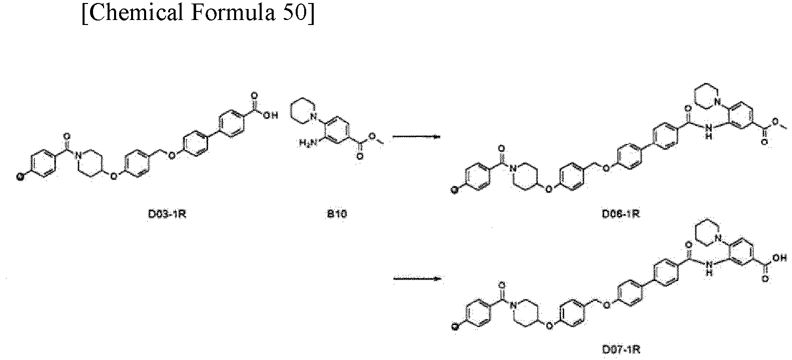

### Amidation reaction

Under a nitrogen atmosphere, to a 20 mL column equipped with a filter, compound D03-1R (0.201 mmol/g, 1.00 g) and NMP (15 mL) were added, and the mixture was shaken at room temperature for 1 hour. To this, methyl 3-amino-4-piperidin-1-yl-benzoate (B10) (94.0 mg, 0.402 mmol), NMI (64.0 µL, 0.804 mmol), and PyClU (0.134 g, 0.402 mmol) were added, and the mixture was shaken at room temperature for 18 hours. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 5 minutes and then filtered, and the filtrate was washed with NMP (0.1 mL). 50 µL of the filtrates combined was diluted with MeCN (0.25 mL) and then subjected to LCMS measurement to measure the progress of the reaction. As a result, D06 was observed as 100%. The analysis was performed by extracting at the wavelength of 299 nm (± 4 nm).

The whole of the suspension of the reaction solution and the solid phase was filtered through the filter of the column, and washed 2 times with NMP (15 mL), 3 times with MeOH (15 mL), and 3 times with DCM (15 mL), and the resulting solid phase was dried under reduced pressure to obtain compound D06-1R (0.193 mmol/g, 1.02 g).

### Hydrolysis

Under nitrogen atmosphere, to a 20 mL column equipped with a filter, the whole of the resulting compound D06-1R and THF (12 mL), MeOH (1.5 mL), and aqueous NaOH solution (5 M, 1.5 mL, 7.5 mmol) were added, and the mixture was shaken at 50°C for 22 hours. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.2 M, 0.05 mL) for 5 minutes and then filtered. The filtrate was washed with NMP (0.05 mL), then the filtrates were combined and diluted with NMP (250 µL). 50 µL of the resultant was diluted with MeCN (0.25 mL) and then subjected to LCMS measurement to measure the progress of the reaction. As a result, D07 was observed as 84%. The analysis was performed by extracting at the wavelength of 299 nm (± 4 nm). The whole of the suspension of the reaction solution and the solid phase was filtered through the filter of the column, and washed 3 times with water (15 mL), 3 times with a solution of HOAt in NMP (0.2 M, 15 mL), 3 times with NMP (15 mL), 3 times with MeOH (15 mL), and 3 times with DCM (15 mL), and the resulting solid phase was dried under reduced pressure to obtain compound D07-1R (0.193 mmol/g).

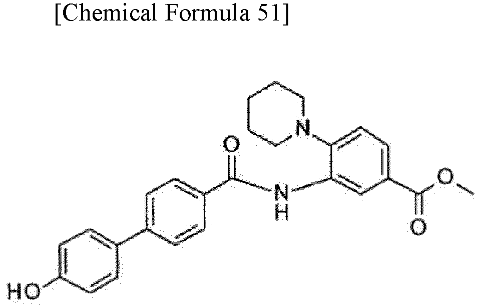

### Compound D06

LRMS: m/z 431 [M+H]⁺
Retention time: 1.330 min (analysis condition FA05-1)

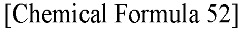

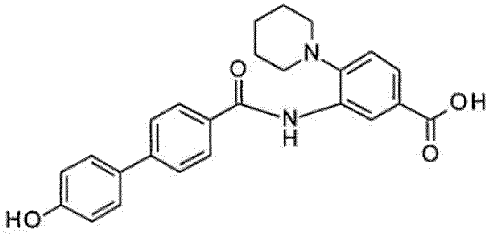

### Compound D07

LRMS: m/z 417 [M+H]⁺
Retention time: 1.120 min (analysis condition FA05-1)

### Example 1-1-4: Synthesis of compound D09-1R

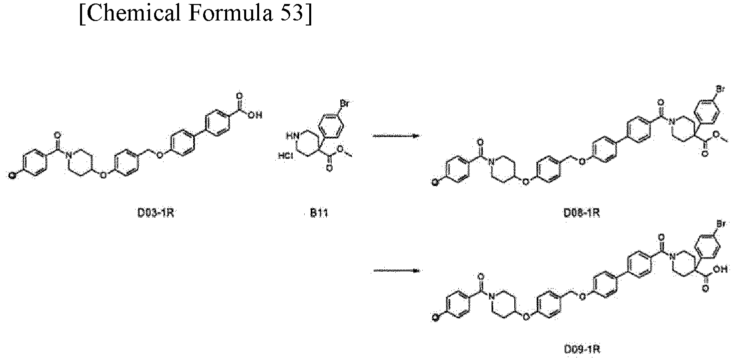

### Amidation reaction

Under a nitrogen atmosphere, to a 20 mL column equipped with a filter, compound D03-1R (0.201 mmol/g, 1.00 g) and NMP (15 mL) were added, and the mixture was shaken at room temperature for 1 hour. To this, methyl 4-(4-bromophenyl)piperidine-4-carboxylate hydrochloride (B11) (135 mg, 0.402 mmol) and NMI (64.0 µL, 0.804 mmol), DIPEA (70.0 µL, 0.402 mmol), and PyClU (0.134 g, 0.402 mmol) were added, and the mixture was shaken at room temperature for 18 hours. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 5 minutes and then filtered, and the filtrate was washed with NMP (0.1 mL). 50 µL of the filtrates combined was diluted with MeCN (0.25 mL) and then subjected to LCMS measurement to measure the progress of the reaction. As a result, D08 was observed as 100%. The analysis was performed by extracting at the wavelength of 290 nm (± 4 nm).

The whole of the suspension of the reaction solution and the solid phase was filtered through the filter of the column, and washed 2 times with NMP (15 mL), 3 times with MeOH (15 mL), and 3 times with DCM (15 mL), and the resulting solid phase was dried under reduced pressure to obtain compound D08-1R (0.190 mmol/g, 1.04 g).

### Hydrolysis

Under nitrogen atmosphere, to a 20 mL column equipped with a filter, the whole of the resulting compound D08-1R and THF (12 mL), MeOH (1.5 mL), and aqueous NaOH solution (5 M, 1.5 mL, 7.5 mmol) were added, and the mixture was shaken at 50°C for 22 hours. 10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.2 M, 0.05 mL) for 5 minutes and then filtered. The filtrate was washed with NMP (0.05 mL), then the filtrates were combined and diluted with NMP (250 µL). 50 µL of the resultant was diluted with MeCN (0.25 mL) and then subjected to LCMS measurement to measure the progress of the reaction. As a result, D09 was observed as 96%. The analysis was performed by extracting at the wavelength of 290 nm (± 4 nm).

The whole of the suspension of the reaction solution and the solid phase was filtered through the filter of the column, and washed 3 times with water (15 mL), 3 times with a solution of HOAt in NMP (0.2 M, 15 mL), 3 times with NMP (15 mL), 3 times with MeOH (15 mL), and 3 times with DCM (15 mL), and the resulting solid phase was dried under reduced pressure to obtain compound D09-1R (0.193 mmol/g).

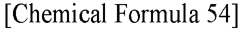

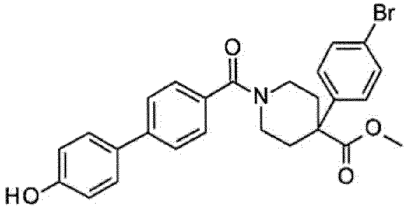

### Compound D08

LRMS: m/z 494, 496 [M+H]⁺
Retention time: 1.198 min (analysis condition FA05-1)

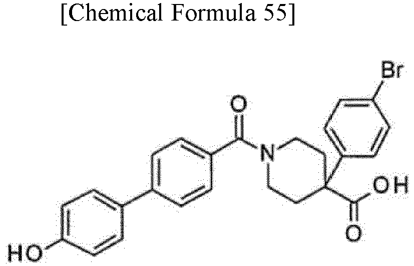

### Compound D09

LRMS: m/z 480, 482 [M+H]⁺
Retention time: 1.028 min (analysis condition FA05-1)

### Example 1-1-5: Synthesis of compound D10-1R

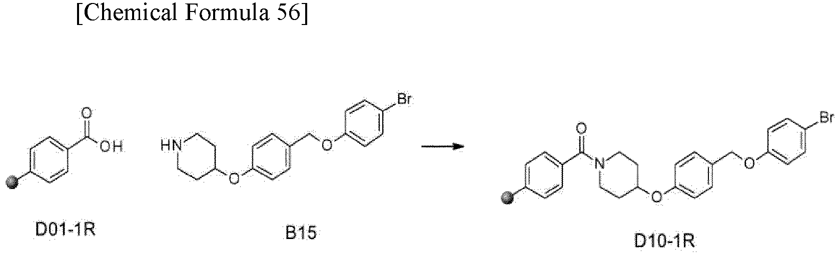

Under a nitrogen atmosphere, to a 60 mL glass vials, Carboxylic Resin (D01-1R) (2.19 mmol/g, 2.00 g) and NMP (30 mL) were added, and the mixture was shaken at room temperature for 1 hour. 4-[4-[(4-Bromophenoxy)methyl]phenoxy]piperidine (B15) (176 mg, 0.486 mmol) and piperidine (0.217 mL, 2.19 mmol), HOAt (0.656 g, 4.82 mmol), and DIC (0.751 mL, 4.82 mmol) were added, and the mixture was shaken at room temperature for 21 hours. Piperidine (0.867 mL, 8.76 mmol) and HOAt (1.19 g, 8.76 mmol), and DIC (1.37 mL, 8.76 mmol) were added, and the mixture was shaken at room temperature for 14 hours.

10 µL of the suspension of the reaction solution and the solid phase was transferred onto a tip equipped with a filter, and washed 3 times with NMP (0.1 mL), 3 times with methanol (0.1 mL), and 3 times with DCM (0.1 mL). The resultant was immersed in a 10% TFA/DCM solution of pentamethylbenzene (0.02 M, 0.05 mL) for 5 minutes and then filtered, and the filtrate was washed with NMP (0.05 mL). The filtrates combined were diluted with MeCN (0.25 mL) and then subjected to LCMS measurement to measure the progress of the reaction. As a result, D10 was observed as 100%. The analysis was performed by extracting at the wavelength of 280 nm (± 4 nm).

The whole of the suspension of the reaction solution and the solid phase was transferred onto a filter, and washed 3 times with NMP (30 mL), 3 times with methanol (30 mL), and 3 times with DCM (30 mL), and the resulting solid phase was dried overnight under reduced pressure to obtain compound D10-1R (the loading rate 0.200 mmol/g, 2.87 g).

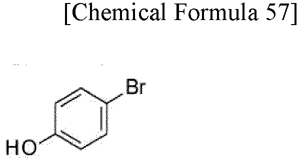

### Compound D10

Maximum wavelength: 226, 281 nm
Retention time: 0.880 min (analysis condition FA05-1)

### Example 1-1-6: Synthesis of compound D11-1R

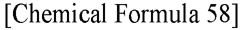

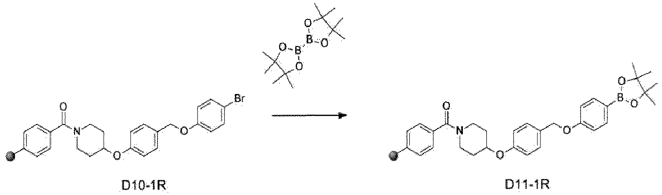

Under a nitrogen atmosphere, to a 30 mL glass vial, compound D10-1R (0.200 mmol/g, 1.05 g) and NMP (21.0 mL) were added, and the mixture was shaken at room temperature for 1 hour. To this, bis(pinacolate)diboron (CAS No. 73183-34-3) (1.07 g, 4.20 mmol) and potassium acetate (618 mg, 6.30 mmol), [1,1'-bis(diphenylphosphino)ferrocene]palladium(II) dichloride dichloromethane adduct (CAS No. 95464-05-4) (171 mg, 0.21 mmol) were added, and the mixture was shaken at 80°C for 2 hours.

12 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.02 M, 0.05 mL) for 5 minutes and then filtered, and the filtrate was washed with NMP (0.05 mL). The filtrates combined were diluted with MeCN (0.25 mL) and then subjected to LCMS measurement to measure the progress of the reaction. As a result, D11 was observed as 99.4%. The analysis was performed by extracting at the wavelength of 250 nm (± 4 nm).

The suspension of the reaction solution and the solid phase was transferred onto a filter using NMP (60 mL), and washed 3 times with NMP (21 mL), 3 times with NMP/water = 1/1 (21 mL), 3 times with water (21 mL), 3 times with NMP/water = 1/1 (21 mL), 3 times with NMP (21 mL), 3 times with methanol (21 mL), 3 times with DCM (21 mL), and 3 times with heptane (21 mL), and the resulting solid phase was dried overnight under reduced pressure to obtain compound D11-1R (the loading rate 0.198 mmol/g, 1.13 g).

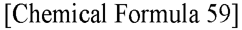

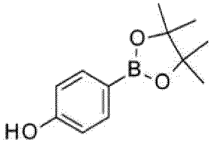

### Compound D11

Maximum wavelength: 238 nm
Retention time: 0.999 min (analysis condition FA05-1)

### Example 1-1-7: Synthesis of compound D12-1R

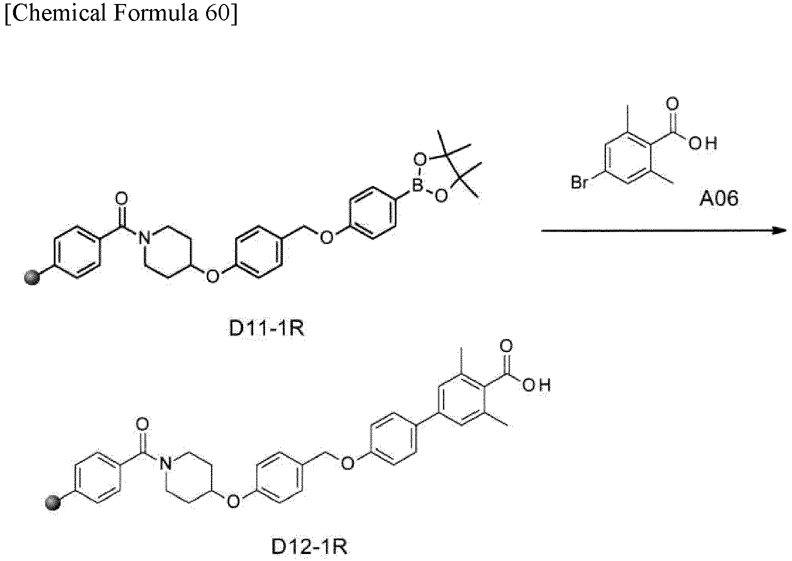

Under a nitrogen atmosphere, to a 20 mL glass vial, compound D11-1R (0.198 mmol/g, 509 mg), 4-bromo-2,6-dimethylbenzoic acid (A06) (69.3 mg, 0.302 mmol) and THF (7.64 mL) were added, and the mixture was shaken at room temperature for 1 hour. Water (27.2 µL, 1.51 mmol), cataCXium Pd G4 (CAS No.: 2230788-67-5, Aldrich Product No.: 900349) (37.4 mg, 50.0 µmol), and P2tBu/THF solution (2.0 M, 0.227 mL, 0.454 mmol) were added, and the mixture was shaken at 60°C for 1 hour.

The suspension of the reaction solution and the solid phase was divided and transferred onto two filters using NMP, and each washed 3 times with NMP (5 mL), 3 times with a solution of N-acetylcysteine in NMP/water = 5/1 (0.2 M, 5 mL), 3 times with NMP (5 mL), 3 times with MeOH (5 mL), 3 times with DCM (5 mL) and 3 times with heptane (5 mL), and the resulting solid phase was dried overnight under reduced pressure to obtain compound D12-1R (the loading rate 0.198 mmol/g, 485 mg).

A portion of compound D12-1R was washed 3 times with DCM (0.10 mL) and immersed in a 10% TFA/DCM solution of 0.05 M pentamethylbenzene (0.05 M, 0.05 mL) for 5 minutes and then filtered. The filtrate was washed with NMP (0.10 mL). The filtrates combined were diluted with MeCN (0.25 mL) and then subjected to LCMS measurement. As a result, the target material D12 was observed as 97.3%. The analysis was performed by extracting at the wavelength of 270 nm (± 4 nm).

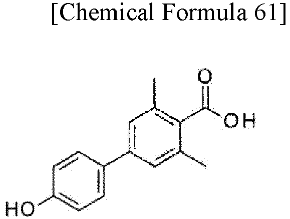

### Compound D12

LRMS: m/z 225 [M+H]⁺
Retention time: 0.823 min (analysis condition FA05-1)

### Example 1-2: Synthesis of solid phase amine compound

### Example 1-2-1: Synthesis of compound F01-1R

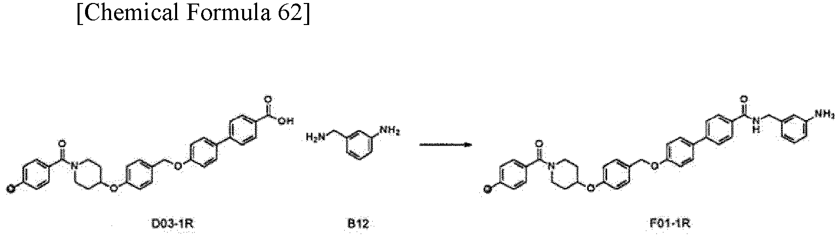

Under a nitrogen atmosphere, to a 20 mL glass vial, compound D03-1R (0.201 mmol/g, 1.00 g) and NMP (15 mL) were added, and the mixture was shaken at room temperature for 1 hour. To this, 3-(aminomethyl)aniline (B12) (197 mg, 1.61 mmol), Oxyma (229 mg, 1.61 mmol) and DIC (252 µL, 1.61 mmol) were added, and the mixture was shaken at room temperature for 15 hours.

The whole of the suspension of the reaction solution and the solid phase was transferred to a column equipped with a filter, and filtered. The filtrate was then washed 3 times with NMP (15 mL), 3 times with MeOH (15 mL), and 3 times with DCM (15 mL), and the resulting solid phase was dried under reduced pressure to obtain compound F01-1R (0.197 mmol/g, 1.01 g). A portion of compound F01-1R was transferred onto a chip with a filter and washed with DCM (0.1 mL). The resultant was immersed in a 10% TFA/DCM solution of pentamethylbenzene (0.02 M, 0.05 mL) for 5 minutes and filtered, and then washed with NMP (0.05 mL). The filtrates were combined and diluted with MeCN (0.25 mL), and then subjected to LCMS measurement. As a result, F01 was observed as 97%. The analysis was performed by extracting at the wavelength of 290 nm (± 4 nm).

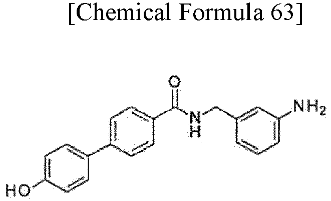

### Compound F01

LRMS: m/z 319 [M+H]⁺
Retention time: 0.708 min (analysis condition RPAmideTFA05)

### Example 1-2-2: Synthesis of compound F02-1R

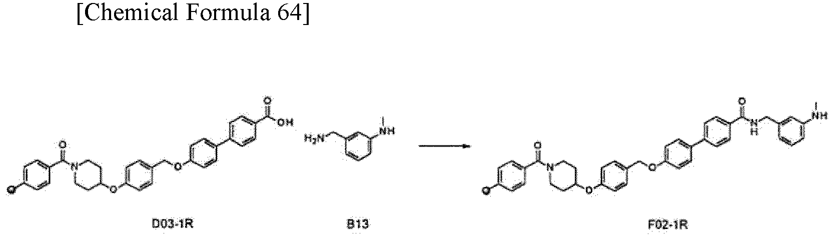

Under a nitrogen atmosphere, to a 20 mL glass vial, compound D03-1R (0.201 mmol/g, 1.00 g) and NMP (15 mL) were added, and the mixture was shaken at room temperature for 1 hour. To this, 3-((methylamino)methyl)aniline (B13) (219 mg, 1.61 mmol), Oxyma (229 mg, 1.61 mmol) and DIC (252 µL, 1.61 mmol) were added, and the mixture was shaken at room temperature for 15 hours.

The whole of the suspension of the reaction solution and the solid phase was transferred to a column equipped with a filter, and filtered. The filtrate was then washed 3 times with NMP (15 mL), 3 times with MeOH (15 mL), and 3 times with DCM (15 mL), and the resulting solid phase was dried under reduced pressure to obtain compound F02-1R (0.196 mmol/g, 1.05 g). A portion of compound F02-1R was transferred onto a chip equipped with a filter and washed with DCM (0.1 mL). The resultant was immersed in a 10% TFA/DCM solution of pentamethylbenzene (0.02 M, 0.05 mL) for 5 minutes and filtered, and then washed with NMP (0.05 mL). The filtrates were combined and diluted with MeCN (0.25 mL), and then subjected to LCMS measurement. As a result, F02 was observed as 98%. The analysis was performed by extracting at the wavelength of 290 nm (± 4 nm).

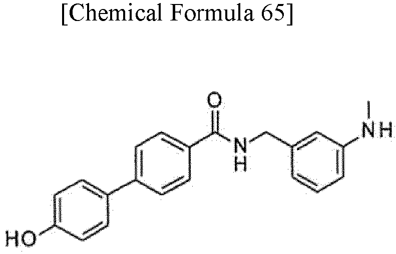

### Compound F02

LRMS: m/z 333 [M+H]⁺
Retention time: 0.723 min (analysis condition RPAmideTFA05)

### Example 1-2-3: Synthesis of compound F04-1R

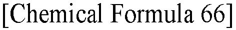

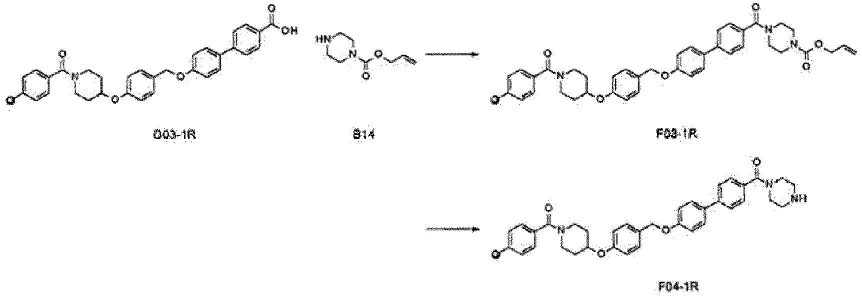

Under a nitrogen atmosphere, to each of twenty-four 4 mL glass vials, compound D03-1R (0.201 mmol/g, 100 mg) and NMP (1.5 mL) were added per vial, and the mixture was shaken at room temperature for 1 hour. To each of the vials, a solution of allyl piperazine-1-carboxylate (B14) in NMP (0.4 M, 101 µL, 0.040 mmol), a solution of NMI in NMP (0.8 M, 101 µL, 0.080 mmol) and a solution of PyClU in NMP (0.4 M, 101 µL, 0.04 mmol) were added, and the mixture was shaken at room temperature for 3 hours.

The suspensions of the reaction solution and the solid phase each were transferred to twenty-four columns equipped with filters using NMP, and filtered. The filtrate was then washed 2 times with NMP (2 mL), 3 times with MeOH (2 mL), 3 times with DCM (2 mL) and 3 times with heptane (2 mL), and the resulting solid phase was dried under reduced pressure. All solid phases were combined to obtain compound F03-1R (0.195 mmol/g, 2.64 g).

Under a nitrogen atmosphere, to a 4 mL glass vial, compound F03-1R (0.195 mmol/g, 108 mg), a solution of pyrrolidine in 1,2-dichloroethane (1 M, 0.632 mL, 0.632 mmol) and 1,2-dichloroethane (0.864 mL) were added, and the mixture was shaken at room temperature for 1 hour. A solution of tetrakis(triphenylphosphine)palladium(0) in 1,2-dichloroethane (0.1 M, 10.5 µL, 1.05 µmol) was added, and the mixture was shaken at room temperature for 7.5 hours.

10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.02 M, 0.05 mL) for 5 minutes and filtered, and then washed with NMP (0.05 mL). The filtrates were combined and diluted with MeCN (0.25 mL), and then subjected to LCMS measurement to measure the progress of the reaction. As a result, F04 was observed as 98%. The analysis was performed by extracting at the wavelength of 290 nm (± 4 nm).

The suspension of the reaction solution and the solid phase was also transferred to a column equipped with a filter and filtered. The filtrate was then washed 3 times with NMP (2 mL), 3 times with a solution of N-acetyl-L-cysteine in NMP/water = 5/1 (0.3 M, 2 mL), 3 times with a solution of DIPEA in NMP (0.2 M, 2 mL), 3 times with NMP (2 mL), 3 times with MeOH (2 mL), 3 times with DCM (2 mL), and 3 times with heptane (2 mL), and the resulting solid phase was dried under reduced pressure. All solid phases were combined to obtain compound F04-1R (0.198 mmol/g, 105 mg).

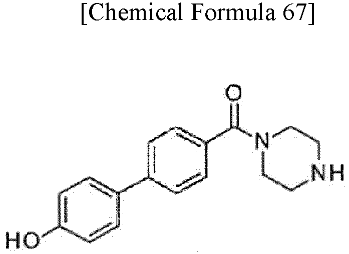

### Compound F04

LRMS: m/z 283 [M+H]⁺
Retention time: 0.549 min (analysis condition FA05-1)

### Example 1-2-4: Synthesis of compound F06-1R

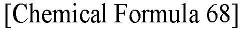

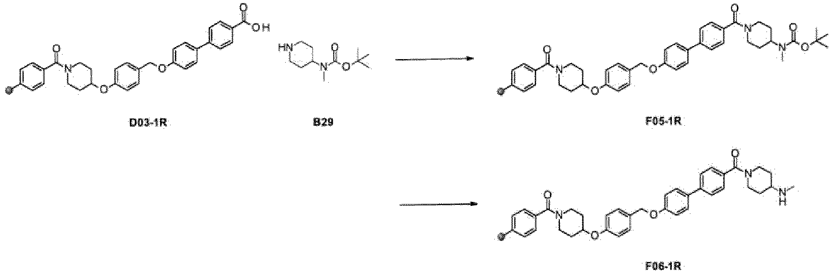

Under a nitrogen atmosphere, to a 30 mL glass vial, compound D03-1R (0.201 mmol/g, 1.00 g) and DCM (15 mL) were added, and the mixture was shaken at room temperature for 1 hour. To this, tert-butyl N-methyl-N-piperidin-4-ylcarbamate (B29) (86 mg, 0.402 mmol), NMI (63.5 µL, 0.804 mmol), DIPEA (70.2 µL, 0.402 mmol), and PipClU (0.145 g, 0.402 mmol) were added, and the mixture was shaken at room temperature for 3.5 hours. 12 µL of the suspension of the reaction solution and the solid phase was transferred onto a tip equipped with a filter, and washed 3 times with DMF (0.1 mL), 3 times with MeOH (0.1 mL), and 3 times with DCM (0.1 mL). The resultant was immersed in a 10% TFA/DCM solution of pentamethylbenzene (0.10 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, F05 was observed as 93%. The analysis was performed by extracting at the wavelength of 299 nm (± 4 nm).

The suspension of the reaction solution and the solid phase was transferred onto a filter using NMP (20 mL), and washed 3 times with NMP/water = 1/1 (20 mL), 3 times with NMP (20 mL), 3 times with methanol (20 mL), 3 times with DCM (20 mL), and 3 times with heptane (20 mL), and the resulting solid phase was dried overnight under reduced pressure to obtain compound F05-1R (the loading rate 0.194 mmol/g, 1.18 g).

Under a nitrogen atmosphere, to a 30 mL glass vial, compound F05-1R (0.194 mmol/g, 1.11 g) and 2-methyltetrahydrofuran (22 mL) were added, and the mixture was shaken at room temperature for 1 hour. Tin(II) trifluoromethanesulfonate (CAS No. 62086-04-8) (1.12 g, 2.68 mmol) and 2,6-lutidine (468 µL, 4.02 mmol) were added, and the mixture was shaken at 80°C for 14.5 hours. To the reaction solution, a solution of 4,4'-di-tert-butyl-2,2' -bipyridine in NMP (0.1 M, 6.0 mL) was added, and the mixture was shaken at room temperature for 1.5 hours.

12 µL of the suspension of the reaction solution and the solid phase was transferred onto a tip equipped with a filter, and washed 3 times with DMF (0.1 mL), 3 times with MeOH (0.1 mL), and 3 times with DCM (0.1 mL). The resultant was immersed in a 10% TFA/DCM solution of pentamethylbenzene (0.10 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, F06 was observed as 99%. The analysis was performed by extracting at the wavelength of 299 nm (± 4 nm).

The suspension of the reaction solution and solid phase was transferred to a column equipped with a filter and filtered, and then washed 3 times with a solution of 2,2'-bipyridine in NMP (0.1 M, 22 mL), 3 times with NMP (22 mL), 3 times with a mixed solution of TBAHSO₄ (tetrabutylammonium hydrogen sulfate) and DTBP (2,6-di-tert-butyl pyridine) in NMP (both 0.05 M, 22 mL), 3 times with a solution of BTMG (2-tert-butyl-1,1,3,3-tetramethylguanidine) in NMP (0.05 M, 22 mL), 3 times with a solution of DTBP in NMP (0.05 M, 22 mL), 3 times with NMP/water = 1/1 (22 mL), 3 times with NMP (22 mL), 3 times with MeOH (22 mL), 3 times with DCM (22 mL), and 3 times with heptane (22 mL). The resulting solid phase was dried under reduced pressure to obtain compound F06-1R (the loading rate 0.197 mmol/g, 1.11 g).

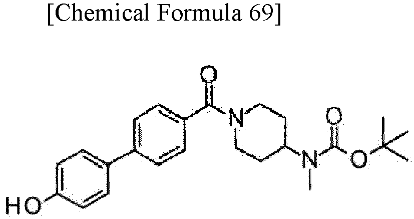

### Compound F05

LRMS: m/z 411 [M+H]⁺
Retention time: 1.057 min (analysis condition FA05-1)

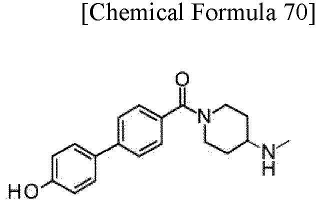

### Compound F06

LRMS: m/z 311 [M+H]⁺
Retention time: 0.537 min (analysis condition FA05-1)

### Example 2: Amidation reaction in liquid phase

### Example 2-1: Study on amidation conditions

### Example 2-1-1: Study on amidation conditions of 1 equivalent of amine substrate (3-aminobiphenyl (B01)) and 3 equivalents of carboxylic acid (2,6-dimethylbenzoic acid (A01))

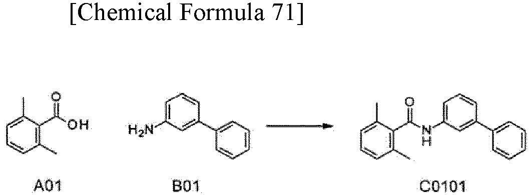

Under a nitrogen atmosphere, to a 0.6 mL glass vial, 3-aminobiphenyl (B01) (3.4 mg, 0.020 mmol, 1 eq.) and 2,6-dimethylbenzoic acid (A01) (9.0 mg, 0.060 mmol, 3 eq.) were added. For Runs 1, 3, and 5 to 9, DCM (0.1 mL) and MeCN (0.1 mL) were added. For Runs 2 and 4, DCM (0.2 mL) and MeCN (0.2 mL) were added. To this, the first and second bases and the condensing agent described in Table 2-1-1 were added, and the mixture was shaken at room temperature for the reaction time described in the Table. It should be noted that the condensing agent was added as a DCM/MeCN = 1/1 solution (0.2 M) for Runs 1, 3, and 6 to 9. For Run 5, DCM (0.1 mL) and MeCN (0.1 mL) were further added. A portion of the reaction solution was dispensed and subjected to LCMS measurement to measure the progress of the reaction. The results are as shown in Table 2-1-1. It should be noted that the yield in Table 2-1-1 is calculated by setting the sum of the portions of the identified peaks excluding the peaks not derived from B01 to 100%. The analysis was performed by extracting at the wavelength of 305 nm (± 95 nm).

**[Table 3]**

| Table 2-1-1 | | | | | | | |
|---|---|---|---|---|---|---|---|
| Run | Condensing agent | First base | Second base | Reaction time (hour) | TM (C0101) | SM (B01) | Other impurities |
| 1 | PyCIU (2 eq.) | DIPEA (9 eq.) | - | 2 | 69.3% | 21.0% | 9.6% |
| 2 | PyCIU (2 eq.) | NMI (9 eq.) | - | 2 | 17.1% | 81.1% | 1.8% |
| 3 | PyCIU (2 eq.) | NMI (2 eq.) | N,N-dimethylaniline (7 eq.) | 2 | 87.8% | 2.1% | 10.1% |
| 4 | PyCIU (2 eq.) | NMI (2 eq.) | N,N-dimethyl-3-(trifluoromethyl)aniline (7 eq.) | 2 | 81.8% | 10.5% | 7.7% |
| 5 | CIP (2 eq.) | NMI (9 eq.) | - | 2 | 26.4% | 71.9% | 1.7% |
| 6 | CIP (2 eq.) | N,N-dimethylaniline (9 eq.) | - | 2 | 64.2% | 0.0% | 35.8% |
| 7 | CIP (2 eq.) | NMI (2 eq.) | N,N-dimethylaniline (7 eq.) | 2 | 83.3% | 4.2% | 12.4% |
| 8 | CIP (2 eq.) | 1-Phenylimidazole (9 eq.) | - | 2 | 44.0% | 44.5% | 11.5% |
| 9 | CIP (2 eq.) | 1-Phenylimidazole (2 eq.) | N,N-dimethylaniline (7 eq.) | 2 | 79.2% | 6.9% | 14.0% |

From the above results, it was shown that the yield in the conditions (Runs 3, 6, and 8) in which 1 equivalent of the first base (NMI or 1-phenylimidazole) was used relative to the halouronium-based condensing agent (PyClU or CIP) and the second base (N,N-dimethylaniline or N,N-dimethyl-3-(trifluoromethyl)aniline) was added is higher than that in the conditions (Runs 1, 2, 4, 5, and 7) in which 4.5 equivalents of the base (NMI, 1-phenylimidazole, N,N-dimethylaniline, or DIPEA) were used relative to the halouronium-based condensing agent (PyClU or CIP).

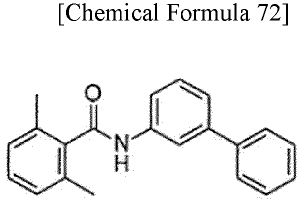

### Compound C0101

LRMS: m/z 302 [M+H]⁺
Retention time: 1.287 min (analysis condition FA05-1)
Retention time: 2.799 min (analysis condition FA05-long)

### Example 2-1-2: Study on amidation conditions of 1 equivalent of carboxylic acid substrate (2,6-dimethylbenzoic acid (A01)) and 2 equivalents of amine (aniline (B02))

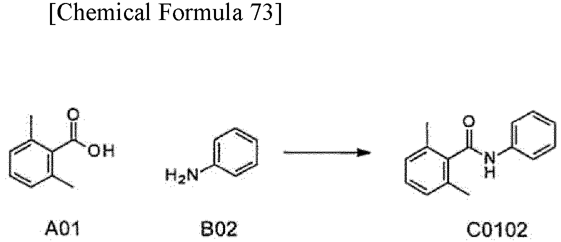

Under a nitrogen atmosphere, to a 0.6 mL glass vial, 2,6-dimethylbenzoic acid (A01) (3.0 mg, 0.020 mmol, 1 eq.), aniline (B02) (3.7 mg, 0.040 mmol, 2 eq.), DCM (0.1 mL), and MeCN (0.1 mL) were added. To this, the first and second bases and the condensing agent described in Table 2-1-2 were added, and the mixture was shaken at room temperature for 24 hours. It should be noted that the condensing agent was added as a DCM/MeCN = 1/1 solution (0.2 M). A portion of the reaction solution was dispensed and subjected to LCMS measurement to measure the progress of the reaction. The results are as shown in Table 2-1-2. It should be noted that the yield in Table 2-1-2 is calculated by setting the sum of the portions of the identified peaks excluding the peaks not derived from A01 to 100%. The analysis was performed by extracting at the wavelength of 270 nm (± 4 nm).

**[Table 4]**

| **Table 2-1-2** | | | | | | | |
|---|---|---|---|---|---|---|---|
| Run | Condensing agent | First base | Second base | Reaction time (hour) | Target material (C0102) | Starting material (A01) | Other impurities |
| 1 | PyCIU (2 eq.) | NMI (4 eq.) | - | 24 | 45.4% | 0.0% | 54.6% |
| 2 | PyCIU (2 eq.) | N,N-dimethylaniline (4 eq.) | - | 24 | 74.1% | 25.9% | 0.0% |
| 3 | PyCIU (2 eq.) | DIPEA (4 eq.) | - | 24 | 83.2% | 0.0% | 16.8% |
| 4 | PyCIU (2 eq.) | NMI (2 eq.) | N,N-dimethylaniline (2 eq.) | 24 | 90.4% | 0.0% | 9.6% |
| 5 | PyCIU (2 eq.) | NMI (1.6 eq.) | N,N-dimethylaniline (2 eq.) | 24 | 94.9% | 0.0% | 5.1% |
| 6 | PyCIU (2 eq.) | N,N-dimethyl-3-(trifluoromethyl)aniline (4 eq.) | - | 24 | 31.3% | 68.7% | 0.0% |
| 7 | PyCIU (2 eq.) | NMI (2 eq.) | N,N-dimethyl-3-(trifluoromethyl)aniline (2 eq.) | 24 | 93.7% | 0.0% | 6.3% |
| 8 | PyCIU (2 eq.) | NMI (1.6 eq.) | N,N-dimethyl-3-(trifluoromethyl)aniline (2 eq.) | 24 | 97.5% | 0.0% | 2.5% |

From the above results, it was shown that the yield in the conditions (Runs 4, 5, 7, and 8) in which 0.8 to 1.0 equivalents of the first base (NMI) were used relative to the halouronium-based condensing agent (PyClU) and the second base (N,N-dimethylaniline or N,N-dimethyl-3-(trifluoromethyl)aniline) was added is higher than that in the conditions (Runs 1, 2, 3, and 6) in which 2 equivalents of the base (NMI, N,N-dimethylaniline, DIPEA, N,N-dimethyl-3-(trifluoromethyl)aniline) were used relative to the halouronium-based condensing agent (PyClU).

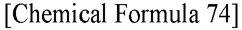

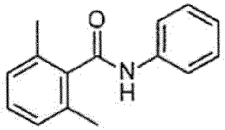

### Compound C0102

LRMS: m/z 226 [M+H]⁺
Retention time: 1.043 min (analysis condition FA05-1)

### Example 2-1-3: Study on amidation conditions of 1 equivalent of amine substrate (3-aminobiphenyl (B01)) and 3 equivalents of carboxylic acid (2,6-dimethylbenzoic acid (A01))

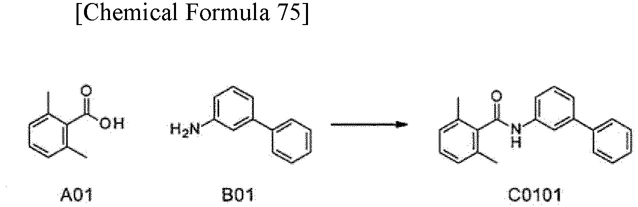

Under a nitrogen atmosphere, to a 0.6 mL glass vial, 3-aminobiphenyl (B01) (3.4 mg, 0.020 mmol, 1 eq.) and 2,6-dimethylbenzoic acid (A01) (9.0 mg, 0.060 mmol, 3 eq.), DCM (0.2 mL) and MeCN (0.2 mL) were added. To this, the first and second bases and the condensing agent described in Table 2-1-3 were added, and the mixture was shaken at room temperature for the reaction time described in the Table. A portion of the reaction solution was dispensed and subjected to LCMS measurement to measure the progress of the reaction. The results are as shown in Table 2-1-3. It should be noted that the yield in Table 2-1-3 is calculated by setting the sum of the portions of the identified peaks excluding the peaks not derived from B01 to 100%. The analysis was performed by extracting at the wavelength of 305 nm (± 95 nm).

**[Table 5]**

| [Table 2-1-3] | | | | | | | |
|---|---|---|---|---|---|---|---|
| Run | Condensing agent | First base | Second base | Reaction time (hour) | Target material (C0101) | Starting material (B01) | Other impurities |
| 1 | TCFH (2 eq.) | NMI (9 eq.) | - | 2 | 19.9% | 78.1% | 2.0% |
| 2 | TCFH (2 eq.) | NMI (9 eq.) | - | 24 | 25.1% | 69.1% | 5.9% |
| 3 | TCFH (2 eq.) | NMI (2 eq.) | N,N-dimethylaniline (7 eq.) | 2 | 86.8% | 3.3% | 9.9% |
| 4 | TCFH (2 eq.) | NMI (2 eq.) | N,N-dimethylaniline (7 eq.) | 24 | 91.3% | 1.1% | 7.5% |
| 5 | BEP (2 eq.) | NMI (9 eq.) | - | 24 | 13.0% | 76.7% | 10.3% |
| 6 | BEP (2 eq.) | NMI (2 eq.) | N,N-dimethylaniline (7 eq.) | 24 | 63.0% | 6.0% | 31.0% |

From the above results, as shown in NPL 3, it was shown that the yield in the conditions (Runs 3, 4, and 6) in which 1 equivalent of the first base (NMI) was used relative to the halouronium-based condensing agent (TCFH) or the Mukaiyama reagent (2-halo-N-alkyl pyridinium salt) (BEP) and the second base (N,N-dimethylaniline) was added is higher than that in the conditions (Runs 1, 2, and 5) in which 4.5 equivalents of base (NMI) were used relative to the halouronium-based condensing agent (TCFH) or the Mukaiyama reagent (2-halo-N-alkyl pyridinium salt) (BEP).

### Reference Example: Amidation reaction of 2,6-dimethylbenzoic acid (A01) and 3-aminobiphenyl (B01) using BTFFH/DIPEA system (conditions in NPL 2)

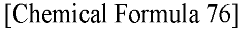

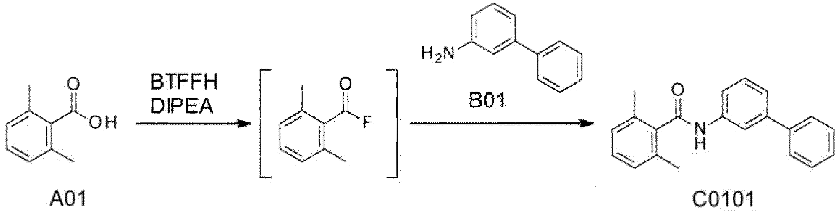

Under a nitrogen atmosphere, to a 0.6 mL glass vial, 2,6-dimethylbenzoic acid (A01) (3.9 mg, 0.026 mmol, 1.3 eq.) and DCM (0.2 mL) were added. To this, BTFFH (9.5 mg, 0.030 mmol, 1.5 eq.) and DIPEA (15.7 µL, 0.090 mmol, 4.5 eq.) were added, and the mixture was shaken at room temperature for 1 hour. A solution of 3-aminobiphenyl (B01) (3.4 mg, 0.020 mmol, 1 eq.) in DCM (0.2 mL) was added, and the mixture was shaken at 80°C for 24 hours. A portion of the reaction solution was dispensed and subjected to LCMS measurement to measure the progress of the reaction. As a result, the target material (C0101), the starting material (B01), and the byproduct believed to be the result of the reaction between the starting material (B01) and the condensing agent BTFFH were observed as 4%, 63%, and 33%, respectively. It should be noted that the yield is calculated by setting the sum of the portions of the identified peaks excluding the peaks not derived from B01 to 100%. The analysis was performed by extracting at the wavelength of 305 nm (± 95 nm).

The results showed that the amidation reaction of 2,6-dimethylbenzoic acid (A01) and 3-aminobiphenyl (B01) could not be performed efficiently in the BTFFH/DIPEA system (conditions in NPL 2)

### Example 2-2: Study on scope of application of halouronium-based condensing agent, first base, and second base

### Example 2-2-1: Study on scope of application of halouronium-based condensing agent, first base, and second base in amidation of 1 equivalent of amine substrate (3-aminobiphenyl (B01)) and 3 equivalents of carboxylic acid (2,6-dimethylbenzoic acid (A01))

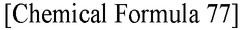

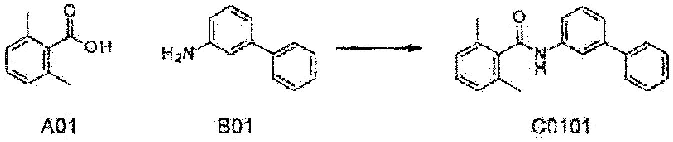

Under a nitrogen atmosphere, to a 0.6 mL glass vial, 3-aminobiphenyl (B01) (3.4 mg, 0.020 mmol, 1 eq.) and 2,6-dimethylbenzoic acid (A01) (9.0 mg, 0.060 mmol, 3 eq.), DCM (0.1 mL) and MeCN (0.1 mL) were added. To this, the first and second bases and the condensing agent described in Table 2-2-1 were added, and the mixture was shaken at room temperature for the reaction time described in the Table. It should be noted that the condensing agent was added as a DCM/MeCN = 1/1 solution (0.2 M) for Runs 1 to 5, 8, 9, 12 and 13. For Runs 6, 7, 10, 11, 14 and 15, the condensing agent was added as a MeCN solution (0.4 M) and the first base was added as a DCM solution (0.4 M). For Runs 16 to 21, the condensing agent was added as a MeCN solution (0.4 M), the first base was added as a DCM solution (0.8 M), and DCM (0.05 mL) was further added. A portion of the reaction solution was dispensed and subjected to LCMS measurement to measure the progress of the reaction. The results are as shown in Table 2-2-1. It should be noted that the yield in Table 2-2-1 is calculated by setting the sum of the portions of the identified peaks excluding the peaks not derived from B01 to 100%. The analysis was performed by extracting at the wavelength of 305 nm (± 95 nm).

**[Table 6]**

| **Table 2-2-1** | | | | | | | |
|---|---|---|---|---|---|---|---|
| Run | Condensing agent | First base | Second base | Reaction time (hour) | Target material (C0101) | Starting material (B01) | Other impurities |
| 1 | CIP (2 eq.) | NMI (2 eq.) | N,N-dimethylaniline (7 eq.) | 2 | 83.3% | 4.2% | 12.4% |
| 2 | CIP (2 eq.) | NMI (2 eq.) | N,N-diethylaniline (7 eq.) | 24 | 75.6% | 12.6% | 11.8% |
| 3 | CIP (2 eq.) | NMI (2 eq.) | N,N,2,4,6-pentamethylaniline (7 eq.) | 24 | 83.7% | 2.8% | 13.6% |
| 4 | CIP (2 eq.) | NMI (2 eq.) | Julolidine (7 eq.) | 24 | 80.3% | 4.0% | 15.7% |
| 5 | CIP (2 eq.) | Tetramethylimidazole (2 eq.) | N,N-dimethylaniline (7 eq.) | 24 | 82.4% | 0.0% | 17.6% |
| 6 | CIP (2 eq.) | Tetramethylimidazole (2 eq.) | N,N,2,4,6-pentamethylaniline (7 eq.) | 24 | 79.9% | 0.0% | 20.1% |
| 7 | CIP (2 eq.) | Tetramethylimidazole (2 eq.) | 2,6-Lutidine (7 eq.) | 24 | 71.3% | 2.8% | 25.9% |
| 8 | PipCIU (2 eq.) | NMI (2 eq.) | N,N-dimethylaniline (7 eq.) | 24 | 91.4% | 0.0% | 8.6% |
| 9 | PipCIU (2 eq.) | Tetramethylimidazole (2 eq.) | N,N-dimethylaniline (7 eq.) | 24 | 91.9% | 0.0% | 8.1% |
| 10 | PipCIU (2 eq.) | Tetramethylimidazole (2 eq.) | N,N,2,4,6-pentamethylaniline (7 eq.) | 24 | 87.0% | 0.0% | 13.0% |
| 11 | PipCIU (2 eq.) | Tetramethylimidazole (2 eq.) | 2,6-Lutidine (7 eq.) | 24 | 75.1% | 3.6% | 21.3% |
| 12 | PyCIU (2 eq.) | NMI (2 eq.) | N,N-dimethylaniline (7 eq.) | 24 | 88.7% | 0.0% | 11.3% |
| 13 | PyCIU (2 eq.) | Tetramethylimidazole (2 eq.) | N,N-dimethylaniline (7 eq.) | 24 | 91.9% | 0.0% | 81% |
| 14 | PyCIU (2 eq.) | Tetramethylimidazole (2 eq.) | N,N,2,4,6-pentamethylaniline (7 eq.) | 24 | 82.7% | 0.0% | 17.3% |
| 15 | PyCIU (2 eq.) | Tetramethylimidazole (2 eq.) | 2,6-Lutidine (7 eq.) | 24 | 75.4% | 3.9% | 20.7% |
| 16 | PyCIU (2 eq.) | Tetramethylimidazole (2 eq.) | 4-Bromo-N,N-dimethylaniline (7 eq.) | 24 | 92.7% | 0.0% | 7.3% |
| 17 | PyCIU (2 eq.) | Tetramethylimidazole (2 eq.) | 4-Fluoro-N,N-dimethylaniline (7 eq.) | 24 | 90.7% | 0.0% | 9.3% |
| 18 | PyCIU (2 eq.) | Tetramethylimidazole (2 eq.) | N,N-Dimethyl-4-(trifluoromethyl)aniline (7 eq.) | 24 | 89.7% | 3.1% | 7.2% |
| 19 | PyCIU (2 eq.) | Tetramethylimidazole (2 eq.) | 3-Bromo-N,N-dimethylaniline (7 eq.) | 24 | 92.9% | 0.0% | 71% |
| 20 | PyCIU (2 eq.) | Tetramethylimidazole (2 eq.) | 3-Chloro-N,N-dimethylaniline (7 eq.) | 24 | 91.6% | 0.0% | 8.4% |
| 21 | PyCIU (2 eq.) | Tetramethylimidazole (2 eq.) | N,N-dimethyl-3-(trifluoromethyl)aniline (7 eq.) | 24 | 94.6% | 0.0% | 5.4% |

From the above results, it was shown that CIP, PyClU, and PipClU can be used as the halouronium-based condensing agent. It was also shown that NMI and tetramethylimidazole can be applicable as the first base. It was further shown that a wide range of bases such as N,N-dimethylaniline, N,N,2,4,6-pentamethylaniline, julolidine, 2,6-lutidine, 4-bromo-N,N-dimethylaniline, 4-fluoro-N,N-dimethylaniline, N,N-dimethyl-4-(trifluoromethyl)aniline, 3-bromo-N,N-dimethylaniline, 3-chloro-N,N-dimethylaniline, and N,N-dimethyl-3-(trifluoromethyl)aniline can be applicable as the second base.

### Example 2-3: Study on scope of application of substrate

### Example 2-3-1: Study on scope of application of substrate when using amine substrate

Under a nitrogen atmosphere, to a 0.6 mL glass vial, carboxylic acid (0.084 mmol) and amine (0.040 mmol) as described in Table 2-3-1 were added. To this, a solution of NMI in DCM (0.8 M, 0.10 mL, 0.080 mmol), a solution of 4-bromo-N,N-dimethylaniline in DCM (1.6 M, 0.10 mL, 0.16 mmol), and a solution of PyClU in DCM (0.4 M, 0.20 mL, 0.080 mmol) were added, and the mixture was shaken at room temperature for the reaction time described in the table. A portion of the reaction solution was dispensed and subjected to LCMS measurement to measure the progress of the reaction. The results are as yields described in Table 2-3-1. It should be noted that the yield in Table 2-3-1 is calculated by setting the sum of the portions of the identified peaks excluding the peaks not derived from amines to 100%. The analysis was performed by extracting at the wavelength of 305 nm (± 95 nm).

**[Table 7]**

| **Table 2-3-1** | | | | | | | |
|---|---|---|---|---|---|---|---|
| Run | | Carboxylic acid | Amine | Reaction time (hour) | | Target material | Yield |
| 1 | Structural formula | 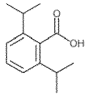 | 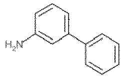 | 2 | Structural formula | 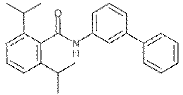 | 69.9 |
| | Compound number | A02 | B01 | | Compound number | C0201 | |
| | Compound name | 2,6-Diisopropylbenzoic acid | 3-Aminobiphenyl | | Compound name | N-(biphenyl-3-yl)-2,6-diisopropyl benzamide | |
| | Amount | 17.3 mg | 6.8 mg | | LRMS | 358 [M+H]⁺ | |
| | Molar amount | 0.084 mmol | 0.040 mmol | | Retention time | 1.492 min (Method: FA05-1) | |
| 2 | Structural formula | 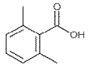 | 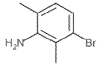 | 67 | Structural formula | 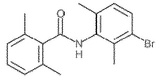 | 88.1 |
| | Compound number | A01 | B04 | | Compound number | C0104 | |
| | Compound name | 2,6-Dimethylbenzoic acid | 3-Bromo-2,6-dimethylaniline | | Compound name | N-(3-bromo-2,6-dimethylphenyl)-2,6-dimethylbenzamide | |
| | Amount | 12.6 mg | 8.0 mg | | LRMS | 332, 334 [M+H]⁺ | |
| | Molar amount | 0.084 mmol | 0.040 mmol | | Retention time | 1.204 min (Method: FA05-1) | |
| 3 | Structural formula | 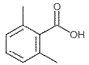 | 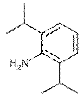 | 2 | Structural formula | 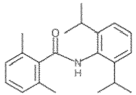 | 58.1 |
| | Compound number | A01 | B03 | | Compound number | C0103 | |
| | Compound name | 2,6-Dimethylbenzoic acid | 2,6-Diisopropylaniline | | Compound name | N-(2,6-diisopropylphenyl)-2,6-dimethylbenzamide | |
| | Amount | 12.6 mg | 7.5 µL | | LRMS | 310 [M+H]⁺ | |
| | Molar amount | 0.084 mmol | 0.040 mmol | | Retention time | 2.837 min (Method: FA05-long) | |
| 4 | Structural formula | 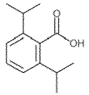 | 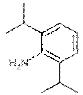 | 24 | Structural formula | 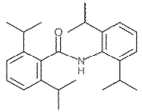 | 61.2 |
| | Compound number | A02 | B03 | | Compound number | C0203 | |
| | Compound name | 2,6-Diisopropylbenzoic acid | 2,6-Diisopropylaniline | | Compound name | N-(2,6-Diisopropylphenyl)-2,6-diisopropylbenzamide | |
| | Amount | 17.3 mg | 7.5 µL | | LRMS | 366 [M+H]⁺ | |
| | Molar amount | 0.084 mmol | 0.040 mmol | | Retention time | 3.391 min (Method: FA05-long) | |
| 5 | Structural formula | 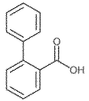 | 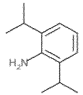 | 67 | Structural formula | 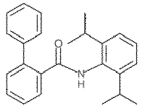 | 100.0 |
| | Compound number | A03 | B03 | | Compound number | C0303 | |
| | Compound name | Biphenyl-2-carboxylic acid | 2,6-Diisopropylaniline | | Compound name | N-(2,6-Diisopropylphenyl)biphenyl-2-carboxamide | |
| | Amount | 16.7 mg | 7.5 µL | | LRMS | 358 [M+H]⁺ | |
| | Molar amount | 0.084 mmol | 0.040 mmol | | Retention time | 3.044 min (Method: FA05-long) | |
| 6 | Structural formula | 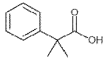 | 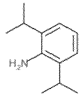 | 67 | Structural formula | 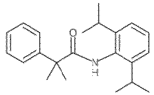 | 100.0 |
| | Compound number | A04 | B03 | | Compound number | C0403 | |
| | Compound name | 2-Methyl-2-phenylpropanoic acid | 2,6-Diisopropylaniline | | Compound name | N-(2,6-Diisopropylphenyl)-2-methyl-2-phenylpropanamide | |
| | Amount | 13.8 mg | 7.5 µL | | LRMS | 324 [M+H]⁺ | |
| | Molar amount | 0.084 mmol | 0.040 mmol | | Retention time | 3.048 min (Method: FA05-long) | |

**[Table 8]**

| | | | | | | | |
|---|---|---|---|---|---|---|---|
| 7 | Structural formula | 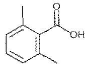 | 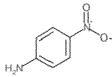 | 24 | Structural formula | 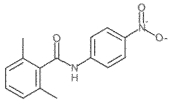 | 74.5 |
| | Compound number | A01 | 805 | | Compound number | C0105 | |
| | Compound name | 2,6-Dimethylbenzoic acid | 4-Nitroaniline | | Compound name | 2,6-Dimethyl-N-(4-nitrophenyl)benzamide | |
| | Amount | 12.6 mg | 5.5 mg | | LRMS | 271 [M+H]⁺ | |
| | Molar amount | 0.084 mmol | 0.040 mmol | | Retention time | 2.316 min (Method: FA05-long) | |
| 8 | Structural formula | 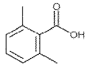 | 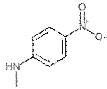 | 24 | Structural formula | 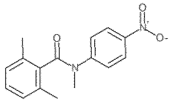 | 90.4 |
| | Compound number | A01 | 806 | | Compound number | C0106 | |
| | Compound name | 2,6-Dimethylbenzoic acid | N-Methyl-4-nitroaniline | | Compound name | N,2,6-trimethyl-N-(4-nitrophenyl)benzamide | |
| | Amount | 12.6 mg | 6.1 mg | | LRMS | 285 [M+H]⁺ | |
| | Molar amount | 0.084 mmol | 0.040 mmol | | Retention time | 2.215 min (Method: FA05-long) | |
| 9 | Structural formula | 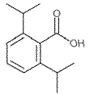 | 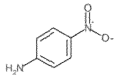 | 24 | Structural formula | 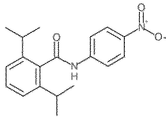 | 82.6 |
| | Compound number | A02 | 805 | | Compound number | C0205 | |
| | Compound name | 2,6-Diisopropylbenzoic acid | 4-Nitroaniline | | Compound name | 2,6-Diisopropyl-N-(4-nitrophenyl)benzamide | |
| | Amount | 17.3 mq | 5.5 mg | | LRMS | 327 [M+H]⁺ | |
| | Molar amount | 0.084 mmol | 0.040 mmol | | Retention time | 3.000-3.055 min (Method: FA05-long) | |
| 10 | Structural formula | 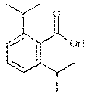 | 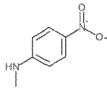 | 24 | Structural formula | 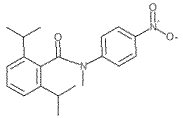 | 62.9 |
| | Compound number | A02 | B06 | | Compound number | C0206 | |
| | Compound name | 2,6-Diisopropylbenzoic acid | N-Methyl-4-nitroaniline | | Compound name | 2,6-Diisopropyl-N-methyl-N-(4-nitrophenyl) benzamide | |
| | Amount | 17.3 mg | 6.1 mg | | LRMS | 341 [M+H]⁺ | |
| | Molar amount | 0.084 mmol | 0.040 mmol | | Retention time | 2.920-3.052 min (Method: FA05-long) | |
| 11 | Structural formula | 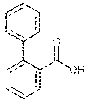 | 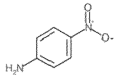 | 24 | Structural formula | 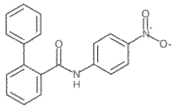 | 68.5 |
| | Compound number | A03 | B05 | | Compound number | C0305 | |
| | Compound name | Biphenyl-2-carboxylic acid | 4-Nitroaniline | | Compound name | N-(4-nitrophenyl)biphenyl-2-carboxamide | |
| | Amount | 16.7 mg | 5.5 mg | | LRMS | 319 [M+H]⁺ | |
| | Molar amount | 0.084 mmol | 0.040 mmol | | Retention time | 1.186 min (Method: FA05) | |
| 12 | Structural formula | 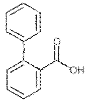 | 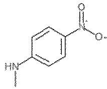 | 24 | Structural formula | 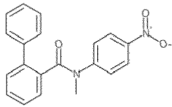 | 57.2 |
| | Compound number | A03 | B06 | | Compound number | C0306 | |
| | Compound name | Biphenyl-2-carboxylic acid | N-Methyl-4-nitroaniline | | Compound name | N-methyl-N-(4-nitrophenyl)biphenyl-2-carboxamide | |
| | Amount | 16.7 mg | 6.1 mg | | LRMS | 333 [M+H]⁺ | |
| | Molar amount | 0.084 mmol | 0.040 mmol | | Retention time | 1.187 min (Method: FA05) | |

**[Table 9]**

| | | | | | | | |
|---|---|---|---|---|---|---|---|
| 18 | Structural formula | 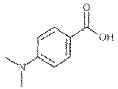 | 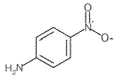 | 24 | Structural formula | 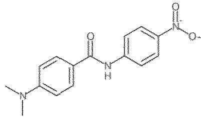 | 100.0 |
| | Compound number | A05 | B05 | | Compound number | CO505 | |
| | Compound name | 4-(Dimethylamino)benzoic acid | 4-Nitroaniline | | Compound name | 4-(Dimethylamino)-N-(4-nitrophenyl) benzamide | |
| | Amount | 13.9 mg | 5.5 mg | | LRMS | 286 [M+H]⁺ | |
| | Molar amount | 0.084 mmol | 0.040 mmol | | Retention time | 2.295 min (Method: FA05-long) | |
| 14 | Structural formula | 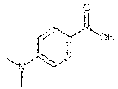 | 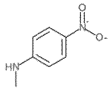 | 24 | Structural formula | 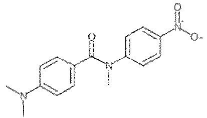 | 93.3 |
| | Compound number | A05 | B06 | | Compound number | C0506 | |
| | Compound name | 4-(Dimethylamino)benzoic acid | N-Methyl-4-nitroaniline | | Compound name | 4-(Dimethylamino)-N-methyl-N-(4-nitrophenyl) benzamide | |
| | Amount | 13.9 mg | 6.1 mg | | LRMS | 300 [M+H]⁺ | |
| | Molar amount | 0.084 mmol | 0.040 mmol | | Retention time | 1.040 min (Method: FA05) | |
| 15 | Structural formula | 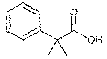 | 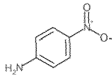 | 24 | Structural formula | 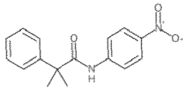 | 97.9 |
| | Compound number | A04 | B05 | | Compound number | C0405 | |
| | Compound name | 2-Methyl-2-phenylpropanoic acid | 4-Nitroaniline | | Compound name | 2-Methyl-N-(4-nitrophenyl)-2-phenylpropanamide | |
| | Amount | 13.8 mg | 5.5 mg | | LRMS | 285 [M+H]⁺ | |
| | Molar amount | 0.084 mmol | 0.040 mmol | | Retention time | 1.207 min (Method: FA05) | |
| 16 | Structural formula | 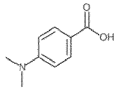 | 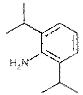 | 24 | Structural formula | 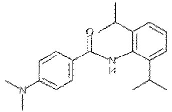 | 98.5 |
| | Compound number | A05 | B03 | | Compound number | C0503 | |
| | Compound name | 4-(Dimethylamino)benzoic acid | 2,6-Diisopropylaniline | | Compound name | N-(2,6-diisopropylphenyl)-4-(dimethylamino)benzamide | |
| | Amount | 13.9 mg | 7.5 µL | | LRMS | 325 [M+H]+ | |
| | Molar amount | 0.084 mmol | 0.040 mmol | | Retention time | 2.837 min (Method: FA05-long) | |

### Example 3: Amidation reaction in solid phase

### Example 3-1: Study on amidation conditions

### Example 3-1-1: Study on amidation conditions of solid phase carboxylic acid substrate (D05-1R) and aniline (B02)

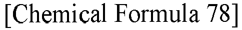

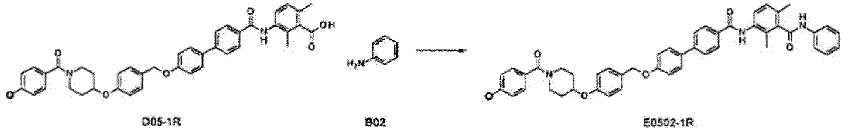

Under a nitrogen atmosphere, to a 0.6 mL glass vial, D05-1R (0.195 mmol/g, 20 mg, 83% purity), aniline (B02) (3.6 µL, 0.039 mmol) and DCM (0.4 mL) were added. DIPEA (4.1 µL, 0.023 mmol) was added for Run 2 and NMI (1.9 µL, 0.023 mmol) was added for Run 3, and the mixture was shaken at room temperature for 1 hour for all of Runs 1 to 3. For Run 1, a mixed solution of PipClU and NMI in MeCN (both 1 M, 7.8 µL, 0.0078 mmol) was added, and the mixture was shaken at room temperature for 24 hours. For Runs 2 and 3, PipClU (2.8 mg, 0.0078 mmol) was added, and the mixture was shaken at room temperature for 24 hours.

12 µL of the suspension of the reaction solution and the solid phase was transferred onto a tip equipped with a filter, and washed 3 times with DMF (0.1 mL), 3 times with MeOH (0.1 mL) (EtOH was used instead of MeOH for Runs 2 and 3), and 3 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. The results are as shown in Table 3-1-1. It should be noted that the yield in Table 3-1-1 is calculated by setting the sum of the portions excluding the peaks derived from impurities originally contained in D05-1R (purity 83%) to 100%. The analysis was performed by extracting at the wavelength of 299 nm (± 4 nm).

**[Table 10]**

| **Table 3-3-1** | | | | | | |
|---|---|---|---|---|---|---|
| Run | Condensing agent | First base | Reaction time (hour) | Target material (E0402) | Starting material (D04) | Other impurities |
| 1 | PipCIU (2 eq.) | NMI (2 eq.) | 24 | 90.9% | 9.1% | 0.0% |
| 2 | PipCIU (2 eq.) | DIPEA (6 eq.) | 24 | 43.0% | 50.7% | 6.4% |
| 3 | PipCIU (2 eq.) | NMI (6 eq.) | 24 | 19.9% | 53.1% | 27.0% |

From the above results, it was shown that the yield in the conditions (Run 1) in which 1 equivalent of the base (NMI) was used relative to the condensing agent is higher than that in the conditions (Runs 2 and 3) in which 3 equivalents of the base (DIPEA or NMI) were used relative to the condensing agent.

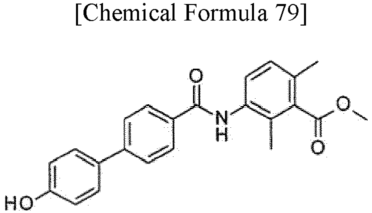

### Compound E0502

LRMS: m/z 437 [M+H]⁺
Retention time: 2.207 min (analysis condition FA05-long)

### Example 3-2: Study on scope of application of halouronium-based condensing agent, first base, second base, and solvent

### Example 3-2-1: Study on scope of application of halouronium-based condensing agent in amidation of solid phase carboxylic acid substrate (D03-1R) and 2,6-diisopropylaniline (B03)

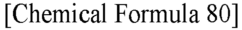

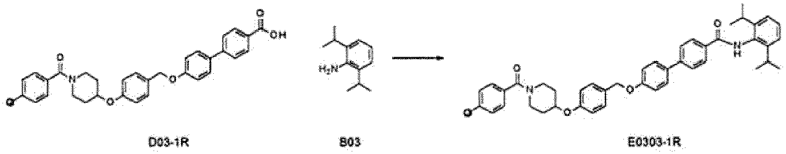

Under a nitrogen atmosphere, to a 0.6 mL glass vial, D03-1R (0.201 mmol/g, 20 mg) and DCM (0.35 mL) were added, and the mixture was shaken at room temperature for 1 hour. To this, a mixed solution of 2,6-diisopropylaniline (B03) (0.4 M, 0.020 mmol), NMI (0.4 M, 0.020 mmol) and N,N-dimethyl-3-(trifluoromethyl)aniline (0.8 M, 0.040 mmol) in DCM (0.050 mL) was added. To this, the halouronium-based condensing agent (0.020 mmol) described in Table 3-2-1 was added and the mixture was shaken at room temperature for 2 hours.

10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 1 minute and filtered, and then washed with NMP (0.05 mL). The filtrates were combined and diluted with MeCN (0.25 mL), and then subjected to LCMS measurement to measure the progress of the reaction. The results are as shown in Table 3-2-1. The analysis was performed by extracting at the wavelength of 290 nm (± 4 nm).

**[Table 11]**

| Table 3-2-1 | | | | | | | |
|---|---|---|---|---|---|---|---|
| Run | Condensing agent | First base | Second base | Reaction time (hour) | Target material (E0303) | Starting material (D03) | Other impurities |
| 1 | PyCIU (5 eq.) | NMI (5 eq.) | N,N-dimethyl-3-(trifluoromethyl)aniline (10 eq.) | 2 | 84.7% | 4.6% | 10.6% |
| 2 | TPyClU (5 eq.) | NMI (5 eq.) | N,N-dimethyl-3-(trifluoromethyl)aniline (10 eq.) | 2 | 83.4% | 6.0% | 10.6% |
| 3 | PipCIU (5 eq.) | NMI (5 eq.) | N,N-dimethyl-3-(trifluoromethyl)aniline (10 eq.) | 2 | 91.2% | 3.0% | 5.8% |
| 4 | CIP (5 eq.) | NMI (5 eq.) | N,N-dimethyl-3-(trifluoromethyl)aniline (10 eq.) | 2 | 68.1% | 12.9% | 19.0% |
| 5 | CIB (5 eq.) | NMI (5 eq.) | N,N-dimethyl-3-(trifluoromethyl)aniline (10 eq.) | 2 | 81.1% | 8.1% | 10.8% |

From the above results, it was shown that PyClU, TPyClU, PipClU, CIP, and CIB can be used as the halouronium-based condensing agent.

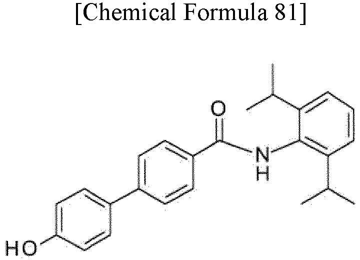

### Compound E0303

LRMS: m/z 374 [M+H]⁺
Retention time: 1.210 min (analysis condition FA05-1)

### Example 3-2-2: Study on scope of application of solvent in amidation of solid phase carboxylic acid substrate (D03-1R) and 2,6-diisopropylaniline (B03)

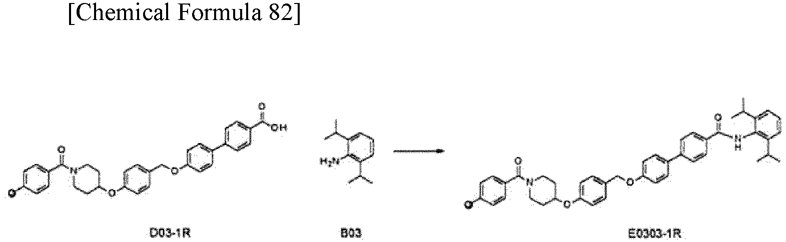

Under a nitrogen atmosphere, to a 0.6 mL glass vial, D03-1R (0.201 mmol/g, 20 mg) and a solvent described in Table 3-2-2 were added, and the mixture was shaken at room temperature for 1 hour. To this, a mixed solution of 2,6-diisopropylaniline (B03) (0.4 M, 0.020 mmol), NMI (0.4 M, 0.020 mmol) and N,N-dimethyl-3-(trifluoromethyl)aniline (0.8 M, 0.040 mmol) in DCM (0.050 mL) was added. To this, PipClU (7.3 mg, 0.020 mmol) was added for Runs 1 to 5, and a solution of PyClU in DCM (0.4 M, 0.050 mL, 0.020 mmol) was added for Run 6 to 14, and the mixture was shaken at room temperature for 2 hours.

10 µL of the suspension of the reaction solution and the solid phase was transferred onto a tip equipped with a filter, and washed 3 times with NMP (0.1 mL), 3 times with MeOH (0.1 mL), and 3 times with DCM (0.1 mL). The resultant was immersed in a 10% TFA/DCM solution of pentamethylbenzene (0.1 M, 0.05 mL) for 1 minute and filtered, and then washed with NMP (0.05 mL). The filtrates were combined and diluted with MeCN (0.25 mL), and then subjected to LCMS measurement to measure the progress of the reaction. The results are as shown in Table 3-2-2. The analysis was performed by extracting at the wavelength of 290 nm (± 4 nm).

**[Table 12]**

| **Table 3-2-2** | | | | | |
|---|---|---|---|---|---|
| Run | Solvent | Reaction time (hour) | Target material (E0303) | Starting material (D03) | Other impurities |
| 1 | DCM (0.35 mL) | 2 | 91.2% | 3.0% | 5.8% |
| 2 | Benzonitrile (0.4 mL) | 2 | 81.4% | 4.7% | 13.9% |
| 3 | 1-Ethyl-2-pyrrolidone (0.4 mL) | 2 | 73.9% | 21.2% | 4.9% |
| 4 | N,N-dimethylpropionamide (0.4 mL) | 2 | 74.1% | 23.8% | 2.2% |
| 5 | N,N-diethylacetamide (0.4 mL) | 2 | 65.5% | 30.1% | 4.4% |
| 6 | NMP (0.3 mL) | 2 | 85.1% | 6.2% | 8.7% |
| 7 | 1-Ethyl-2-pyrrolidone (0.3 mL) | 2 | 84.5% | 7.3% | 8.2% |
| 8 | 1-Octyl-2-pyrrolidone (0.3 mL) | 2 | 84.4% | 10.4% | 5.2% |
| 9 | 1-Cyclohexyl-2-pyrrolidone (0.3 mL) | 2 | 76.4% | 9.7% | 14.0% |
| 10 | N-methylcaprolactam (0.3 mL) | 2 | 75.9% | 9.2% | 14.9% |
| 11 | N,N-dimethylpropionamide (0.3 mL) | 2 | 88.9% | 7.4% | 3.7% |
| 12 | N,N-dimethylisobutylamide (0.3 mL) | 2 | 86.7% | 7.3% | 6.0% |
| 13 | N,N-diethylacetamide (0.3 mL) | 2 | 87.7% | 3.9% | 8.4% |
| 14 | N,N-diethylpropionamide (0.3 mL) | 2 | 85.3% | 6.2% | 8.6% |

### Example 3-3: Study on scope of application of substrate

### Example 3-3-1: Synthesis of E0305-1R by amidation of compound D03-1R and 4-nitroaniline (B05)

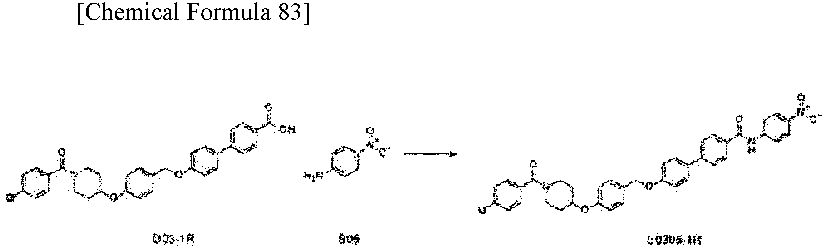

Under a nitrogen atmosphere, to a 0.6 mL glass vial, compound D03-1R (0.201 mmol/g, 20 mg) and DCM (0.4 mL) were added, and the mixture was shaken at room temperature for 1 hour. To this, 4-nitroaniline (B05) (5.6 mg, 0.040 mmol) was added. To this, a mixed solution of PipClU (1.1 M, 0.0088 mmol) and NMI (1.0 M, 0.0080 mmol) in MeCN (8.0 µL) was added, and the mixture was shaken at room temperature for 28 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0305 was observed as 98%. The analysis was performed by extracting at the wavelength of 290 nm (± 4 nm).

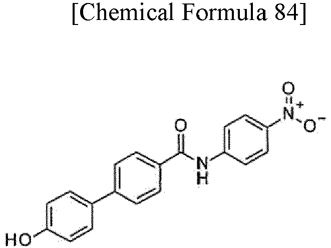

### Compound E0305

LRMS: m/z 335 [M+H]⁺
Retention time: 1.085 min (analysis condition FA05-1)

### Example 3-3-2: Synthesis ofE0306-1R by amidation of compound D03-1R and N-methyl-4-nitroaniline (B06)

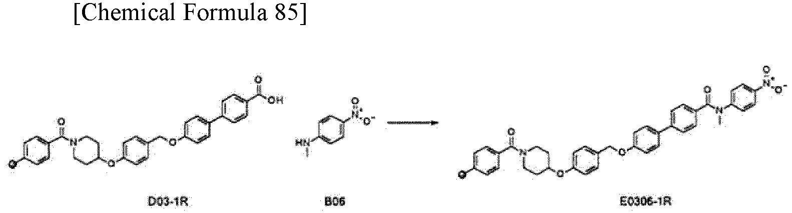

Under a nitrogen atmosphere, to a 0.6 mL glass vial, compound D03-1R (0.201 mmol/g, 20 mg) and DCM (0.4 mL) were added, and the mixture was shaken at room temperature for 1 hour. To this, N-methyl-4-nitroaniline (B06) (6.1 mg, 0.040 mmol) was added. To this, a mixed solution of PipClU (1.1 M, 0.0088 mmol) and NMI (1.0 M, 0.0080 mmol) in MeCN (8.0 µL) was added, and the mixture was shaken at room temperature for 28 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0306 was observed as 99%. The analysis was performed by extracting at the wavelength of 290 nm (± 4 nm).

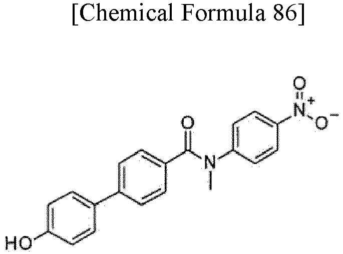

### Compound E0306

LRMS: m/z 349 [M+H]⁺
Retention time: 0.999 min (analysis condition FA05-1)

### Example 3-3-3: Synthesis of E0307-1R by amidation of compound D03-1R and N-isopropylbenzylamine (B07)

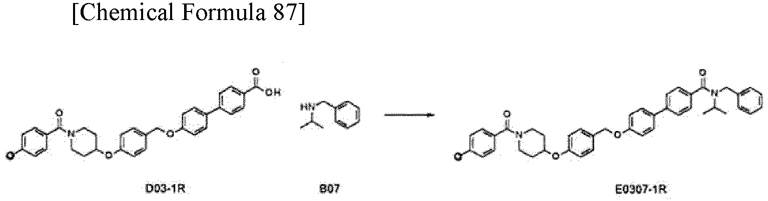

Under a nitrogen atmosphere, to a 0.6 mL glass vial, compound D03-1R (0.201 mmol/g, 20 mg) and DCM (0.4 mL) were added, and the mixture was shaken at room temperature for 1 hour. To this, N-isopropylbenzylamine (B07) (6.6 µL, 0.040 mmol) was added. To this, a mixed solution of PipClU (1.1 M, 0.0088 mmol) and NMI (1.0 M, 0.0080 mmol) in MeCN (8.0 µL) was added, and the mixture was shaken at room temperature for 2 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0307 was observed as 99%. The analysis was performed by extracting at the wavelength of 290 nm (± 4 nm).

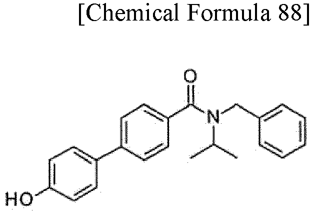

### Compound E0307

LRMS: m/z 346 [M+H]⁺
Retention time: 1.137 min (analysis condition FA05-1)

### Example 3-3-4: Synthesis of E0703-1R by amidation of compound D07-1R and 2,6-diisopropylaniline (B03)

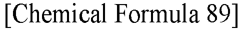

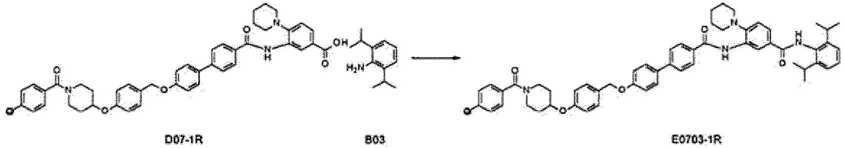

Under a nitrogen atmosphere, to a 0.6 mL glass vial, D07-1R (0.193 mmol/g, 20 mg, 84% purity) and DCM (0.4 mL) were added, and the mixture was shaken at room temperature for 1 hour. To this, 2,6-diisopropylaniline (B03) (7.3 µL, 0.039 mmol) was added. To this, a mixed solution of PipClU (1.1 M, 0.0085 mmol) and NMI (1.0 M, 0.0077 mmol) in MeCN (7.7 µL) was added, and the mixture was shaken at room temperature for 2 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0703 was observed as 99%. It should be noted that the yield is calculated by setting the sum of the portions excluding the peaks derived from impurities originally contained in D07-1R (purity 84%) to 100%. The analysis was performed by extracting at the wavelength of 299 nm (± 4 nm).

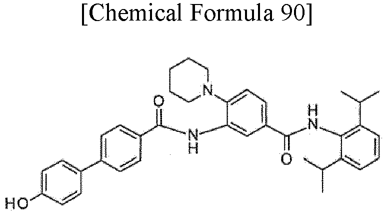

### Compound E0703

LRMS: m/z 576 [M+H]⁺
Retention time: 1.461 min (analysis condition FA05-1)

### Example 3-3-5: Synthesis of E0705-1R by amidation of compound D07-1R and 4-nitroaniline (B05)

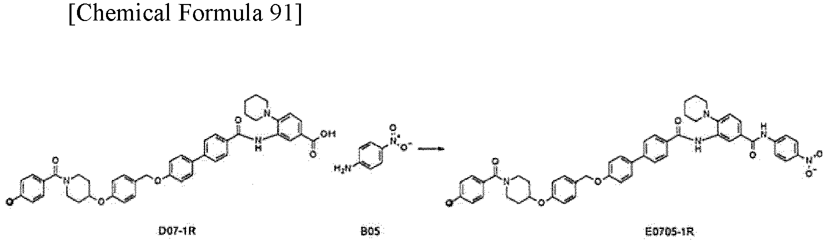

Under a nitrogen atmosphere, to a 0.6 mL glass vial, D07-1R (0.193 mmol/g, 20 mg, 84% purity) and DCM (0.4 mL) were added, and the mixture was shaken at room temperature for 1 hour. To this, 4-nitroaniline (B05) (5.3 mg, 0.039 mmol) was added. To this, a mixed solution of PipClU (1.1 M, 0.0085 mmol) and NMI (1.0 M, 0.0077 mmol) in MeCN (7.7 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0705 was observed as 99%. It should be noted that the yield is calculated by setting the sum of the portions excluding the peaks derived from impurities originally contained in D07-1R (purity 84%) to 100%. The analysis was performed by extracting at the wavelength of 299 nm (± 4 nm).

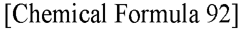

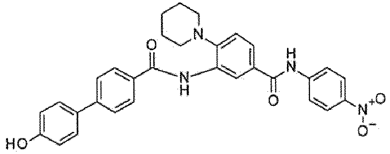

### Compound E0705

LRMS: m/z 537 [M+H]⁺
Retention time: 1.365 min (analysis condition FA05-1)

### Example 3-3-6: Synthesis of E0706-1R by amidation of compound D07-1R and N-methyl-4-nitroaniline (B06)

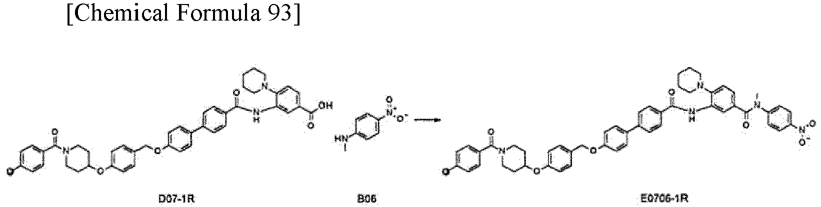

Under a nitrogen atmosphere, to a 0.6 mL glass vial, D07-1R (0.193 mmol/g, 20 mg, 84% purity) and DCM (0.4 mL) were added, and the mixture was shaken at room temperature for 1 hour. To this, N-methyl-4-nitroaniline (B06) (5.9 mg, 0.039 mmol) was added. To this, a mixed solution of PipClU (1.1 M, 0.0085 mmol) and NMI (1.0 M, 0.0077 mmol) in MeCN (7.7 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0706 was observed as 100%. It should be noted that the yield is calculated by setting the sum of the portions excluding the peaks derived from impurities originally contained in D07-1R (purity 84%) to 100%. The analysis was performed by extracting at the wavelength of 299 nm (± 4 nm).

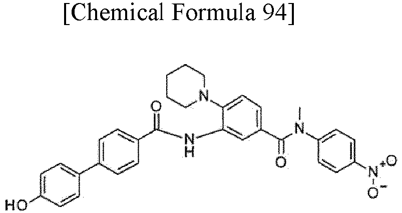

### Compound E0706

LRMS: m/z 551 [M+H]⁺
Retention time: 1.284 min (analysis condition FA05-1)

### Example 3-3-7: Synthesis of E0707-1R by amidation of compound D07-1R and N-isopropylbenzylamine (B07)

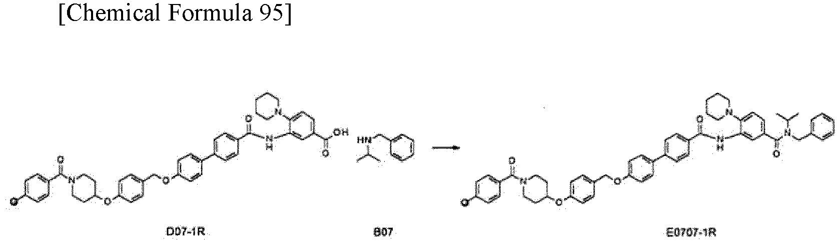

Under a nitrogen atmosphere, to a 0.6 mL glass vial, D07-1R (0.193 mmol/g, 20 mg, 84% purity) and DCM (0.4 mL) were added, and the mixture was shaken at room temperature for 1 hour. To this, N-isopropylbenzylamine (B07) (6.3 µL, 0.039 mmol) was added. To this, a mixed solution of PipClU (1.1 M, 0.0085 mmol) and NMI (1.0 M, 0.0077 mmol) in MeCN (7.7 µL) was added, and the mixture was shaken at room temperature for 30 minutes.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0707 was observed as 99%. It should be noted that the yield is calculated by setting the sum of the portions excluding the peaks derived from impurities originally contained in D07-1R (purity 84%) to 100%. The analysis was performed by extracting at the wavelength of 299 nm (± 4 nm).

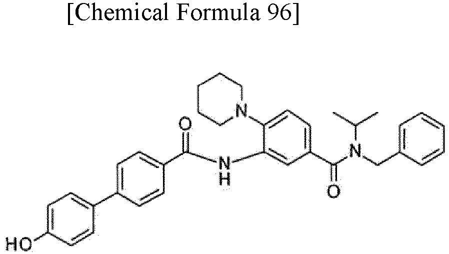

### Compound E0707

LRMS: m/z 548 [M+H]⁺
Retention time: 1.420 min (analysis condition FA05-1)

### Example 3-3-8: Synthesis ofE0903-1R by amidation of compound D09-1R and 2,6-diisopropylaniline (B03)

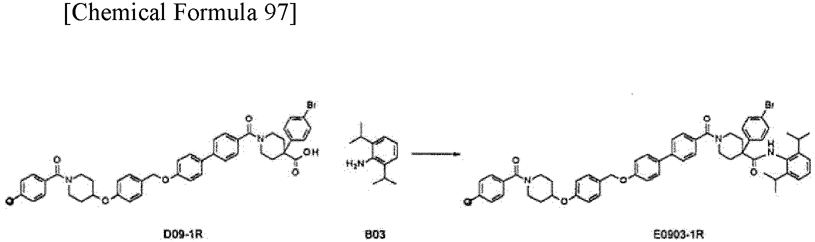

Under a nitrogen atmosphere, to a 0.6 mL glass vial, D09-1R (0.191 mmol/g, 20 mg, 96% purity) and DCM (0.4 mL) were added, and the mixture was shaken at room temperature for 1 hour. To this, 2,6-diisopropylaniline (B03) (7.2 µL, 0.038 mmol) was added. To this, a mixed solution of PipClU (1.1 M, 0.0084 mmol) and NMI (1.0 M, 0.0076 mmol) in MeCN (7.6 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0903 was observed as 94%. It should be noted that the yield is calculated by setting the sum of the portions excluding the peaks derived from impurities originally contained in D09-1R (purity 96%) to 100%. The analysis was performed by extracting at the wavelength of 290 nm (± 4 nm).

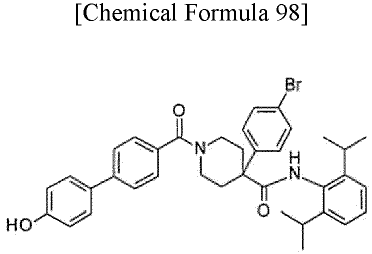

Compound E0903
LRMS: m/z 639, 641 [M+H]⁺
Retention time: 1.359 min (analysis condition FA05-1)

### Example 3-3-9: Synthesis ofE0905-1R by amidation of compound D09-1R and 4-nitroaniline (B05)

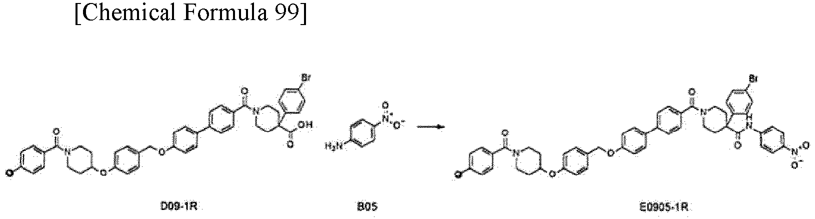

Under a nitrogen atmosphere, to a 0.6 mL glass vial, D09-1R (0.191 mmol/g, 20 mg, 96% purity) and DCM (0.4 mL) were added, and the mixture was shaken at room temperature for 1 hour. To this, 4-nitroaniline (B05) (5.3 mg, 0.038 mmol) was added. To this, a mixed solution of PipClU (1.1 M, 0.0084 mmol) and NMI (1.0 M, 0.0076 mmol) in MeCN (7.6 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E0905 was observed as 95%. It should be noted that the yield is calculated by setting the sum of the portions excluding the peaks derived from impurities originally contained in D09-1R (purity 96%) to 100%. The analysis was performed by extracting at the wavelength of 290 nm (± 4 nm).

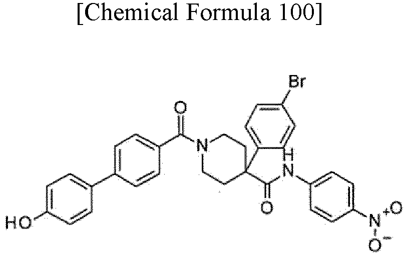

### Compound E0905

LRMS: m/z 600, 602 [M+H]⁺
Retention time: 1.253 min (analysis condition FA05-1)

### Example 3-3-10: Synthesis of G0101-1R by amidation of compound F01-1R and 2,6-dimethylbenzoic acid (A01)

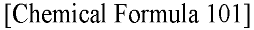

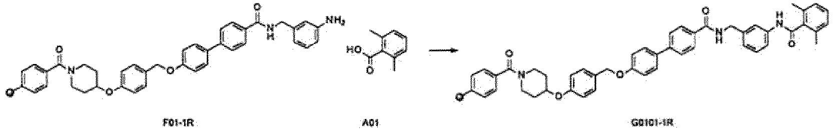

Under a nitrogen atmosphere, to a 0.6 mL glass vial, F01-1R (0.197 mmol/g, 20 mg, 97% purity) and DCM (0.4 mL) were added, and the mixture was shaken at room temperature for 1 hour. To this, 2,6-dimethylbenzoic acid (A01) (7.1 mg, 0.047 mmol) and N,N-dimethyl-p-toluidine (17.0 µL, 0.118 mmol) were added. To this, a mixed solution of PipClU (1.0 M, 0.039 mmol) and NMI (1.1 M, 0.043 mmol) in MeCN (39.4 µL) was added, and the mixture was shaken at room temperature for 90 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0101 was observed as 69%. It should be noted that the yield is calculated by setting the sum of the portions excluding the peaks derived from impurities originally contained in F01-1R (purity 97%) to 100%. The analysis was performed by extracting at the wavelength of 290 nm (± 4 nm).

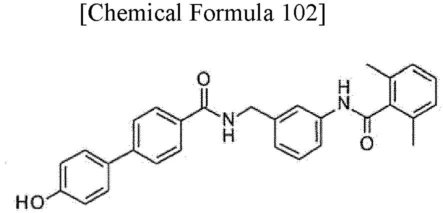

### Compound G0101

LRMS: m/z 451 [M+H]⁺
Retention time: 1.037 min (analysis condition FA05-1)

### Example 3-3-11: Synthesis of G0105-1R by amidation of compound F01-1R and 4-dimethylaminobenzoic acid (A05)

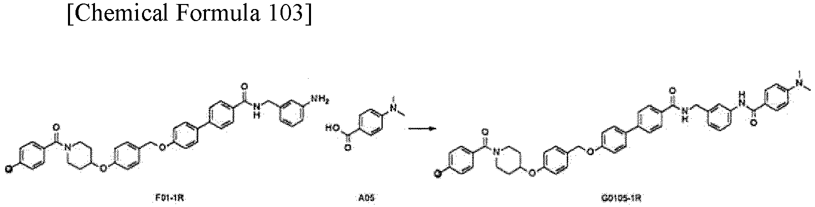

Under a nitrogen atmosphere, to a 0.6 mL glass vial, F01-1R (0.197 mmol/g, 20 mg, 97% purity) and DCM (0.4 mL) were added, and the mixture was shaken at room temperature for 1 hour. To this, 4-dimethylaminobenzoic acid (A05) (7.8 mg, 0.047 mmol) and N,N-dimethyl-p-toluidine (17.0 µL, 0.118 mmol) were added. To this, a mixed solution of PipClU (1.0 M, 0.039 mmol) and NMI (1.1 M, 0.043 mmol) in MeCN (39.4 µL) was added, and the mixture was shaken at room temperature for 90 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0105 was observed as 98%. It should be noted that the yield is calculated by setting the sum of the portions excluding the peaks derived from impurities originally contained in F01-1R (purity 97%) to 100%. The analysis was performed by extracting at the wavelength of 290 nm (± 4 nm).

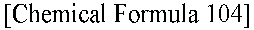

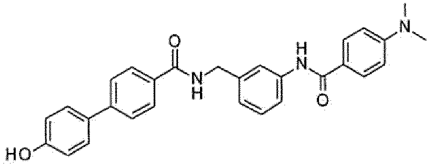

### Compound G0105

LRMS: m/z 466 [M+H]⁺
Retention time: 1.023 min (analysis condition FA05-1)

### Example 3-3-12: Synthesis of G0104-1R by amidation of compound F01-1R and 2-methyl-2-phenylpropanoic acid (A04)

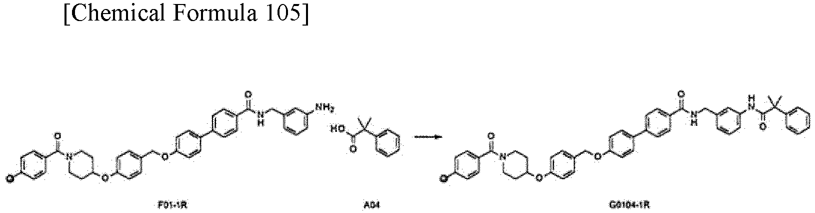

Under a nitrogen atmosphere, to a 0.6 mL glass vial, F01-1R (0.197 mmol/g, 20 mg, 97% purity) and DCM (0.4 mL) were added, and the mixture was shaken at room temperature for 1 hour. To this, 2-methyl-2-phenylpropanoic acid (A04) (7.8 mg, 0.047 mmol) and N,N-dimethyl-p-toluidine (17.0 µL, 0.118 mmol) were added. To this, a mixed solution of PipClU (1.0 M, 0.039 mmol) and NMI (1.1 M, 0.043 mmol) in MeCN (39.4 µL) was added, and the mixture was shaken at room temperature for 30 minutes.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0104 was observed as 100%. It should be noted that the yield is calculated by setting the sum of the portions excluding the peaks derived from impurities originally contained in F01-1R (purity 97%) to 100%. The analysis was performed by extracting at the wavelength of 290 nm (± 4 nm).

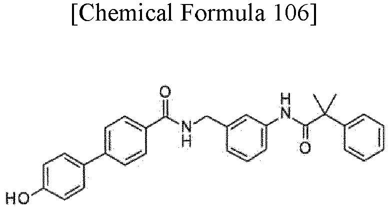

### Compound G0104

LRMS: m/z 465 [M+H]⁺
Retention time: 1.105 min (analysis condition FA05-1)

### Example 3-3-13: Synthesis of G0201-1R by amidation of compound F02-1R and 2,6-dimethylbenzoic acid (A01)

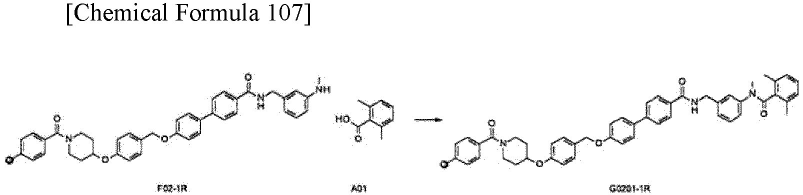

Under a nitrogen atmosphere, to a 0.6 mL glass vial, F02-1R (0.196 mmol/g, 20 mg, 98% purity) and DCM (0.4 mL) were added, and the mixture was shaken at room temperature for 1 hour. To this, 2,6-dimethylbenzoic acid (A01) (7.1 mg, 0.047 mmol) and N,N-dimethyl-p-toluidine (16.9 µL, 0.118 mmol) were added. To this, a mixed solution of PipClU (1.0 M, 0.039 mmol) and NMI (1.1 M, 0.043 mmol) in MeCN (39.2 µL) was added, and the mixture was shaken at room temperature for 90 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0201 was observed as 91%. It should be noted that the yield is calculated by setting the sum of the portions excluding the peaks derived from impurities originally contained in F02-1R (purity 98%) to 100%. The analysis was performed by extracting at the wavelength of 290 nm (± 4 nm).

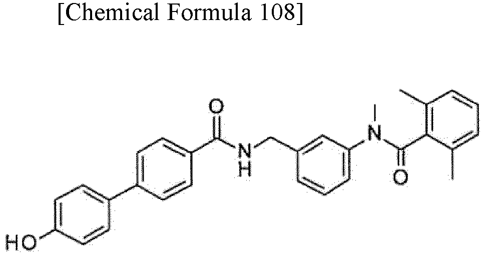

### Compound G0201

LRMS: m/z 465 [M+H]⁺
Retention time: 1.000 min, 1.060 min (bimodal) (analysis condition FA05-1)

### Example 3-3-14: Synthesis of G0205-1R by amidation of compound F02-1R and 4-dimethylaminobenzoic acid (A05)

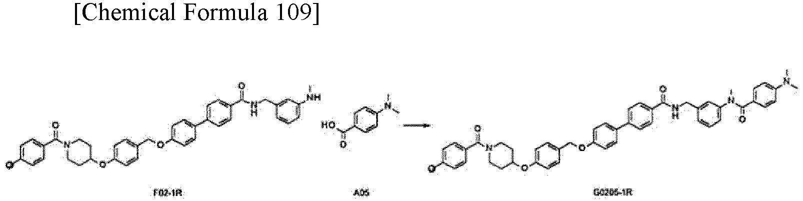

Under a nitrogen atmosphere, to a 0.6 mL glass vial, F02-1R (0.196 mmol/g, 20 mg, 98% purity) and DCM (0.4 mL) were added, and the mixture was shaken at room temperature for 1 hour. To this, 4-dimethylaminobenzoic acid (A05) (7.8 mg, 0.047 mmol) and N,N-dimethyl-p-toluidine (16.9 µL, 0.118 mmol) were added. To this, a mixed solution of PipClU (1.0 M, 0.039 mmol) and NMI (1.1 M, 0.043 mmol) in MeCN (39.2 µL) was added, and the mixture was shaken at room temperature for 90 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0205 was observed as 98%. It should be noted that the yield is calculated by setting the sum of the portions excluding the peaks derived from impurities originally contained in F02-1R (purity 98%) to 100%. The analysis was performed by extracting at the wavelength of 290 nm (± 4 nm).

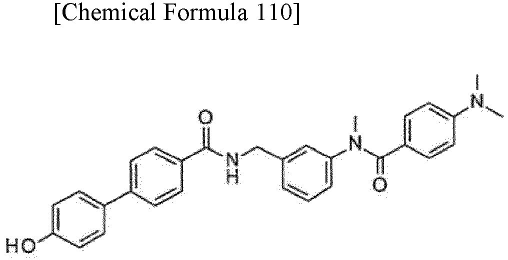

### Compound G0205

LRMS: m/z 480 [M+H]⁺
Retention time: 0.975 min (analysis condition FA05-1)

### Example 3-3-15: Synthesis of G0204-1R by amidation of compound F02-1R and 2-methyl-2-phenylpropanoic acid (A04)

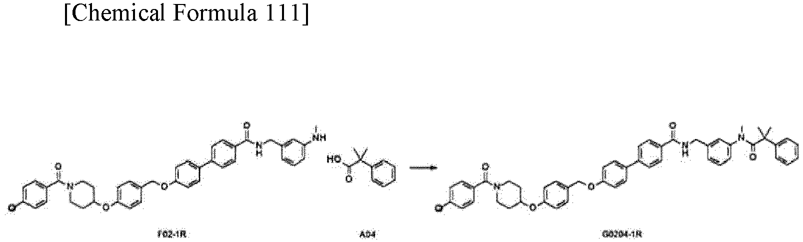

Under a nitrogen atmosphere, to a 0.6 mL glass vial, F02-1R (0.196 mmol/g, 20 mg, 98% purity) and DCM (0.4 mL) were added, and the mixture was shaken at room temperature for 1 hour. To this, 2-methyl-2-phenylpropanoic acid (A04) (7.7 mg, 0.047 mmol) and N,N-dimethyl-p-toluidine (16.9 µL, 0.118 mmol) were added. To this, a mixed solution of PipClU (1.0 M, 0.039 mmol) and NMI (1.1 M, 0.043 mmol) in MeCN (39.2 µL) was added, and the mixture was shaken at room temperature for 30 minutes.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0204 was observed as 99%. It should be noted that the yield is calculated by setting the sum of the portions excluding the peaks derived from impurities originally contained in F02-1R (purity 98%) to 100%. The analysis was performed by extracting at the wavelength of 290 nm (± 4 nm).

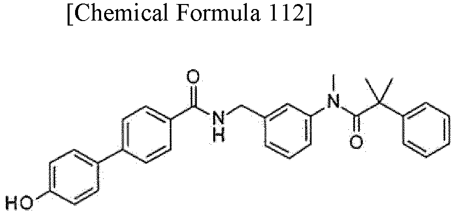

### Compound G0204

LRMS: m/z 479 [M+H]⁺
Retention time: 1.113 min (analysis condition FA05-1)

### Example 3-3-16: Synthesis of G0401-1R by amidation of compound F04-1R and 2,6-dimethylbenzoic acid (A01)

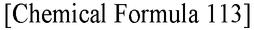

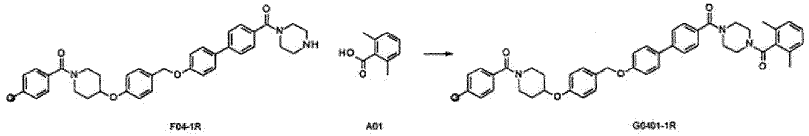

Under a nitrogen atmosphere, to a 0.6 mL glass vial, F04-1R (0.198 mmol/g, 20 mg) and DCM (0.4 mL) were added, and the mixture was shaken at room temperature for 1 hour. To this, 2,6-dimethylbenzoic acid (A01) (7.1 mg, 0.048 mmol) and N-methylmorpholine (13.1 µL, 0.119 mmol) were added. To this, a mixed solution of PipClU (1.0 M, 0.040 mmol) and NMI (1.1 M, 0.044 mmol) in MeCN (39.6 µL) was added, and the mixture was shaken at room temperature for 2 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0401 was observed as 95%. The analysis was performed by extracting at the wavelength of 290 nm (± 4 nm).

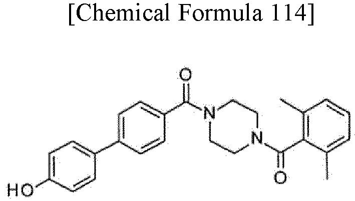

### Compound G0401

LRMS: m/z 415 [M+H]⁺
Retention time: 0.941 min (analysis condition FA05-1)

### Example 3-3-17: Synthesis of G0405-1R by amidation of compound F04-1R and 4-dimethylaminobenzoic acid (A05)

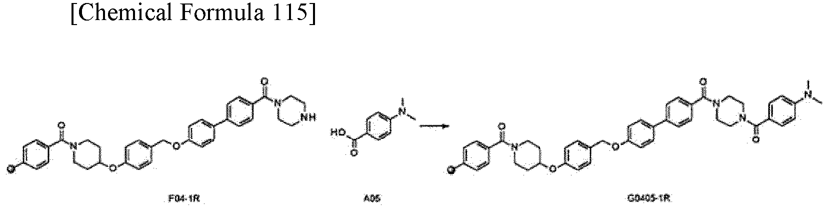

Under a nitrogen atmosphere, to a 0.6 mL glass vial, F04-1R (0.198 mmol/g, 20 mg) and DCM (0.4 mL) were added, and the mixture was shaken at room temperature for 1 hour. To this, 4-dimethylaminobenzoic acid (A05) (7.9 mg, 0.048 mmol) and N-methylmorpholine (13.1 µL, 0.119 mmol) were added. To this, a mixed solution of PipClU (1.0 M, 0.040 mmol) and NMI (1.1 M, 0.044 mmol) in MeCN (39.6 µL) was added, and the mixture was shaken at room temperature for 2 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0405 was observed as 98%. The analysis was performed by extracting at the wavelength of 290 nm (± 4 nm).

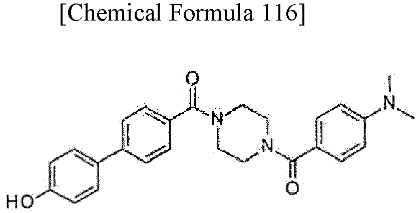

### Compound G0405

LRMS: m/z 430 [M+H]⁺
Retention time: 0.885 min (analysis condition FA05-1)

### Example 3-3-18: Synthesis of G0404-1R by amidation of compound F04-1R and 2-methyl-2-phenylpropanoic acid (A04)

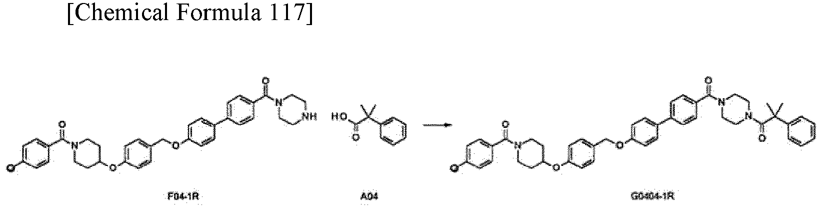

Under a nitrogen atmosphere, to a 0.6 mL glass vial, F04-1R (0.198 mmol/g, 20 mg) and DCM (0.4 mL) were added, and the mixture was shaken at room temperature for 1 hour. 2-methyl-2-phenylpropanoic acid (A04) (7.8 mg, 0.048 mmol) and N-methylmorpholine (13.1 µL, 0.119 mmol) were added. To this, a mixed solution of PipClU (1.0 M, 0.040 mmol) and NMI (1.1 M, 0.044 mmol) in MeCN (39.6 µL) was added, and the mixture was shaken at room temperature for 30 minutes.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0404 was observed as 100%. The analysis was performed by extracting at the wavelength of 290 nm (± 4 nm).

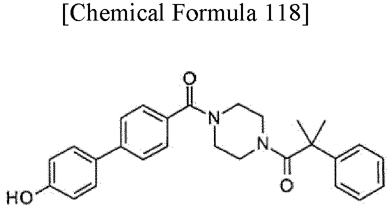

### Compound G0404

LRMS: m/z 429 [M+H]⁺
Retention time: 1.027 min (analysis condition FA05-1)

### Example 3-3-19: Synthesis of E1202-1R by amidation of compound D12-1R and aniline (B02)

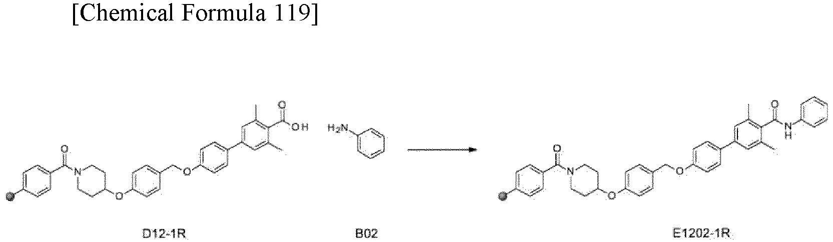

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, aniline (B02) (1.8 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1202 was observed as 96%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

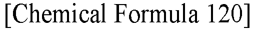

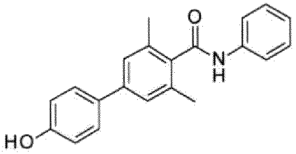

### Compound E1202

LRMS: m/z 318 [M+H]⁺
Retention time: 1.065 min (analysis condition FA05-1)

### Example 3-3-20: Synthesis of E1216-1R by amidation of compound D12-1R and N-methylaniline (B16)

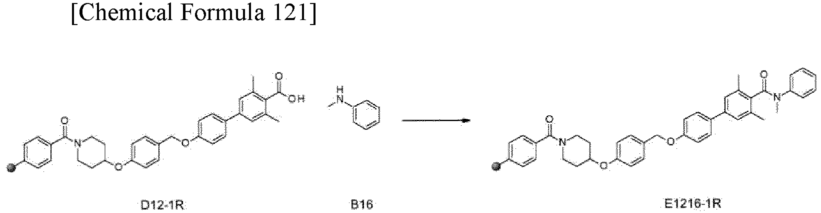

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, N-methylaniline (B16) (2.0 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1216 was observed as 95%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

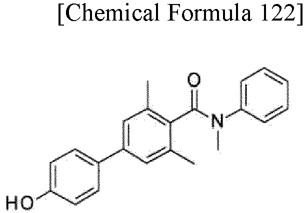

### Compound E1216

LRMS: m/z 332 [M+H]⁺
Retention time: 1.023 min, 1.045 min (bimodal) (analysis condition FA05-1).

### Example 3-3-21: Synthesis of E1217-1R by amidation of compound D12-1R and 2-methylaniline (B17)

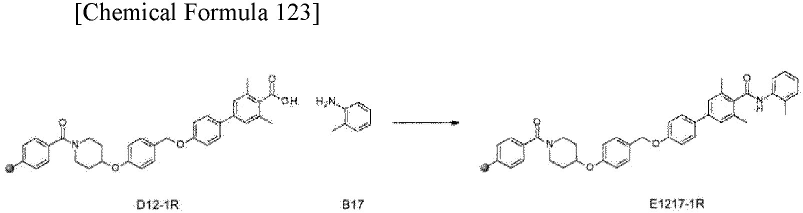

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 2-methylaniline (B17) (2.0 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1217 was observed as 90%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

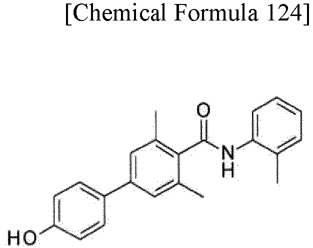

### Compound E1217

LRMS: m/z 332 [M+H]⁺
Retention time: 1.065 min (analysis condition FA05-1)

### Example 3-3-22: Synthesis of E1219-1R by amidation of compound D12-1R and 2,6-dimethylaniline (B19)

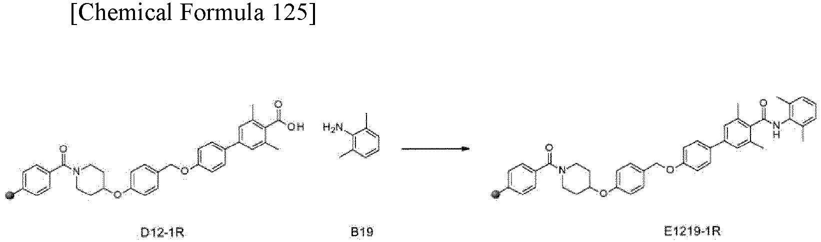

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 2,6-dimethylaniline (B19)(2.3 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1219 was observed as 90%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

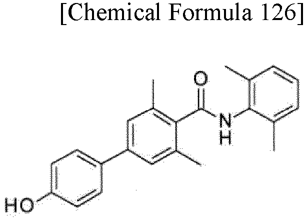

### Compound E1219

LRMS: m/z 346 [M+H]⁺
Retention time: 1.081 min (analysis condition FA05-1)

### Example 3-3-23: Synthesis of E1220-1R by amidation of compound D12-1R with N-propan-2-ylaniline (B20)

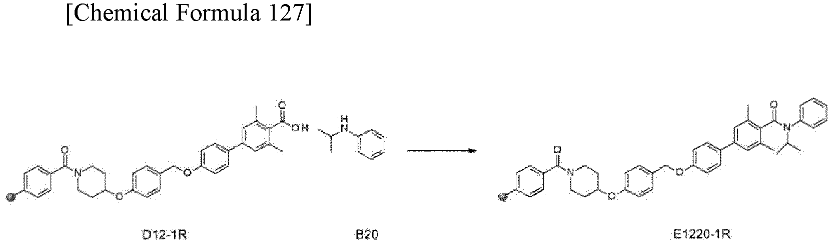

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, N-propan-2-ylaniline (B20) (2.6 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1220 was observed as 88%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

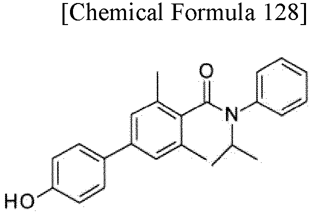

### Compound E1220

LRMS: m/z 360 [M+H]⁺
Retention time: 1.135 min (analysis condition FA05-1)

### Example 3-3-24: Synthesis of E1221-1R by amidation of Compound D12-1R and 2-tert-butyl-N-methylaniline (B21)

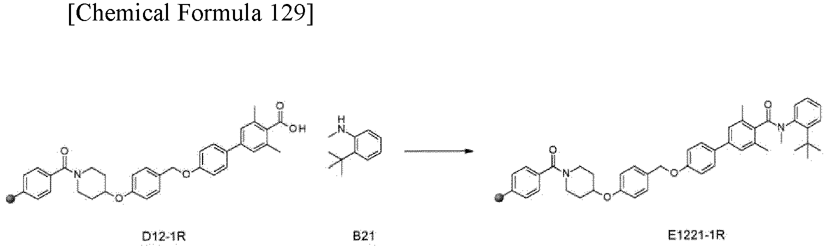

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 2-tert-butyl-N-methylaniline (B21) (3.1 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1221 was observed as 87%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

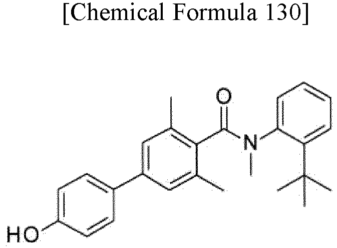

### Compound E1221

LRMS: m/z 388 [M+H]⁺
Retention time: 1.281 min (analysis condition FA05-1)

### Example 3-3-25: Synthesis of E1222-1R by amidation of compound D12-1R and 4-(methylamino)benzonitrile (B22)

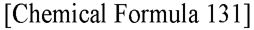

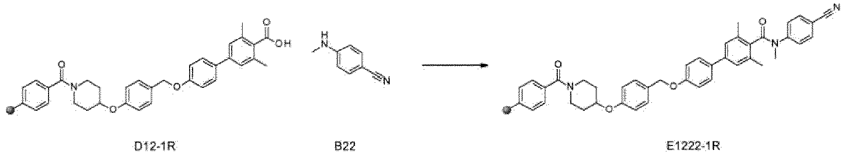

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 4-(methylamino)benzonitrile (B22) (2.5 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1222 was observed as 62%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

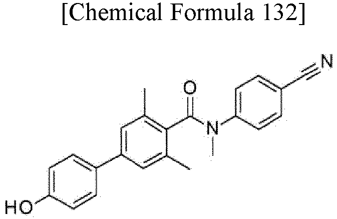

### Compound E1222

LRMS: m/z 357 [M+H]⁺
Retention time: 2.033 min (analysis condition FA05-long)

### Example 3-3-26: Synthesis of E1223-1R by amidation of compound D12-1R and 2,6-diethylaniline (B23)

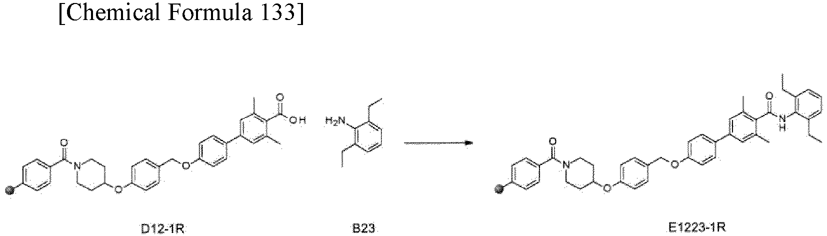

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 2,6-diethylaniline (B23)(2.8 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1223 was observed as 91%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

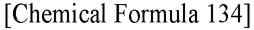

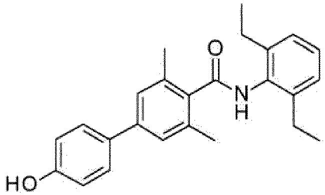

### Compound E1223

LRMS: m/z 374 [M+H]⁺
Retention time: 1.176 min (analysis condition FA05-1)

### Example 3-3-27: Synthesis of E1224-1R by amidation of compound D12-1R and 4-(ethylamino)benzonitrile (B24)

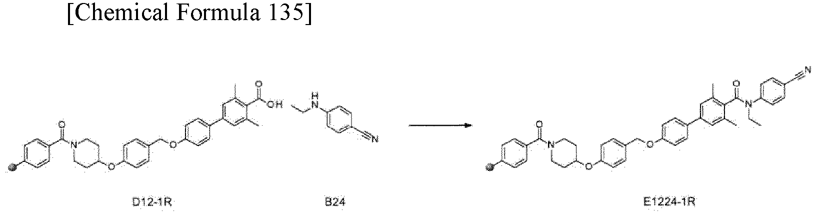

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 4-(ethylamino)benzonitrile (B24) (2.8 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1224 was observed as 54%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

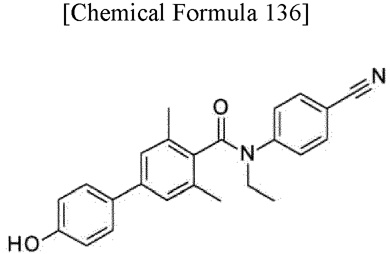

### Compound E1224

LRMS: m/z 371 [M+H]⁺
Retention time: 1.043 min (analysis condition FA05-1)

### Example 3-3-28: Synthesis of E1225-1R by amidation of Compound D12-1R and 4-(propan-2-ylamino)benzonitrile (B25)

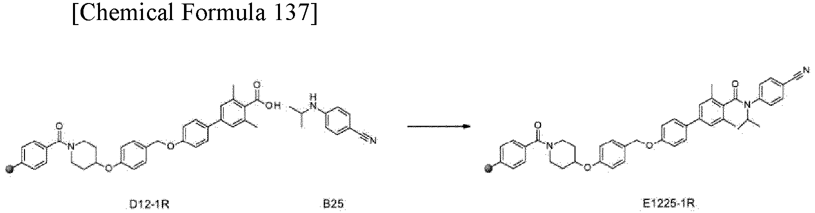

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 4-(propan-2-ylamino)benzonitrile (B25) (3.0 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1225 was observed as 15%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

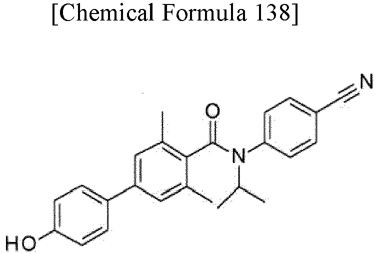

### Compound E1225

LRMS: m/z 385 [M+H]⁺
Retention time: 2.269 min (analysis condition FA05-long)

### Example 3-3-29: Synthesis of E1226-1R by amidation of compound D12-1R and 4-amino-3-methylbenzonitrile (B26)

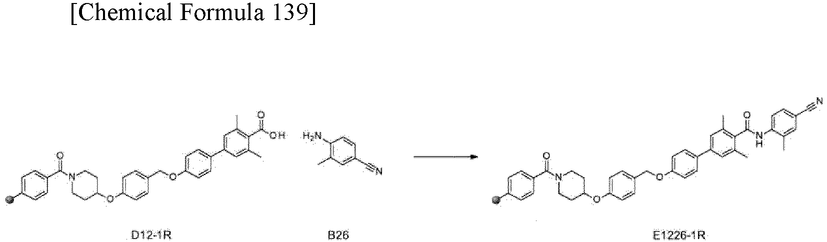

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 4-amino-3-methylbenzonitrile (B26) (2.5 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1226 was observed as 66%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

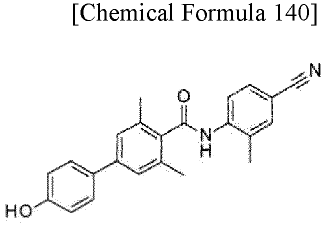

### Compound E1226

LRMS: m/z 357 [M+H]⁺
Retention time: 1.055 min (analysis condition FA05-1)

### Example 3-3-30: Synthesis of E1227-1R by amidation of compound D12-1R and N-methyl-4-(trifluoromethyl)aniline (B27)

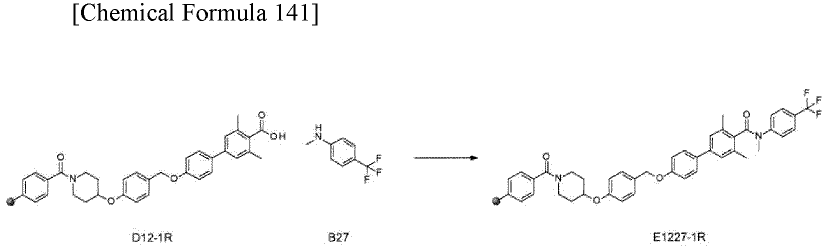

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, N-methyl-4-(trifluoromethyl)aniline (B27) (3.3 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1227 was observed as 83%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

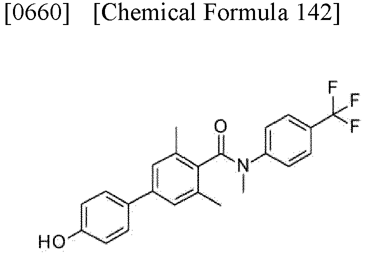

### Compound E1227

LRMS: m/z 400 [M+H]⁺
Retention time: 1.153 min (analysis condition FA05-1)

### Example 3-3-31: Synthesis of E1206-1R by amidation of compound D12-1R and N-methyl-4-nitroaniline (B06)

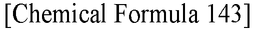

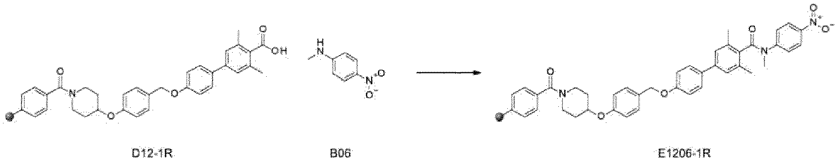

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, N-methyl-4-nitroaniline (B06) (2.9 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1206 was observed as 58%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

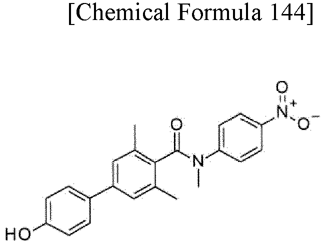

### Compound E1206

LRMS: m/z 377 [M+H]⁺
Retention time: 1.051 min (analysis condition FA05-1)

### Example 3-3-32: Synthesis of E1228-1R by amidation of compound D12-1R and N-ethyl-4-nitroaniline (B28)

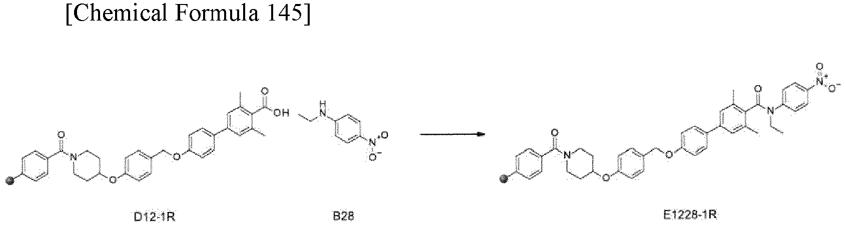

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, N-ethyl-4-nitroaniline (B28) (3.2 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1228 was observed as 34%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

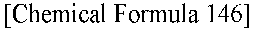

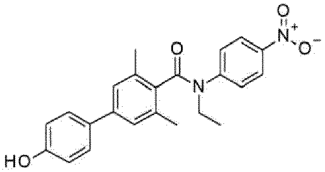

### Compound E1228

LRMS: m/z 391 [M+H]⁺
Retention time: 1.093 min (analysis condition FA05-1)

### Example 3-3-33: Synthesis of E1218-1R by amidation of compound D12-1R with 4-aminobenzonitrile (B18)

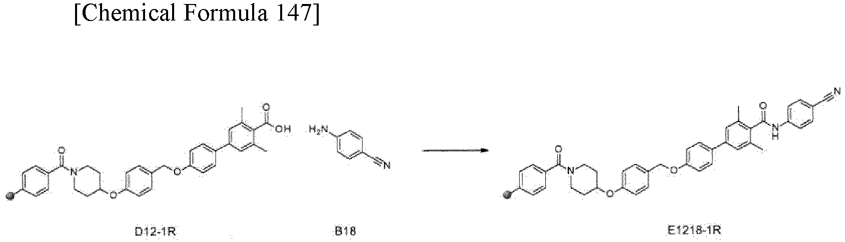

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 4-aminobenzonitrile (B18) (2.2 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1218 was observed as 92%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

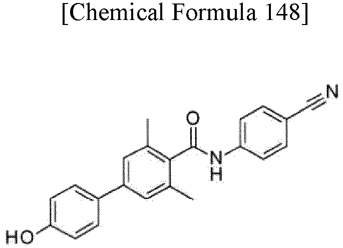

### Compound E1218

LRMS: m/z 343 [M+H]⁺
Retention time: 1.052 min (analysis condition FA05-1)

### Example 3-3-34: Synthesis of E1203-1R by amidation of compound D12-1R and 2,6-di(propan-2-yl)aniline (B03)

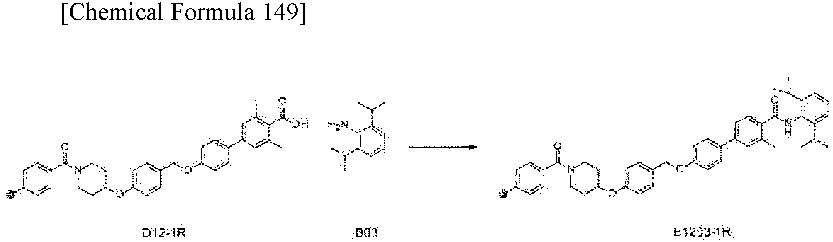

Under a nitrogen atmosphere, to a 0.6 mL glass vial, D12-1R (0.190 mmol/g, 10 mg, 96% purity) and DCM (0.2 mL) were added, and the mixture was shaken at room temperature for 1 hour. To this, 2,6-di(propan-2-yl)aniline (B03) (3.4 mg, 0.019 mmol) was added. To this, a mixed solution of PipClU (1.0 M, 0.0038 mmol) and NMI (1.0 M, 0.0038 mmol) in MeCN (3.8 µL) was added, and the mixture was shaken at room temperature for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, E1203 was observed as 79%. It should be noted that the yield is calculated by setting the sum of the portions excluding the peaks derived from impurities originally contained in D12-1R (purity 96%) and the peak of pentamethylbenzene to 100%. The analysis was performed by extracting at the wavelength of 270 nm (± 4 nm).

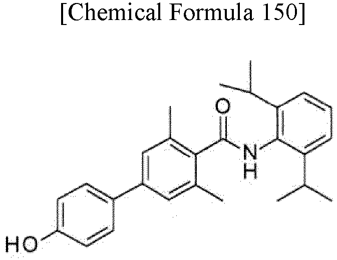

### Compound E1203

LRMS: m/z 402 [M+H]⁺
Retention time: 1.247 min (analysis condition FA05-1)

### Example 3-3-35: Synthesis of G0607-1R by amidation of compound F06-1R and 2-methylbenzoic acid (A07)

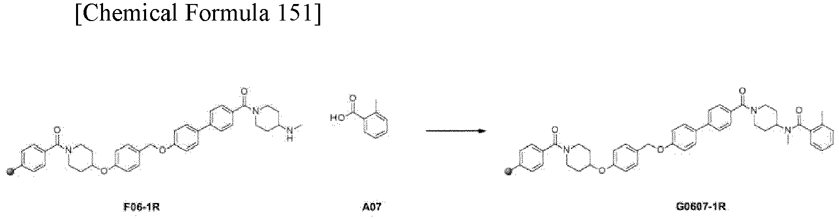

Under a nitrogen atmosphere, to a 0.6 mL glass vial, F06-1R (0.197 mmol/g, 10 mg, 99% purity) and DCM (0.15 mL) were added, and the mixture was shaken at room temperature for 1 hour. To this, a solution of 2-methylbenzoic acid (A07) (0.3 M, 0.012 mmol) in THF (39.5 µL) was added. To this, a solution of PipClU (1.0 M, 0.0099 mmol) and NMI (1.0 M, 0.0099 mmol) in MeCN (9.87 µL) and DIPEA (2.068 µL, 0.012 mmol) were added, and the mixture was shaken at 40°C for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0607 was observed as 79%. The analysis was performed by extracting at the wavelength of 299 nm (± 4 nm).

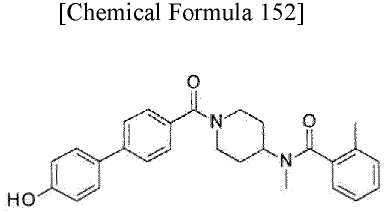

### Compound G0607

LRMS: m/z 429 [M+H]⁺
Retention time: 0.932 min (analysis condition FA05-1)

### Example 3-3-36: Synthesis of G0608-1R by amidation of compound F06-1R and 4-methoxybenzoic acid (A08)

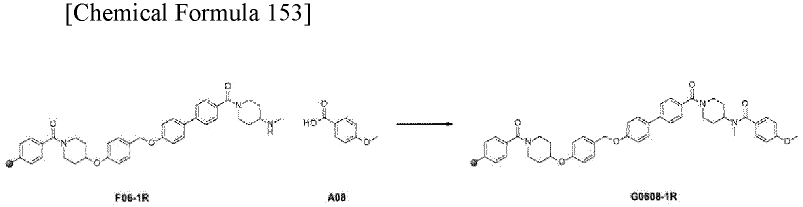

Under a nitrogen atmosphere, to a 0.6 mL glass vial, F06-1R (0.197 mmol/g, 10 mg, 99% purity) and DCM (0.15 mL) were added, and the mixture was shaken at room temperature for 1 hour. To this, a solution of 4-methoxybenzoic acid (A08) (0.3 M, 0.012 mmol) in THF (39.5 µL) was added. To this, a solution of PipClU (1.0 M, 0.0099 mmol) and NMI (1.0 M, 0.0099 mmol) in MeCN (9.87 µL) and DIPEA (2.068 µL, 0.012 mmol) were added, and the mixture was shaken at 40°C for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0608 was observed as 100%. It should be noted that the yield is calculated by setting the sum of the portions excluding the peaks derived from the identifiable impurities to 100%. The analysis was performed by extracting at the wavelength of 299 nm (± 4 nm).

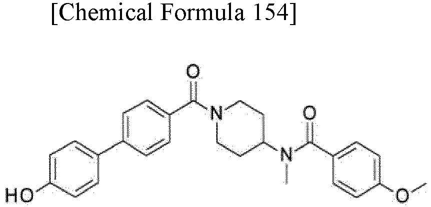

### Compound G0608

LRMS: m/z 445 [M+H]⁺
Retention time: 0.908 min (analysis condition FA05-1)

### Example 3-3-37: Synthesis of G0609-1R by amidation of compound F06-1R and (2S)-2-[9H-fluoren-9-ylmethoxycarbonyl(methyl)amino]-3-methylbutanoic acid (A09)

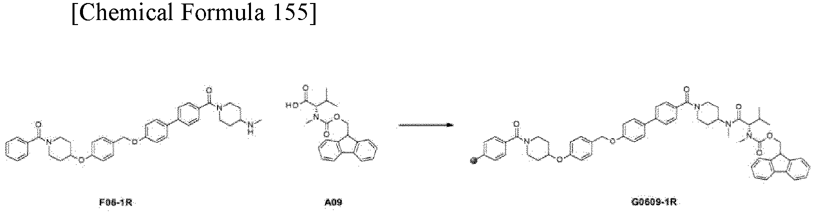

Under a nitrogen atmosphere, to a 0.6 mL glass vial, F06-1R (0.197 mmol/g, 10 mg, 99% purity) and DCM (0.15 mL) were added, and the mixture was shaken at room temperature for 1 hour. To this, a solution of (2S)-2-[9H-fluoren-9-ylmethoxycarbonyl(methyl)amino]-3-methylbutanoic acid (A09)(0.3 M, 0.012 mmol) in THF (39.5 µL) was added. To this, a solution of PipClU (1.0 M, 0.0099 mmol) and NMI (1.0 M, 0.0099 mmol) in MeCN (9.87 µL) and DIPEA (2.068 µL, 0.012 mmol) were added, and the mixture was shaken at 40°C for 24 hours.

12 µL of the suspension of the reaction solution and the solid phase was transferred onto a tip equipped with a filter, and washed 5 times with DMF (0.1 mL), 5 times with MeOH (0.1 mL), and 5 times with DCM (0.1 mL). The resultant was immersed in a 10% TFA/DCM solution of pentamethylbenzene (0.1 M, 0.05 mL) for 1 minute and filtered, and then washed with DMF (0.05 mL). The filtrates were combined and diluted with MeCN (0.25 mL), and then subjected to LCMS measurement to measure the progress of the reaction. As a result, G0609 was observed as 89%. It should be noted that the yield is calculated by setting the sum of the portions excluding the peaks derived from the identifiable impurities to 100%. The analysis was performed by extracting at the wavelength of 299 nm (± 4 nm).

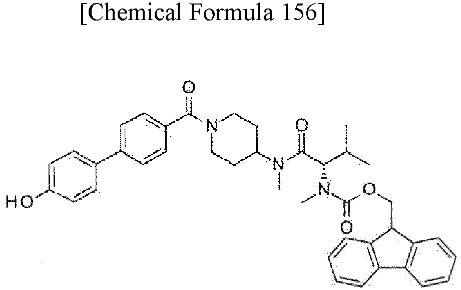

### Compound G0609

LRMS: m/z 646 [M+H]⁺
Retention time: 1.271 min (analysis condition FA05-1)

### Example 4: Application of amidation conditions to mixture

### Example 4-1: Synthesis and confirmation of production of mixture 2-2-c1D01-0 by amidation reaction of mixture 2-2-D00-0

### Example 4-1-1: Synthesis of mixture 2-2-c1D01-0 by amidation reaction of mixture 2-2-D00-0

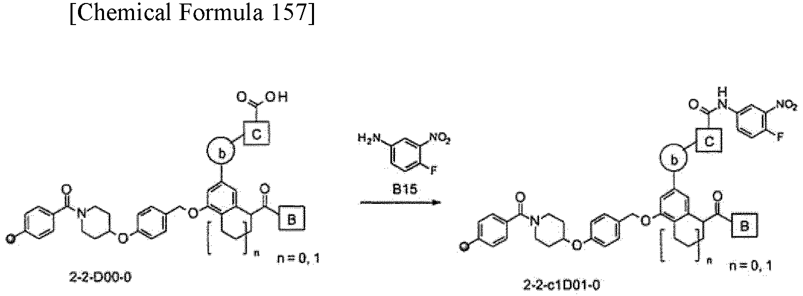

The compounds that may be contained in the mixture 2-2-D00-0 that was separately prepared are as shown in Table 4-1-1-1. In Table 4-1-1-1, each compound that may be contained in the mixture is represented by ID, and the structure is represented by indicating the corresponding n-number and the combination of part B, part b, and part c in the structural formula of the mixture by symbols.

Herein, the correspondence of symbols in parts B, b, C and c, and the structural formulas is shown below.

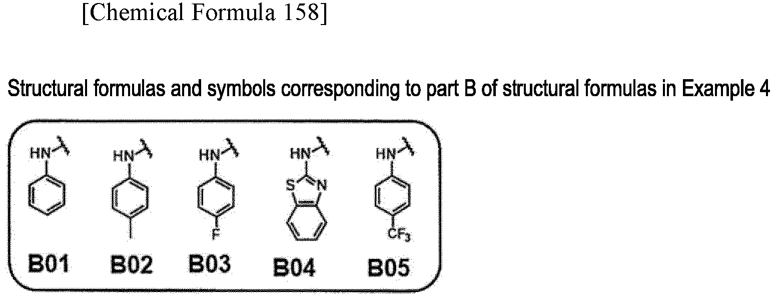

Structural formulas and symbols corresponding to part B of structural formulas in Example 4

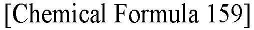

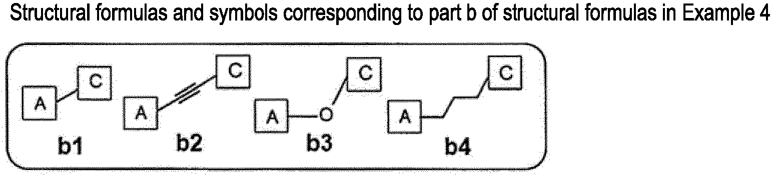

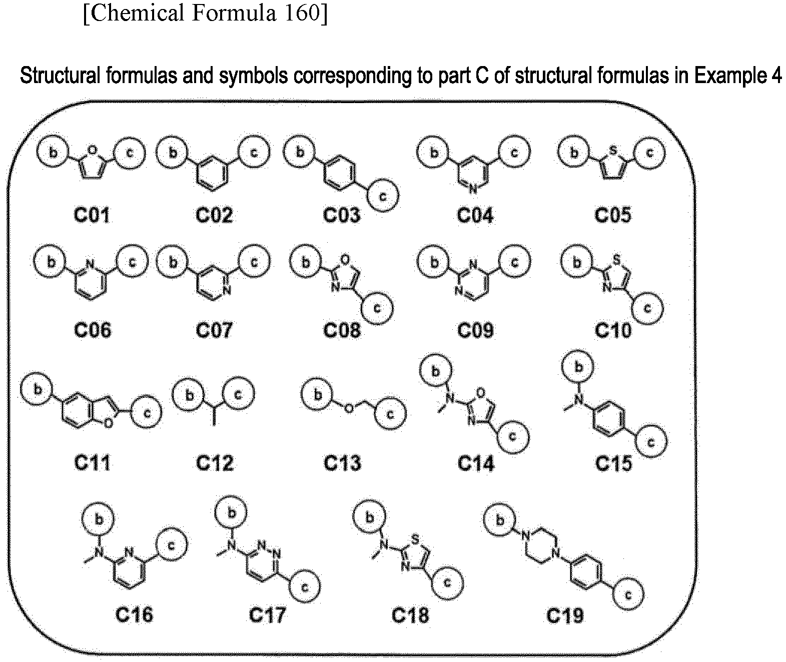

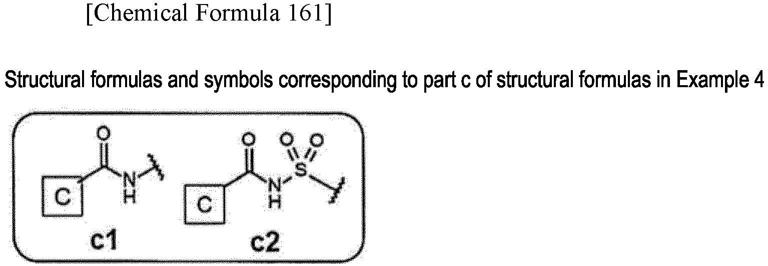

For example, compound 2-2-D00-0-0001 has the n-number of 0, part B of B01, part b of b 1, and part C of C01, and is represented by the following structure.

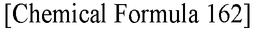

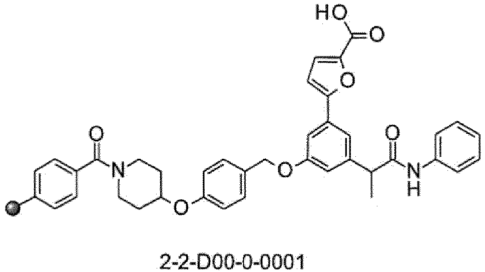

For example, compound 2-2-D00-0-0049 has the n-number of 1, part B of B02, part b of b4, and part C of C12, and is represented by the following structure.

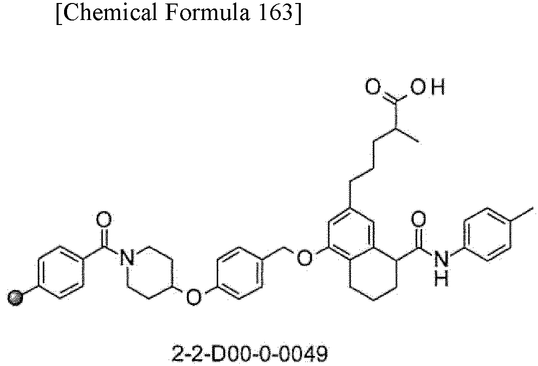

Twenty-five mixtures were added to a 20 mL glass vial to prepare mixture 2-2-D00-0 (800 mg), then DCM (12.0 mL) was added, and the mixture was shaken at room temperature for 1 hour. To this, 4-fluoro-3-nitroaniline (B15) (0.242 g, 1.55 mmol) was added. To this, a mixed solution of PipClU (0.224 g, 0.621 mmol) and NMI (0.049 mL, 0.621 mmol) in MeCN (0.50 mL) was added, and the mixture was shaken at room temperature for 17 hours.

The suspension of the reaction solution and solid phase was transferred to a column equipped with a filter, and filtered, and then washed 3 times with NMP/water = 1/1 (16 mL), 3 times with a mixed solution of tetrabutylammonium hydrogen sulfate and 2,6-di-tert-butyl pyridine in NMP (both 0.05 M, 16 mL), 3 times with a solution of 4-methylmorpholine in NMP (0.05 M, 16 mL), 3 times with NMP/water = 1/1 (16 mL), 3 times with NMP (16 mL), 3 times with MeOH (16 mL), 3 times with DCM (16 mL), and 3 times with heptane (16 mL). The resulting solid phase was dried under reduced pressure to obtain mixture 2-2-c1D01-0.

The compounds that may be contained in the mixture 2-2-c1D01-0 are as shown in Table 4-1-1-2. In Table 4-1-1-2, each compound that may be contained in the mixture is represented by ID, and the structure is represented by indicating the corresponding n-number and the combination of part B, part b, and part c in the structural formula of the mixture by symbols. The correspondence of symbols in parts B, b, and C, and the structural formulas is as shown above.

**[Table 13]**

| [Table 4-1-1-1] Compounds that may be contained in mixture 2-2-D00-0 | | | | |
|---|---|---|---|---|
| ID | n-number | B | b | C |
| 2-2-D00-0-0001 | 0 | B01 | b1 | C01 |
| 2-2-D00-0-0002 | 0 | B01 | b4 | C12 |
| 2-2-D00-0-0003 | 0 | B01 | b4 | C13 |
| 2-2-D00-0-0004 | 0 | B01 | b1 | C02 |
| 2-2-D00-0-0005 | 0 | B01 | b1 | C03 |
| 2-2-D00-0-0006 | 0 | B01 | b1 | C04 |
| 2-2-D00-0-0007 | 0 | B02 | b1 | C01 |
| 2-2-D00-0-0008 | 0 | B01 | b1 | C05 |
| 2-2-D00-0-0009 | 0 | 801 | b3 | C08 |
| 2-2-D00-0-0010 | 0 | B03 | b1 | C01 |
| 2-2-D00-0-0011 | 0 | B02 | b4 | C12 |
| 2-2-D0-0-0012 | 0 | B02 | b4 | C13 |
| 2-2-D00-0-0013 | 0 | B03 | b4 | C12 |
| 2-2-D00-0-0014 | 0 | B02 | bl | C02 |
| 2-2-D00-0-0015 | 0 | B02 | b1 | C03 |
| 2-2-D00-0-0016 | 0 | B03 | b4 | C13 |
| 2-2-D00-0-0017 | 0 | B02 | b1 | 004 |
| 2-2-D00-0-0018 | 1 | B01 | b1 | C01 |
| 2-2-D00-0-0019 | 0 | B01 | b3 | C09 |
| 2-2-D00-0-0020 | 0 | B03 | b1 | C02 |
| 2-2-D00-0-0021 | 0 | B03 | b1 | C03 |
| 2-2-D00-0-0022 | 0 | B03 | b1 | C04 |
| 2-2-D00-0-0023 | 0 | B02 | b1 | C05 |
| 2-2-D00-0-0024 | 0 | B01 | b1 | C14 |
| 2-2-D00-0-0025 | 1 | B01 | b4 | C12 |
| 2-2-D00-0-0026 | 0 | B02 | b3 | C08 |
| 2-2-D00-0-0027 | 1 | B01 | b4 | C13 |
| 2-2-D00-0-0028 | 0 | B01 | b3 | C10 |
| 2-2-D00-0-0029 | 0 | B03 | b1 | C05 |
| 2-2-D00-0-0030 | 0 | B01 | b2 | C02 |

**[Table 14]**

| | | | | |
|---|---|---|---|---|
| 2-2-D00-0-0031 | 0 | B01 | b2 | C03 |
| 2-2-D00-0-0032 | 0 | B03 | b3 | C08 |
| 2-2-D00-0-0033 | 0 | B01 | b2 | C04 |
| 2-2-D00-0-0034 | 0 | B01 | b2 | C06 |
| 2-2-D00-0-0035 | 0 | B01 | b2 | C07 |
| 2-2-D00-0-0036 | 1 | B01 | b1 | C02 |
| 2-2-D00-0-0037 | 1 | BO1 | b1 | C03 |
| 2-2-D00-0-0038 | 1 | B01 | b1 | C04 |
| 2-2-D00-0-0039 | 0 | B01 | b1 | C15 |
| 2-2-D00-0-0040 | 1 | B02 | b1 | C01 |
| 2-2-D00-0-0041 | 0 | B01 | b1 | C16 |
| 2-2-D00-0-0042 | 0 | B01 | b1 | C17 |
| 2-2-D00-0-0043 | 1 | B01 | b1 | C05 |
| 2-2-D00-0-0044 | 0 | B02 | b3 | C09 |
| 2-2-D00-0-0045 | 0 | B01 | b4 | C01 |
| 2-2-D00-0-0046 | 1 | B01 | b3 | C08 |
| 2-2-D00-0-0047 | 1 | B03 | b1 | C01 |
| 2-2-D00-0-0048 | 0 | B02 | b1 | C14 |
| 2-2-D00-0-0049 | 1 | B02 | b4 | C12 |
| 2-2-D00-0-0050 | 0 | B03 | b3 | C09 |
| 2-2-D00-0-0051 | 0 | B01 | b1 | C18 |
| 2-2-D00-0-0052 | 1 | B02 | b4 | C13 |
| 2-2-D00-0-0053 | 0 | B02 | b3 | C10 |
| 2-2-D00-0-0054 | 0 | B03 | b1 | C14 |
| 2-2-D00-0-0055 | 0 | B02 | b2 | C02 |
| 2-2-D00-0-0056 | 0 | B02 | b2 | C03 |
| 2-2-D00-0-0057 | 1 | B03 | b4 | C12 |
| 2-2-D00-0-0058 | 0 | B02 | b2 | C04 |
| 2-2-D00-0-0059 | 0 | B02 | b2 | C06 |
| 2-2-D00-0-0060 | 0 | B02 | b2 | C07 |
| 2-2-D00-0-0061 | 1 | B02 | b1 | C02 |
| 2-2-D00-0-0062 | 1 | B02 | b1 | C03 |
| 2-2-D00-0-0063 | 1 | B03 | b4 | C13 |
| 2-2-D00-0-0064 | 0 | B03 | b3 | C10 |

**[Table 15]**

| | | | | |
|---|---|---|---|---|
| 2-2-D00-0-0065 | 1 | B02 | b1 | C04 |
| 2-2-D00-0-0066 | 0 | B03 | b2 | C02 |
| 2-2-D00-0-0067 | 0 | B03 | b2 | C03 |
| 2-2-D00-0-0068 | 0 | B01 | b4 | C02 |
| 2-2-D00-0-0069 | 0 | B03 | b2 | C04 |
| 2-2-D00-0-0070 | 0 | B03 | b2 | C06 |
| 2-2-D00-0-0071 | 0 | B03 | b2 | C07 |
| 2-2-D00-0-0072 | 0 | B02 | b1 | C15 |
| 2-2-D00-0-0073 | 1 | B01 | b3 | C09 |
| 2-2-D00-0-0074 | 1 | B03 | b1 | C02 |
| 2-2-D00-0-0075 | 1 | B03 | bl | C03 |
| 2-2-D00-0-0076 | 0 | B02 | b1 | C16 |
| 2-2-D00-0-0077 | 1 | B03 | b1 | C04 |
| 2-2-D00-0-0078 | 0 | B02 | b1 | C17 |
| 2-2-D00-0-0079 | 1 | B02 | b1 | C05 |
| 2-2-D00-0-0080 | 1 | B01 | b1 | C14 |
| 2-2-D00-0-0081 | 0 | B02 | b4 | C01 |
| 2-2-D00-0-0082 | 0 | B04 | b1 | C01 |
| 2-2-D00-0-0083 | 1 | B02 | b3 | C08 |
| 2-2-D00-0-0084 | 0 | B03 | b1 | C15 |
| 2-2-D00-0-0085 | 0 | B01 | b4 | C05 |
| 2-2-D00-0-0086 | 0 | B03 | b1 | C16 |
| 2-2-D00-0-0087 | 1 | B01 | b3 | C10 |
| 2-2-D00-0-0088 | 0 | B03 | b1 | C17 |
| 2-2-D00-0-0089 | 1 | B03 | b1 | C05 |
| 2-2-D00-0-0090 | 0 | B02 | b1 | C18 |
| 2-2-D00-0-0091 | 1 | B01 | b2 | C02 |
| 2-2-D00-0-0092 | 1 | B01 | b2 | C03 |
| 2-2-D00-0-0093 | 0 | B03 | b4 | C01 |
| 2-2-D00-0-0094 | 1 | B03 | b3 | C08 |
| 2-2-D00-0-0095 | 1 | B01 | b2 | C04 |
| 2-2-D00-0-0096 | 1 | B01 | b2 | C06 |
| 2-2-D00-0-0097 | 1 | B01 | b2 | C07 |
| 2-2-D00-0-0098 | 0 | B04 | b4 | C12 |

**[Table 16]**

| | | | | |
|---|---|---|---|---|
| 2-2-D00-0-0099 | 0 | B04 | b4 | C13 |
| 2-2-D00-0-0100 | 0 | B03 | b1 | C18 |
| 2-2-D00-0-0101 | 1 | B01 | b1 | C15 |
| 2-2-D00-0-0102 | 0 | B01 | b3 | C11 |
| 2-2-D00-0-0103 | 1 | B01 | b1 | C16 |
| 2-2-D00-0-0104 | 0 | B02 | b4 | C02 |
| 2-2-D00-0-0105 | 0 | B04 | b1 | C02 |
| 2-2-D00-0-0106 | 0 | B04 | b1 | C03 |
| 2-2-D00-0-0107 | 1 | B01 | b1 | C17 |
| 2-2-D00-0-0108 | 0 | B04 | b1 | C04 |
| 2-2-D00-0-0109 | 0 | B05 | b1 | C01 |
| 2-2-D00-0-0110 | 1 | B02 | b3 | C09 |
| 2-2-D00-0-0111 | 1 | B01 | b4 | C01 |
| 2-2-D00-0-0112 | 1 | B02 | b1 | C14 |
| 2-2-D00-0-0113 | 0 | BOS | b4 | C02 |
| 2-2-D00-0-0114 | 1 | B03 | b3 | C09 |
| 2-2-D00-0-0115 | 1 | B01 | b1 | C18 |
| 2-2-D00-0-0116 | 0 | B02 | b4 | C05 |
| 2-2-D00-0-0117 | 0 | B05 | b4 | C12 |
| 2-2-D00-0-0118 | 0 | B04 | b1 | C05 |
| 2-2-D00-0-0119 | 1 | B02 | b3 | C10 |
| 2-2-D00-0-0120 | 0 | B04 | b3 | C08 |
| 2-2-D00-0-0121 | 1 | B03 | b1 | C14 |
| 2-2-D00-0-0122 | 0 | B05 | b4 | C13 |
| 2-2-D00-0-0123 | 1 | B02 | b2 | C02 |
| 2-2-D00-0-0124 | 1 | B02 | b2 | C03 |
| 2-2-D00-0-0125 | 1 | B02 | b2 | C04 |
| 2-2-D00-0-0126 | 1 | B02 | b2 | C06 |
| 2-2-D00-0-0127 | 1 | B02 | b2 | C07 |
| 2-2-D00-0-0128 | 0 | B03 | b4 | C05 |
| 2-2-D00-0-0129 | 1 | B03 | b3 | C10 |
| 2-2-D00-0-0130 | 0 | B05 | b1 | C02 |
| 2-2-D00-0-0131 | 0 | B05 | b1 | C03 |
| 2-2-D00-0-0132 | 1 | B03 | b2 | C02 |

**[Table 17]**

| | | | | |
|---|---|---|---|---|
| 2-2-D00-0-0133 | 1 | B03 | b2 | C03 |
| 2-2-D00-0-0134 | 1 | B01 | b4 | C02 |
| 2-2-D00-0-0135 | 0 | B05 | b1 | C04 |
| 2-2-D00-0-0136 | 1 | B03 | b2 | C04 |
| 2-2-D00-0-0137 | 1 | B03 | b2 | C06 |
| 2-2-D00-0-0138 | 1 | B03 | b2 | C07 |
| 2-2-D00-0-0139 | 1 | B02 | b1 | C15 |
| 2-2-D00-0-0140 | 0 | B02 | b3 | C11 |
| 2-2-D00-0-0141 | 1 | B02 | b1 | C16 |
| 2-2-D00-0-0142 | 1 | B02 | b1 | C17 |
| 2-2-D00-0-0143 | 1 | B02 | b4 | C01 |
| 2-2-D00-0-0144 | 1 | B04 | b1 | C01 |
| 2-2-D00-0-0145 | 1 | B03 | b1 | C15 |
| 2-2-D00-0-0146 | 0 | B05 | b1 | C05 |
| 2-2-D00-0-0147 | 0 | B03 | b3 | C11 |
| 2-2-D00-0-0148 | 1 | B01 | b4 | C05 |
| 2-2-D00-0-0149 | 1 | B03 | b1 | C16 |
| 2-2-D00-0-0150 | 0 | B04 | b3 | C09 |
| 2-2-D00-0-0151 | 0 | B05 | b3 | C08 |
| 2-2-D00-0-0152 | 1 | B03 | b1 | C17 |
| 2-2-D00-0-0153 | 1 | B02 | b1 | C18 |
| 2-2-D00-0-0154 | 1 | B03 | b4 | C01 |
| 2-2-D00-0-0155 | 0 | B04 | b1 | C14 |
| 2-2-D00-0-0156 | 1 | B04 | b4 | C12 |
| 2-2-D00-0-0157 | 1 | B04 | b4 | C13 |
| 2-2-D00-0-0158 | 0 | B04 | b3 | C10 |
| 2-2-D00-0-0159 | 1 | B03 | b1 | C18 |
| 2-2-D00-0-0160 | 0 | B04 | b2 | C02 |
| 2-2-D00-0-0161 | 0 | B04 | b2 | C03 |
| 2-2-D00-0-0162 | 0 | B04 | b2 | C04 |
| 2-2-D00-0-0163 | 0 | B04 | b2 | C06 |
| 2-2-D00-0-0164 | 0 | B04 | b2 | C07 |
| 2-2-D00-0-0165 | 1 | B01 | b3 | C11 |
| 2-2-D00-0-0166 | 1 | B02 | b4 | C02 |

**[Table 18]**

| | | | | |
|---|---|---|---|---|
| 2-2-D00-0-0167 | 1 | B04 | b1 | C02 |
| 2-2-D00-0-0168 | 1 | B04 | b1 | C03 |
| 2-2-D00-0-0169 | 1 | B04 | b1 | C04 |
| 2-2-D00-0-0170 | 1 | B05 | b1 | C01 |
| 2-2-D00-0-0171 | 0 | B01 | b1 | C19 |
| 2-2-D00-0-0172 | 0 | B05 | b3 | C09 |
| 2-2-D00-0-0173 | 0 | B04 | b1 | C15 |
| 2-2-D00-0-0174 | 1 | B03 | b4 | C02 |
| 2-2-D00-0-0175 | 0 | B04 | b1 | C16 |
| 2-2-D00-0-0176 | 0 | B04 | b1 | C17 |
| 2-2-D00-0-0177 | 0 | B05 | b1 | C14 |
| 2-2-D00-0-0178 | 1 | B02 | b4 | C05 |
| 2-2-D00-0-0179 | 1 | B05 | b4 | C12 |
| 2-2-D00-0-0180 | 1 | B04 | b1 | C05 |
| 2-2-D00-0-0181 | 0 | B04 | b4 | C01 |
| 2-2-D00-0-0182 | 1 | B04 | b3 | C08 |
| 2-2-D00-0-0183 | 1 | B05 | b4 | C13 |
| 2-2-D00-0-0184 | 0 | B05 | b3 | C10 |
| 2-2-D00-0-0185 | 0 | B05 | b2 | C02 |
| 2-2-D00-0-0186 | 0 | B05 | b2 | C03 |
| 2-2-D00-0-0187 | 1 | B03 | b4 | C05 |
| 2-2-D00-0-0188 | 0 | B04 | b1 | C18 |
| 2-2-D00-0-0189 | 0 | B05 | b2 | C04 |
| 2-2-D00-0-0190 | 0 | B05 | b2 | C06 |
| 2-2-D00-0-0191 | 0 | B05 | b2 | C07 |
| 2-2-D00-0-0192 | 1 | B05 | b1 | C02 |
| 2-2-D00-0-0193 | 1 | B05 | b1 | C03 |
| 2-2-D00-0-0194 | 1 | B05 | b1 | C04 |
| 2-2-D00-0-0195 | 1 | B02 | b3 | C11 |
| 2-2-D00-0-0196 | 0 | B05 | b1 | C15 |
| 2-2-D00-0-0197 | 0 | B05 | b1 | C16 |
| 2-2-D00-0-0198 | 0 | B02 | b1 | C19 |
| 2-2-D00-0-0199 | 0 | B05 | b1 | C17 |
| 2-2-D00-0-0200 | 0 | B04 | b4 | C02 |

**[Table 19]**

| | | | | |
|---|---|---|---|---|
| 2-2-D00-0-0201 | 1 | B05 | b1 | C05 |
| 2-2-D00-0-0202 | 1 | B03 | b3 | C11 |
| 2-2-D00-0-0203 | 0 | B05 | b4 | C01 |
| 2-2-D00-0-0204 | 1 | B04 | b3 | C09 |
| 2-2-D00-0-0205 | 1 | B05 | b3 | C08 |
| 2-2-D00-0-0206 | 0 | B03 | b1 | C19 |
| 2-2-D00-0-0207 | 1 | B04 | b1 | C14 |
| 2-2-D00-0-0208 | 0 | B05 | b1 | C18 |
| 2-2-D00-0-0209 | 0 | B04 | b4 | C05 |
| 2-2-D00-0-0210 | 1 | B04 | b3 | C10 |
| 2-2-D00-0-0211 | 1 | B04 | b2 | C02 |
| 2-2-D00-0-0212 | 1 | B04 | b2 | C03 |
| 2-2-D00-0-0213 | 1 | B04 | b2 | C04 |
| 2-2-D00-0-0214 | 1 | B04 | b2 | C06 |
| 2-2-D00-0-0215 | 1 | B04 | b2 | C07 |
| 2-2-D00-0-0216 | 0 | B05 | b4 | C02 |
| 2-2-D00-0-0217 | 1 | B01 | b1 | C19 |
| 2-2-D00-0-0218 | 1 | B05 | b3 | C09 |
| 2-2-D00-0-0219 | 1 | B04 | b1 | C15 |
| 2-2-D00-0-0220 | 0 | B04 | b3 | C11 |
| 2-2-D00-0-0221 | 1 | B04 | b1 | C16 |
| 2-2-D00-0-0222 | 1 | B04 | b1 | C17 |
| 2-2-D00-0-0223 | 1 | B05 | b1 | C14 |
| 2-2-D00-0-0224 | 1 | B04 | b4 | C01 |
| 2-2-D00-0-0225 | 0 | B05 | b4 | C05 |
| 2-2-D00-0-0226 | 1 | B05 | b3 | C10 |
| 2-2-D00-0-0227 | 1 | B05 | b2 | C02 |
| 2-2-D00-0-0228 | 1 | B05 | b2 | C03 |
| 2-2-D00-0-0229 | 1 | B04 | b1 | C18 |
| 2-2-D00-0-0230 | 1 | B05 | b2 | C04 |
| 2-2-D00-0-0231 | 1 | B05 | b2 | C06 |
| 2-2-D00-0-0232 | 1 | B05 | b2 | C07 |
| 2-2-D00-0-0233 | 1 | B05 | b1 | C15 |
| 2-2-D00-0-0234 | 0 | B05 | b3 | C11 |

**[Table 20]**

| | | | | |
|---|---|---|---|---|
| 2-2-D00-0-0235 | 1 | B05 | b1 | C16 |
| 2-2-D00-0-0236 | 1 | B02 | b1 | C19 |
| 2-2-D00-0-0237 | 1 | B05 | b1 | C17 |
| 2-2-D00-0-0238 | 1 | B04 | b4 | C02 |
| 2-2-D00-0-0239 | 1 | B05 | b4 | C01 |
| 2-2-D00-0-0240 | 1 | B03 | b1 | C19 |
| 2-2-D00-0-0241 | 1 | B05 | b1 | C18 |
| 2-2-D00-0-0242 | 1 | B04 | b4 | C05 |
| 2-2-D00-0-0243 | 1 | B05 | b4 | C02 |
| 2-2-D00-0-0244 | 1 | B04 | b3 | C11 |
| 2-2-D00-0-0245 | 0 | B04 | b1 | C19 |
| 2-2-D00-0-0246 | 1 | B05 | b4 | C05 |
| 2-2-D00-0-0247 | 1 | B05 | b3 | C11 |
| 2-2-D00-0-0248 | 0 | B05 | b1 | C19 |
| 2-2-D00-0-0249 | 1 | B04 | b1 | C19 |
| 2-2-D00-0-0250 | 1 | B05 | b1 | C19 |

**[Table 21]**

| [Table 4-1-1-2] Compounds that may be contained in mixture 2-2-c1D01 -0 | | | | |
|---|---|---|---|---|
| ID | n-number | B | b | C |
| 2-2-c1D01-0-0001 | 0 | B01 | b1 | C01 |
| 2-2-c1D01-0-0002 | 0 | B01 | b4 | C12 |
| 2-2-c1D01-0-0003 | 0 | B01 | b4 | C13 |
| 2-2-c1D01-0-0004 | 0 | B01 | b1 | C02 |
| 2-2-c1D01-0-0005 | 0 | B01 | b1 | C03 |
| 2-2-c1D01-0-0006 | 0 | B01 | b1 | C04 |
| 2-2-c1D01-0-0007 | 0 | B02 | b1 | C01 |
| 2-2-c1D01-0-0008 | 0 | B01 | b1 | C05 |
| 2-2-c1D01-0-0009 | 0 | B01 | b3 | C08 |
| 2-2-c1D01-0-0010 | 0 | B03 | b1 | C01 |
| 2-2-c1D01-0-0011 | 0 | B02 | b4 | C12 |
| 2-2-c1D01-0-0012 | 0 | B02 | b4 | C13 |
| 2-2-c1D01-0-0013 | 0 | B03 | b4 | C12 |
| 2-2-c1D01-0-0014 | 0 | B02 | b1 | C02 |
| 2-2-c1D01-0-0015 | 0 | B02 | b1 | C03 |
| 2-2-c1D01-0-0016 | 0 | B03 | b4 | C13 |
| 2-2-c1D01-0-0017 | 0 | B02 | b1 | C04 |
| 2-2-c1D01-0-0018 | 1 | B01 | b1 | C01 |
| 2-2-c1D01-0-0019 | 0 | B01 | b3 | C09 |
| 2-2-c1D01-0-0020 | 0 | B03 | b1 | C02 |
| 2-2-c1D01-0-0021 | 0 | B03 | b1 | 003 |
| 2-2-c1D01-0-0022 | 0 | B03 | b1 | C04 |
| 2-2-c1D01-0-0023 | 0 | B02 | b1 | C05 |
| 2-2-c1D01-0-0024 | 0 | B01 | b1 | C14 |
| 2-2-c1D01-0-0025 | 1 | B01 | b4 | C12 |
| 2-2-c1D01-0-0026 | 0 | B02 | b3 | C08 |
| 2-2-c1D01-0-0027 | 1 | B01 | b4 | C13 |
| 2-2-c1D01-0-0028 | 0 | B01 | b3 | C10 |
| 2-2-c1D01-0-0029 | 0 | B03 | b1 | C05 |
| 2-2-c1D01-0-0030 | 0 | B01 | b2 | C02 |

**[Table 22]**

| | | | | |
|---|---|---|---|---|
| 2-2-c1D01-0-0031 | 0 | B01 | b2 | C03 |
| 2-2-c1D01-0-0032 | 0 | B03 | b3 | C08 |
| 2-2-c1D01-0-0033 | 0 | B01 | b2 | C04 |
| 2-2-c1D01-0-0034 | 0 | B01 | b2 | C06 |
| 2-2-c1D01-0-0035 | 0 | B01 | b2 | C07 |
| 2-2-c1D01-0-0036 | 1 | B01 | b1 | C02 |
| 2-2-c1D01-0-0037 | 1 | B01 | b1 | C03 |
| 2-2-c1D01-0-0038 | 1 | B01 | b1 | C04 |
| 2-2-c1D01-0-0039 | 0 | B01 | b1 | C15 |
| 2-2-c1D01-0-0040 | 1 | B02 | b1 | C01 |
| 2-2-c1D01-0-0041 | 0 | B01 | b1 | C16 |
| 2-2-c1D01-0-0042 | 0 | B01 | b1 | C17 |
| 2-2-c1D01-0-0043 | 1 | B01 | b1 | C05 |
| 2-2-c1D01-0-0044 | 0 | B02 | b3 | C09 |
| 2-2-c1D01-0-0045 | 0 | B01 | b4 | C01 |
| 2-2-c1D01-0-0046 | 1 | B01 | b3 | C08 |
| 2-2-c1D01-0-0047 | 1 | B03 | b1 | C01 |
| 2-2-c1D01-0-0048 | 0 | B02 | b1 | C14 |
| 2-2-c1D01-0-0049 | 1 | B02 | b4 | C12 |
| 2-2-c1D01-0-0050 | 0 | B03 | b3 | C09 |
| 2-2-c1D01-0-0051 | 0 | B01 | b1 | C18 |
| 2-2-c1D01-0-0052 | 1 | B02 | b4 | C13 |
| 2-2-c1D01-0-0053 | 0 | B02 | b3 | C10 |
| 2-2-c1D01-0-0054 | 0 | B03 | b1 | C14 |
| 2-2-c1D01-0-0055 | 0 | B02 | b2 | C02 |
| 2-2-c1D01-0-0056 | 0 | B02 | b2 | C03 |
| 2-2-c1D01-0-0057 | 1 | B03 | b4 | C12 |
| 2-2-c1D01-0-0058 | 0 | B02 | b2 | C04 |
| 2-2-c1D01-0-0059 | 0 | B02 | b2 | C06 |
| 2-2-c1D01-0-0060 | 0 | B02 | b2 | C07 |
| 2-2-c1D01-0-0061 | 1 | B02 | b1 | C02 |
| 2-2-c1D01-0-0062 | 1 | B02 | b1 | C03 |
| 2-2-c1D01-0-0063 | 1 | B03 | b4 | C13 |
| 2-2-c1D01-0-0064 | 0 | B03 | b3 | C10 |

**[Table 23]**

| | | | | |
|---|---|---|---|---|
| 2-2-c1D01-0-0065 | 1 | B02 | b1 | C04 |
| 2-2-c1D01-0-0066 | 0 | B03 | b2 | C02 |
| 2-2-c1D01-0-0067 | 0 | B03 | b2 | C03 |
| 2-2-c1D01-0-0068 | 0 | B01 | b4 | C02 |
| 2-2-c1D01-0-0069 | 0 | B03 | b2 | C04 |
| 2-2-c1D01-0-0070 | 0 | B03 | b2 | C06 |
| 2-2-c1D01-0-0071 | 0 | B03 | b2 | C07 |
| 2-2-c1D01-0-0072 | 0 | B02 | b1 | C15 |
| 2-2-c1D01-0-0073 | 1 | B01 | b3 | C09 |
| 2-2-c1D01-0-0074 | 1 | B03 | b1 | C02 |
| 2-2-c1D01-0-0075 | 1 | B03 | b1 | C03 |
| 2-2-c1D01-0-0076 | 0 | B02 | b1 | C16 |
| 2-2-c1D01-0-0077 | 1 | B03 | b1 | C04 |
| 2-2-c1D01-0-0078 | 0 | B02 | b1 | C17 |
| 2-2-c1D01-0-0079 | 1 | B02 | b1 | C05 |
| 2-2-c1D01-0-0080 | 1 | B01 | b1 | C14 |
| 2-2-c1D01-0-0081 | 0 | B02 | b4 | C01 |
| 2-2-c1D01-0-0082 | 0 | B04 | b1 | C01 |
| 2-2-c1D01-0-0083 | 1 | B02 | b3 | C08 |
| 2-2-c1D01-0-0084 | 0 | B03 | b1 | C15 |
| 2-2-c1D01-0-0085 | 0 | B01 | b4 | C05 |
| 2-2-c1D01-0-0086 | 0 | B03 | b1 | C16 |
| 2-2-c1D01-0-0087 | 1 | B01 | b3 | C10 |
| 2-2-c1D01-0-0088 | 0 | B03 | b1 | C17 |
| 2-2-c1D01-0-0089 | 1 | B03 | b1 | C05 |
| 2-2-c1D01-0-0090 | 0 | B02 | b1 | C18 |
| 2-2-c1D01-0-0091 | 1 | B01 | b2 | C02 |
| 2-2-c1D01-0-0092 | 1 | B01 | b2 | C03 |
| 2-2-c1D01-0-0093 | 0 | B03 | b4 | C01 |
| 2-2-c1D01-0-0094 | 1 | B03 | b3 | C08 |
| 2-2-c1D01-0-0095 | 1 | B01 | b2 | C04 |
| 2-2-c1D01-0-0096 | 1 | B01 | b2 | C06 |
| 2-2-c1D01-0-0097 | 1 | B01 | b2 | C07 |
| 2-2-c1D01-0-0098 | 0 | B04 | b4 | C12 |

**[Table 24]**

| | | | | |
|---|---|---|---|---|
| 2-2-c1D01-0-0099 | 0 | B04 | b4 | C13 |
| 2-2-c1D01-0-0100 | 0 | B03 | b1 | C18 |
| 2-2-c1D01-0-0101 | 1 | B01 | b1 | C15 |
| 2-2-c1D01-0-0102 | 0 | B01 | b3 | C11 |
| 2-2-c1D01-0-0103 | 1 | B01 | b1 | C16 |
| 2-2-c1D01-0-0104 | 0 | B02 | b4 | C02 |
| 2-2-c1D01-0-0105 | 0 | B04 | b1 | C02 |
| 2-2-c1D01-0-0106 | 0 | B04 | b1 | C03 |
| 2-2-c1D01-0-0107 | 1 | B01 | b1 | C17 |
| 2-2-c1D01-0-0108 | 0 | B04 | b1 | C04 |
| 2-2-c1D01-0-0109 | 0 | B05 | b1 | C01 |
| 2-2-c1D01-0-0110 | 1 | B02 | b3 | C09 |
| 2-2-c1D01-0-0111 | 1 | B01 | b4 | C01 |
| 2-2-c1D01-0-0112 | 1 | B02 | b1 | C14 |
| 2-2-c1D01-0-0113 | 0 | B03 | b4 | C02 |
| 2-2-c1D01-0-0114 | 1 | B03 | b3 | C09 |
| 2-2-c1D01-0-0115 | 1 | B01 | b1 | C18 |
| 2-2-c1D01-0-0116 | 0 | B02 | b4 | C05 |
| 2-2-c1D01-0-0117 | 0 | B05 | b4 | C12 |
| 2-2-c1D01-0-0118 | 0 | B04 | b1 | C05 |
| 2-2-c1D01-0-0119 | 1 | B02 | b3 | C10 |
| 2-2-c1D01-0-0120 | 0 | B04 | b3 | C08 |
| 2-2-c1D01-0-0121 | 1 | B03 | b1 | C14 |
| 2-2-c1D01-0-0122 | 0 | B05 | b4 | C13 |
| 2-2-c1D01-0-0123 | 1 | B02 | b2 | C02 |
| 2-2-c1D01-0-0124 | 1 | B02 | b2 | C03 |
| 2-2-c1D01-0-0125 | 1 | B02 | b2 | C04 |
| 2-2-c1D01-0-0126 | 1 | B02 | b2 | C06 |
| 2-2-c1D01-0-0127 | 1 | B02 | b2 | C07 |
| 2-2-c1D01-0-0128 | 0 | B03 | b4 | C05 |
| 2-2-c1D01-0-0129 | 1 | B03 | b3 | C10 |
| 2-2-c1D01-0-0130 | 0 | B05 | b1 | C02 |
| 2-2-c1D01-0-0131 | 0 | B05 | b1 | C03 |
| 2-2-c1D01-0-0132 | 1 | B03 | b2 | C02 |

**[Table 25]**

| | | | | |
|---|---|---|---|---|
| 2-2-c1D01-0-0133 | 1 | B03 | b2 | C03 |
| 2-2-c1D01-0-0134 | 1 | B01 | b4 | C02 |
| 2-2-c1D01-0-0135 | 0 | B05 | b1 | C04 |
| 2-2-c1D01-0-0136 | 1 | B03 | b2 | C04 |
| 2-2-c1D01-0-0137 | 1 | B03 | b2 | C06 |
| 2-2-c1D01-0-0138 | 1 | B03 | b2 | C07 |
| 2-2-c1D01-0-0139 | 1 | B02 | b1 | C15 |
| 2-2-c1D01-0-0140 | 0 | B02 | b3 | C11 |
| 2-2-c1D01-0-0141 | 1 | B02 | b1 | C16 |
| 2-2-c1D01-0-0142 | 1 | B02 | b1 | C17 |
| 2-2-c1D01-0-0143 | 1 | B02 | b4 | C01 |
| 2-2-c1D01-0-0144 | 1 | B04 | b1 | C01 |
| 2-2-c1D01-0-0145 | 1 | B03 | b1 | C15 |
| 2-2-c1D01-0-0146 | 0 | B05 | b1 | C05 |
| 2-2-c1D01-0-0147 | 0 | B03 | b3 | C11 |
| 2-2-c1D01-0-0148 | 1 | B01 | b4 | C05 |
| 2-2-c1D01-0-0149 | 1 | B03 | b1 | C18 |
| 2-2-c1D01-0-0150 | 0 | B04 | b3 | C09 |
| 2-2-c1D01-0-0151 | 0 | B05 | b3 | C08 |
| 2-2-c1D01-0-0152 | 1 | B03 | b1 | C17 |
| 2-2-c1D01-0-0153 | 1 | B02 | b1 | C18 |
| 2-2-c1D01-0-0154 | 1 | B03 | b4 | C01 |
| 2-2-c1D01-0-0155 | 0 | B04 | b1 | C14 |
| 2-2-c1D01-0-0156 | 1 | B04 | b4 | C12 |
| 2-2-c1D01-0-0157 | 1 | B04 | b4 | C13 |
| 2-2-c1D01-0-0158 | 0 | B04 | b3 | C10 |
| 2-2-c1D01-0-0159 | 1 | B03 | b1 | C18 |
| 2-2-c1D01-0-0160 | 0 | B04 | b2 | C02 |
| 2-2-c1D01-0-0161 | 0 | B04 | b2 | C03 |
| 2-2-c1D01-0-0162 | 0 | B04 | b2 | C04 |
| 2-2-c1D01-0-0163 | 0 | B04 | b2 | C06 |
| 2-2-c1D01-0-0164 | 0 | B04 | b2 | C07 |
| 2-2-c1D01-0-0165 | 1 | B01 | b3 | C11 |
| 2-2-c1D01-0-0166 | 1 | B02 | b4 | C02 |

**[Table 26]**

| | | | | |
|---|---|---|---|---|
| 2-2-c1D01-0-0167 | 1 | B04 | b1 | C02 |
| 2-2-c1D01-0-0168 | 1 | B04 | b1 | C03 |
| 2-2-c1D01-0-0169 | 1 | B04 | b1 | C04 |
| 2-2-c1D01-0-0170 | 1 | B05 | b1 | C01 |
| 2-2-c1D01-0-0171 | 0 | B01 | b1 | C19 |
| 2-2-c1D01-0-0172 | 0 | B05 | b3 | C09 |
| 2-2-c1D01-0-0173 | 0 | B04 | b1 | C15 |
| 2-2-c1D01-0-0174 | 1 | B03 | b4 | C02 |
| 2-2-c1D01-0-0175 | 0 | B04 | b1 | C16 |
| 2-2-c1D01-0-0176 | 0 | B04 | b1 | C17 |
| 2-2-c1D01-0-0177 | 0 | B05 | b1 | C14 |
| 2-2-c1D01-0-0178 | 1 | B02 | b4 | C05 |
| 2-2-c1D01-0-0179 | 1 | B05 | b4 | C12 |
| 2-2-c1D01-0-0180 | 1 | B04 | b1 | C05 |
| 2-2-c1D01-0-0181 | 0 | B04 | b4 | C01 |
| 2-2-c1D01-0-0182 | 1 | B04 | b3 | C08 |
| 2-2-c1D01-0-0183 | 1 | B05 | b4 | C13 |
| 2-2-c1D01-0-0184 | 0 | B05 | b3 | C10 |
| 2-2-c1D01-0-0185 | 0 | B05 | b2 | C02 |
| 2-2-c1D01-0-0186 | 0 | B05 | b2 | C03 |
| 2-2-c1D01-0-0187 | 1 | B03 | b4 | C05 |
| 2-2-c1D01-0-0188 | 0 | B04 | b1 | C18 |
| 2-2-c1D01-0-0189 | 0 | B05 | b2 | C04 |
| 2-2-c1D01-0-0190 | 0 | B05 | b2 | C06 |
| 2-2-c1D01-0-0191 | 0 | B05 | b2 | C07 |
| 2-2-c1D01-0-0192 | 1 | B05 | b1 | C02 |
| 2-2-c1D01-0-0193 | 1 | B05 | b1 | C03 |
| 2-2-c1D01-0-0194 | 1 | B05 | b1 | C04 |
| 2-2-c1D01-0-0195 | 1 | B02 | b3 | C11 |
| 2-2-c1D01-0-0196 | 0 | B05 | b1 | C15 |
| 2-2-c1D01-0-0197 | 0 | B05 | b1 | C16 |
| 2-2-c1D01-0-0198 | 0 | B02 | b1 | C19 |
| 2-2-c1D01-0-0199 | 0 | B05 | b1 | C17 |
| 2-2-c1D01-0-0200 | 0 | B04 | b4 | C02 |

**[Table 27]**

| | | | | |
|---|---|---|---|---|
| 2-2-c1D01-0-0201 | 1 | B05 | b1 | C05 |
| 2-2-c1D01-0-0202 | 1 | B03 | b3 | C11 |
| 2-2-c1D01-0-0203 | 0 | B05 | b4 | C01 |
| 2-2-c1D01-0-0204 | 1 | B04 | b3 | C09 |
| 2-2-c1D01-0-0205 | 1 | B05 | b3 | C08 |
| 2-2-c1D01-0-0206 | 0 | B03 | b1 | C19 |
| 2-2-c1D01-0-0207 | 1 | B04 | b1 | C14 |
| 2-2-c1D01-0-0208 | 0 | B05 | b1 | C18 |
| 2-2-c1D01-0-0209 | 0 | B04 | b4 | C05 |
| 2-2-c1D01-0-0210 | 1 | B04 | b3 | C10 |
| 2-2-c1D01-0-0211 | 1 | B04 | b2 | C02 |
| 2-2-c1D01-0-0212 | 1 | B04 | b2 | C03 |
| 2-2-c1D01-0-0213 | 1 | B04 | b2 | C04 |
| 2-2-c1D01-0-0214 | 1 | B04 | b2 | C06 |
| 2-2-c1D01-0-0215 | 1 | B04 | b2 | C07 |
| 2-2-c1D01-0-0216 | 0 | B05 | b4 | C02 |
| 2-2-c1D01-0-0217 | 1 | B01 | b1 | C19 |
| 2-2-c1D01-0-0218 | 1 | B05 | b3 | C09 |
| 2-2-c1D01-0-0219 | 1 | B04 | b1 | C15 |
| 2-2-c1D01-0-0220 | 0 | B04 | b3 | C11 |
| 2-2-c1D01-0-0221 | 1 | B04 | b1 | C16 |
| 2-2-c1D01-0-0222 | 1 | B04 | b1 | C17 |
| 2-2-c1D01-0-0223 | 1 | B05 | b1 | C14 |
| 2-2-c1D01-0-0224 | 1 | B04 | b4 | C01 |
| 2-2-c1D01-0-0225 | 0 | B05 | b4 | C05 |
| 2-2-c1D01-0-0226 | 1 | B05 | b3 | C10 |
| 2-2-c1D01-0-0227 | 1 | B05 | b2 | C02 |
| 2-2-c1D01-0-0228 | 1 | B05 | b2 | C03 |
| 2-2-c1D01-0-0229 | 1 | B04 | b1 | C18 |
| 2-2-c1D01-0-0230 | 1 | B05 | b2 | C04 |
| 2-2-c1D01-0-0231 | 1 | B05 | b2 | C06 |
| 2-2-c1D01-0-0232 | 1 | B05 | b2 | C07 |
| 2-2-c1D01-0-0233 | 1 | B05 | b1 | C15 |
| 2-2-c1D01-0-0234 | 0 | B05 | b3 | C11 |

**[Table 28]**

| | | | | |
|---|---|---|---|---|
| 2-2-c1D01-0-0235 | 1 | B05 | b1 | C16 |
| 2-2-c1D01-0-0236 | 1 | B02 | b1 | C19 |
| 2-2-c1D01-0-0237 | 1 | B05 | b1 | C17 |
| 2-2-c1D01-0-0238 | 1 | B04 | b4 | C02 |
| 2-2-c1D01-0-0239 | 1 | B05 | b4 | C01 |
| 2-2-c1D01-0-0240 | 1 | B03 | b1 | C19 |
| 2-2-c1D01-0-0241 | 1 | B05 | b1 | C18 |
| 2-2-c1D01-0-0242 | 1 | B04 | b4 | C05 |
| 2-2-c1D01-0-0243 | 1 | B05 | b4 | C02 |
| 2-2-c1D01-0-0244 | 1 | B04 | b3 | C11 |
| 2-2-c1D01-0-0245 | 0 | B04 | b1 | C19 |
| 2-2-c1D01-0-0246 | 1 | B05 | b4 | C05 |
| 2-2-c1D01-0-0247 | 1 | B05 | b3 | C11 |
| 2-2-c1D01-0-0248 | 0 | B05 | b1 | C19 |
| 2-2-c1D01-0-0243 | 1 | B04 | b1 | C19 |
| 2-2-c1D01-0-0250 | 1 | B05 | b1 | C19 |

### Example 4-1-2: Synthesis of mixture 2-2-c2D02-0 by acylsulfonamidation reaction of mixture 2-2-D00-0

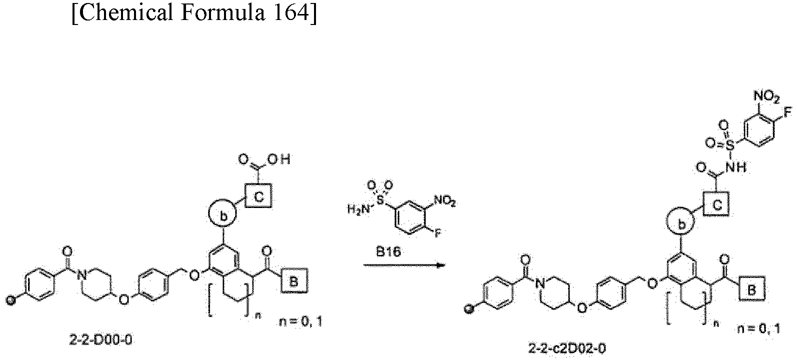

Twenty-five mixtures were added to a 20 mL glass vial to prepare mixture 2-2-D00-0 (832 mg), then NMP (12.0 mL) was added, and the mixture was shaken at room temperature for 1 hour. To this, 2,6-lutidine (0.108 mL, 0.931 mmol) and HATU (1-[bis(dimethylamino)methylene]-1H-1,2,3-triazolo[4,5-b]pyridinium 3-oxide hexafluorophosphate, CAS number: 148893-10-1) (0.354 g, 0.931 mmol) were added and the mixture was shaken at 45°C for 17 hours. To this, 4-fluoro-3-nitrobenzenesulfonamide (B16) (0.239 g, 1.09 mmol) and PltBu (tert-butylimino-tris(dimethylamino)phosphorane, CAS No. 81675-81-2) (0.592 mL, 2.33 mmol) were added, and the mixture was shaken at room temperature for 18 hours.

The suspension of the reaction solution and solid phase was transferred to a column equipped with a filter and filtered, and then washed 3 times with NMP/water = 1/1 (16 mL), 3 times with a mixed solution of tetrabutylammonium hydrogen sulfate and 2,6-di-tert-butyl pyridine in NMP (both 0.05 M, 16 mL), 3 times with a solution of 4-methylmorpholine in NMP (0.05 M, 16 mL), 3 times with NMP/water = 1/1 (16 mL), 3 times with NMP (16 mL), 3 times with MeOH (16 mL), 3 times with DCM (16 mL), and 3 times with heptane (16 mL). The resulting solid phase was dried under reduced pressure to obtain mixture 2-2-c2D02-0.

The compounds that may be contained in the mixture 2-2-c2D02-0 are as shown in Table 4-1-2-1. In Table 4-1-2-1, each compound that may be contained in the mixture is represented by ID, and the structure is represented by indicating the corresponding n-number and the combination of parts B, b, and C in the structural formula of the mixture by symbols. The correspondence of symbols in parts B, b, and C, and the structural formulas is as shown above.

**[Table 29]**

| [Table 4-1-2-1] Compounds that may be contained in mixture 2-2-c2002-0 | | | | |
|---|---|---|---|---|
| ID | n-number | B | b | C |
| 2-2-c2D02-0-0001 | 0 | B01 | b1 | C01 |
| 2-2-c2D02-0-0002 | 0 | B01 | b4 | C12 |
| 2-2-c2D02-0-0003 | 0 | B01 | b4 | C13 |
| 2-2-c2D02-0-0004 | 0 | B01 | b1 | C02 |
| 2-2-c2D02-0-0005 | 0 | B01 | b1 | C03 |
| 2-2-c2D02-0-0006 | 0 | B01 | b1 | C04 |
| 2-2-c2D02-0-0007 | 0 | B02 | b1 | C01 |
| 2-2-c2D02-0-0008 | 0 | B01 | b1 | C05 |
| 2-2-c2D02-0-0009 | 0 | B01 | b3 | C08 |
| 2-2-c2D02-0-0010 | 0 | B03 | b1 | C01 |
| 2-2-c2D02-0-0011 | 0 | B02 | b4 | C12 |
| 2-2-c2D02-0-0012 | 0 | B02 | b4 | C13 |
| 2-2-c2D02-0-0013 | 0 | B03 | b4 | C12 |
| 2-2-c2D02-0-0014 | 0 | B02 | b1 | C02 |
| 2-2-c2D02-0-0015 | 0 | B02 | b1 | C03 |
| 2-2-c2D02-0-0016 | 0 | B03 | b4 | C13 |
| 2-2-c2D02-0-0017 | 0 | B02 | b1 | C04 |
| 2-2-c2D02-0-0018 | 1 | B01 | b1 | C01 |
| 2-2-c2D02-0-0019 | 0 | B01 | b3 | C09 |
| 2-2-c2D02-0-0020 | 0 | B03 | b1 | C02 |
| 2-2-c2D02-0-0021 | 0 | B03 | b1 | C03 |
| 2-2-c2D02-0-0022 | 0 | B03 | b1 | C04 |
| 2-2-c2D02-0-0023 | 0 | B02 | b1 | C05 |
| 2-2-c2D02-0-0024 | 0 | B01 | b1 | C14 |
| 2-2-c2D02-0-0025 | 1 | B01 | b4 | C12 |
| 2-2-c2D02-0-0026 | 0 | B02 | b3 | C08 |
| 2-2-c2D02-0-0027 | 1 | B01 | b4 | C13 |
| 2-2-c2D02-0-0028 | 0 | B01 | b3 | C10 |
| 2-2-c2D02-0-0029 | 0 | B03 | b1 | C05 |
| 2-2-c2D02-0-0030 | 0 | B01 | b2 | C02 |

**[Table 30]**

| | | | | |
|---|---|---|---|---|
| 2-2-c2D02-0-0031 | 0 | B01 | b2 | C03 |
| 2-2-c2D02-0-0032 | 0 | B03 | b3 | C08 |
| 2-2-c2D02-0-0033 | 0 | B01 | b2 | C04 |
| 2-2-c2D02-0-0034 | 0 | B01 | b2 | C06 |
| 2-2-c2D02-0-0035 | 0 | B01 | b2 | C07 |
| 2.2-c2D02-0-0036 | 1 | B01 | b1 | C02 |
| 2-2-c2D02-0-0037 | 1 | B01 | b1 | C03 |
| 2-2-c2D02-0-0038 | 1 | B01 | b1 | C04 |
| 2-2-c2D02-0-0039 | 0 | B01 | b1 | C15 |
| 2-2-c2D02-0-0040 | 1 | B02 | b1 | C01 |
| 2-2-c2D02-0-0041 | 0 | B01 | b1 | C16 |
| 2-2-c2D02-0-0042 | 0 | B01 | b1 | C17 |
| 2-2-c2D02-0-0043 | 1 | B01 | b1 | C05 |
| 2-2-c2D02-0-0044 | 0 | B02 | b3 | C09 |
| 2-2-c2D02-0-0045 | 0 | B01 | b4 | C01 |
| 2-2-c2D02-0-0046 | 1 | B01 | b3 | C08 |
| 2-2-c2D02-0-0047 | 1 | B03 | b1 | C01 |
| 2-2-c2D02-0-0048 | 0 | B02 | b1 | C14 |
| 2-2-c2D02-0-0049 | 1 | B02 | b4 | C12 |
| 2-2-c2D02-0-0050 | 0 | B03 | b3 | C09 |
| 2-2-c2D02-0-0051 | 0 | B01 | b1 | C18 |
| 2-2-c2D02-0-0052 | 1 | B02 | b4 | C13 |
| 2-2-c2D02-0-0063 | 0 | B02 | b3 | C10 |
| 2-2-c2D02-0-0054 | 0 | B 03 | b1 | C14 |
| 2-2-c2D02-0-0055 | 0 | B02 | b2 | C02 |
| 2-2-c2D02-0-0056 | 0 | B02 | b2 | C03 |
| 2-2-c2D02-0-0057 | 1 | B03 | b4 | C12 |
| 2-2-c2D02-0-0058 | 0 | B02 | b2 | C04 |
| 2-2-c2D02-0-0059 | 0 | B 03 | b2 | C 06 |
| 2-2-c2D02-0-0060 | 0 | B02 | b2 | C07 |
| 2-2-c2D02-0-0061 | 1 | B02 | b1 | C02 |
| 2-2-c2D02-0-0062 | 1 | B02 | b1 | C03 |
| 2-2-c2D02-0-0063 | 1 | B 03 | b4 | C13 |
| 2-2-c2D02-0-0064 | 0 | B03 | b3 | C10 |

**[Table 31]**

| | | | | |
|---|---|---|---|---|
| 2-2-c2D02-0-0065 | 1 | B02 | b1 | C04 |
| 2-2-c2D02-0-0066 | 0 | B03 | b2 | C02 |
| 2-2-c2D02-0-0067 | 0 | B03 | b2 | C03 |
| 2-2-c2D02-0-0068 | 0 | B01 | b4 | C02 |
| 2-2-c2D02-0-0069 | 0 | B03 | b2 | C04 |
| 2-2-c2D02-0-0070 | 0 | B03 | b2 | C06 |
| 2-2-c2D02-0-0071 | 0 | B03 | b2 | C07 |
| 2-2-c2D02-0-0072 | 0 | B02 | b1 | C16 |
| 2-2-c2D02-0-0073 | 1 | B01 | b3 | C09 |
| 2-2-c2D02-0-0074 | 1 | B03 | b1 | C02 |
| 2-2-c2D02-0-0075 | 1 | B03 | b1 | C03 |
| 2-2-c2D02-0-0076 | 0 | B02 | b1 | C16 |
| 2-2-c2D02-0-0077 | 1 | B03 | b1 | C04 |
| 2-2-c2D02-0-0078 | 0 | B02 | b1 | C17 |
| 2-2-c2D02-0-0079 | 1 | B02 | b1 | C05 |
| 2-2-c2D02-0-0080 | 1 | B01 | b1 | C14 |
| 2-2-c2D02-0-0081 | 0 | B02 | b4 | C01 |
| 2-2-c2D02-0-0082 | 0 | B04 | b1 | C01 |
| 2-2-c2D02-0-0083 | 1 | B02 | b3 | C08 |
| 2-2-c2D02-0-0084 | 0 | B03 | b1 | C15 |
| 2-2-c2D02-0-0085 | 0 | B01 | b4 | C05 |
| 2-2-c2D02-0-0086 | 0 | B03 | b1 | C16 |
| 2-2-c2D02-0-0087 | 1 | B01 | b3 | C10 |
| 2-2-c2D02-0-0088 | 0 | B03 | b1 | C17 |
| 2-2-c2D02-0-0089 | 1 | B03 | b1 | C05 |
| 2-2-c2D02-0-0090 | 0 | B02 | b1 | C18 |
| 2-2-c2D02-0-0091 | 1 | B01 | b2 | C02 |
| 2-2-c2D02-0-0092 | 1 | B01 | b2 | C03 |
| 2-2-c2D02-0-0093 | 0 | B03 | b4 | C01 |
| 2-2-c2D02-0-0094 | 1 | B03 | b3 | C08 |
| 2-2-c2D02-0-0095 | 1 | B01 | b2 | C04 |
| 2-2-c2D02-0-0096 | 1 | B01 | b2 | C06 |
| 2-2-c2D02-0-0097 | 1 | B01 | b2 | C07 |
| 2-2-c2D02-0-0098 | 0 | B04 | b4 | C12 |

**[Table 32]**

| | | | | |
|---|---|---|---|---|
| 2-2-c2D02-0-0099 | 0 | B04 | b4 | C13 |
| 2-2-c2D02-0-0100 | 0 | B03 | b1 | C18 |
| 2-2-c2D02-0-0101 | 1 | B01 | b1 | C15 |
| 2-2-c2D02-0-0102 | 0 | B01 | b3 | C11 |
| 2-2-c2D02-0-0143 | 1 | B01 | b1 | C16 |
| 2-2-c2D02-0-0104 | 0 | B02 | b4 | C02 |
| 2-2-c2D02-0-0105 | 0 | B04 | b1 | C02 |
| 2-2-c2D02-0-0106 | 0 | B04 | b1 | C03 |
| 2-2-c2D02-0-0107 | 1 | B01 | b1 | C17 |
| 2-2-c2D02-0-0108 | 0 | B04 | b1 | C04 |
| 2-2-c2D02-0-0109 | 0 | B05 | b1 | C01 |
| 2-2-c2D02-0-0110 | 1 | B02 | b3 | C09 |
| 2-2-c2D02-0-0111 | 1 | B01 | b4 | C01 |
| 2-2-c2D02-0-0112 | 1 | B02 | b1 | C14 |
| 2-2-c2D02-0-0113 | 0 | B03 | b4 | C02 |
| 2-2-c2D02-0-0114 | 1 | B03 | b3 | C09 |
| 2-2-c2D02-0-0115 | 1 | B01 | b1 | C18 |
| 2-2-c2D02-0-0116 | 0 | 802 | b4 | C05 |
| 2-2-c2D02-0-0117 | 0 | B05 | b4 | C12 |
| 2-2-c2D02-0-0118 | 0 | B04 | b1 | C05 |
| 2-2-c2D02-0-0119 | 1 | B02 | b3 | C10 |
| 2-2-c2D02-0-0120 | 0 | B04 | b3 | C08 |
| 2-2-c2D02-0-0121 | 1 | B03 | b1 | C14 |
| 2-2-c2D02-0-0122 | 0 | B05 | b4 | C13 |
| 2-2-c2D02-0-0123 | 1 | B02 | b2 | C02 |
| 2-2-c2D02-0-0124 | 1 | B02 | b2 | C03 |
| 2-2-c2D02-0-0125 | 1 | B02 | b2 | C04 |
| 2-2-c2D02-0-0126 | 1 | B02 | b2 | C06 |
| 2-2-c2D02-0-0127 | 1 | B02 | b2 | C07 |
| 2-2-c2D02-0-0128 | 0 | B03 | b4 | C05 |
| 2-2-c2D02-0-0129 | 1 | B03 | b3 | C10 |
| 2-2-c2D02-0-0130 | 0 | B05 | b1 | C02 |
| 2-2-c2D02-0-0131 | 0 | B05 | b1 | C03 |
| 2-2-c2D02-0-0132 | 1 | B03 | b2 | C02 |

**[Table 33]**

| | | | | |
|---|---|---|---|---|
| 2-2-c2D02-0-0133 | 1 | B03 | b2 | C03 |
| 2-2-c2D02-0-0134 | 1 | B01 | b4 | C02 |
| 2-2-c2D02-0-0135 | 0 | B05 | b1 | C04 |
| 2-2-c2D02-0-0136 | 1 | B03 | b2 | C04 |
| 2-2-c2D02-0-0137 | 1 | B03 | b2 | C06 |
| 2-2-c2D02-0-0138 | 1 | B03 | b2 | C07 |
| 2-2-c2D02-0-0139 | 1 | B02 | b1 | C15 |
| 2-2-c2D02-0-0140 | 0 | B02 | b3 | C11 |
| 2-2-c2D02-0-0141 | 1 | B02 | b1 | C16 |
| 2-2-c2D02-0-0142 | 1 | B02 | b1 | C17 |
| 2-2-c2D02-0-0143 | 1 | B02 | b4 | C01 |
| 2-2-c2D02-0-0144 | 1 | B04 | b1 | C01 |
| 2-2-c2D02-0-0145 | 1 | B03 | b1 | C15 |
| 2-2-c2D02-0-0146 | 0 | B05 | b1 | C05 |
| 2-2-c2D02-0-0147 | 0 | B03 | b3 | C11 |
| 2-2-c2D02-0-0148 | 1 | B01 | b4 | C05 |
| 2-2-c2D02-0-0149 | 1 | B03 | b1 | C16 |
| 2-2-c2D02-0-0150 | 0 | B04 | b3 | C09 |
| 2-2-c2D02-0-0151 | 0 | B05 | b3 | C08 |
| 2-2-c2D02-0-0152 | 1 | B03 | b1 | C17 |
| 2-2-c2D02-0-0153 | 1 | B02 | b1 | C18 |
| 2-2-c2D02-0-0154 | 1 | B03 | b4 | C01 |
| 2-2-c2D02-0-0155 | 0 | B04 | b1 | C14 |
| 2-2-c2D02-0-0156 | 1 | B04 | b4 | C12 |
| 2-2-c2D02-0-0157 | 1 | B04 | b4 | C13 |
| 2-2-c2D02-0-0158 | 0 | B04 | b3 | C10 |
| 2-2-c2D02-0-0159 | 1 | B03 | b1 | C18 |
| 2-2-c2D02-0-0160 | 0 | B04 | b2 | C02 |
| 2-2-c2D02-0-0161 | 0 | B04 | b2 | C03 |
| 2-2-c2D02-0-0162 | 0 | B04 | b2 | C04 |
| 2-2-c2D02-0-0163 | 0 | B04 | b2 | C06 |
| 2-2-c2D02-0-0164 | 0 | B04 | b2 | C07 |
| 2-2-c2D02-0-0165 | 1 | B01 | b3 | C11 |
| 2-2-c2D02-0-0166 | 1 | B02 | b4 | C02 |

**[Table 34]**

| | | | | |
|---|---|---|---|---|
| 2-2-c2D02-0-0167 | 1 | B04 | b1 | C02 |
| 2-2-c2D02-0-0168 | 1 | B04 | b1 | C03 |
| 2-2-c2D02-0-0169 | 1 | B04 | b1 | C04 |
| 2-2-c2D02-0-0170 | 1 | B05 | b1 | C01 |
| 2-2-c2D02-0-0171 | 0 | B01 | b1 | C19 |
| 2-2-c2D02-0-0172 | 0 | B05 | b3 | C09 |
| 2-2-c2D02-0-0173 | 0 | B04 | b1 | C15 |
| 2-2-c2D02-0-0174 | 1 | B03 | b4 | C02 |
| 2-2-c2D02-0-0175 | 0 | B04 | b1 | C16 |
| 2-2-c2D02-0-0176 | 0 | B04 | b1 | C17 |
| 2-2-c2D02-0-0177 | 0 | B05 | b1 | C14 |
| 2-2-c2D02-0-0178 | 1 | B02 | b4 | C05 |
| 2-2-c2D02-0-0179 | 1 | B05 | b4 | C12 |
| 2-2-c2D02-0-0180 | 1 | B04 | b1 | C05 |
| 2-2-c2D02-0-0181 | 0 | B04 | b4 | C01 |
| 2-2-c2D02-0-0182 | 1 | B04 | b3 | C08 |
| 2-2-c2D02-0-0183 | 1 | B05 | b4 | C13 |
| 2-2-c2D02-0-0184 | 0 | B05 | b3 | C10 |
| 2-2-c2D02-0-0185 | 0 | B05 | b2 | C02 |
| 2-2-c2D02-0-0186 | 0 | B05 | b2 | C03 |
| 22-c2D02-0-0187 | 1 | B03 | b4 | C05 |
| 2-2-c2D02-0-0188 | 0 | B04 | b1 | C18 |
| 2-2-c2D02-0-0189 | 0 | B05 | b2 | C04 |
| 2-2-c2D02-0-0190 | 0 | B05 | b2 | C06 |
| 2-2-c2D02-0-0191 | 0 | B05 | b2 | C07 |
| 2-2-c2D02-0-0192 | 1 | B05 | b1 | C02 |
| 2-2-c2D02-0-0193 | 1 | B05 | b1 | C03 |
| 2-2-c2D02-0-0194 | 1 | B05 | b1 | C04 |
| 2-2-c2D02-0-0195 | 1 | B02 | b3 | C11 |
| 2-2-c2D02-0-0196 | 0 | B05 | b1 | C15 |
| 2-2-c2D02-0-0197 | 0 | B05 | b1 | C16 |
| 2-2-c2D02-0-0198 | 0 | B02 | b1 | C19 |
| 2-2-c2D02-0-0199 | 0 | B05 | b1 | C17 |
| 2-2-c2D02-0-0200 | 0 | B04 | b4 | C02 |

**[Table 35]**

| | | | | |
|---|---|---|---|---|
| 2-2-c2D02-0-0201 | 1 | B05 | b1 | C05 |
| 2-2-c2D02-0-0202 | 1 | B03 | b3 | C11 |
| 2-2-c2D02-0-0203 | 0 | B05 | b4 | C01 |
| 2-2-c2D02-0-0204 | 1 | B04 | b3 | C09 |
| 2-2-c2D02-0-0205 | 1 | B05 | b3 | C08 |
| 2-2-c2D02-0-0206 | 0 | B03 | b1 | C19 |
| 2-2-c2D02-0-0207 | 1 | B04 | b1 | C14 |
| 2-2-c2D02-0-0208 | 0 | B05 | b1 | C18 |
| 2-2-c2D02-0-0209 | 0 | B04 | b4 | C05 |
| 2-2-c2D02-0-0210 | 1 | B04 | b3 | C10 |
| 2-2-c2D02-0-0211 | 1 | B04 | b2 | C02 |
| 2-2-c2D02-0-0212 | 1 | B04 | b2 | C03 |
| 2-2-c2D02-0-0213 | 1 | B04 | b2 | C04 |
| 2-2-c2D02-0-0214 | 1 | B04 | b2 | C06 |
| 2-2-c2D02-0-0215 | 1 | B04 | b2 | C07 |
| 2-2-cD02-0-0216 | 0 | B05 | b4 | C02 |
| 2-2-c2D02-0-0217 | 1 | B01 | b1 | C19 |
| 2-2-c2D02-0-0218 | 1 | B05 | b3 | C09 |
| 2-2-c2D02-0-0219 | 1 | B04 | b1 | C15 |
| 2-2-c2D02-0-0220 | 0 | B04 | b3 | C11 |
| 2-2-c2D02-0-0221 | 1 | B04 | b1 | C16 |
| 2-2-c2D02-0-0222 | 1 | B04 | b1 | C17 |
| 2-2-c2D02-0-0223 | 1 | B05 | b1 | C14 |
| 2-2-c2D02-0-0224 | 1 | B04 | b4 | C01 |
| 2-2-c2D02-0-0225 | 0 | B05 | b4 | C05 |
| 2-2-c2D02-0-0226 | 1 | B05 | b3 | C10 |
| 2-2-c2D02-0-0227 | 1 | B05 | b2 | C02 |
| 2-2-c2D02-0-0228 | 1 | B05 | b2 | C03 |
| 2-2-c2D02-0-0229 | 1 | B04 | b1 | C18 |
| 2-2-c2D02-0-0230 | 1 | B05 | b2 | C04 |
| 2-2-c2D02-0-0231 | 1 | B05 | b2 | C06 |
| 2-2-c2D02-0-0232 | 1 | B05 | b2 | C07 |
| 2-2-c2D02-0-0233 | 1 | B05 | b1 | C15 |
| 2-2-c2D02-0-0234 | 0 | B05 | b3 | C11 |

**[Table 36]**

| | | | | |
|---|---|---|---|---|
| 2-2-c2D02-0-0235 | 1 | B05 | b1 | C16 |
| 2-2-c2D02-0-0236 | 1 | B02 | b1 | C19 |
| 2-2-c2D02-0-0237 | 1 | B05 | b1 | C17 |
| 2-2-c2D02-0-0238 | 1 | B04 | b4 | C02 |
| 2-2-c2D02-0-0239 | 1 | B05 | b4 | C01 |
| 2-2-c2D02-0-0240 | 1 | B03 | b1 | C19 |
| 2-2-c2D02-0-0241 | 1 | B05 | b1 | C18 |
| 2-2-c2D02-0-0242 | 1 | B04 | b4 | C05 |
| 2-2-c2D02-0-0243 | 1 | B05 | b4 | C02 |
| 2-2-c2D02-0-0244 | 1 | B04 | b3 | C11 |
| 2-2-c2D02-0-0245 | 0 | B04 | b1 | C19 |
| 2-2-c2D02-0-0246 | 1 | B05 | b4 | C05 |
| 2-2-c2D02-0-0247 | 1 | B05 | b3 | C11 |
| 2-2-c2D02-0-0248 | 0 | B05 | b1 | C19 |
| 2-2-c2D02-0-0249 | 1 | B04 | b1 | C19 |
| 2-2-c2D02-0-0250 | 1 | B05 | b1 | C19 |

### Example 4-1-3: Synthesis of mixture 2-2-d1E01-0

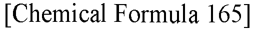

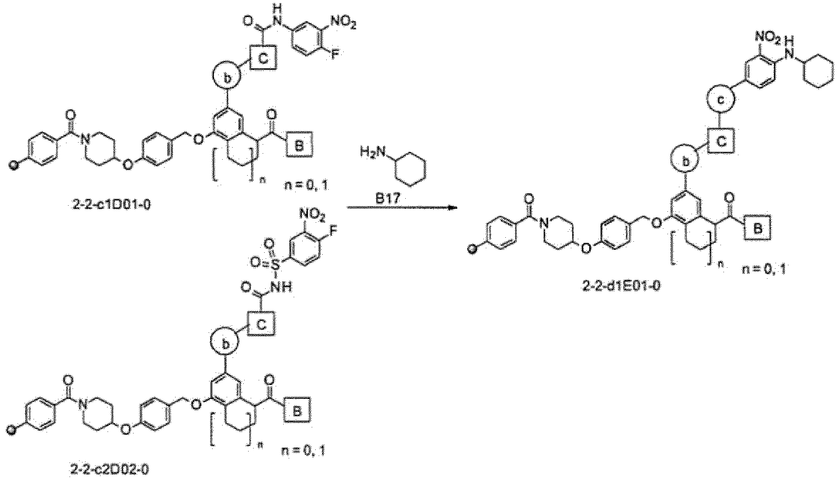

The mixture 2-2-c1D01-0 (100 mg) and the mixture 2-2-c2D02-0 (100 mg) were added to a 4 mL glass vial, then NMP (2.0 mL) was added, and the mixture was shaken at room temperature for 1 hour. To this, cyclohexylamine (B 17) (37.2 mg, 0.375 mmol) and 1,8-bis(dimethylamino)naphthalene (121 mg, 0.563 mmol) were added, and the mixture was shaken at 80°C for 18 hours.

The suspension of the reaction solution and solid phase was transferred to a column equipped with a filter and filtered, and then washed 3 times with NMP/water = 1/1 (4 mL), 3 times with NMP (4 mL), 3 times with a mixed solution of tetrabutylammonium hydrogen sulfate and 2,6-di-tert-butyl pyridine in NMP (both 0.05 M, 4 mL), 3 times with a solution of 4-methylmorpholine in NMP (0.05 M, 4 mL), 3 times with NMP/water = 1/1 (4 mL), 3 times with NMP (4 mL), 3 times with MeOH (4.5 mL), 3 times with DCM (4.5 mL), and 3 times with heptane (4.5 mL). The resulting solid phase was dried under reduced pressure to obtain mixture 2-2-d1E01-0.

The compounds that may be contained in the mixture 2-2-d1E01-0 are as shown in Table 4-1-3-1. In Table 4-1-3-1, each compound that may be contained in the mixture is represented by ID, and the structure is represented by indicating the corresponding n-number and the combination of parts B, b, C and c in the structural formula of the mixture by symbols. The correspondence of symbols in parts B, b, C and c, and the structural formulas is as shown above. For example, compound 2-2-d1E01-0-0001 has the n-number of 0, part B of B01, part b of b 1, and part C of C01, and part c of c01, and is represented by the following structure.

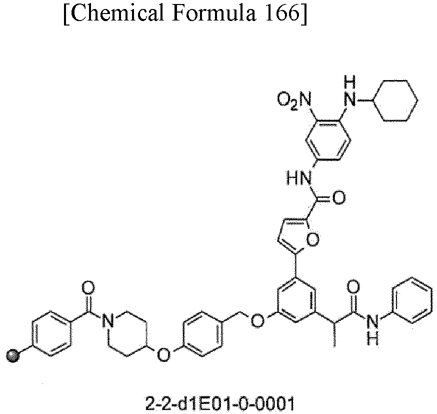

**[Table 37]**

| [Table 4-1-3-1] | | | | | |
|---|---|---|---|---|---|
| ID | n-number | B | b | C | c |
| 2-2-d1E01-0-0001 | 0 | B01 | b1 | C01 | c1 |
| 2-2-d1E01-0-0002 | 0 | B01 | b4 | C12 | c1 |
| 2-2-d1E01-0-0003 | 0 | B01 | b4 | C13 | c1 |
| 2-2-d1E01-0-0004 | 0 | B01 | b1 | C02 | c1 |
| 2-2-d1E01-0-0005 | 0 | B01 | b1 | C03 | c1 |
| 2-2-d1E01-0-0006 | 0 | B01 | b1 | C04 | c1 |
| 2-2-d1E01-0-0007 | 0 | B02 | b1 | C01 | c1 |
| 2-2-d1E01-0-0008 | 0 | B01 | b1 | C05 | c1 |
| 2-2-d1E01-0-0009 | 0 | B01 | b3 | C08 | c1 |
| 2-2-d0E01-0-0010 | 0 | B01 | b1 | C01 | c1 |
| 2-2-d1E01-0-0011 | 0 | B02 | b4 | C12 | c1 |
| 2-2-d1E01-0-0012 | 0 | B02 | b4 | C13 | c1 |
| 2-2-d1E01-0-0013 | 0 | B03 | b4 | C12 | c1 |
| 2-2-d1E01-0-0014 | 0 | B02 | b1 | C02 | c1 |
| 2-2-d1E01-0-0015 | 0 | B02 | b1 | C03 | c1 |
| 2-2-d1E01-0-0016 | 0 | B03 | b4 | C13 | c1 |
| 2-2-d1E01-0-0017 | 0 | B02 | b1 | C04 | c1 |
| 2-2-d1E01-0-0018 | 1 | B01 | b1 | C01 | c1 |
| 2-2-d1E01-0-0019 | 0 | B01 | b3 | C09 | c1 |
| 2-2-d1E01-0-0020 | 0 | B03 | b1 | C02 | c1 |
| 2-2-d1E01-0-0021 | 0 | B03 | b1 | C03 | c1 |
| 2-2-d17E01-0-0022 | 0 | B03 | b1 | C04 | c1 |
| 2-2-d1E01-0-0023 | 0 | B02 | b1 | C05 | c1 |
| 2-2-d1E01-0-0024 | 0 | B01 | b1 | C14 | c1 |
| 2-2-d1E01-0-0025 | 1 | B01 | b4 | C12 | c1 |
| 2-2-dE01-0-0026 | 0 | B02 | b3 | C08 | c1 |
| 2-2-d1E01-0-0027 | 1 | B01 | b4 | C13 | c1 |
| 2-2-d1E01-0-0028 | 0 | B01 | b3 | C10 | c1 |
| 2-2-d1E01-0-0029 | 0 | B03 | b1 | C05 | c1 |
| 2-2-d1E01-0-0030 | 0 | B01 | b2 | C02 | c1 |

**[Table 38]**

| | | | | | |
|---|---|---|---|---|---|
| 2-2-d1E01-0-0031 | 0 | B01 | b2 | C03 | c1 |
| 2.2-d1E01-0-0032 | 0 | B03 | b3 | C08 | c1 |
| 2-2-d1E01-0-0033 | 0 | B01 | b2 | C04 | c1 |
| 2-2-d1E01-0-0034 | 0 | B01 | b2 | C06 | c1 |
| 2-2-d1E01-0-0035 | 0 | B01 | b2 | C07 | c1 |
| 2-2-d1E01-0-0036 | 1 | B01 | b1 | C02 | c1 |
| 2-2-d1E01-0-0037 | 1 | B01 | b1 | C03 | c1 |
| 2-2-d1E01-0-0038 | 1 | B01 | b1 | C04 | c1 |
| 2-2-d1E01-0-0039 | 0 | B01 | b1 | C15 | c1 |
| 2-2-d1E01-0-0040 | 1 | B02 | b1 | C01 | c1 |
| 2-2-d1E01-0-0041 | 0 | B01 | b1 | C16 | c1 |
| 2-2-d1E01-0-0042 | 0 | B01 | b1 | C17 | c1 |
| 2-2-d1E01-0-0043 | 1 | B01 | b1 | C05 | c1 |
| 2-2-d1E01-0-0044 | 0 | B02 | b3 | C09 | c1 |
| 2-2-d1E01-0-0045 | 0 | B01 | b4 | C01 | c1 |
| 2-2-d1E01-0-0046 | 1 | B01 | b3 | C08 | c1 |
| 2-2-d1E01-0-0047 | 1 | B03 | b1 | C01 | c1 |
| 2-2-d1E01-0-0048 | 0 | B02 | b1 | C14 | c1 |
| 2-2-d1E01-0-0049 | 1 | B02 | b4 | C12 | c1 |
| 2-2-d1E01-0-0050 | 0 | B03 | b3 | C09 | c1 |
| 2-2-d1E01-0-0051 | 0 | B01 | b1 | C18 | c1 |
| 2-2-d1E01-0-0052 | 1 | B02 | b4 | C13 | c1 |
| 2-2-d1E01-0-0053 | 0 | B02 | b3 | C10 | c1 |
| 2-2-d1E01-0-0054 | 0 | B03 | b1 | C14 | c1 |
| 2-2-d1E01-0-0055 | 0 | B02 | b2 | C02 | c1 |
| 2-2-d1E01-0-0056 | 0 | B02 | b2 | C03 | c1 |
| 2-2-d1E01-0-0057 | 1 | B03 | b4 | C12 | c1 |
| 2-2-d1E01-0-0058 | 0 | B02 | b2 | C04 | c1 |
| 2-2-d1E01-0-0059 | 0 | B02 | b2 | C06 | c1 |
| 2-2-d1E01-0-0060 | 0 | B02 | b2 | C07 | c1 |
| 2-2-d1E01-0-0061 | 1 | B02 | b1 | C02 | c1 |
| 2-2-d1E01-0-0062 | 1 | B02 | b1 | C03 | c1 |
| 2-2-d1E01-0-0063 | 1 | B03 | b4 | C13 | c1 |
| 2-2-d1E01-0-0064 | 0 | B03 | b3 | C10 | c1 |

**[Table 39]**

| | | | | | |
|---|---|---|---|---|---|
| 2-2-d1E01-0-0065 | 1 | B02 | b1 | C04 | c1 |
| 2-2-d1E01-0-0066 | 0 | B03 | b2 | C02 | c1 |
| 2-2-d1E01-0-0067 | 0 | B03 | b2 | C03 | c1 |
| 2-2-d1E01-0-0068 | 0 | B01 | b4 | C02 | c1 |
| 2-2-d1E01-0-0069 | 0 | B03 | b2 | C04 | c1 |
| 2-2-d1E01-0-0070 | 0 | B03 | b2 | C06 | c1 |
| 2-2-d1E01-0-0071 | 0 | B03 | b2 | C07 | c1 |
| 2-2-d1E01-0-0072 | 0 | B02 | b1 | C15 | c1 |
| 2-2-d1E01-0-0073 | 1 | B01 | b3 | C09 | c1 |
| 2-2-d1E01-0-0074 | 1 | B03 | b1 | C02 | c1 |
| 2-2-d1E01-0-0075 | 1 | B03 | b1 | C03 | c1 |
| 2-2-d1E01-0-0076 | 0 | B02 | b1 | C16 | c1 |
| 2-2-d1E01-0-0077 | 1 | B03 | b1 | C04 | c1 |
| 2-2-d1E01-0-0078 | 0 | B02 | b1 | C17 | c1 |
| 2-2-d1E01-0-0079 | 1 | B02 | b1 | C05 | c1 |
| 2-2-d1E01-0-0080 | 1 | B01 | b1 | C14 | c1 |
| 2-2-d1E01-0-0081 | 0 | B02 | b4 | C01 | c1 |
| 2-2-d1E01-0-0082 | 0 | B04 | b1 | C01 | c1 |
| 2-2-d1E01-0-0083 | 1 | B02 | b3 | C08 | c1 |
| 2-2-d1E01-0-0084 | 0 | B03 | b1 | C15 | c1 |
| 2-2-d1E01-0-0085 | 0 | B01 | b4 | C05 | c1 |
| 2-2-d1E01-0-0086 | 0 | B03 | b1 | C16 | c1 |
| 2-2-d1E01-0-0087 | 1 | B01 | b3 | C10 | c1 |
| 2-2-d1E01-0-0088 | 0 | B03 | b1 | C17 | c1 |
| 2-2-d1E01-0-0089 | 1 | B03 | b1 | C05 | c1 |
| 2-2-d1E01-0-0090 | 0 | B02 | b1 | C18 | c1 |
| 2-2-d1E01-0-0091 | 1 | B01 | b2 | C02 | c1 |
| 2-2-d1E01-0-0092 | 1 | B01 | b2 | C03 | c1 |
| 2-2-d1E01-0-0093 | 0 | B03 | b4 | C01 | c1 |
| 2-2-d1E01-0-0094 | 1 | B03 | b3 | C08 | c1 |
| 2-2-d1E01-0-0095 | 1 | B01 | b2 | C04 | c1 |
| 2-2-d1E01-0-0096 | 1 | B01 | b2 | C06 | c1 |
| 2-2-d1E01-0-0097 | 1 | B01 | b2 | C07 | c1 |
| 2-2-d1E01-0-0098 | 0 | B04 | b4 | C12 | c1 |

**[Table 40]**

| | | | | | |
|---|---|---|---|---|---|
| 2-2-d1E01-0-0099 | 0 | B04 | b4 | C13 | c1 |
| 2-2-d1E01-0-0100 | 0 | B01 | b1 | C01 | c2 |
| 2-2-d1E01-0-0101 | 0 | B03 | b1 | C18 | c1 |
| 2-2-d1E01-0-0102 | 1 | B01 | b1 | C15 | c1 |
| 2-2-d1E01-0-0103 | 0 | B01 | b3 | C11 | c1 |
| 2-2-d1E01-0-0104 | 1 | B01 | b1 | C16 | c1 |
| 2-2-d1E01-0-0105 | 0 | B02 | b4 | C02 | c1 |
| 2-2-d1E01-0-0106 | 0 | B04 | b1 | C02 | c1 |
| 2-2-d1E01-0-0107 | 0 | B04 | b1 | C03 | c1 |
| 2-2-d1E01-0-0108 | 1 | B01 | b1 | C17 | c1 |
| 2-2-d1E01-0-0109 | 0 | B04 | b1 | C04 | c1 |
| 2-2-d1E01-0-0110 | 0 | B05 | b1 | C01 | c1 |
| 2-2-d1E01-0-0111 | 0 | B01 | b4 | C12 | c2 |
| 2-2-d1E01-0-0112 | 1 | B02 | b3 | C09 | c1 |
| 2-2-d1E01-0-0113 | 1 | B01 | b4 | C01 | c1 |
| 2-2-d1E01-0-0114 | 0 | B01 | b4 | C13 | c2 |
| 2-2-d1E01-0-0115 | 1 | B02 | b1 | C14 | c1 |
| 2-2-d1E01-0-0116 | 0 | B03 | b4 | C02 | c1 |
| 2-2-d1E01-0-0117 | 1 | B03 | b3 | C09 | c1 |
| 2-2-d1E01-0-0118 | 1 | B01 | b1 | C18 | c1 |
| 2-2-d1E01-0-0119 | 0 | B02 | b4 | C05 | c1 |
| 2-2-d1E01-0-0120 | 0 | B05 | b4 | C12 | c1 |
| 2-2-d1E01-0-0121 | 0 | B04 | b1 | C05 | c1 |
| 2-2-d1E01-0-0122 | 1 | B02 | b3 | C10 | c1 |
| 2-2-d1E01-0-0123 | 0 | B04 | b3 | C08 | c1 |
| 2-2-d1E01-0-0124 | 0 | B01 | b1 | C02 | c2 |
| 2-2-d1E01-0-0125 | 0 | B01 | b1 | C03 | c2 |
| 2-2-d1E01-0-0126 | 1 | B03 | b1 | C14 | c1 |
| 2-2-d1E01-0-0127 | 0 | B05 | b4 | C13 | c1 |
| 2-2-d1E01-0-0128 | 1 | B02 | b2 | C02 | c1 |
| 2-2-d1E01-0-0129 | 1 | B02 | b2 | C03 | c1 |
| 2-2-d1E01-0-0130 | 0 | B01 | b1 | C04 | c2 |
| 2-2-d1E01-0-0131 | 1 | B02 | b2 | C04 | c1 |
| 2-2-d1E01-0-0132 | 1 | B02 | b2 | C06 | c1 |

**[Table 41]**

| | | | | | |
|---|---|---|---|---|---|
| 2-2-d1E01-0-0133 | 1 | B02 | b2 | C07 | c1 |
| 2-2-d1E01-0-0134 | 0 | B03 | b4 | C05 | c1 |
| 2-2-d1E01-0-0135 | 1 | B03 | b3 | C10 | c1 |
| 2-2-d1E01-0-0136 | 0 | B02 | b1 | C01 | c2 |
| 2-2-d1E01-0-0137 | 0 | B05 | b1 | C02 | c1 |
| 2-2-d1E01-0-0138 | 0 | B05 | b1 | C03 | c1 |
| 2-2-d1E01-0-0139 | 1 | B03 | b2 | C02 | c1 |
| 2-2-d1E01-0-0140 | 1 | B03 | b2 | C03 | c1 |
| 2-2-d1E01-0-0141 | 1 | B01 | b4 | C02 | c1 |
| 2-2-d1E01-0-0142 | 0 | B05 | b1 | C04 | c1 |
| 2-2-d1E01-0-0143 | 1 | B03 | b2 | C04 | c1 |
| 2-2-d1E01-0-0144 | 1 | B03 | b2 | C06 | c1 |
| 2-2-d1E01-0-0145 | 1 | B03 | b2 | C07 | c1 |
| 2-2-d1E01-0-0146 | 1 | B02 | b1 | C15 | c1 |
| 2-2-d1E01-0-0147 | 0 | B01 | b1 | C05 | c2 |
| 2-2-d1E01-0-0148 | 0 | B02 | b3 | C11 | c1 |
| 2-2-d1E01-0-0149 | 1 | B02 | b1 | C16 | c1 |
| 2-2-d1E01-0-0150 | 0 | B01 | b3 | C08 | c2 |
| 2-2-d1E01-0-0151 | 1 | B02 | b1 | C17 | c1 |
| 2-2-d1E01-0-0152 | 0 | B03 | b1 | C01 | c2 |
| 2-2-d1E01-0-0153 | 0 | B02 | b4 | C12 | c2 |
| 2-2-d1E01-0-0154 | 1 | B02 | b4 | C01 | c1 |
| 2-2-d1E01-0-0155 | 1 | B04 | b1 | C01 | c1 |
| 2-2-d1E01-0-0156 | 1 | B03 | b1 | C15 | c1 |
| 2-2-d1E01-0-0157 | 0 | B05 | b1 | C05 | c1 |
| 2-2-d1E01-0-0158 | 0 | B03 | b3 | C11 | c1 |
| 2-2-d1E01-0-0159 | 0 | B02 | b4 | C13 | c2 |
| 2-2-d1E01-0-0160 | 1 | B01 | b4 | C05 | c1 |
| 2-2-d1E01-0-0161 | 1 | B03 | b1 | C16 | c1 |
| 2-2-d1E01-0-0162 | 0 | B04 | b3 | C09 | c1 |
| 2-2-d1E01-0-0163 | 0 | B05 | b3 | C08 | c1 |
| 2-2-d1E01-0-0164 | 1 | B03 | b1 | C17 | c1 |
| 2-2-d1E01-0-0165 | 0 | B03 | b4 | C12 | c2 |
| 2-2-d1E01-0-0166 | 1 | B02 | b1 | C18 | c1 |

**[Table 42]**

| | | | | | |
|---|---|---|---|---|---|
| 2-2-d1E01-0-0167 | 1 | B03 | b4 | C01 | c1 |
| 2-2-d1E01-0-0168 | 0 | B04 | b1 | C14 | c1 |
| 2-2-d1E01-0-0169 | 1 | B04 | b4 | C12 | c1 |
| 2-2-d1E01-0-0170 | 0 | B02 | b1 | C02 | c2 |
| 2-2-d1E01-0-0171 | 0 | B02 | b1 | C03 | c2 |
| 2-2-d1E01-0-0172 | 0 | B03 | b4 | C13 | c2 |
| 2-2-d1E01-0-0173 | 0 | B02 | b1 | C04 | c2 |
| 2-2-d1E01-0-0174 | 1 | B04 | b4 | C13 | c1 |
| 2-2-d1E01-0-0175 | 0 | B04 | b3 | C10 | c1 |
| 2-2-d1E01-0-0176 | 1 | B01 | b1 | C01 | c2 |
| 2-2-d1E01-0-0177 | 1 | B03 | b1 | C18 | c1 |
| 2-2-d1E01-0-0178 | 0 | B04 | b2 | C02 | c1 |
| 2-2-d1E01-0-0179 | 0 | B04 | b2 | C03 | c1 |
| 2-2-d1E01-0-0180 | 0 | B01 | b3 | C09 | c2 |
| 2-2-d1E01-0-0181 | 0 | B03 | b1 | C02 | c2 |
| 2-2-d1E01-0-0182 | 0 | B03 | b1 | C03 | c2 |
| 2-2-d1E01-0-0183 | 0 | B04 | b2 | C04 | c1 |
| 2-2-d1E01-0-0184 | 0 | B04 | b2 | C06 | c1 |
| 2-2-d1E01-0-0185 | 0 | B04 | b2 | C07 | c1 |
| 2-2-d1E01-0-0186 | 1 | B01 | b3 | C11 | c1 |
| 2-2-d1E01-0-0187 | 1 | B02 | b4 | C02 | c1 |
| 2-2-d1E01-0-0188 | 0 | B03 | b1 | C04 | c2 |
| 2-2-d1E01-0-0189 | 1 | B04 | b1 | C02 | c1 |
| 2-2-d1E01-0-0190 | 1 | B04 | b1 | C03 | c1 |
| 2-2-d1E01-0-0191 | 0 | B02 | b1 | C05 | c2 |
| 2-2-d1E01-0-0192 | 0 | B01 | b1 | C14 | c2 |
| 2-2-d1E01-0-0193 | 1 | B04 | b1 | C04 | c1 |
| 2-2-d1E01-0-0194 | 1 | B05 | b1 | C01 | c1 |
| 2-2-d1E01-0-0195 | 1 | B01 | b4 | C12 | c2 |
| 2-2-d1E01-0-0196 | 0 | B01 | b1 | C19 | c1 |
| 2-2-d1E01-0-0197 | 0 | B02 | b3 | C08 | c2 |
| 2-2-d1E01-0-0198 | 0 | B05 | b3 | C09 | c1 |
| 2-2-d1E01-0-0199 | 0 | B04 | b1 | C15 | c1 |
| 2-2-d1E01-0-0200 | 1 | B01 | b4 | C13 | c2 |

**[Table 43]**

| | | | | | |
|---|---|---|---|---|---|
| 2-2-d1E01-0-0201 | 1 | B03 | b4 | C02 | c1 |
| 2-2-d1E01-0-0202 | 0 | B01 | b3 | C10 | c2 |
| 2-2-d1E01-0-0203 | 0 | B04 | b1 | C16 | c1 |
| 2-2-d1E01-0-0204 | 0 | B03 | b1 | C05 | c2 |
| 2-2-d1E01-0-0205 | 0 | B01 | b2 | C02 | c2 |
| 2-2-d1E01-0-0206 | 0 | B01 | b2 | C03 | c2 |
| 2-2-d1E01-0-0207 | 0 | B04 | b1 | C19 | c1 |
| 2-2-d1E01-0-0208 | 0 | B05 | b1 | C14 | c1 |
| 2-2-d1E01-0-0209 | 1 | B02 | b4 | C05 | c1 |
| 2-2-d1E01-0-0210 | 1 | B05 | b4 | C12 | c1 |
| 2-2-d1E01-0-0211 | 0 | B03 | b3 | C08 | c2 |
| 2-2-d1E01-0-0212 | 1 | B04 | b1 | C05 | c1 |
| 2-2-d1E01-0-0213 | 0 | B01 | b2 | C04 | c2 |
| 2-2-d1E01-0-0214 | 0 | B01 | b2 | C06 | c2 |
| 2-2-d1E01-0-0215 | 0 | B01 | b2 | C07 | c2 |
| 2-2-d1E01-0-0216 | 0 | B04 | b4 | C01 | c1 |
| 2-2-d1E01-0-0217 | 1 | B04 | b3 | C08 | c1 |
| 2-2-d1E01-0-0218 | 1 | B01 | b1 | C02 | c2 |
| 2-2-d1E01-0-0219 | 1 | B01 | b1 | C03 | c2 |
| 2-2-d1E01-0-0220 | 1 | B05 | b4 | C13 | c1 |
| 2-2-d1E01-0-0221 | 0 | B05 | b3 | C10 | c1 |
| 2-2-d1E01-0-0222 | 1 | B01 | b1 | C04 | c2 |
| 2-2-d1E01-0-0223 | 0 | B05 | b2 | C02 | c1 |
| 2-2-d1E01-0-0224 | 0 | B05 | b2 | C03 | c1 |
| 2-2-d1E01-0-0225 | 1 | B03 | b4 | C05 | c1 |
| 2-2-d1E01-0-0226 | 0 | B04 | b1 | C18 | c1 |
| 2-2-d1E01-0-0227 | 0 | B05 | b2 | C04 | c1 |
| 2-2-d1E01-0-0228 | 0 | B05 | b2 | C06 | c1 |
| 2-2-d1E01-0-0229 | 0 | B05 | b2 | C07 | c1 |
| 2-2-d1E01-0-0230 | 0 | B01 | b1 | C15 | c2 |
| 2-2-d1E01-0-0231 | 1 | B02 | b1 | C01 | c2 |
| 2-2-d1E01-0-0232 | 0 | B01 | b1 | C16 | c2 |
| 2-2-d1E01-0-0233 | 1 | B05 | b1 | C02 | c1 |
| 2-2-d1E01-0-0234 | 1 | B05 | b1 | C03 | c1 |

**[Table 44]**

| | | | | | |
|---|---|---|---|---|---|
| 2-2-d1E01-0-0235 | 0 | B01 | b1 | C17 | c2 |
| 2-2-d1E01-0-0236 | 1 | B05 | b1 | C04 | c1 |
| 2-2-d1E01-0-0237 | 1 | B01 | b1 | C05 | c2 |
| 2-2-d1E01-0-0238 | 0 | B02 | b3 | C09 | c2 |
| 2-2-d1E01-0-0239 | 0 | B01 | b4 | C01 | c2 |
| 2-2-d1E01-0-0240 | 1 | B02 | b3 | C11 | c1 |
| 2-2-d1E01-0-0241 | 1 | B01 | b3 | C08 | c2 |
| 2-2-d1E01-0-0242 | 0 | B05 | b1 | C15 | c1 |
| 2-2-d1E01-0-0243 | 1 | B03 | b1 | C01 | c2 |
| 2-2-d1E01-0-0244 | 0 | B02 | b1 | C14 | c2 |
| 2-2-d1E01-0-0245 | 0 | B05 | b1 | C16 | c1 |
| 2-2-d1E01-0-0246 | 1 | B02 | b4 | C12 | c2 |
| 2-2-d1E01-0-0247 | 0 | B02 | b1 | C19 | c1 |
| 2-2-d1E01-0-0248 | 0 | B05 | b1 | C17 | c1 |
| 2-2-d1E01-0-0249 | 0 | B04 | b4 | C02 | c1 |
| 2-2-d1E01-0-0250 | 0 | B03 | b3 | C09 | c2 |
| 2-2-d1E01-0-0251 | 0 | B01 | b1 | C18 | c2 |
| 2-2-d1E01-0-0252 | 1 | B05 | b1 | C05 | c1 |
| 2-2-d1E01-0-0253 | 1 | B03 | b3 | C11 | c1 |
| 2-2-d1E01-0-0254 | 0 | B05 | b4 | C01 | c1 |
| 2-2-d1E01-0-0255 | 1 | B02 | b4 | C13 | c2 |
| 2-2-d1E01-0-0256 | 0 | B02 | b3 | C10 | c2 |
| 2-2-d1E01-0-0257 | 1 | B04 | b3 | C09 | c1 |
| 2-2-d1E01-0-0258 | 1 | B05 | b3 | C08 | c1 |
| 2-2-d1E01-0-0259 | 0 | B03 | b1 | C14 | c2 |
| 2-2-d1E01-0-0260 | 0 | B02 | b2 | C02 | c2 |
| 2-2-d1E01-0-0261 | 0 | B02 | b2 | C03 | c2 |
| 2-2-d1E01-0-0262 | 1 | B03 | b4 | C12 | c2 |
| 2-2-d1E01-0-0263 | 0 | B03 | b1 | C19 | c1 |
| 2-2-d1E01-0-0264 | 0 | B02 | b2 | C04 | c2 |
| 2-2-d1E01-0-0265 | 0 | B02 | b2 | C06 | c2 |
| 2-2-d1E01-0-0266 | 0 | B02 | b2 | C07 | c2 |
| 2-2-d1E01-0-0267 | 1 | B04 | b1 | C14 | c1 |
| 2-2-d1E01-0-0268 | 0 | B05 | b1 | C18 | c1 |

**[Table 45]**

| | | | | | |
|---|---|---|---|---|---|
| 2-2-d1E01-0-0269 | 1 | B02 | b1 | C02 | c2 |
| 2-2-d1E01-0-0270 | 1 | B02 | b1 | C03 | c2 |
| 2-2-d1E01-0-0271 | 1 | B03 | b4 | C13 | c2 |
| 2-2-d1E01-0-0272 | 0 | B03 | b3 | C10 | c2 |
| 2-2-d1E01-0-0273 | 0 | B04 | b4 | C05 | c1 |
| 2-2-d1E01-0-0274 | 1 | B02 | b1 | C04 | c2 |
| 2-2-d1E01-0-0275 | 1 | B04 | b3 | C10 | c1 |
| 2-2-d1E01-0-0276 | 0 | B03 | b2 | C02 | c2 |
| 2-2-d1E01-0-0277 | 0 | B03 | b2 | C03 | c2 |
| 2-2-d1E01-0-0278 | 0 | B01 | b4 | C02 | c2 |
| 2-2-d1E01-0-0279 | 0 | B03 | b2 | C04 | c2 |
| 2-2-d1E01-0-0280 | 0 | B03 | b2 | C06 | c2 |
| 2-2-d1E01-0-0281 | 0 | B03 | b2 | C07 | c2 |
| 2-2-d1E01-0-0282 | 1 | B04 | b2 | C02 | c1 |
| 2-2-d1E01-0-0283 | 1 | B04 | b2 | C03 | c1 |
| 2-2-d1E01-0-0284 | 0 | B02 | b1 | C15 | c2 |
| 2-2-d1E01-0-0285 | 1 | B01 | b3 | C09 | c2 |
| 2-2-d1E01-0-0286 | 1 | B03 | b1 | C02 | c2 |
| 2-2-d1E01-0-0287 | 1 | B03 | b1 | C03 | c2 |
| 2-2-d1E01-0-0288 | 1 | B04 | b2 | C04 | c1 |
| 2-2-d1E01-0-0289 | 1 | B04 | b2 | C06 | c1 |
| 2-2-d1E01-0-0290 | 1 | B04 | b2 | C07 | c1 |
| 2-2-d1E01-0-0291 | 0 | B02 | b1 | C16 | c2 |
| 2-2-d1E01-0-0292 | 1 | B03 | b1 | C04 | c2 |
| 2-2-d1E01-0-0293 | 0 | B02 | b1 | C17 | c2 |
| 2-2-d1E01-0-0294 | 1 | B02 | b1 | C05 | c2 |
| 2-2-d1E01-0-0295 | 1 | B01 | b1 | C14 | c2 |
| 2-2-d1E01-0-0296 | 0 | B02 | b4 | C01 | c2 |
| 2-2-d1E01-0-0297 | 0 | B05 | b4 | C02 | c1 |
| 2-2-d1E01-0-0298 | 1 | B01 | b1 | C19 | c1 |
| 2-2-d1E01-0-0299 | 0 | B04 | b1 | C01 | c2 |
| 2-2-d1E01-0-0300 | 1 | B02 | b3 | C08 | c2 |
| 2-2-d1E01-0-0301 | 0 | B03 | b1 | C15 | c2 |
| 2-2-d1E01-0-0302 | 0 | B01 | b4 | C05 | c2 |

**[Table 46]**

| | | | | | |
|---|---|---|---|---|---|
| 2-2-d1E01-0-0303 | 0 | B03 | b1 | C16 | c2 |
| 2-2-d1E01-0-0304 | 1 | B05 | b3 | C09 | c1 |
| 2-2-d1E01-0-0305 | 1 | B04 | b1 | C15 | c1 |
| 2-2-d1E01-0-0306 | 1 | B01 | b3 | C10 | c2 |
| 2-2-d1E01-0-0307 | 0 | B04 | b3 | C11 | c1 |
| 2-2-d1E01-0-0308 | 0 | B03 | b1 | C17 | c2 |
| 2-2-d1E01-0-0309 | 1 | B04 | b1 | C16 | c1 |
| 2-2-d1E01-0-0310 | 1 | B03 | b1 | C05 | c2 |
| 2-2-d1E01-0-0311 | 0 | B02 | b1 | C18 | c2 |
| 2-2-d1E01-0-0312 | 1 | B01 | b2 | C02 | c2 |
| 2-2-d1E01-0-0313 | 1 | B01 | b2 | C03 | c2 |
| 2-2-d1E01-0-0314 | 0 | B03 | b4 | C01 | c2 |
| 2-2-d1E01-0-0315 | 1 | B04 | b1 | C17 | c1 |
| 2-2-d1E01-0-0316 | 1 | B05 | b1 | C14 | c1 |
| 2-2-d1E01-0-0317 | 1 | B03 | b3 | C08 | c2 |
| 2-2-d1E01-0-0318 | 1 | B01 | b2 | C04 | c2 |
| 2-2-d1E01-0-0319 | 1 | B01 | b2 | C06 | c2 |
| 2-2-d1E01-0-0320 | 1 | B01 | b2 | C07 | c2 |
| 2-2-d1E01-0-0321 | 0 | B04 | b4 | C12 | c2 |
| 2-2-d1E01-0-0322 | 1 | B04 | b4 | C01 | c1 |
| 2-2-d1E01-0-0323 | 0 | B05 | b4 | 005 | c1 |
| 2-2-d1E01-0-0324 | 1 | B05 | b3 | C10 | c1 |
| 2-2-d1E01-0-0325 | 0 | B04 | b4 | C13 | c2 |
| 2-2-d1E01-0-0326 | 0 | B03 | b1 | C18 | c2 |
| 2-2-d1E01-0-0327 | 1 | B05 | b2 | C02 | c1 |
| 2-2-d1E01-0-0328 | 1 | B05 | b2 | C03 | c1 |
| 2-2-d1E01-0-0329 | 1 | B04 | b1 | C18 | c1 |
| 2-2-d1E01-0-0330 | 1 | B05 | b2 | C04 | c1 |
| 2-2-d1E01-0-0331 | 1 | B05 | b2 | C06 | c1 |
| 2-2-d1E01-0-0332 | 1 | B05 | b2 | C07 | c1 |
| 2-2-d1E01-0-0333 | 1 | B01 | b1 | C15 | c2 |
| 2-2-d1E01-0-0334 | 0 | B01 | b3 | C11 | c2 |
| 2-2-d1E01-0-0335 | 1 | B01 | b1 | 016 | c2 |
| 2-2-d1E01-0-0336 | 0 | B02 | b4 | C02 | c2 |

**[Table 47]**

| | | | | | |
|---|---|---|---|---|---|
| 2-2-d1E01-0-0337 | 0 | B04 | b1 | C02 | c2 |
| 2-2-d1E01-0-0338 | 0 | B04 | b1 | C03 | c2 |
| 2-2-d1E01-0-0339 | 1 | B01 | b1 | C17 | c2 |
| 2-2-d1E01-0-0340 | 0 | B04 | b1 | C04 | c2 |
| 2-2-d1E01-0-0341 | 0 | B05 | b1 | C01 | c2 |
| 2-2-d1E01-0-0342 | 1 | B02 | b3 | C09 | c2 |
| 2-2-d1E01-0-0343 | 1 | B01 | b4 | C01 | c2 |
| 2-2-d1E01-0-0344 | 1 | B05 | b1 | C15 | c1 |
| 2-2-d1E01-0-0345 | 0 | B05 | b3 | C11 | c1 |
| 2-2-d1E01-0-0346 | 1 | B02 | b1 | C14 | c2 |
| 2-2-d1E01-0-0347 | 0 | B03 | b4 | C02 | c2 |
| 2-2-d1E01-0-0348 | 1 | B05 | b1 | C16 | c1 |
| 2-2-d1E01-0-0349 | 1 | B02 | b1 | C19 | c1 |
| 2-2-d1E01-0-0350 | 1 | B05 | b1 | C17 | c1 |
| 2-2-d1E01-0-0351 | 1 | B04 | b4 | C02 | c1 |
| 2-2-d1E01-0-0352 | 1 | B03 | b3 | C09 | c2 |
| 2-2-d1E01-0-0353 | 1 | B01 | b1 | C18 | c2 |
| 2-2-d1E01-0-0354 | 0 | B02 | b4 | C05 | c2 |
| 2-2-d1E01-0-0355 | 0 | B05 | b4 | C12 | c2 |
| 2-2-d1E01-0-0356 | 1 | B05 | b4 | C01 | c1 |
| 2-2-d1E01-0-0357 | 0 | B04 | b1 | C05 | c2 |
| 2-2-d1E01-0-0358 | 1 | B02 | b3 | C10 | c2 |
| 2-2-d1E01-0-0359 | 0 | B04 | b3 | C08 | c2 |
| 2-2-d1E01-0-0360 | 1 | B03 | b1 | C14 | c2 |
| 2-2-d1E01-0-0361 | 0 | B05 | b4 | C13 | c2 |
| 2-2-d1E01-0-0362 | 1 | B02 | b2 | C02 | c2 |
| 2-2-d1E01-0-0363 | 1 | B02 | b2 | C03 | c2 |
| 2-2-d1E01-0-0364 | 1 | B03 | b1 | C19 | c1 |
| 2-2-d1E01-0-0365 | 1 | B02 | b2 | C04 | c2 |
| 2-2-d1E01-0-0366 | 1 | B02 | b2 | C06 | c2 |
| 2-2-d1E01-0-0367 | 1 | B02 | b2 | C07 | c2 |
| 2-2-d1E01-0-0368 | 0 | B03 | b4 | C05 | c2 |
| 2-2-d1E01-0-0369 | 1 | B05 | b1 | C18 | c1 |
| 2-2-d1E01-0-0370 | 1 | B03 | b3 | C10 | c2 |

**[Table 48]**

| | | | | | |
|---|---|---|---|---|---|
| 2-2-d1E01-0-0371 | 1 | B04 | b4 | C05 | c1 |
| 2-2-d1E01-0-0372 | 0 | B05 | b1 | C02 | c2 |
| 2-2-d1E01-0-0373 | 0 | B05 | b1 | C03 | c2 |
| 2-2-d1E01-0-0374 | 1 | B03 | b2 | C02 | c2 |
| 2-2-d1E01-0-0375 | 1 | B03 | b2 | C03 | c2 |
| 2-2-d1E01-0-0376 | 1 | B01 | b4 | C02 | c2 |
| 2-2-d1E01-0-0377 | 0 | B05 | b1 | C04 | c2 |
| 2-2-d1E01-0-0378 | 1 | B03 | b2 | C04 | c2 |
| 2-2-d1E01-0-0379 | 1 | B03 | b2 | C06 | c2 |
| 2-2-d1E01-0-0380 | 1 | B03 | b2 | C07 | c2 |
| 2-2-d1E01-0-0381 | 1 | B02 | b1 | C15 | c2 |
| 2-2-d1E01-0-0382 | 0 | B02 | b3 | C11 | c2 |
| 2-2-d1E01-0-0383 | 1 | B02 | b1 | C16 | c2 |
| 2-2-d1E01-0-0384 | 1 | B02 | b1 | C17 | c2 |
| 2-2-d1E01-0-0385 | 1 | B02 | b4 | C01 | c2 |
| 2-2-d1E01-0-0386 | 1 | B05 | b4 | C02 | c1 |
| 2-2-d1E01-0-0387 | 1 | B04 | b1 | C01 | c2 |
| 2-2-d1E01-0-0388 | 1 | B03 | b1 | C15 | c2 |
| 2-2-d1E01-0-0389 | 0 | B05 | b1 | C05 | c2 |
| 2-2-d1E01-0-0390 | 0 | B03 | b3 | C11 | c2 |
| 2-2-d1E01-0-0391 | 1 | B01 | b4 | C05 | c2 |
| 2-2-d1E01-0-0392 | 1 | B03 | b1 | C16 | c2 |
| 2-2-d1E01-0-0393 | 0 | B04 | b3 | C09 | c2 |
| 2-2-d1E01-0-0394 | 0 | B05 | b3 | C08 | c2 |
| 2-2-d1E01-0-0395 | 1 | B04 | b3 | C11 | c1 |
| 2-2-d1E01-0-0396 | 1 | B03 | b1 | C17 | c2 |
| 2-2-d1E01-0-0397 | 1 | B02 | b1 | C18 | c2 |
| 2-2-d1E01-0-0398 | 1 | B03 | b4 | C01 | c2 |
| 2-2-d1E01-0-0399 | 0 | B04 | b1 | C14 | c2 |
| 2-2-d1E01-0-0400 | 1 | B04 | b4 | C12 | c2 |
| 2-2-d1E01-0-0401 | 0 | B04 | b1 | C19 | c1 |
| 2-2-d1E01-0-0402 | 1 | B05 | b4 | C05 | c1 |
| 2-2-d1E01-0-0403 | 1 | B04 | b4 | C13 | c2 |
| 2-2-d1E01-0-0404 | 0 | B04 | b3 | C10 | c2 |

**[Table 49]**

| | | | | | |
|---|---|---|---|---|---|
| 2-2-d1E01-0-0405 | 1 | B03 | b1 | C18 | c2 |
| 2-2-d1E01-0-0406 | 0 | B04 | b2 | C02 | c2 |
| 2-2-d1E01-0-0407 | 0 | B04 | b2 | C03 | c2 |
| 2-2-d1E01-0-0408 | 0 | B04 | b2 | C04 | c2 |
| 2-2-d1E01-0-0409 | 0 | B04 | b2 | C06 | c2 |
| 2-2-d1E01-0-0410 | 0 | B04 | b2 | C07 | c2 |
| 2-2-d1E01-0-0411 | 1 | B01 | b3 | C11 | c2 |
| 2-2-d1E01-0-0412 | 1 | B02 | b4 | C02 | c2 |
| 2-2-d1E01-0-0413 | 1 | B04 | b1 | C02 | c2 |
| 2-2-d1E01-0-0414 | 1 | B04 | b1 | C03 | c2 |
| 2-2-d1E01-0-0415 | 1 | B04 | b1 | C04 | c2 |
| 2-2-d1E01-0-0416 | 1 | B05 | b1 | C01 | c2 |
| 2-2-d1E01-0-0417 | 0 | B01 | b1 | C19 | c2 |
| 2-2-d1E01-0-0418 | 0 | B05 | b3 | C09 | c2 |
| 2-2-d1E01-0-0419 | 0 | B04 | b1 | C15 | c2 |
| 2-2-d1E01-0-0420 | 1 | B05 | b3 | C11 | c1 |
| 2-2-d1E01-0-0421 | 1 | B03 | b4 | C02 | c2 |
| 2-2-d1E01-0-0422 | 0 | B04 | b1 | C16 | c2 |
| 2-2-d1E01-0-0423 | 0 | B04 | b1 | C17 | c2 |
| 2-2-d1E01-0-0424 | 0 | B05 | b1 | C14 | c2 |
| 2-2-d1E01-0-0425 | 1 | B02 | b4 | C05 | c2 |
| 2-2-d1E01-0-0426 | 1 | B05 | b4 | C12 | c2 |
| 2-2-d1E01-0.0427 | 0 | B05 | b1 | C19 | c1 |
| 2-2-d1E01-0-0428 | 1 | B04 | b1 | C05 | c2 |
| 2-2-d1E01-0-0429 | 0 | B04 | b4 | C01 | c2 |
| 2-2-d1E01-0-0430 | 1 | B04 | b3 | C08 | c2 |
| 2-2-d1E01-0-0431 | 1 | B05 | b4 | C13 | c2 |
| 2-2-d1E01-0-0432 | 0 | B05 | b3 | C10 | c2 |
| 2-2-d1E01-0-0433 | 0 | B05 | b2 | C02 | c2 |
| 2-2-d1E01-0-0434 | 0 | B05 | b2 | C03 | c2 |
| 2.2-d1E01-0-0435 | 1 | B03 | b4 | C05 | c2 |
| 2-2-d1E01-0-0436 | 0 | B04 | b1 | C18 | c2 |
| 2-2-d1E01-0-0437 | 0 | B05 | b2 | C04 | c2 |
| 2-2-d1E01-0-0438 | 0 | B05 | b2 | COS | c2 |

**[Table 50]**

| | | | | | |
|---|---|---|---|---|---|
| 2-2-d1E01-0-0439 | 0 | B05 | b2 | C07 | c2 |
| 2-2-d1E01-0-0440 | 1 | B05 | b1 | C02 | c2 |
| 2-2-d1E01-0-0441 | 1 | B05 | b1 | C03 | c2 |
| 2-2-d1E01-0-0442 | 1 | B05 | b1 | C04 | c2 |
| 2-2-d1E01-0-0443 | 1 | B02 | b3 | C11 | c2 |
| 2-2-d1E01-0-0444 | 0 | B05 | b1 | C15 | c2 |
| 2-2-d1E01-0-0445 | 0 | B05 | b1 | C16 | c2 |
| 2-2-d1E01-0-0446 | 0 | B02 | b1 | C19 | c2 |
| 2-2-d1E01-0-0447 | 0 | B05 | b1 | C17 | c2 |
| 2-2-d1E01-0-0448 | 0 | B04 | b4 | C02 | c2 |
| 2-2-d1E01-0-0449 | 1 | B05 | b1 | C05 | c2 |
| 2-2-d1E01-0-0450 | 1 | B05 | b3 | C11 | c2 |
| 2-2-d1E01-0-0451 | 0 | B05 | b4 | C01 | c2 |
| 2-2-d1E01-0-0452 | 1 | B04 | b3 | C09 | c2 |
| 2-2-d1E01-0-0453 | 1 | B05 | b3 | C08 | c2 |
| 2-2-d1E01-0-0454 | 0 | B03 | b1 | C19 | c2 |
| 2-2-d1E01-0-0456 | 1 | B04 | b1 | C14 | c2 |
| 2-2-d1E01-0-0456 | 1 | B04 | b1 | C19 | c1 |
| 2-2-d1E01-0-0457 | 0 | B05 | b1 | C18 | c2 |
| 2-2-d1E01-0-0458 | 0 | B04 | b4 | C05 | c2 |
| 2-2-d1E01-0-0459 | 1 | B04 | b3 | C10 | c2 |
| 2-2-d1E01-0-0460 | 1 | B04 | b2 | C02 | c2 |
| 2-2-d1E01-0-0461 | 1 | B04 | b2 | C03 | c2 |
| 2-2-d1E01-0-0462 | 1 | B04 | b2 | C04 | c2 |
| 2-2-d1E01-0-0463 | 1 | B04 | b2 | C06 | c2 |
| 2-2-d1E01-0-0464 | 1 | B04 | b2 | C07 | c2 |
| 2-2-d1E01-0-0465 | 0 | B05 | b4 | C02 | c2 |
| 2-2-d1E01-0-0466 | 1 | B01 | b1 | C19 | c2 |
| 2-2-d1E01-0-0467 | 1 | B05 | b3 | C09 | c2 |
| 2-2-d1E01-0-0468 | 1 | B04 | b1 | C15 | c2 |
| 2-2-d1E01-0-0469 | 0 | B04 | b3 | C11 | c2 |
| 2-2-d1E01-0-0470 | 1 | B04 | b1 | C16 | c2 |
| 2-2-d1E01-0-0471 | 1 | B04 | b1 | C17 | c2 |
| 2-2-d1E01-0-0472 | 1 | B05 | b1 | C14 | c2 |

**[Table 51]**

| | | | | | |
|---|---|---|---|---|---|
| 2-2-d1E01-0-0473 | 1 | B05 | b1 | C19 | c1 |
| 2-2-d1E01-0-0474 | 1 | B04 | b4 | C01 | c2 |
| 2-2-d1E01-0-0475 | 0 | B05 | b4 | C05 | c2 |
| 2-2-d1E01-0-0476 | 1 | B05 | b3 | C10 | c2 |
| 2-2-d1E01-0-0477 | 1 | B05 | b2 | C02 | c2 |
| 2-2-d1EC1-0-0478 | 1 | B05 | b2 | C03 | c2 |
| 2-2-d1E01-0-0479 | 1 | B04 | b1 | C18 | c2 |
| 2-2-d1E01-0-0480 | 1 | B05 | b2 | C04 | c2 |
| 2-2-d1E01-0-0481 | 1 | B05 | b2 | C06 | c2 |
| 2-2-d1E01-0-0482 | 1 | B05 | b2 | C07 | c2 |
| 2-2-d1E01-0-0483 | 1 | B05 | b1 | C15 | c2 |
| 2-2-d1E01-0-0484 | 0 | B05 | b3 | C11 | c2 |
| 2-2-d1E01-0-0485 | 1 | B05 | b1 | C16 | c2 |
| 2-2-d1E01-0-0486 | 1 | B02 | b1 | C19 | c2 |
| 2-2-d1E01-0-0487 | 1 | B05 | b1 | C17 | c2 |
| 2-2-d1E01-0-0488 | 1 | B04 | b4 | C02 | c2 |
| 2-2-d1E01-0-0489 | 1 | B05 | b4 | C01 | c2 |
| 2-2-d1E01-0-0490 | 1 | B03 | b1 | C19 | c2 |
| 2-2-d1E01-0-0491 | 1 | B05 | b1 | C18 | c2 |
| 2-2-d1E01-0-0492 | 1 | B04 | b4 | C05 | c2 |
| 2-2-d1E01-0-0493 | 1 | B05 | b4 | C02 | c2 |
| 2-2-d1E01-0-0494 | 1 | B04 | b3 | C11 | c2 |
| 2-2-d1E01-0-0495 | 0 | B04 | b1 | C19 | c2 |
| 2-2-d1E01-0-0496 | 1 | B05 | b4 | C05 | c2 |
| 2-2-d1E01-0-0497 | 1 | B05 | b3 | C11 | c2 |
| 2-2-d1E01-0-0498 | 0 | B05 | b1 | C19 | c2 |
| 2-2-d1E01-0-0499 | 1 | B04 | b1 | C19 | c2 |
| 2-2-d1E01-0-0500 | 1 | B05 | b1 | C19 | c2 |

### Example 4-1-4: Confirmation of progress of amidation reaction of Example 4-1-1 by analysis of mixture 2-2-d1E01-1

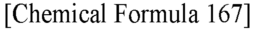

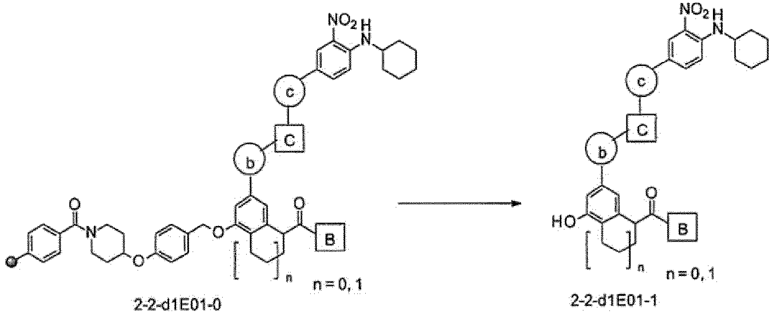

To a 2 mL glass vial, the mixture 2-2-d1E01-0 (50 mg) was added. To this, a solution of pentamethylbenzene in DCM (0.08 M, 1.0 mL) was added, and the mixture was shaken at room temperature for 30 minutes. To this, TFA (0.10 mL) was added, and the mixture was shaken at room temperature for 30 minutes. The suspension of the reaction solution and solid phase was transferred to a 3 mL column equipped with a filter using DMI (0.5 mL) twice and filtered. The filtrate was washed twice with DMI (0.5 mL), and the filtrates were combined and transferred to a 15 mm * 75 mm tube. The solution was distilled off with Genevac. Morpholine supported on solid phase (Aldrich Cat. No. 493813) (80 mg) and DMI (0.4 mL) were added, and the mixture was shaken at 1500 rpm for 15 minutes. The suspension of the solution and solid phase was transferred to a 3 mL column equipped with a filter using DMI (0.4 mL) twice and filtered. The filtrate was washed twice with DMI (0.2 mL) and twice with MeCN (0.2 mL), and the filtrates were combined and transferred to a 0.5-2 mL microwave vessel. The solution was distilled off under reduced pressure (Genevac vacuum concentrator was used). The residue was dissolved in dimethyl sulfoxide (0.185 mL) to obtain a solution of the mixture 2-2-d1E01-1. The compounds in mixture 2-2-d1E01-1 were subjected to measurements of retention time and mass spectrometry under the analysis conditions shown below and analyzed using Compound Discover 3.2 (Thermo Fisher Scientific).

**[Table 52]**

| Reverse phase LC/MS conditions | |
|---|---|
| Vanquish UHPLC (Thermo Fisher Scientific) | |
| Oven (°C) | 35 |
| Column | Ascentis Express C18 |
| | 2. 1mmI. D. x 150mm, 2. 7*µ*m |
| Auto Sampl er (°C) | 20 |
| Mobile phase | A) 0.1%FA H2O |
| | B) 0. 1%FA MeCN |
| Gradient | A/B = 95/5 → 0/100 (18min) → 0/ 100 (20min) |
| Flow rate | 0.5mL/min |

| Orbitrap Fusion Lumos Tribrid Mass spectrometer (Thermo Fisher Scientific) | |
|---|---|
| Polarity | Positive / Negative mode |
| Data Type | profile |

As a result, the m/z and retention times of 499 out of 500 compounds that could be contained in the mixture 2-2-d1E01-1 were observed as shown in Table 4-1-4. Thus, it was shown that 99.8% of the mixture 2-2-d1E01-0 was synthesized, and 99.6% or more of the mixture 2-2-c1D01-0, which is the previous stage mixture of the mixture 2-2-d1E01-0, was also synthesized.

The above results showed that the amidation reaction of the mixture 2-2-D00-0 in Example 4-1-1 proceeds without problems. The results also showed that the amidation reaction conditions are applicable even when the substrate is not a single compound, but a mixture of a plurality of compounds.

**[Table 53]**

| [Table 4-1-4] | | | |
|---|---|---|---|
| Ion species | m/z [M+H] or [M-H]- | Retention time of MS peak (min) | Attributed compound number (noted without "2-2-d1E01-1-") |
| M+H | 569.2389 | 12.807 | 0001 |
| M+H | 573.3063 | 12.662 | 0002 |
| M-H | 573.2697 | 12.323 | 0003 |
| M+H | 579.2597 | 13.22 | 0004,0005 |
| M+H | 579.2597 | 13.111 | 0004,0005 |
| M-H | 578.2389 | 12.008 | 0006 |
| M+H | 583.2546 | 14.995 | 0007 |
| M+H | 585.2164 | 13.193 | 0008 |
| M+H | 586.2295 | 12.594 | 0009 |
| M+H | 587.2292 | 12.896 | 0010 |
| M+H | 587.3219 | 13.083 | 0011 |
| M+H | 589.3017 | 12.745 | 0012 |
| M-H | 589.2813 | 12.812 | 0013 |
| M+H | 593.2765 | 12.427 | 0014,0015,0016 |
| M+H | 593.2755 | 13.511 | 0014,0015,0016 |
| M-H | 591.2591 | 13.358 | 0014,0015,0016 |
| M+H | 594.2734 | 12.422 | 0017 |
| M+H | 595.2543 | 13.05 | 0018 |
| M+H | 597.2454 | 12.566 | 0019 |
| M+H | 597.2501 | 13.2 | 0019,0020,0021 |
| M+H | 597.2502 | 13.272 | 0019,0020,0021 |
| M-H | 596.2294 | 12.134 | 0022 |
| M+H | 599.2315 | 13.535 | 0023 |
| M+H | 599.2609 | 12.642 | 0024 |
| M+H | 599.3216 | 13.072 | 0026 |
| M+H | 600.2446 | 13.001 | 0026 |
| M+H | 601.3012 | 12.731 | 0027 |
| M+H | 602.2062 | 13.044 | 0028 |
| M+H | 603.2068 | 13.232 | 0029 |
| M+H | 603.2596 | 13.778 | 0030,0031 |
| M-H | 601.2431 | 13.67 | 0030,0031 |
| M+H | 604.2196 | 12.677 | 0032 |
| M+H | 604.2550 | 12.862 | 0033,0034,0035 |
| M+H | 604.2549 | 13.956 | 0033,0034,0035 |
| M+H | 604.2549 | 14.295 | 0033,0034,0035 |

**[Table 54]**

| | | | |
|---|---|---|---|
| M+H | 605.2763 | 13.33 | 0036,0037 |
| M+H | 605.2747 | 13.229 | 0036,0037 |
| M+H | 606.2704 | 12.241 | 0038 |
| M+H | 608.2858 | 13.059 | 0039 |
| M+H | 609.2705 | 13.454 | 0040 |
| M+H | 609.2821 | 13.634 | 0041 |
| M+H | 610.2766 | 12.735 | 0042 |
| M+H | 611.2301 | 14.63 | 0043 |
| M+H | 611.2604 | 13.006 | 0044 |
| M+H | 611.2855 | 13.279 | 0045 |
| M+H | 612.2452 | 12.902 | 0046 |
| M+H | 613.2453 | 13.082 | 0047 |
| M+H | 613.2763 | 13.056 | 0048 |
| M+H | 613.3377 | 13.512 | 0049 |
| M+H | 615.2351 | 12.66 | 0050,0051 |
| M+H | 615.2390 | 13.193 | 0050,0051 |
| M+H | 615.3165 | 13.136 | 0052 |
| M+H | 616.2222 | 13.408 | 0053 |
| M+H | 617.2511 | 12.74 | 0054 |
| M+H | 617.2750 | 14.155 | 0055,0056 |
| M+H | 617.2750 | 14.056 | 0055,0056 |
| M+H | 617.3118 | 11.17 | 0057 |
| M+H | 618.2703 | 13.28 | 0058,0059,0060 |
| M-H | 616.2540 | 14.364 | 0058,0059,0060 |
| M+H | 618.2705 | 14.675 | 0058,0059,0060 |
| M+H | 619.2922 | 12.799 | 0061,0062,0063 |
| M+H | 619.2924 | 12.854 | 0061,0062,0063 |
| M+H | 619.2908 | 13.76 | 0061,0062,0063 |
| M+H | 620.1971 | 13.083 | 0064 |
| M+H | 620.2859 | 12.723 | 0065 |
| M+H | 621.2503 | 13.759 | 0066,0067 |
| M+H | 621.2503 | 13.847 | 0066,0067 |
| M+H | 621.3066 | 13.667 | 0068 |
| M+H | 622.2456 | 12.964 | 0069,0070,0071 |
| M+H | 622.2454 | 14.403 | 0069,0070,0071 |
| M+H | 622.2453 | 14.028 | 0069,0070,0071 |
| M+H | 622.3008 | 13.419 | 0072 |
| M+H | 623.2598 | 13.049 | 0073 |
| M+H | 623.2661 | 13.391 | 0073,0074,0075 |
| M+H | 623.2656 | 13.249 | 0073,0074,0075 |
| M+H | 623.2970 | 14.045 | 0076 |
| M-H | 622.2445 | 12.35 | 0077 |

**[Table 55]**

| | | | |
|---|---|---|---|
| M+H | 624.2924 | 13.151 | 0078 |
| M-H | 623.2343 | 11.393 | 0079 |
| M+H | 625.2756 | 12.876 | 0080 |
| M-H | 623.2840 | 13.679 | 0081 |
| M-H | 624.1891 | 13.317 | 0082 |
| M-H | 624.2445 | 12.009 | 0083 |
| M+H | 626.2756 | 11.537 | 0084 |
| M+H | 627.2665 | 13.663 | 0085 |
| M+H | 627.2723 | 13.719 | 0086 |
| M+H | 628.2235 | 11.147 | 0087 |
| M+H | 628.2678 | 14.14 | 0088 |
| M+H | 629.2223 | 13.4 | 0089 |
| M+H | 629.2540 | 13.594 | 0090 |
| M+H | 629.2770 | 13.356 | 0091,0092,0093 |
| M+H | 629.2752 | 13.939 | 0091,0092,093 |
| M+H | 629.2750 | 13.827 | 0091,0092,0093 |
| M+H | 630.2362 | 12.887 | 0094 |
| M+H | 630.2712 | 13.117 | 0095,0096,0097,0098 |
| M+H | 630.2705 | 14.474 | 0095,0096,0097,0098 |
| M+H | 630.2708 | 14.428 | 0095,0096,0097,0098 |
| M+H | 630.2735 | 13.223 | 0095,0096,0097,0098 |
| M-H | 630.2362 | 12.898 | 0099 |
| M+H | 633.2008 | 12.5 | 0100 |
| M+H | 633.2282 | 13.236 | 0101 |
| M+H | 634.3016 | 8.625 | 0102 |
| M+H | 635.2495 | 13.699 | 0103 |
| M+H | 635.2972 | 13.946 | 0104 |
| M+H | 635.3227 | 14.208 | 0105 |
| M+H | 636.2277 | 13.487 | 0106,0107 |
| M+H | 636.2278 | 13.634 | 0106,0107 |
| M+H | 636.2921 | 12.919 | 0108 |
| M+H | 637.2220 | 12.563 | 0109,0110 |
| M+H | 637.2257 | 13.829 | 0109,0110 |
| M+H | 637.2682 | 12.257 | 0111 |
| M+H | 637.2759 | 13.496 | 0112 |
| M+H | 637.3028 | 13.715 | 0113 |
| M+H | 639.2481 | 12.058 | 0114 |
| M+H | 639.2915 | 13.33 | 0115 |
| M+H | 639.2973 | 13.883 | 0115,0116 |
| M+H | 641.2505 | 13.09 | 0117,0118 |
| M+H | 641.2525 | 14.583 | 0117,0118 |
| M+H | 641.2786 | 13.981 | 0119 |

**[Table 56]**

| | | | |
|---|---|---|---|
| M+H | 641.2941 | 13.765 | 0120 |
| M+H | 642.1835 | 13.629 | 0121 |
| M+H | 642.2376 | 13.679 | 0122 |
| M-H | 641.1796 | 13.102 | 0123 |
| M+H | 643.2190 | 12.921 | 0124,0125 |
| M-H | 641.2046 | 12.524 | 0124,0125 |
| M+H | 643.2671 | 12.933 | 0126 |
| M+H | 643.2738 | 13.404 | 0127 |
| M+H | 643.2908 | 14.383 | 0128,0129 |
| M+H | 643.2908 | 14.253 | 0128,0129 |
| M+H | 644.2167 | 11.628 | 0130 |
| M+H | 644.2863 | 14.559 | 0131,0132,0133 |
| M+H | 644.2864 | 14.962 | 0131,0132,0133 |
| M-H | 642.2690 | 14.859 | 0131,0132,0133 |
| M+H | 645.2536 | 13.732 | 0134 |
| M+H | 646.2123 | 13.291 | 0135 |
| M+H | 647.2155 | 14.679 | 0136 |
| M+H | 647.2470 | 14.125 | 0137,0138 |
| M+H | 647.2484 | 13.982 | 0137,0138 |
| M+H | 647.2659 | 10.425 | 0139,0140 |
| M-H | 645.2486 | 13.901 | 0139,0140 |
| M-H | 645.3043 | 14.117 | 0141 |
| M-H | 646.2254 | 13.171 | 0142 |
| M+H | 648.2617 | 14.501 | 0143,0144,0145 |
| M+H | 648.2602 | 14.164 | 0143,0144,0145 |
| M+H | 648.2616 | 14.193 | 0143,0144,0145 |
| M+H | 648.3170 | 13.398 | 0146 |
| M+H | 649.1776 | 12.652 | 0147 |
| M+H | 649.2664 | 13.19 | 0148 |
| M+H | 649.3138 | 14.403 | 0149 |
| M-H | 648.1750 | 12.412 | 0150 |
| M-H | 648.2912 | 13.415 | 0151 |
| M-H | 649.1745 | 12.613 | 0152 |
| M+H | 651.2835 | 12.65 | 0153 |
| M+H | 651.3167 | 13.686 | 0154 |
| M+H | 652.2223 | 13.525 | 0155 |
| M+H | 652.2933 | 13.634 | 0156 |
| M+H | 653.2031 | 14.152 | 0157 |
| M+H | 653.2404 | 13.679 | 0158 |
| M+H | 653.2634 | 12.367 | 0159 |
| M+H | 653.2774 | 13.169 | 0160 |
| M+H | 653.2907 | 12.435 | 0161 |

**[Table 57]**

| | | | |
|---|---|---|---|
| M+H | 654.2130 | 12.99 | 0162,0163 |
| M+H | 654.2163 | 13.666 | 0162,0163 |
| M+H | 654.2824 | 12.999 | 0164 |
| M-H | 653.2424 | 12.326 | 0165 |
| M-H | 653.2518 | 12.808 | 0166 |
| M-H | 653.2738 | 13.679 | 0167 |
| M+H | 656.2272 | 13,179 | 0168 |
| M+H | 656.2900 | 13.763 | 0169 |
| M-H | 655.2220 | 12.184 | 0170,0171,0172 |
| M+H | 657.2356 | 13.124 | 0170,0171,0172 |
| M+H | 657.2384 | 12.138 | 0170,0171,0172 |
| M+H | 658.2332 | 12.126 | 0173 |
| M-H | 656.2516 | 13.286 | 0174 |
| M-H | 657.1558 | 13.531 | 0175 |
| M+H | 659.2161 | 12.766 | 0176 |
| M+H | 659.2442 | 13,424 | 0177 |
| M+H | 660.2271 | 14.28 | 0178,0179 |
| M+H | 660.2270 | 14.169 | 0178,0179 |
| M-H | 659.1907 | 12.422 | 0180 |
| M+H | 661.2123 | 12.839 | 0180,0181,0182 |
| M+H | 661.2120 | 12.619 | 0180,0181,0182, |
| M+H | 661.2223 | 14.471 | 0183,0184,0185 |
| M+H | 661.2222 | 14.832 | 0183,0184,0185 |
| M+H | 661.2223 | 13.399 | 0183,0184,0185 |
| M+H | 661.2651 | 13.74 | 0186 |
| M+H | 661.3385 | 14.512 | 0187 |
| M+H | 662.2075 | 11.799 | 0188 |
| M+H | 662.2432 | 13.872 | 0189,0190 |
| M+H | 662.2431 | 13.698 | 0189,0190 |
| M+H | 663.1931 | 13.056 | 0191 |
| M+H | 663.2224 | 12.257 | 0192 |
| M+H | 663.2378 | 12.853 | 0193,0194 |
| M+H | 663.2416 | 14.051 | 0193,0194 |
| M-H | 661.2681 | 12.78 | 0195 |
| M+H | 663.3280 | 12.897 | 0196 |
| M-H | 662.1916 | 10.825 | 0197 |
| M+H | 665.2324 | 13.662 | 0198 |
| M+H | 665.2570 | 13.985 | 0199 |
| M+H | 665.2632 | 12.482 | 0200 |
| M+H | 665.3124 | 14.153 | 0201 |
| M+H | 666.1661 | 11.045 | 0202 |
| M+H | 666.2485 | 14.157 | 0203 |

**[Table 58]**

| | | | |
|---|---|---|---|
| M+H | 667.1682 | 12.761 | 0204 |
| M+H | 667.2208 | 13.162 | 0205,0206 |
| M+H | 667.2210 | 13.225 | 0205,0206 |
| M+H | 667.2439 | 13.398 | 0207,0208 |
| M+H | 667.2481 | 13.793 | 0207,0208 |
| M+H | 667.2935 | 14.387 | 0209 |
| M+H | 667.3102 | 14.237 | 0210 |
| M-H | 666.1652 | 12.521 | 0211 |
| M+H | 668.1998 | 13.842 | 0212 |
| M+H | 668.2161 | 12.962 | 0213,0214,0215 |
| M-H | 666.1998 | 13.417 | 0213,0214,0215 |
| M+H | 668.2171 | 12.917 | 0213,0214,0215 |
| M+H | 668.2532 | 13.821 | 0216 |
| M+H | 669.2098 | 13.713 | 0217 |
| M+H | 669.2373 | 13.009 | 0218,0219 |
| M+H | 669.2365 | 12.81 | 0218,0219 |
| M+H | 669.2884 | 13.842 | 0220 |
| M+H | 670.1936 | 14.086 | 0221 |
| M+H | 670.2325 | 11.909 | 0222 |
| M+H | 671.2470 | 14.691 | 0223,0224 |
| M+H | 671.2471 | 14.617 | 0223,0224 |
| M+H | 671.2691 | 14.023 | 0225 |
| M+H | 672.2052 | 13.707 | 0226 |
| M+H | 672.2424 | 13.901 | 0227,0228,0229 |
| M+H | 672.2422 | 14.912 | 0227,0228,0229 |
| M+H | 672.2424 | 15.237 | 0227,0228,0229 |
| M+H | 672.2474 | 12.549 | 0227,0228,0229,0230 |
| M-H | 671.2173 | 11.717 | 0231 |
| M+H | 673.2419 | 13.231 | 0232 |
| M+H | 673.2633 | 14.341 | 0233,0234 |
| M+H | 673.2632 | 14.232 | 0233,0234 |
| M+H | 674.2388 | 12.068 | 0235 |
| M+H | 674.2576 | 13.42 | 0236 |
| M+H | 675.1918 | 12.345 | 0237 |
| M-H | 673.2056 | 12.84 | 0238 |
| M+H | 675.2474 | 12.667 | 0239 |
| M+H | 675.2836 | 12.948 | 0240 |
| M+H | 676.2071 | 12.742 | 0241 |
| M-H | 674.2597 | 13.12 | 0242 |
| MM-H | 675.1900 | 12.807 | 0243 |
| M-H | 675.2207 | 12.671 | 0244 |
| M+H | 677.2705 | 14.612 | 0245 |

**[Table 59]**

| | | | |
|---|---|---|---|
| M+H | 677.2994 | 13.165 | 0246 |
| M+H | 677.3435 | 13.283 | 0247 |
| M+H | 678.2642 | 13.886 | 0248 |
| M+H | 678.2740 | 14.333 | 0249 |
| M+H | 679.1996 | 12,791 | 0250,0251 |
| M+H | 679.1997 | 12.743 | 0250,0251 |
| M+H | 679.2195 | 14.357 | 0252 |
| M+H | 679.2575 | 12.412 | 0253 |
| M-H | 677.2615 | 12.961 | 0254,0255 |
| M-H | 677.2626 | 12.447 | 0254,0255 |
| M+H | 680.1835 | 13.062 | 0256 |
| M+H | 680.2290 | 13.486 | 0257,0258 |
| M+H | 680.2321 | 13.938 | 0257,0258 |
| M+H | 681.2131 | 12.377 | 0259 |
| M+H | 681.2365 | 13.599 | 0260,0261 |
| M+H | 681.2359 | 13.648 | 0260,0261 |
| M+H | 681.2768 | 13.306 | 0262 |
| M-H | 679.3018 | 13 | 0263 |
| M+H | 682.2323 | 13.748 | 0264,0265,0266 |
| M-H | 680.2147 | 13.796 | 0264,0265,0266 |
| M+H | 682.2329 | 11.955 | 0264,0265,0266 |
| M+H | 682.2418 | 13.419 | 0267 |
| M+H | 683.2255 | 14.196 | 0268 |
| M+H | 683.2566 | 10.467 | 0269,0270,0271 |
| M+H | 683.2566 | 10.441 | 0269,0270,0271 |
| M+H | 683.2541 | 12.605 | 0269,0270,0271 |
| M+H | 684.1577 | 12.729 | 0272 |
| M+H | 684.2307 | 14.401 | 0273 |
| M+H | 684.2478 | 12.447 | 0274 |
| M-H | 683.1715 | 13.706 | 0275 |
| M+H | 685.2119 | 13.349 | 0276,0277 |
| M+H | 685.2119 | 13.256 | 0276,0277 |
| M-H | 683.2507 | 13.169 | 0278 |
| M+H | 686.2070 | 13.47 | 0279,0280,0281 |
| M+H | 686.2068 | 13.086 | 0279,0280,0281 |
| M-H | 684.1893 | 13.515 | 0279,0280,0281 |
| M+H | 686.2430 | 14.437 | 0282,0283 |
| M+H | 686.2423 | 14.301 | 0282,0283 |
| M+H | 686.2635 | 12.943 | 0284 |
| M+H | 687.2234 | 12.818 | 0285,0286,0287 |
| M+H | 687.2277 | 13.034 | 0285,0286,0287 |
| M+H | 687.2274 | 12.863 | 0285,0286,0287 |

**[Table 60]**

| | | | |
|---|---|---|---|
| M+H | 687.2382 | 14.625 | 0288,0289,0290 |
| M+H | 687.2382 | 14.932 | 0288,0289,0290 |
| M+H | 687.2378 | 13.631 | 0288,0289,0290 |
| M+H | 687.2593 | 13.668 | 0291 |
| M+H | 688.2234 | 12.062 | 0292 |
| M+H | 688.2575 | 8.242 | 0293 |
| M+H | 689.2094 | 10.752 | 0294 |
| M+H | 689.2382 | 12.771 | 0295 |
| M+H | 689.2634 | 13.132 | 0296 |
| M+H | 689.2945 | 14.701 | 0297 |
| M+H | 689.3437 | 12.908 | 0298 |
| M+H | 690.1676 | 12.982 | 0299 |
| M+H | 690,2223 | 13.187 | 0300 |
| M-H | 688.2218 | 12.663 | 0301 |
| M+H | 691.2239 | 12.988 | 0302 |
| M+H | 691.2336 | 13.35 | 0303 |
| M+H | 691.2483 | 14.084 | 0304 |
| M+H | 691.2687 | 13.819 | 0305 |
| M+H | 692.1815 | 12.901 | 0306 |
| M+H | 692.2166 | 14.268 | 0307 |
| M-H | 690.2134 | 12.224 | 0308 |
| M+H | 692.2641 | 14.487 | 0309 |
| M+H | 693.1831 | 13.048 | 0310 |
| M+H | 693,27.55 | 13.186 | 0311 |
| M-H | 691.2205 | 12.788 | 0312,0313,0314 |
| M-H | 691.2212 | 12.832 | 0312,0313,0314 |
| M+H | 693,2378 | 12.872 | 0312,0313,0314 |
| M+H | 693.2602 | 13.564 | 0315,0316 |
| M+H | 693.2640 | 13.942 | 0315,0316 |
| M+H | 694.1979 | 12.827 | 0317 |
| M+H | 694.2355 | 12.732 | 0318,0319,0320,0321 |
| M+H | 694.2342 | 13.044 | 0318,0319,0320,0321 |
| M-H | 692.2166 | 13.645 | 0318,0319,0320,0321 |
| M+H | 694.2355 | 12.769 | 0318,0319,0320,0321 |
| M+H | 694.2685 | 14.154 | 0322 |
| M+H | 695.2503 | 14.577 | 0323 |
| M-H | 694.1912 | 14.272 | 0324 |
| M+H | 696.2149 | 12.583 | 0325 |
| M+H | 697.1932 | 12.266 | 0326 |
| M+H | 697.2624 | 14.789 | 0327,0328 |
| M+H | 697.2623 | 14.878 | 0327,0328 |
| M+H | 698.2207 | 13.995 | 0329 |

**[Table 61]**

| | | | |
|---|---|---|---|
| M+H | 698.2586 | 15.063 | 0330,0331,0332 |
| M+H | 698.2587 | 15.342 | 0330,0331,0332 |
| M+H | 698.2587 | 15.376 | 0330,0331,0332 |
| M+H | 698.2643 | 13.304 | 0333 |
| M+H | 699.2105 | 13.157 | 0334 |
| M+H | 699.2602 | 15.45 | 0335 |
| M+H | 699.2834 | 13.668 | 0336 |
| M+H | 700.1890 | 13.235 | 0337,0338 |
| M+H | 700.1891 | 13.015 | 0337,0338 |
| M+H | 700.2517 | 13.144 | 0339 |
| M-H | 699.1675 | 12.258 | 0340 |
| M-H | 699.1701 | 13.575 | 0340,0341 |
| M-H | 699.2214 | 13.273 | 0342 |
| M+H | 701.2625 | 13.176 | 0343 |
| M+H | 702.2888 | 14.294 | 0344 |
| M-H | 701.2192 | 12.206 | 0345 |
| M+H | 703.2538 | 13.155 | 0346 |
| M+H | 703.2587 | 13.321 | 0346,0347 |
| M+H | 703.2843 | 14.969 | 0348 |
| M+H | 703.3594 | 13.345 | 0349 |
| M+H | 704.2795 | 14.082 | 0350 |
| M+H | 704.2886 | 14.673 | 0351 |
| M+H | 705.2109 | 13.053 | 0352 |
| M-H | 703.2003 | 10.481 | 0352,0353 |
| M+H | 705.2407 | 13.398 | 0354 |
| M-H | 703.2379 | 13.328 | 0355 |
| M-H | 703.2725 | 14.59 | 0356 |
| M+H | 706.1453 | 13.148 | 0357 |
| M+H | 706.1997 | 13.362 | 0358 |
| M+H | 707.1579 | 12.952 | 0359 |
| M+H | 707.2288 | 12.699 | 0360 |
| M-H | 705.2179 | 13.15 | 0360,0361 |
| M+H | 707.2528 | 13.796 | 0362,0363 |
| M+H | 707.2521 | 13.927 | 0362,0363 |
| M+H | 707.3342 | 12.997 | 0364 |
| M+H | 708.2478 | 14.053 | 0365,0366,0367 |
| M+H | 708.2490 | 13.819 | 0365,0366,0367 |
| M-H | 706.2330 | 13.74 | 0365,0366,0367 |
| M+H | 709.2151 | 13.087 | 0368 |
| M+H | 709.2405 | 14.411 | 0369 |
| M+H | 710.1733 | 13.034 | 0370 |
| M+H | 710.2463 | 14.491 | 0371 |

**[Table 62]**

| | | | |
|---|---|---|---|
| M+H | 711.2082 | 13.76 | 0372,0373 |
| M+H | 711.2086 | 13.716 | 0372,0373 |
| M+H | 711.2274 | 13.581 | 0374,0375 |
| M+H | 711.2273 | 13.469 | 0374,0375 |
| M+H | 711.2828 | 13.585 | 0376 |
| M+H | 712.2037 | 13.005 | 0377 |
| M+H | 712.2249 | 13.45 | 0378,0379,0380 |
| M+H | 712.2249 | 13.407 | 0378,0379,0380 |
| M+H | 712.2249 | 13.351 | 0378,0379,380 |
| M-H | 710.2636 | 10.962 | 0381 |
| M+H | 713.2261 | 13.691 | 0382 |
| M+H | 713.2787 | 8.5 | 0383 |
| M+H | 714.2679 | 12.754 | 0384 |
| M+H | 715.2797 | 13.545 | 0385 |
| M+H | 715.3103 | 15.147 | 0386 |
| M+H | 716.1835 | 13.279 | 0387 |
| M+H | 716.2548 | 13.986 | 0388 |
| M+H | 717.1650 | 13.732 | 0389 |
| M-H | 715.1850 | 14.212 | 0390 |
| M+H | 717.2403 | 13.39 | 0391 |
| M+H | 717.2499 | 13.564 | 0392 |
| M+H | 718.1743 | 12.958 | 0393,0394 |
| M+H | 718.1795 | 13.347 | 0393,0394 |
| M+H | 718.2331 | 14.261 | 0395 |
| M-H | 716.2303 | 13.923 | 0396 |
| M-H | 717.2185 | 12.706 | 0397 |
| M+H | 719.2527 | 13.259 | 0398 |
| M+H | 720.1894 | 12.789 | 0399 |
| M+H | 720.2515 | 13.412 | 0400 |
| M+H | 720.2958 | 13.495 | 0401 |
| M+H | 721.2660 | 14.927 | 0402 |
| M+H | 722.2301 | 13.067 | 0403 |
| M+H | 723.1356 | 13.18 | 0404 |
| M+H | 723.2053 | 13.149 | 0405 |
| M+H | 724.1890 | 13.741 | 0406,0407 |
| M+H | 724.1890 | 13.63 | 0406,0407 |
| M+H | 725.1842 | 13.515 | 0408,0409,0410 |
| M+H | 725.1845 | 13.869 | 0408,0409,0410 |
| M+H | 725.1847 | 13.978 | 0408,0409,0410 |
| M-H | 723.2095 | 13.311 | 0411 |
| M+H | 725.2996 | 14.037 | 0412 |
| M+H | 726.2044 | 13.345 | 0413,0414 |

**[Table 63]**

| | | | |
|---|---|---|---|
| M+H | 726.2038 | 13.525 | 0413,0414 |
| M+H | 727.1992 | 12.574 | 0415 |
| M+H | 727.2021 | 13.853 | 0415,0416 |
| M+H | 727.2901 | 12.454 | 0417 |
| M-H | 727.1795 | 13.011 | 0418 |
| M+H | 729.2146 | 13.082 | 0419 |
| M+H | 729.2516 | 14.676 | 0420 |
| M+H | 729.2740 | 13.69 | 0421 |
| M+H | 730.2107 | 13.794 | 0422 |
| M+H | 731.2064 | 12.817 | 0423 |
| M+H | 731.2134 | 13.848 | 0424 |
| M+H | 731.2555 | 11.309 | 0425 |
| M-H | 729.2538 | 13.868 | 0426 |
| M+H | 731.3156 | 13.932 | 0427 |
| M+H | 732.1612 | 13.467 | 0428 |
| M+H | 732.2152 | 13.292 | 0429 |
| M+H | 733.1731 | 13.209 | 0430 |
| M-H | 731.2336 | 13.618 | 0431 |
| M+H | 735.2085 | 14.222 | 0433,0434 |
| M-H | 733.1923 | 14.115 | 0433,0434 |
| M-H | 733.2146 | 13.482 | 0435 |
| M+H | 736.1667 | 13.304 | 0436 |
| M+H | 736.2058 | 14.336 | 0437,0438,0439 |
| M+H | 736.2066 | 9.571 | 0437,0438,0439 |
| M+H | 736.2069 | 14.137 | 0437,0438,0439 |
| M+H | 737.2243 | 14.044 | 0440,0441 |
| M+H | 737.2240 | 13.84 | 0440,0441 |
| M-H | 736.2026 | 13.312 | 0442 |
| M-H | 737.2255 | 13.788 | 0443 |
| M+H | 740.2327 | 13.524 | 0444 |
| M+H | 741.2306 | 14.247 | 0445 |
| M+H | 741.3047 | 12.82 | 0446 |
| M+H | 742.2254 | 13.177 | 0447 |
| M+H | 742.2360 | 13.794 | 0448 |
| M+H | 743.1813 | 14.047 | 0449 |
| M+H | 743.2165 | 13.396 | 0450 |
| M+H | 743.2385 | 13.979 | 0451 |
| M+H | 744.1894 | 13.303 | 0452 |
| M-H | 742.1765 | 13.861 | 0452,0453 |
| M+H | 745.2805 | 12.562 | 0454 |
| M+H | 746.2058 | 13.119 | 0455 |
| M+H | 746.3111 | 13.584 | 0456 |

**[Table 64]**

| | | | |
|---|---|---|---|
| M+H | 747.1866 | 13.797 | 0457 |
| M+H | 748.1928 | 13.563 | 0458 |
| M+H | 749.1504 | 13.389 | 0459 |
| M+H | 750.2024 | 13.851 | 0460,0461 |
| M+H | 750.2041 | 13.977 | 0460,0461 |
| M+H | 751.1999 | 13.917 | 0462,0463,0464 |
| M+H | 751.1997 | 14.099 | 0462,0463,0464 |
| M+H | 751.1992 | 13.972 | 0462,0463,0464 |
| M-H | 751.2386 | 14.211 | 0465 |
| M+H | 753.3071 | 15.473 | 0466 |
| M+H | 755.2070 | 13.874 | 0467 |
| M+H | 755.2303 | 13.389 | 0488 |
| M+H | 756.1791 | 13.689 | 0469 |
| M+H | 756.2257 | 14.039 | 0470 |
| M+H | 757.2258 | 13.678 | 0471,0472 |
| M-H | 755.2112 | 13.77 | 0471,0472 |
| M+H | 757.3310 | 14.053 | 0473 |
| M+H | 758.2310 | 13.711 | 0474 |
| M-H | 757.1965 | 13.456 | 0475 |
| M+H | 760.1706 | 13.973 | 0476 |
| M+H | 761.2238 | 14.472 | 0477,0478 |
| M+H | 761.2237 | 14.349 | 0477,0478 |
| M+H | 762.1827 | 13.566 | 0479 |
| M+H | 762.2191 | 14.568 | 0480,0481,0482 |
| M+H | 762.2221 | 11.105 | 0480,0481,0482 |
| M+H | 762.2216 | 10.042 | 0480,0481,0482 |
| M+H | 766.2501 | 13.884 | 0483 |
| M+H | 767.1978 | 14.197 | 0484 |
| M+H | 767.2459 | 14.498 | 0485 |
| M-H | 765.3033 | 12.964 | 0486 |
| M+H | 768.2422 | 13.447 | 0487 |
| M+H | 768.2511 | 14.169 | 0488 |
| M-H | 767.2327 | 14.187 | 0489 |
| M+H | 771.2961 | 12.651 | 0490 |
| M+H | 773.2024 | 14.07 | 0491 |
| M+H | 774.2084 | 13.94 | 0492 |
| M-H | 777.2529 | 14.607 | 0493 |
| M+H | 782.1941 | 13.91.5 | 0494 |
| M+H | 784.2573 | 12.999 | 0495 |
| M-H | 783.2105 | 14.372 | 0496 |
| M-H | 791.1957 | 14.267 | 0497 |
| M+H | 795.2775 | 13.456 | 0498 |

**[Table 65]**

| | | | |
|---|---|---|---|
| M+H | 810.2732 | 13.184 | 0499 |
| M+H | 821.2937 | 13.649 | 0500 |

It was also shown that in the production of compound libraries with various structures, it is possible to efficiently construct a compound library by performing an amidation step with a plurality of compounds by applying the reaction conditions shown in Examples.

## Claims

1. A method for producing an amide compound, comprising reacting a carboxylic acid compound and an amine compound in the presence of a condensing agent and a first base to obtain an amide compound,
wherein the first base is represented by formula A: 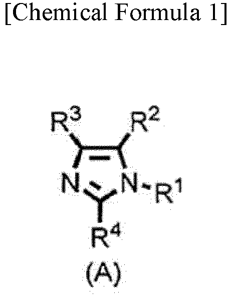
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
two or more of the first base may be used, and
wherein a molar ratio of the first base to the condensing agent (first base/condensing agent) is 1.8 or less.

2. The method according to claim 1, wherein the condensing agent is a uronium-based condensing agent or a 2-halo-N-alkyl pyridinium-based condensing agent.

3. The method according to claim 1 or 2, wherein the reaction is performed further in the presence of a second base.

4. The method according to any one of claims 1 to 3, wherein the reaction is performed in the presence of an excess amount of the amine compound as a second base.

5. The method according to claim 3 or 4, wherein the second base is an organic base having a pKa of a conjugated acid in water of 11 or less.

6. The method according to any one of claims 3 to 5, wherein the second base is selected from the group consisting of formulas B1 and B2: 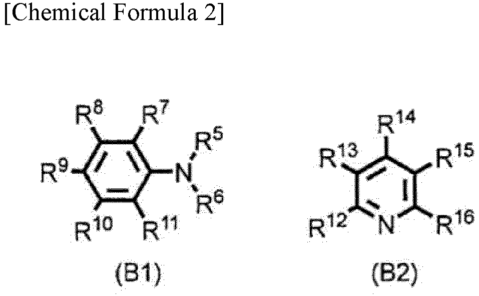
wherein R⁵ and R⁶ together with a nitrogen atom to which they are attached form a 5- to 7-membered saturated heterocycle, and the heterocycle may further comprise a heteroatom selected from O and S; or
R⁵ and R⁷, and/or R⁶ and R¹¹ together with a nitrogen atom and a carbon atom to which they are attached form a 5- to 7-membered non-aromatic heterocycle, and the heterocycle may further comprise a heteroatom selected from O and S; or
R⁵ and R⁶ are each independently selected from C₁₋₆ alkyl and C₆₋₁₀ aryl;
R⁷, R⁸, R⁹, R¹⁰, and R¹¹ are each independently a hydrogen atom, C₁₋₆ alkyl, C₆₋₁₀ aryl, a halogen atom, or cyano;
R¹², R¹³, R¹⁴, R¹⁵, and R¹⁶ are each independently selected from a hydrogen atom, C₁₋₆ alkyl, and C₆₋₁₀ aryl; and
the C₁₋₆ alkyl and C₆₋₁₀ aryl are optionally substituted with one or more halogen atoms, and
two or more of the second base may be used.

7. The method according to any one of claims 1 to 6, wherein the first base is selected from the group consisting of N-methylimidazole, tetramethylimidazole, and N-phenylimidazole, and two or more of the first base may be used.

8. The method according to any one of claims 1 to 7, wherein the reaction is performed in the presence of a solvent, and wherein the solvent is selected from the group consisting of a halogen-based solvent, a nitrile-based solvent, an amide-based solvent, an ether-based solvent, and an aromatic hydrocarbon-based solvent, and two or more of the solvent may be used.

9. The method according to any one of claims 1 to 8, wherein the carboxylic acid compound is a resin for solid-phase synthesis to which the carboxylic acid compound is attached via a linker, or the amine compound is a resin for solid-phase synthesis to which the amine compound is attached via a linker.

10. The method according to any one of claims 1 to 9,
wherein the carboxylic acid compound is a compound or a mixture of two or more compounds represented by formula A1 or formula A2: 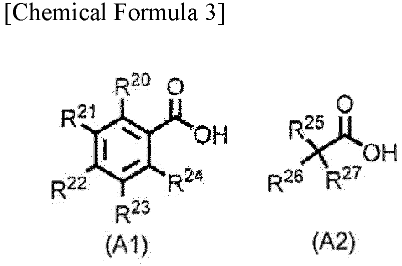
wherein R²⁰, R²¹, R²², R²³, R²⁴, R²⁵, R²⁶, and R²⁷ are each independently selected from a hydrogen atom, a halogen atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the carboxylic acid compound does not have a group involved in an amide bond formation reaction other than a carboxy group represented by formula A1 or formula A2.

11. The method according to any one of claims 1 to 10,
wherein the amine compound is a compound or a mixture of two or more compounds represented by formula A3 or formula A4: 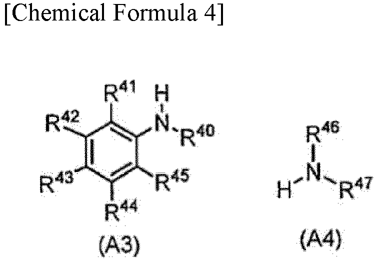
wherein R⁴⁰, R⁴⁶, and R⁴⁷ are each independently selected from a hydrogen atom, C₁₋₆ alkyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N, and S; or
R⁴⁰ and R⁴⁵ together with a nitrogen atom and a carbon atom to which they are attached form a 5- to 7-membered non-aromatic heterocycle, and the heterocycle may further comprise a heteroatom selected from O and S; or
R⁴⁶ and R⁴⁷ together with a nitrogen atom to which they are attached form a 5- to 7-membered saturated heterocycle, and the heterocycle may further comprise a heteroatom selected from O and S; and
R⁴¹, R⁴², R⁴³, R⁴⁴, and R⁴⁵ are each independently selected from a hydrogen atom, a halogen atom, cyano, nitro, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, 4- to 8-membered cyclic aminocarbonyl, C₂₋₆ alkenyl, C₂₋₆ alkynyl, C₃₋₈ cycloalkyl, C₇₋₁₄ aralkyl, C₆₋₁₀ aryl, and 5- to 10-membered heteroaryl comprising one or more ring heteroatoms independently selected from O, N and S, each of which is optionally substituted with a substituent, or
a resin for solid-phase synthesis to which the compound is attached via a linker, and
wherein the amine compound does not have a group involved in an amide bond formation reaction other than an amino group represented by formula A3 or formula A4.

12. The method according to any one of claims 1 to 11,
wherein the uronium-based condensing agent is a compound or a mixture of two or more compounds represented by formula C 1: 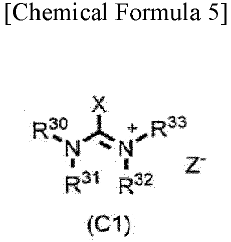
wherein R³⁰, R³¹, R³², and R³³ are each independently C₁₋₆ alkyl; or
R³⁰ and R³¹ and/or R³² and R³³ form a 5- to 7-membered saturated heterocycle, wherein the heterocycle may comprise one ring heteroatom selected from O or S; or
R³¹ and R³² form a 5- to 7-membered saturated heterocycle, wherein the heterocycle may comprise one ring heteroatom selected from O or S;
X is a leaving group; and
Z is a counter anion, or
the 2-halo-N-alkyl pyridinium-based condensing agent is a compound or a mixture of two or more compounds represented by formula C3: 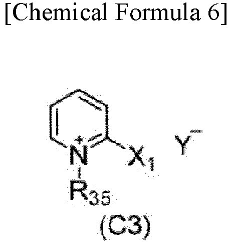
wherein R³⁵ is C₁₋₆ alkyl, X₁ is halogen, and Y is a counter anion.

13. The method according to any one of claims 1 to 12, wherein the reaction is performed in a mixture comprising two or more different carboxylic acid compounds and/or two or more different amine compounds as substrates.

14. A method for producing a compound constituting a compound library, the method comprising producing an amide compound by the method according to any one of claims 1 to 13.

15. A method for forming an amide bond by dehydration condensation of a carboxy group and an amino group, comprising reacting a carboxylic acid compound and an amine compound in the presence of a condensing agent and a first base to form an amide bond,
wherein the first base is a compound or a mixture of two or more compounds represented by formula A: 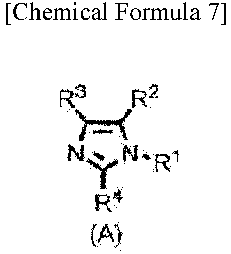
wherein R¹ is selected from C₁₋₆ alkyl and C₆₋₁₀ aryl, and R², R³, and R⁴ are each independently selected from a hydrogen atom, a halogen atom, C₁₋₆ alkyl, and C₆₋₁₀ aryl; or
R¹ and R² together with a nitrogen atom to which R¹ is attached and a carbon atom to which R² is attached form a 5- to 6-membered non-aromatic heterocycle, and
wherein a molar ratio of the first base to the condensing agent (first base/condensing agent) is 1.8 or less.
